# EUROPEAN PATENT SPECIFICATION

(11) **EP 1 690 207 B1**
(45) Date of publication and mention of the grant of the patent: **29.09.2010**
(21) Application number: 04701071.5
(22) Date of filing: 09.01.2004
(51) Int. Cl.: G06F 19/00

(54) **A COMPUTER BASED VERSATILE METHOD FOR IDENTIFYING PROTEIN CODING DNA SEQUENCES USEFUL AS DRUG TARGETS**
EIN AUF COMPUTERVERWENDUNG BASIERTES VERFAHREN ZUR IDENTIFIZIERUNG VON DNA-SEQUENZEN, DIE FÜR ALS "DRUG TARGETS" NUTZBARE PROTEINE CODIEREN
PROCEDE INFORMATIQUE POLYVALENT PERMETTANT L'IDENTIFICATION DE SEQUENCES D'ADN CODANT DES PROTEINES ET UTILISABLES COMME CIBLES MEDICAMENTEUSES

(30) Priority: 05.12.2003 US 727989
(43) Date of publication of application: 16.08.2006
(73) Proprietor: Council of Scientific and Industrial Research, New Delhi 110 001 (IN)
(72) Inventor: BRAHMACHARI, Samir Kumar Institute of Genomics and, Technology Mall Road New Delhi 110 007 (IN); DASH, Debasis, Instit. of Genomics and Integrative, Mall Road, New Delhi 110 007 (IN); SHARMA, Ramakant Inst. of Genomics and Integratove, Mall Road, New Delhi 110 007 (IN); MAHESHWARI, Jitendra Kumar, Inst. of Genomics and, Technology, Mall Road, New Delhi 110 007 (IN)
(74) Representative: Srinivasan, Ravi Chandran
(86) International application number: PCT/IB2004/000453
(87) International publication number: WO 2005/057464

(56) References cited:
- WO-A-01/74130
- CHEN LING-LING ET AL: "ZCURVE_CoV: A new system to recognize protein coding genes in coronavirus genomes, and its applications in analyzing SARS-CoV genomes." BIOCHEMICAL AND BIOPHYSICAL RESEARCH COMMUNICATIONS, vol. 307, no. 2, 25 July 2003 (2003-07-25), pages 382-388, XP004435314 ISSN: 0006-291X
- GUO FENG-BIAO ET AL: "ZCURVE: A new system for recognizing protein-coding genes in bacterial and archaeal genomes." NUCLEIC ACIDS RESEARCH, vol. 31, no. 6, March 2003 (2003-03), pages 1780-1789, XP002317383 ISSN: 0305-1048
- LUKASHIN ALEXANDER V ET AL: "GeneMark.hmm: New solutions for gene finding" NUCLEIC ACIDS RESEARCH, vol. 26, no. 4, 15 February 1998 (1998-02-15), pages 1107-1115, XP002317114 ISSN: 0305-1048
- DELCHER ARTHUR L ET AL: "Improved microbial gene identification with Glimmer" NUCLEIC ACIDS RESEARCH, vol. 27, no. 23, 1 December 1999 (1999-12-01), pages 4636-4641, XP002317115 ISSN: 0305-1048
- SHARMA RAMAKANT ET AL: "Recognition and analysis of protein-coding genes in severe acute respiratory syndrome associated coronavirus" BIOINFORMATICS (OXFORD), vol. 20, no. 7, 1 May 2004 (2004-05-01), pages 1074-1080, XP002317116 ISSN: 1367-4803

## Description

### Field of the present invention

This invention relates to a versatile method for identifying protein coding DNA sequences useful as drug targets. More particularly this invention relates to a method for identification of novel genes in genome sequence data of various organisms, useful as potential drug targets. This invention further provides a method for assignment of function to hypothetical Open Reading Frames (proteins) of unknown function through exact amino acid sequence identity signature.

Emergence of high throughput sequencing technologies has necessitated identification of novel protein coding DNA sequences (genes) in newly sequenced genomes. The invention provides a novel method of converting DNA sequence to alphanumeric sequence by the use of peptide library. The invention also provides a method for use of artificial neural network (feed forward back propagation topology) with one input layer, one hidden layer with 30 neurons and one output layer for identification protein coding DNA sequences. The invention further provides a method for training of neural networks using sigmoid as a learning function with five parameters namely total score, mean, fraction of zeroes, maximum continuous non-zero stretch and variance for identification of protein coding DNA sequence.

### Background and prior art references of the present invention

The most reliable way to identify a protein coding DNA sequence (gene) in a newly sequenced genome is to find a close homolog from other organisms (BLAST (Altschul,S.F et al., 1990) and FASTA (Pearson,W.R., 1995)). Four nucleotides in a DNA sequence are not randomly distributed. The statistical distribution of nucleotides within a coding region is significantly different from the non-coding (Bird,A., 1987). Methods based on Hidden Markov Models (HMM) have used these statistical properties most efficiently (Salzberg,S.L et al., 1998; Delcher,A.L et al., 1999; Lukashin,A.V. and Borodovsky,M., 1998) and are able to predict ∼97-98 % of all the genes in a genome when compared with published annotations (Delcher,A.L et al., 1999). Using HMM, various algorithms like GeneMark, Glimmer etc. have been developed to predict genes in prokaryotes. Glimmer 2.0 is the most successful method among all existing methods (Delcher,A.L et al., 1999).

However, Glimmer also predicts 7-20% additional genes (false positives).

Each gene prediction method has its own strengths and weaknesses (Mathe,C. et al., 2002).

Since the prediction is usually dependent on the training set, shortcomings arise because statistics for a coding region vary across various genomes. Also, these methods are unable to efficiently predict genes small in length (< 100 amino acids), because it's very difficult to detect these genes by similarity searches or by statistical analysis. The problem becomes more severe in case of horizontal gene transfer (Kehoe, M.A et al.,1996). In this case statistical distribution of the nucleotide sequence of these genes differs within a genome itself.

The said method of the invention is based upon the observation that the difference between total number of theoretically possible peptides of a given length and that which are actually observed in nature, increases drastically as this length of peptide increases. For example, only about 2% of the theoretically possible heptapeptides are observed in a pool of 56 completely sequenced prokaryotic genomes. At octapeptide level this number reduces to even less than 0.1%. Moreover, it is interesting to note that most of these peptides selected by nature are found only in the coding regions and very rarely in theoretically translated non-coding regions. This observation has prompted us to exploit this exclusivity of natural selection of peptides that are present in protein coding sequences to differentiate between coding and non-coding regions.

In principle, using longer peptides to score a query ORF is always preferable to using shorter ones (Salzberg,S.L. et al., 1998), but only if sufficient data is available to estimate statistical parameters required to train the prediction algorithm. In case we use peptides of length 8 or more amino acids, it is difficult to get sufficient data to estimate the training parameters. This is because likelihood of an octapeptide being shared between two polypeptides is less than that of a heptapeptide. So we consider the length of 7 amino acids as optimum for scoring of an ORF.

The novelty of the said method is that it works on the basis of protein coding sequences at amino acid, not at nucleotide sequence level. It is noteworthy that the method does not need an organism specific training set, which is an obvious advantage over other methods.

Unlike other methods, GeneDecipher does not employ any landmarks like ribosome binding sites, promoter sequences, transcription start sites or codon usage biases to predict the coding genes and their start locations. In addition, this method overcomes the difficulties of gene prediction for smaller genomes (Chen,L et.al., 2003) like SARS-CoV. Other than gene prediction, this method can also be utilized for similarity searches for polypeptides, putative functional assignment to proteins (based on presence of the oligopeptide motifs), and in phylogenetic domain analysis, indicating the generic-ness and versatility of the method.

Current computational methods like GeneMark.hmm (Lukashin and Borodovsky, 1998), Glimmer (Salzberg et al., 1998), etc. face difficulty in analyzing the small genomes such as of SARS. Methods based on Hidden Markov Models (HMM) require thousands of parameters for training. This makes these methods less suitable for analyzing smaller genomes. The problem compounds in the case of SARS-CoV genomes, which are about 30kb length. Even the method most suitable for viral gene prediction till date ZCURVE_CoV (Chen et al., 2003) needs 33 parameters for training. GeneDecipher needs only 5 parameters and can analyze smaller genomes too. The applicants have trained the Artificial Neural Network on ecoli-k12 genome coding and non-coding regions (ORFs not reported as a gene). To predict protein coding genes using GeneDecipher on viral genomes no additional training is required. This is an obvious advantage of this method over other methods.

### Objects of the present invention

The main object of the present invention is to provide a computer based method for predicting protein coding DNA sequences (genes) useful as drug targets.

Another main object of the present invention is to develop a versatile method of identifying genes using oligopeptides that are found to occur in the ORFs of other genomes using software GeneDecipher.

Still another object of the present invention is to develop a method applicable in the management of the diseases caused by the pathogenic organisms.

Still another object of the present invention is to develop a computer based system for performing the aforementioned methods.

Yet another object of the present invention is to develop a method useful for identification of novel protein coding DNA sequences useful as potential drug targets and can serve as drug screen for broad spectrum antibacterial as well as for specific diagnosis of infection. Still another object of the present invention is to identify strain specific or organism specific protein coding genes.

Yet another object of the method of invention is to identify protein coding DNA sequences (exons) in eukaryotic organisms.

Another object of the present invention is to assignment of function to hypothetical Open Reading Frames (proteins) of unknown function through exact amino acid sequence identity signature.

### Summary of the present invention

The present invention relates to a versatile method of identifying genes using oligopeptides that are found to occur in the ORFs of other genomes and is also suitable for analyzing small genomes using software GeneDecipher, said method comprising steps of generating peptide libraries from the known genomes with peptide of length 'N' computationally arranged in an alphabetical order, artificially translating the test genome to obtain a polypeptide in each reading frame, converting each polypeptide sequence into an alphanumeric sequence with one corresponding to each reading frame on the basis of overlappings with the peptide libraries, training Artificial Neural Network (ANN) with sigmoidal learning function to the alphanumeric sequence, deciphering the protein coding regions in the test genome, thus, identifying longer streches of peptides mapping to large number of known genes and their corresponding proteins and lastly, a method of the management of the diseases caused by the pathogenic organisms comprising a step of evaluation of the proposed drug candidate by inhibiting the functioning of one or more proteins identified by the steps of the invention.

### Detailed description of the present invention

Accordingly, the present invention relates to a versatile method of identifying protein coding DNA sequences (genes) useful as drug targets in a genome using specially developed software GeneDecipher, said method comprising steps of generating peptide libraries from the known genomes with peptide of length 'N' computationally arranged in an alphabetical order, artificially translating the test genome to obtain a polypeptide corresponding to each reading frame, converting each polypeptide sequence into an alphanumeric sequence one corresponding to each reading frame on the basis of overlappings with the peptide libraries, training Artificial Neural Network (ANN) with sigmoidal learning function to the alphanumeric sequence, deciphering the protein coding regions in the test genome, thus, identifying longer streches of peptides mapping to large number of known genes and their corresponding proteins and lastly, a method of the management of the diseases caused by the pathogenic organisms comprising a step of evaluation of the proposed drug candidate by inhibiting the functioning of one or more proteins identified by the steps of the invention.

Accordingly, the present invention provides a computer based versatile method for identifying protein coding DNA sequences useful as drug targets said method comprising steps of:
- generating peptide libraries from the known genomes with oligopeptide of length 'N' computationally arranged in an alphabetical order,
- artificially translating the test genome to obtain a polypeptide in each reading frame,
- converting each polypeptide sequence into an alphanumeric sequence with one corresponding to each reading frame on the basis of occurrence of these oligopeptides in the peptide libraries,
- training Artificial Neural Network (ANN) with sigmoidal learning function to the alphanumeric sequences corresponding to known protein coding DNA sequences and known non-coding regions,
- deciphering the protein coding regions in the test genome by applying the trained Artificial Neural Network, and
- identifying stretches of peptides longer or equal to octomers mapped to large number of known genes serving as functional signatures.

In another embodiment of the present invention, wherein the artificial neural network has one or more input layer, one or more hidden layer with varying number of neurons, and one or more output layer.

In yet another embodiment of the present invention, wherein the number of neurons in the hidden layer is preferably 30.

In still another embodiment of the present invention, wherein the value of the 'N' is 4 or more.

In still another embodiment of the present invention, wherein the sigmoidal learning function has five parameters comprising total score, mean, fraction of zeroes, maximum continuous non-zero stretch, and variance.'

In still another embodiment of the present invention, wherein the method of identifying genes using oligopeptides that are found to occur in the ORFs of other genomes but not limited to genomes such as *H.influenzae, M. genitalium, E.coli, B.subtilis, A.fulgidis. M.tuberculosis. T.pallidum, T.maritima, Synecho cystis, H.pylori, and SARS-CoV.*

In still another embodiment of the present invention, wherein a method claimed in claim 1, wherein the peptide library data may be taken from any organism but not specifically limited to those used in the invention.

Emergence of high throughput sequencing technologies has necessitated identification of novel protein coding DNA sequences (genes) in newly sequenced genomes. The invention provides a novel method of converting DNA sequence to alphanumeric sequence by the use of peptide library. The invention also provides a method for use of artificial neural network (feed forward back propagation topology) with one input layer, one hidden layer with 30 neurons and one output layer.for identification protein coding DNA sequences. The invention further provides a method for training of neural networks using sigmoid as a learning function with five parameters namely total score, mean, fraction of zeroes, maximum continuous non-zero stretch and variance for identification of protein coding DNA sequence.

The applicants have invented a novel computer based method to identify protein coding DNA sequences by comparing with peptide library containing millions of peptides obtained from protein sequences of many organisms that has withstood natural selection.

The method describes a generic and versatile new approach for gene identification. The computational method determines gene candidates among all possible Open Reading Frames (ORF) of a given DNA sequence through the use of a peptide library and an artificial neural network. The peptide library consists of all possible overlapping heptapeptides derived from proteins of completely sequenced 56 or more prokaryotic genomes. A given query ORF qualifies as a gene based upon the abundance and distribution pattern of library heptapeptides (heptapeptides present in library) along the ORF. Performance of the method is characterized by simultaneous high values of sensitivity and specificity. An analysis of 10 completely sequenced prokaryotic genomes is provided to demonstrate the capabilities of the method of the invention.

The present method also allows prediction of alternate target against a specific peptide motif of a pathogenic organism or any host protein target responsible for a disease process.

The method could be extended with different peptide lengths to obtain larger number of protein coding genes and also for eukaryotes and multicellular organisms.

The invention relates to a novel method of converting DNA sequence to alphanumeric sequence by the use of peptide library and the invention also provides a method for use of artificial neural network (feed forward back propagation topology) with one input layer, one hidden layer with 30 neurons and one output layer for identification protein coding DNA sequences. The invention further relates to a method for training of neural networks using sigmoid as a learning function with five parameters namely total score, mean, fraction of zeroes, maximum continuous non-zero stretch and variance for identification of protein coding DNA sequence and the present method is useful for identification of new protein coding regions which can serve as drug screen for broad-spectrum antibacterials as well as for specific diagnosis of infections, and in addition, for assignment of function to newly identified proteins of yet unknown functions. The method allows identification of species or strain specific protein coding genes. This method also can be extended to any protein coding sequence identification even in eukaryotic genomes.

Accordingly, present invention discloses a computer based versatile method for identifying protein coding DNA sequences useful as drug targets, said method comprising steps of:
a. generating peptide libraries from the known genomes with oligopeptide of length 'N' computationally arranged in an alphabetical order,
b. artificially translating the test genome to obtain a polypeptide in each reading frame,
c. converting each polypeptide sequence into an alphanumeric sequence with one corresponding to each reading frame on the basis of occurrence of these oligopeptides in the peptide libraries,
d. training Artificial Neural Network (ANN) with sigmoidal learning function to the alphanumeric sequences corresponding to known protein coding DNA sequences and known non-coding regions,
e. deciphering the protein coding regions in the test genome by applying the trained Artificial Neural Network, and
f. identifying stretches of peptides longer or equal to octomers (evolutionary conserved oligopeptides) mapped to large number of known genes serving as functional signatures.

In yet another embodiment of the present invention the ANN has one or more input layer, one or more hidden layer with varying number of neurons, and one or more output layer.

In still another embodiment of the present invention the number of neurons in the hidden layer is preferably 30.

In yet another embodiment of the present invention the value of the 'N' is 4 or more.

In yet another embodiment of the present invention the sigmoidal learning function has five parameters comprising total score, mean, fraction of zeroes, maximum continuous non-zero stretch, and variance.

One more embodiment of the present invention a method of identifying genes having evolutionary conserved peptide sequences which occur in ORFs of various genomes but not limited to genomes such as *H.influenzae, M.genita*/*ium, E.coli, B.subtilis, A.fulgidis. M.tuberculosis. T.pallidum, T.maritima, Synecho cystis, H.pylori and* SARS-CoV.

In still another embodiment of the present invention the method identifies 169 novel genes identified in genomes of SARS-corona virus and *H.influenzae, M.tuberculosis, H.pylori* of SEQ IDs 1 to 169.

In further embodiment of the present invention, a method of the management of the diseases caused by the pathogenic organisms such as SARS-corona virus, *H.influenzae, M.tuberculosis and H. pylori,* said method comprising step of evaluation of the proposed drug candidate for inhibition of the functioning of one or more evolutionary conserved peptide sequences identified by the instant method and selected from a group comprising proteins of SEQ IDs 170 to 338 corresponding to the novel genes of SEQ IDs 1 to 169. In yet another embodiment of the present invention the peptide library data may be taken from any organism but not specifically limited to those used in the invention.

### Detailed methodology:

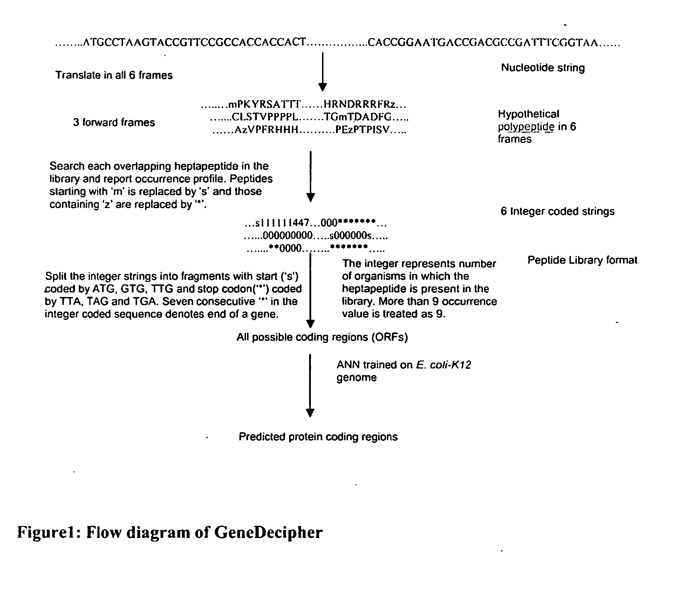

The method has been described in five major steps (as shown in Figure 1):
1. Generation of a peptide library
2. Artificial translation of a given genome into 6 reading frames
3. Conversion of each translated sequence into an alphanumeric sequence. (one corresponding to each reading frame)
4. Training of artificial neural network (ANN).
5. Deciphering genes using trained ANN.

### 1. Generation of peptide library

The method requires a reference peptide library to predict genes in a given genome. In the present invention, the applicants have used proteins from 56 completely sequenced prokaryotic genomes. The protein files for our database were obtained in FASTA format from ftp://ftp.ncbi.nlm.nih.gov/genomes. To prepare a peptide library for deciphering genes in a particular genome, the applicants exclude protein file(s) belonging to that particular species from our database in order to avoid any bias. For example, when analyzing *E.coli*-k12 genome the protein files corresponding to all strains of *E.coli* were excluded from the database to create the peptide library. This has been done to eliminate the signal that is obtained from peptides of that organism, which would be the case while analyzing a newly sequenced genome. This strengthens the method in terms of gene prediction on a newly sequenced genome for which annotated protein file is not available. While creating peptide library all possible overlapping heptapeptides have been taken care of by shifting the window by one amino acid. Redundant peptides were eliminated from the peptide library and each peptide is given an occurrence value based on number of discrete organisms in which it is present.

This occurrence value is a measure of conservation of a heptapetide in coding regions. Presence of a heptapeptide with high occurrence value in an ORF increases the likelihood of that ORF being a protein coding gene. In our algorithm, occurrence value of 9 or more is treated as 9 based on the assumption that if a heptapeptide is present in 9 or more than 9 different organisms' protein files, it can be considered as highly conserved heptapeptide. It is not worthwhile to use any higher value to further discriminate the amount of conservation.

The heptapeptide library database consists of two columns, first for heptapeptide sequence and second for score (occurrence value) of that heptapeptide. Heptapeptides are sorted in dictionary order. The peptide library database also retains other information about the heptapeptides, like the accession number and NCBI annotation of all proteins containing the particular heptapeptide. This can be utilized for putative function prediction of a given ORF. Same approach can be used for phylogenetic domain analysis also.

### 2. Artificial translation of a given genome into 6 reading frames

Second step in the algorithm is artificial translation of the whole query genome in all six reading frames using a standard codon table. However user specified codon table may be used wherever necessary. Applicants used letter 'z' corresponding to the stop codons TTA, TAG and TGA, and letter 'b' for all triplets containing any non standard nucleotide(s) (K, N, W, R, and S etc.) while artificially translating the genome.

### 3. Conversion of each translated sequence into an alphanumeric sequence (one corresponding to each reading frame)

The next step in our algorithm is to convert artificially translated amino acid sequence with stop codon (z) interruption, into an alphanumeric sequence. Applicants search each overlapping heptapeptide in the peptide library, assign a corresponding number

(occurrence value), and append it to the alphanumeric sequence. If a heptapeptide is not present in the library applicants assign the number 0. If a heptapeptide begins with an amino acid corresponding to any of the start codon ATG, GTG and TTG applicants append character 's' in the alphanumeric sequence. This will be helpful to detect the location of a probable start codon. In case a heptapeptide contains character 'z' applicants append a character '*' corresponding to that heptapeptide. Thus consecutive seven '*' (*******) in the alphanumeric sequence is a signal for stop codon. Applicants append '-' character for any heptapeptide containing character 'b'. This signals the presence of a non standard nucleotide character and conveys no information about sequence being a part of gene or non-gene. So, the alphanumeric sequence thus generated contain 13 characters viz. any integer (0-9), 's', '*', and '-'. In this way, applicants convert all six translated protein files into six alphanumeric sequences.

### 4. Training of artificial neural network (ANN)

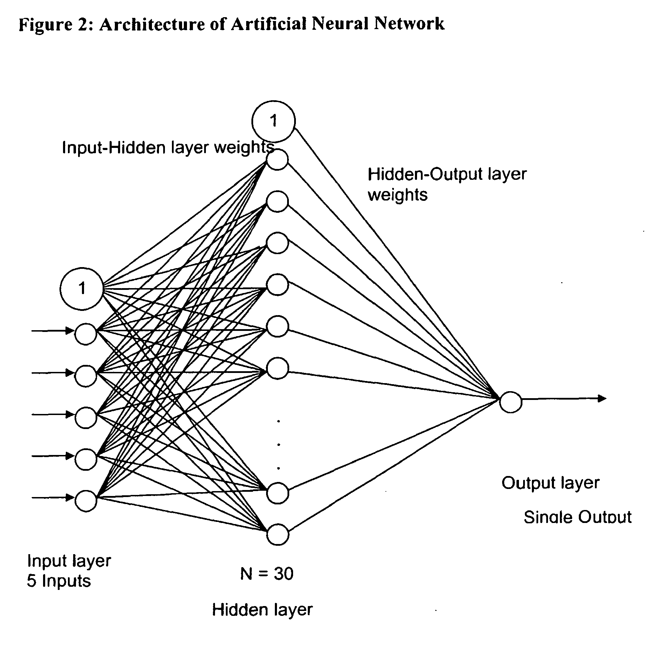

The neural network used here has a multi-layer feed-forward topology. It consists of one input layer, one hidden layer, and an output layer. This is a 'fully-connected' neural network where each neuron i is connected to each unit *j* of the next layer (Figure 2). The weight of each connection is denoted by *wᵢⱼ*. The state Iᵢ of each neuron in the input layer is assigned directly from the input data, whereas the states of hidden layer neurons are computed by using the sigmoid function, hⱼ = I / (1 + exp - λ (wⱼ₀ + Σ wᵢⱼ Iᵢ)), where, *wⱼ₀* is the bias weight, and λ = 1.

The back propagation algorithm is used to minimize the differences between the computed output and the desired output. One thousand cycles (epochs) of iterations are performed.

Subsequently, the epoch with minimum error in validation set is identified and the corresponding weights (*wᵢⱼ*) are assigned as the final weights for the ANN. The network trains on the training set, checks error and optimizes using the validation set through back propagation.

The 'training set' consists of 1610 *E.coli*-k12 NCBI listed protein coding genes and 3000 *E.coli*-k12 ORFs (a stretch of sequence of length more than 20 amino acids and having .start codon, stop codon in the same frame) which have not been reported as genes (non-genes). The 'validation set' has 1000 known genes and 1000 non-genes from *E.coli*-k12, distinct from those used in the training set. The 'test set' contains another 1000 genes and 1000 non-genes from the same organism. For training of the ANN, genes and the non-genes are assigned a probability value of 1 and 0 respectively.

To train the neural network, first applicants convert all the *E.coli*-k12 genes and non-genes into corresponding alphanumeric strings by the method described above (steps 2 and 3).

Here it is important to note that the alphanumeric sequences corresponding to a gene is number rich compared to the alphanumeric sequences corresponding to non-genes. To quantify this number richness of an alphanumeric sequence, five parameters derived from the alphanumeric sequence have been selected. These five parameters are as follows:

### (i). Total Score

This is an algebraic sum of all the integers of a given alphanumeric sequence. Here rule of thumb is higher the score, more are the chances to qualify as a gene.

### (ii). Fraction of zeroes

Fraction of zeroes equals to total no. of zero characters in the alphanumeric sequence divided by total no. of characters in the sequence. More the fraction of zeros, lesser is the chance to qualify as a gene.

### (iii). Mean

Mean equals to total score divided by total length of the sequence. Higher the Mean, more is the chance to qualify as a gene. Virtually this parameter seems same as a total score but it is important because this incorporates the length of the sequence also (score per unit length)

### (iv). Variance

It is the variance of occurrence values about the mean occurrence value for the whole ORF.

### (v). Length of the maximum continuous non zero stretch

Higher the value of this parameter more is the chance to qualify as a gene. Consider a sequence region like '45'. Here, '4'denotes a heptapeptide conserved in 4 organisms, and the succeeding '5' denotes an overlapping heptapeptide conserved in 5 organisms. So if there exists at least one organism which is common between these two sets, eventually applicants have an octapeptide common between that organism and the query ORF. This raises our confidence level in prediction of the coding region. For example, sequence 's45467000000*******' is more likely to be a gene when compared to sequence 's40540607000*******'. This is because there are greater chances of presence of conserved longer peptide in the first sequence. Value of the parameter is 5 for first string and 2 for second one. However, other parameters used in the algorithm can not discriminate between these two sequences.

While calculating these parameters from the alphanumeric sequences, characters such as 's', '*' and '-' have been excluded.

To find an optimum combination, the neural network is trained using all the five parameters together. Parameters corresponding to alphanumeric sequences of genes and non-genes are calculated. The training, validation and test sets contain 6 columns, first 5 columns contains values of the 5 parameters and the last column contains the number '1' for genes and the number '0' for non-genes.

The number of neurons in the input layer was equal to the number of input data points. The optimal number of neurons in the hidden layer was determined by hit and trial while minimizing the error at the best epoch for the network. Computer program to compute all 5 parameters and for the artificial neural network are written in C and executed on a PC under Red Hat Linux version 7.3 or 8.0.

Training of the ANN (step 4 of the algorithm) is generally executed only once, and the same trained neural network can be utilized to execute the method on any prokaryotic genome. Although if applicants use organism specific training set, results might improve in some cases, but it would be marginal. This is because our method predicts gene on the basis of the number distribution of the alphanumeric sequence of an ORF. So the gene prediction is more dependent on the peptide library used rather than training set.

### 5. Deciphering genes using trained ANN

While creation of peptide library (step 1) and training of ANN (step 4) are considered as preparatory phases for executing the method of invention, step 2 and step 3 are mandatory for each genome sequence. After translating computationally a genome into all six reading frames and converting them into six alphanumeric sequences, deciphering genes using ANN is executed. This step can be further divided into following five sub-steps:
1. Breaking of all the six alphanumeric sequences into possible ORFs. ( all possible fragments starting with 's' and ending with '*')
2. Calculate all the five parameters (total score, fraction of zeroes, mean, variance, and length of maximum continuous non zero stretch) for all possible ORFs (all the alphanumeric string sequences between 's' and '*').
3. Calculate the probability of the ORF corresponding to a given alphanumeric string as a protein coding gene, using the trained ANN.
4. Filter out the protein coding ORFs from the non coding ones by using a cutoff probability value.
5. Remove all the encapsulated protein coding regions (Shibuya,T. and Rigoutsos,I., 2002).
   If two ORFs are predicted in distinct translation frames, such that one's span completely encapsulates other, it is a commonly believed that only one of them can be an actual gene. In this case the applicants report the ORF with a higher probability value as a gene. In case of same probability value applicants take longer ORF as a gene.

The method of the invention predicts a probability value corresponding to a query ORF being a protein coding region. The training of ANN is done using a sigmoid learning function with = 1 (probability '1' for genes and '0' for non-genes); therefore most of the time this probability value lies either below 0.1 or above 0.9. Due to this any cutoff value lying between 0.1 and 0.9 generate very similar results. In our analysis applicants use a default cutoff value of 0.5. It's important to note that the method does not require a trade-off between sensitivity and specificity because the choice of cut-off probability has no major consequences on the results.

Other and further aspects, features and advantages of the present invention will be apparent from the following description of the presently preferred embodiments of the invention given for the purpose of disclosures.

### Brief description of the computer programs:

### 1. File Name: genedcodchr.cxx

```
                 Application: Translation of nucleotide sequence (FASTA file format) into 6
 hypothetical polypeptides in 6 respective frames.
                 Input format : <Program_name> <Nucleotide_file> <Output1> <Output2>
 <frame> e.g., ./genedcodchr ecoli.fna pf1 pr1 0
                 Output format:
 AGTFYRYmGHVNMKIYTASLPTYRYGYFSHRED.....HGOIEKSDWEzDFGTRE
```

### 2. File Name: searchchr.cxx

```
                Application:Converts the polypeptide file into an alphanumeric sequence through a
                heptapetide library (given as an input) search.
                 Input format :< Program_name> 7 <peptide library file name> out Y <Input1>
 <Input2> <Output1> <Output 2>
                e.g., ./searchchr 7 ecoli.peplib out Y pf1 pr1 bf1 br1
                Output format:
                s1124500001090003000020000023000000000*******0001000..........
```

### 3. File Name: cutf.c

```
                 Application: Cuts all possible ORFs (i.e., all 's' to '*' regions) from the
                alphanumeric sequence of forward strand and generates a file containing locations
                 of all the 's' in alphanumeric sequence.
                 Input format :< Program_name> <Input file name> <Output1> <Output2>
                e.g../cutf bf1 unknown_bf1 bf1_location
                Output format: output1- s1111000s00000000563*, output2- starting locations of 's'
                 in a column.
```

### 4. File Name: cutr.c

Application: Cuts the all possible ORFs ( all 's' to '* regions) from the reverse strand's alphanumeric sequences and produces a file which contains the starting locations in alphanumeric sequence file for all 3 forward frames corresponding to all ORFs.

```
                 Input format :< Program_name> <Input file name> <Output1> <Output2>
                e.g../cutr br1 unknown_br1 br1_location
                Outputformat: output 1 -*010340000222200067900000s000001000200s00230000s,
                  output2- starting location of 's'
```

### 5. File Name: stat.c

```
                Application: Calculates the five parameters:fraction of zeros, mean, total score,
                length of maximum continuous stretch, and variance for a given alphanumeric
                sequence.
                Input format :< Program_name> <Input file name><Output> 1.
                e.g../stat unknown_bf1 bf1.data 1
                Output format: 0.334 3.2 48 15 0.452 1
```

### 6. File Name: train .c

Application: Training of Artificial Neural Network (single hidden layer, 1 input and I output layer) with feed forward back propagation algorithm and using sigmoid ( = 1) as a learning function.

```
                  Input format :< Program_name> <Input specification file name> <Input1>
 <Input2> <Input3>> output
                  e.g../train train.spec.fast trainset.data validateset.data testset.data >
 train.net
                  Output format: output containing the final neural network wieghts in a single
 column.
```

### 7. File Name: recognize.c

Application: Recognizes a given pattern on the basis of trained weights and generates a probability value as output.

```
                  Input format :< Program_name> <Input specification file name> <Input1>
 <Input2>
                  <Output>
                  e.g../recognize recognize.spec bf1.data train.net f1.out
                  Output format: pat1 probability <value>
```

### 8. File Name: Filter_prediction.c

Application: Filters out the completely overlapping ORFs in same frame based on probability and length parameter.

```
                  Input format :< Program_name> <Input1> <Input2> <Output>
                  e.g. ./Filter_prediction f1.out unknown_bf1 bf1.out.res
                  Output format: pat1 probability <value> <integer string>
```

### 9. File Name: locationf.c

Application: Filters out the genes of length <20 amino acids, and reports starting location of the remaining ones with the alphanumeric sequence for all 3 forward frames.

```
                  Input format :< Program_name> <Input1> <Output> <Input2>
                 e.g. ./locationf bf1.out.res bf1.out.resl bf1_location
                 Output format:<Pattern No> <Probability value> <integer string> <Start> <End>
```

### 10. File Name: locationr.c

Application: Filters out the genes of length <20 amino acids, and reports starting location of the remaining ones with the alphanumeric sequence for all 3 reverse frames.

```
                  Input format :< Program_name> <Input1> <Output> <Input2>
                 e.g. ./locationr br1.out.res br1.out.res1 br1_location
                 Output format:<Pattern No> <Probability value> <integer string> <Start> <End>
```

### 11. File Name: finalf.c

Application: Converts the start and end locations of the alphanumeric sequence into the corresponding genome locations for 3 forward frames.

```
                 Input format :< Program_name> <Input1> <Input2> <Input3> <Output>
                 e.g. ./finalf bf1.out.res1 bf2.out.res1 bf3.out.res1 Final_outputf
                 Output format:<Start> <End> <frame> <length> <Probability value> <integer
 string>
 12. File Name: finalr.c
```

Application: Converts the start and end locations of the alphanumeric sequence into the corresponding genome locations for 3 reverse frames.

```
                 Input format :< Program_name> <Input1> <Input2> <Input3> <Output>
                 e.g. ./final fbr1.out.res1 br2.out.res1 br3.out.res1 Final_outputr
                 Output format:<Start> <End> <frame> <length> <Probability value> <integer
 string>
```

### 13. File Name: sort.c

### File Name: sort.c

Applications: Prints the finally predicted genes into descending order along the genome start location.

```
                  Input format :< Program_name> <Input1> <Input2> <Input3> <Output>
                  e.g../sort Final_outputf Final_outputr OUTPUTF_with_encap
                  OUTPUTR_with_encap OUTPUT
                  Output format:<Start> <End> <Probability value>
```

### 14. File Name: removeencap.c

Application: Removes encapsulated genes found in other five frames.

```
                  Input format :< Program_name> <Input1> <Input2> <Input3> <Output>
                  e.g. ./removeencap OUTPUTF_with_encap OUTPUTR_with_encap
                  OUTPUT OUTPUTF OUTPUTR
                  Output format:<Start> <End> <frame> <length> <Probability value> <integer
 string>
```

The present invention relates to a novel computer based method for predicting protein coding DNA sequences useful as drug targets. In this method occurrence of oligopeptide signatures have been used as probes. The method is versatile and does not necessarily require organism specific training set for the Artificial Neural Network. The method is not only dependent on statistical analysis but also integrates with the biological information that is retained in the conserved peptides, which withstood evolutionary pressure. Logical extension of the method will be to predict protein coding DNA sequences (exons) in eukaryotic genomes.

### Brief description of the accompanying drawings

**Figure 1** shows a logic circuit of GeneDecipher.
**Figure 2** shows a architecture of neural network.
**Figure 3** shows analysis of results of GeneDecipher on 10 organisms.

The particulars of the organisms used for the invention comprising name, strain, accession number and other details are given below.

### S.No. Genome Strain Accession Number Total Base Sequences Date of Completion

| | | | | |
|---|---|---|---|---|
| 1 | *H.Influenzae* | Rd | NC_000907 | 1830138 |
| | Sep30,1996 | | | |
| Fleischmann,R.D. et.al Science 269 (5223), 496-512 (1995) | | | | |
| 2 | *M.Genitalium* | -- | NC_000908 | 580074 |
| | Jan8,2001 | | | |
| Fraser,C.M., et.al Science 270 (5235), 397-403 (1995 | | | | |
| 3 | *E.coli* | K-12 | NC_000913 | 4639221 |
| | Oct 15, 2001. | | | |
| Blattner,F.R. et. al Science 277 (5331), 1453-1474 (1997) | | | | |
| 4 | *B. Subtilis* | 168 | NC_000964 | 4214814 |
| | Nov 20,1997 | | | |
| Kunst,F. et.al Nature 390 (6657), 249-256 (1997) | | | | |
| 5 | *A.Fulgidis* | DSM 4304 | NC_000917 | 2178400 |
| | Dec.17,1997 | | | |
| Klenk,H.P.et.al Nature 390 (6658), 364-370 (1997) | | | | |
| 6 | *M.Tuberculosis* | H37RV | NC_000962 | 4411529 |
| | Sep.7,2001 | | | |
| Cole,S.T. et.al Nature 393 (6685), 537-544 (1998) | | | | |
| 7 | *T.Pallidum* | -- | NC_000919 | 1138011 |
| | Sep 7, 2001 | | | |
| Fraser,C.M.,et.al Science 281 (5375), 375-388 (1998) | | | | |
| 8 | *T.Maritima* | -- | NC_000853 | 1860725 |
| | Sep 10, 2001. | | | |
| Nelson,K.E. et.al Nature 399 (6734), 323-329 (1999) | | | | |
| 9 | *Synecho cystis* | PCC6803 | NC_000911 | 3573470 |
| | Oct 30,1996 | | | |
| Kaneko,T. et.al DNA Res. 3(3), 109-136 (1996) | | | | |
| 10 | *H.Pylori* | 26695 | NC_000915 | 1667867 |
| | Sep7,2001 | | | |
| Tomb,J.-F. et.al Nature 388 (6642), 539-547 (1997) | | | | |

The following examples are given by way of illustration of the present invention and should not be construed to limit the scope of the present invention

### Example 1

### Conversion of DNA sequence into alphanumeric sequence

The purpose of this module in our software is to translate computationally the whole query genome (DNA sequence) in all six reading frames using a specified codon table.

Applicants used letter 'z' corresponding to the stop codons TTA, TAG and TGA, and letter 'b' for all triplets containing any non standard nucleotide(s) (K, N, W, R, and S etc.) while artificially translating the genome. Subsequently the translated genome sequence is converted computationally into an alphanumeric sequence ([0-9], 's', '*', and '-'.). Applicants search each overlapping heptapeptide in the peptide library, assign a corresponding number (occurrence value), and append it to the alphanumeric sequence. If a heptapeptide is not present in the library applicants assign the number 0. If a heptapeptide begins with an amino acid corresponding to any of the start codon ATG,GTG and TTG

Applicants append character 's' in the alphanumeric sequence. This will be helpful to detect the location of a probable start codon. In case a heptapeptide contains character 'z' applicants append a character '*' corresponding to that heptapeptide. Thus consecutive seven '*' (*******) in the alphanumeric sequence is a signal for stop codon. Applicants append a '-' character for any heptapeptide containing character 'b'. This signals the presence of a non-standard nucleotide character.

The aforementioned conversion is further elaborated with the help of following six sequences.

### • SEQ ID No. 12

| | | | | | |
|---|---|---|---|---|---|
| GDC_HINF_243018 | 243018 | 243215 | 65 | + | Cell wall-associated hydrolase |

### >gi_GDC_HINF_243018

GTGATGAGCCGACATCGAGGTGCCAAACACCGCCGTCGATATGAACTCTTGGG
CGGTATCAGCCTGTTATCCCCGGAGTACCTTTTATCCGTTGAGCGATGGCCCTT
CCATTCAGAACCACCGGATCACTATGACCTACTTTCGTACCTGCTCGACTTGTC
TGTCTCGCAGTTAAGCTTGCTTATACCATTGCACTAA

### Computationally translated protein sequence

### >gi_GDC_HINF_243018

VMSRHRGAKHRRRYELLGGISLLSPEYLLSVERWPFHSEPPDHYDLLSYLLDLSVS QLSLLIPLH

### Computationally generated alphanumeric sequence

ss10000000000001s03111431000000000000000000110000100s001030*

### • SEQ ID No. 4

| | | | | | |
|---|---|---|---|---|---|
| GDC_HINF_170553 | 170553 | 170732 | 59 | - | dicarboxylate transport protein homolog H10153 |

### >gi_GDC_HINF_170553

GTGTTTATGCTTTATTTAGAATTTTTATTTTACTATTAATGCTCTATATCGGTA
GCCGTTACGGCGGTATCGGATTAGGTGTTGTTTCTGGTATCGGTCTTGCTATCG
AGGTTTTCGTATTTCGTATGCCAGTGGGGAAGCACCGATTGATGTTATGCTTAT
CATTCTTGCAGTGGTGA

### Computationally translated protein sequence

### >gi_GDC_HINF_170553

VFMLYLEFLFLLLMLYIGSRYGGIGLGVVSGIGLAIEVFVFRMPVGKHRLMLCLSF
LQW

### Computationally generated alphanumeric sequence

s0s1131231142s1111445232254238000000000000s0s0000ss00*

### • SEQ ID No. 73

| | | | | | |
|---|---|---|---|---|---|
| GDC_MTUB_688806 | 688806 | 689060 | 84 | + | MCE-FAMILY PROTEIN MCE2B |

>gi_GDC_MTUB_688806

TTGCTGCACAGCAGCTTCGGGCACCTCGAGGGCATCCAGCAGCCGCTCATAGA
CGAGCTGGCAGAACTCGACCACGTGTTGGGCAAGCTGCCGGACGCCTACCGGA
TCATCGGCCGCGCCGGCGGCATATACGGTGACTTCTTCAACTTCTATCTGTGTG
ACATCTCACTGAAAGTCAACGGATTACAGCCTGGAGGTCCGGTACGCACCGTC
AAGTTGTTCGGCCAGCCGACCGGCAGGTGCACACCGCAATGA

### Computationally translated protein sequence

### >gi_GDC_MTUB_688806

LLHSSFGHLEGIQQPLIDELAELDHVLGKLPDAYRIIGRAGGIYGDFFNFYLCDISLK
VNGLQPGGPVRTVKLFGQPTGRCTPQ

### Computationally generated alphanumeric sequence s000000000110110530100000ss000000000000100000000000000000001111210000000s0 0100*

### • SEQ ID No. 92

| | | | | | |
|---|---|---|---|---|---|
| GDC_MTUB_1286282 | 1286282 | 1286587 | 101 | - | pterin-4-alpha-carbinolamine dehydratase |

### >gi_GDC_MTUB_1286282

GTGACGGTATACCGTCGAGGTATGGCTGTGTTAACGGATGAGCAGGTCGACGC
CGCACTGCACGACCTCAACGGCTGGCAGCGCCGGTGGTGTCCTGCGTAGGT
CAATCAAGTTTCCGACGTTTATGGCCGGTATCGACGCCGTACGCCGGGTGGCC
GAGCGAGCCGAGGAGGTAAATCATCATCCGGACATCGATATCCGTTGGCGAAC

AGTAACTTTCGCGCTGGTTACGCATGCGGTAGGTGGTATCACGGAAAACGACA
TTGCGATGGCGCACGATATCGACGCAATGTTTGGGGCCTAA

### Computationally translated protein sequence

### >gi_GDC_MTUB_1286282

VTVYRRGMAVLTDEQVDAALHDLNGWQRAGGVLRRSIKFPTFMAGIDAVRRVA
ERAEEVNHHPDIDIRWRTVTFALVTHAVGGITENDIAMAHDIDAMFGA

### Computationally generated alphanumeric sequence

s000000s0s21110001000000300000000011000000s01031100s0002000011000000003000 0000013310000000s0001*

### • SEQ ID No. 49

| | | | | | |
|---|---|---|---|---|---|
| GDC_HPYL_583607 | 583607 | 583876 | 89 | + | probable DNA helicase |

### >gi_GDC_HPYL_583607

TTGATGGAATTTGATGTTACCATCATAGATGAGACAGGCAGGGCCACAGCACC
AGAAATCTTGATTCCTGCACTTCGCACTAAAAAACTGATCTTAATAGGCGATC
ACAACCAGCTCCCACCTAGCATTGATAGGTACCTCCTAGAACAATTAGAGAGC
GATGATATTCAAAACTTGGATGCCATTGATCGCCAATTATTGGAAGAGAGTTT
TTTTGAAAATCTCTATAAGTATATTCCAGAGAGTAATAAGGCCATGCTTAATG
AGTAA

### Computationally translated protein sequence

### >gi_GDC_HPYL_583607

LMEFDVTIIDETGRATAPEILIPALRTKKLILIGDHNQLPPSIDRYLLEQLESDDIQNL
DAIDRQLLEESFFENLYKYIPESNKAMLNE

### Computationally generated alphanumeric sequence

ss001000000001000000s0000011000020000000000030310000000002s0003020s0000000 000000000*

### • SEQ ID No. 54

| | | | | | |
|---|---|---|---|---|---|
| GDC_HPYL_954846 | 954846 | 955217 | 123 | - | PHOSPHOTRANSACETY LASE |

### >gi_GDC_HPYL_954846

GTGAGCCTGGTTTCAAGCGTGTTTTAATGTGTTTAGACACTCAAGTGCTAGTC
TTTGGGGATTGCGCGATTATCCCTAACCCTAGCCCTAAAGAATTAGCCGAGAT
CGCTACCACTTCCGCACAAACCGCCAAGCAATTCAATATTGCGCCTAAAGTGG
CCTTGCTTTCTTATGCGACAGGCGATTCCGCTCAAGGCGAAATGATAGACAAA
ATCAACGAAGCTTTAACAATCGCTCAAAAGTTGGATCCCCAATTAGAAATTGA
TGGCCCCTTACAATTTGACGCTTCCATTGATAAAAGCGTAGCCAAGAAAAAAT
GCCTAACAGCCAAGTGGCTGGGCAAGCTAGCGTTTTATTTCCCGGATTTAA

### Computationally translated protein sequence

### >gi_GDC_HPYL_954846

VSLVSSVFLMCLDTQVLVFGDCAIIPNPSPKELAEIATTSAQTAKQFNIAPKVALLS
YATGDSAQGEMIDKINEALTIAQKLDPQLEIDGPLQFDASIDKSVAKKKCLTAKWL
GKLAFLFSRI

### Computationally generated alphanumeric sequence

s80000s00s00002s200222000000003100000000000000000010s0s100000000000s000000 0100000s00000000000000000000000000030000010*

### Example 2

### Training of artificial neural network (ANN)

The purpose of this module in the software is to train the designed neural network (fig 2) with a specified no. of genes and non-genes. In this example the training set consists of 1610 *E.coli-k12* NCBI listed protein coding genes and 3000 *E.coli-k12* ORFs which have not been reported as genes (non-genes). The validation set has 1000 known genes and 1000 non-genes from *E.coli-k12,* distinct from those used in the training set. The test set contains another 1000 genes and 1000 non-genes from the same organism. For training of the ANN, genes and the non-genes are assigned a probability value of 1 and 0 respectively.

To train the neural network, first applicants convert all the *E.coli-k12* genes and non-genes into corresponding alphanumeric strings by the method described above (steps 2 and 3).

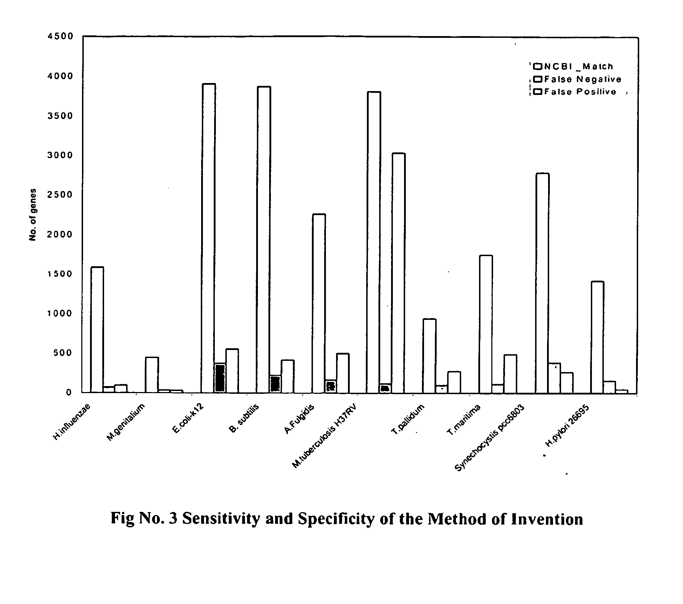

Samples of two *E.coli-k12* genes and two non-genes in alphanumeric sequence format are shown in figure 3. Here it is important to note that the alphanumeric sequences corresponding to a gene is number rich compared to the alphanumeric sequences corresponding to non-genes. This supports our hypothesis. To quantify this number richness of an alphanumeric sequence, five parameters derived from the alphanumeric sequence have been selected. These five parameters are as follows:
*Total Score* (algebraic sum of all the integers of a given alphanumeric sequence), *Fraction of zeroes* (total no. of zero characters in the alphanumeric sequence divided by total no. of characters in the sequence), *Mean (* total score divided by total length of the sequence), *Variance* (variance of occurrence values about the mean occurrence value for the whole ORF), *Length of the maximum continuous non zero stretch* (represents the occupancy of uninterrupted non-zero numbers in a sequence) as explained in table 1(a) and 1(b).

**Table 1(a): Training of ANN (genes)**

| S.No | Fraction of Zeros | Total Score | Average | Biggest Continuous stretch | Variance | Probability |
|---|---|---|---|---|---|---|
| 1 | 0.663116 | 587 | 0.7816 | 19 | 2.10146 | 1 |
| 2 | 0.693950 | 214 | 0.7616 | 18 | 2.43068 | 1 |
| 3 | 0.597436 | 412 | 1.0590 | 13 | 3.16832 | 1 |
| 4 | 0.898876 | 12 | 0.1348 | 4 | 0.20654 | 1 |

**Table 1(b): Training of ANN (Non-genes)**

| S.No | Fraction of Zeros | Total Score | Average | Biggest Continuous stretch | Variance | Probability |
|---|---|---|---|---|---|---|
| 1 | 0.946429 | 3 | 0.0536 | 2 | 0.05070 | 0 |
| 2 | 1.000000 | 0 | 0.0000 | 0 | 0.00000 | 0 |
| 3 | 0.955556 | 2 | 0.0444 | 1 | 0.04247 | 0 |
| 4 | 0.956522 | 2 | 0.0435 | 1 | 0.04159 | 0 |

While calculating these parameters from the alphanumeric sequences characters's', '*' and '-' have been excluded. To determine the contribution of each parameter towards discriminating genes from non-genes, the neural network is trained using all the five parameters together. Parameters corresponding to alphanumeric sequences of genes and non-genes are calculated. The training, validation and test sets contain 6 columns, first 5 columns contains values of the 5 parameters and the last column contains the number '1' for genes and the number '0' for non-genes.

### Example 3

The applicants have analyzed 10 prokaryotic genomes using the method of invention.

Efficiency of the method has been defined as percentage of the NCBI listed protein coding regions predicted by said method. All the encapsulated protein coding regions have been eliminated automatically by a specifically developed program. The method is able to predict on an average 92.7% of the NCBI listed genes with a standard deviation of 2.8%. Both sensitivity and specificity values of the method are high except in *M.tuberculosis* H37RV genome (as shown in figure No. 3).

### Example 4

### Prediction of start site of protein coding DNA sequences

Correct start site prediction rate of the method of invention varies from 49.5 % in *M.tuberculosis* H37Rv (where specificity is also least) to 81.1 % in *H.pylori* 26695. The applicants method decides start location based on the presence of start codon plus conservation of the surrounding heptapeptides. This method can also be utilized to predict the start site of a query protein coding DNA sequences predicted by some other method. This can be done by simply converting the protein sequence into corresponding integer sequence and then deciding the valid start site 's' on the basis of surrounding heptapeptides. The applicants report three such cases from *E.coli* K-12 genome (two from the forward strand and one from the reverse strand), to exemplify the start site prediction (as shown below). In prediction of start site there is a trade-off between number richness and length of the ORF. In Case 1(PID 16132273), the start location of the gene has been shifted from location 85540 to 85630 by NCBI. By visual inspection of the integer sequences corresponding to this gene it is evident that earlier there was a region after 's' which was full of zeroes; or in other terms not a number rich region (bold region in Case 1 of figure shown below). The start site has now been shifted so that it now lies before a number rich region as predicted by the said method of invention. Case 2 is an example of 5' upstream shifting of the start codon because there is a number rich region ('2011111' and one '3' and one '2') upstream of this start codon. So this has been shifted to location 4611050 from 4611194. Case 3 is another example of shifting of start site in the reverse strand where there is a number rich region ('16531311' and many other numbers in the string) upstream of the earlier NCBI start location.

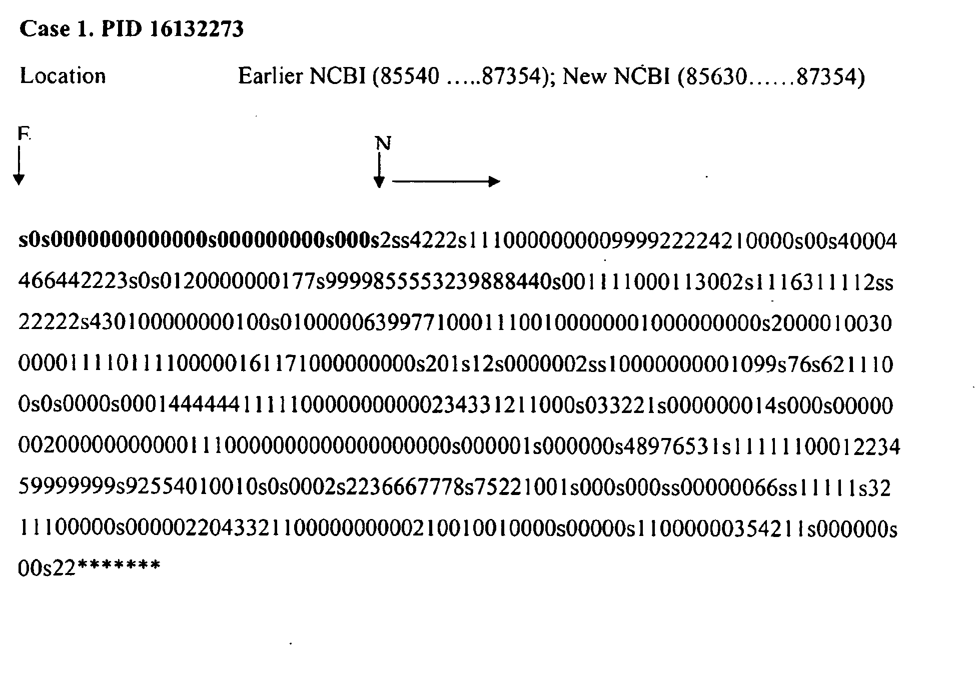
Case 1. PID 16132273

Location Earlier NCBI (85540 .....87354); New NCBI (85630......87354)

F
N

s0s000000000000s000000000s000s2ss4222s11100000000009999222242l0000s00s40004
466442223s0s0120000000177s99998555323988844 0s001111000113002s1116311112ss
22222s4301000000000100s01000006399771000111001000000001000000000s2000010030
00001111011110000016117100000000 0s201s12s0000002ss1000000000 1099s76s621110
0s0s0000s00014444441111100000000000023433121100 0s03322 1s000000014s000s00000
0020000000000011100000000000000000000s000001s000000s4897653 1s1111100012234
59999999s92554010010s0s0002s2236667778s7522100 1s000s000ss00000066ss11111s32
11100000s000002204332110000000000210010010000s00000s1100000035421 1s000000s
00s22*******

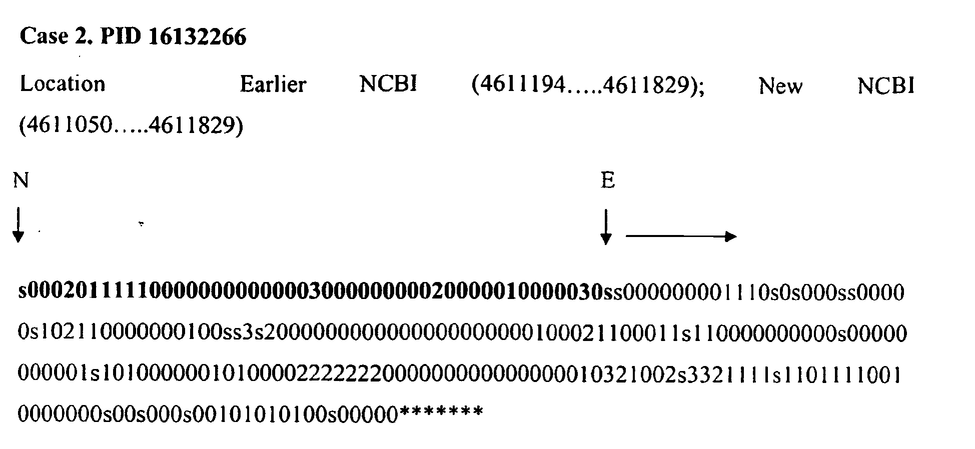
Case 2. PID 16132266

Location Earlier NCBI (4611194.....4611829); New NCBI

(4611050.....4611829)

N E


s000201111100000000000000300000000020000010000030ss000000001110s0s000ss0000

0s10211000000100ss3s20000000000000000000000100021100011s11000000000s00000

000001s1010000001010000222222000000000000000000010321002s332111s110111001

0000000s00s000s00101010100s00000*******

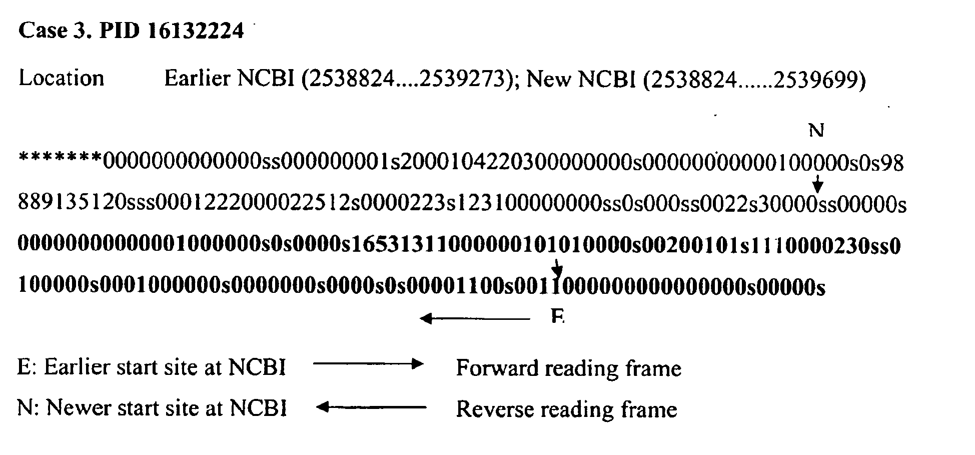
Case 3. PID 16132224

Location Earlier NCBI (2538824....2539273); New NCBI (2538824......2539699)

N

*******0000000000000ss000000001s20001042203000000000s000000000000100000s0s98
889135120sss00012220000022512s0000223s123100000000ss0s000ss0022s30000ss00000s
00000000000001000000s0s0000s16531311000000101010000s00200101s110000230ss0
100000s0001000000s0000000s0000s0s00001100s0011000000000000000s00000s

 E

E: Earlier start site at NCBI Forward reading frame

N: Newer start site at NCBI Reverse reading frame

### Example 5

### Prediction of protein coding DNA sequences

The method is utilized for prediction of protein coding DNA sequences for various genomes in a publicly available database (NCBI) by employing the following steps:
i) generating computationally overlapping peptide libraries from all the protein sequences of the selected organisms available at http://www.ncbi.nlm.nih.gov,
ii) sorting computationally the peptides of length 'N' obtained as above, alphabetically, according to single letter amino acid code,
iii) cataloging every peptide and their unique occurrence different organisms,
iv) converting DNA sequence to alphanumeric sequence using peptide library obtained from steps I and 2,
v) retrieving all possible open reading frames (ORFs) from the alphanumeric sequence,
vi) training of the modified neural network for discriminating protein coding and non-coding DNA sequences,
vii) predicting DNA coding sequences in the open reading frames (obtained in step 4) using trained neural network,
viii) removing the encapsulated protein coding DNA sequences (genes within genes).

Using the steps of the invention the inventors have arrived at disclosure of novel 169 genes from the genomes of organisms selected from SARS-corona virus, *H.influenzae, M.tuberculosis, and H.pylori* as detailed in the table 2. The Table No. 2 provides the said novel genes in the sequence of SEQ ID No. 1 to SEQ ID No. 169.

**Table 2**

| | | | | | | |
|---|---|---|---|---|---|---|
| 1 | GDC_HINF_5641 | 5641 | 6273 | 210 | + | Formate dehydrogenase major subunit |
| 2 | GDC_HINF_6322 | 6322 | 8748 | 808 | + | Formate dehydrogenase major subunit |
| 3 | GDC_HINF_124181 | 124181 | 124378 | 65 | + | Cell wall-associated hydrolase |
| 4 | GDC_HINF_170553 | 170553 | 170732 | 59 | - | dicarboxylate transport protein homolog HI0153 |
| 5 | GDC_HINF_231874 | 231874 | 232173 | 99 | + | type I restriction system adenine methylase |
| 6 | GDC_HINF_232170 | 232170 | 232991 | 273 | + | type I restriction system adenine methylase |
| 7 | GDC_HINF_232813 | 232813 | 233139 | 108 | + | type I restriction system adenine methylase |
| 8 | GDC_HINF_233190 | 233190 | 233393 | 67 | + | Type I restriction enzyme EcoprrI M protein |
| 9 | GDC_HINF_235441 | 235441 | 235932 | 163 | + | prrD protein homolog |
| 10 | GDC_HINF_235913 | 235913 | 238519 | 868 | + | Type I restriction enzyme EcoR124II R protein |
| 11 | GDC_HINF_240336 | 240336 | 241379 | 347 | - | Aerobic respiration control sensor protein |
| 12 | GDC_HINF_243018 | 243018 | 243215 | 65 | + | Cell wall-associated hydrolase |
| 13 | GDC_HINF_274892 | 274892 | 276853 | 653 | - | Adhesion and penetration protein precursor |
| 14 | GDC_HINF_276992 | 276992 | 279121 | 709 | - | Adhesion and penetration protein precursor |
| 15 | GDC_HINF_370413 | 370413 | 370808 | 131 | + | NapA |
| 16 | GDC_HINF_370747 | 370747 | 372912 | 721 | + | NapA |
| 17 | GDC_HINF_628407 | 628407 | 628604 | 65 | - | Cell wall-associated hydrolase |
| 18 | GDC_HINF_654365 | 654365 | 655015 | 216 | - | Probable D-methionine transport system permease |
| 19 | GDC_HINF_661444 | 661444 | 661641 | 65 | - | Cell wall-associated hydrolase |
| 20 | GDC_HINF_737160 | 737160 | 737297 | 45 | + | glycerophosphodiester phosphodiesterase |
| 21 | GDC_HINF_775792 | 775792 | 775989 | 65 | - | Cell wall-associated hydrolase |
| 22 | GDC_HINF_848166 | 848166 | 848678 | 170 | - | ribosomal protein |
| 23 | GDC_HINF_928073 | 928073 | 929080 | 335 | + | Peptidase B (Aminopeptidase B) |
| 24 | GDC_HINF_929037 | 929037 | 929402 | 121 | + | Peptidase B (Aminopeptidase B) |
| 25 | GDC_HINF_1018846 | 1018846 | 1021371 | 841 | - | Isoleucyl-tRNA synthetase |
| 26 | GDC_HINF_1021582 | 1021582 | 1021683 | 33 | - | Isoleucyl-tRNA synthetase |
| 27 | GDC_HINF_1082407 | 1082407 | 1082514 | 35 | - | protein V6, truncated Haemophilus influenzae |
| 28 | GDC_HINF_1144501 | 1144501 | 1145004 | 167 | - | PnuC transporter |
| 29 | GDC_HINF_11279189 279189 | 1279189 | 1279935 | 248 | - | Peptide chain release factor 2 (RF-2) |
| 30 | GDC_HINF_1347200 | 1347200 | 1347445 | 81 | + | putative ABC transport protein |
| 31 | GDC_HINF_1347942 | 1347942 | 1348478 | 178 | + | putative iron compound ABC transporter |
| 32 | GDC_HINF_1476415 | 1476415 | 1476615 | 66 | - | PstB |
| 33 | GDC_HINF_1476557 | 1476557 | 1477183 | 208 | - | PstB |
| 34 | GDC_HINF_1505851 | 1505851 | 1506048 | 65 | - | terminase large subunit |
| 35 | GDC_HINF_1524561 | 1524561 | 1525421 | 286 | - | ThiI |
| 36 | GDC_HINF_1568974 | 1568974 | 1569300 | 108 | + | DNA-binding protein rdgB homolog |
| 37 | GDC_HINF_1586944 | 1586944 | 1587765 | 273 | + | putative tail protein |
| 38 | GDC_HINF_1594339 | 1594339 | 1594854 | 171 | - | NifC |
| 39 | GDC_HINF_1634710 | 1634710 | 1636722 | 670 | + | Probable hemoglobin and hemoglobin-haptoglobin |
| 40 | GDC_HINF_1638626 | 1638626 | 1639372 | 248 | - | Putative integrase/recombinase HI1572 |
| 41 | GDC_HINF_1639409 | 1639409 | 1639726 | 105 | - | Putative integrase/recombinase HI1572 |
| 42 | GDC_HINF_1660491 | 1660491 | 1662080 | 529 | - | Cell division protein ftsK homolog |
| 43 | GDC_HINF_1807963 | 1807963 | 1808859 | 298 | - | adhesin homolog HI1732 |
| 44 | GDC_HINF_1817220 | 1817220 | 1817417 | 65 | + | Cell wall-associated hydrolase |
| 45 | GDC_HPYL_51094 | 51094 | 51432 | 112 | - | putative HP0052-like protein |
| 46 | GDC_HPYL_155367 | 155367 | 156164 | 265 | - | 2-oxoglutarate/malate translocator |
| 47 | GDC_HPYL_447632 | 447632 | 447850 | 72 | - | Cell wall-associated hydrolase |
| 48 | GDC_HPYL_506250 | 506250 | 507134 | 294 | + | site-specific DNA-methyltransferase |
| 49 | GDC_HPYL_583607 | 583607 | 583876 | 89 | + | probable DNA helicase |
| 50 | GDC_HPYL_583883 | 583883 | 584437 | 184 | + | probable DNA helicase |
| 51 | GDC_HPYL_665045 | 665045 | 665695 | 216 | + | putative lipopolysaccharide biosynthesis protein |
| 52 | GDC_HPYL_953783 | 953783 | 954664 | 293 | - | acetate kinase |
| 53 | GDC_HPYL_954679 | 954679 | 954900 | 73 | - | phosphate acetyltransferase |
| 54 | GDC_HPYL_954846 | 954846 | 955217 | 123 | - | PHOSPHOTRANSACETYL ASE |
| 55 | GDC_HPYL_955261 | 955261 | 955557 | 98 | - | phosphate acetyltransferase |
| 56 | GDC_HPYL_1068602 | 1068602 | 1069459 | 285 | - | IS606 TRANSPOSASE |
| 57 | GDC_HPYL_1069456 | 1069456 | 1069929 | 157 | - | transposase-like protein, PS31S |
| 58 | GDC_HPYL_1376803 | 1376803 | 1377126 | 107 | + | ribosomal protein |
| 59 | GDC_HPYL_1474291 | 1474291 | 1474509 | 72 | + | Cell wall-associated hydrolase |
| 60 | GDC_HPYL_1600102 | 1600102 | 1600689 | 195 | - | TYPE III DNA MODIFICATION ENZYME |
| 61 | GDC_MTUB_26830 | 26830 | 27534 | 234 | - | putative protoporphyrinogen oxidase |
| 62 | GDC_MTUB_36276 | 36276 | 36785 | 169 | - | fibronectin-attachment protein FAP-P |
| 63 | GDC_MTUB_6032 | 76032 7 | 76595 | 187 | + | retinoblastoma inhibiting gene 1 |
| 64 | GDC_MTUB_80423 | 80423 | 81214 | 263 | - | mucin 5 |
| 65 | GDC_MTUB_167239 | 167239 | 168084 | 281 | + | putative secreted peptidase |
| 66 | GDC_MTUB_214625 | 214625 | 215116 | 163 | - | glycoprotein gp2 |
| 67 | GDC_MTUB_424142 | 424142 | 424657 | 171 | - | PPE FAMILY PROTEIN |
| 68 | GDC_MTUB_459316 | 459316 | 461076 | 586 | + | 63 kDa protein |
| 69 | GDC_MTUB_549643 | 549643 | 550758 | 371 | - | carR |
| 70 | GDC_MTUB_566823 | 566823 | 567284 | 153 | + | MAPK-interacting and spindle-stabilizing protein |
| 71 | GDC_MTUB_591109 | 591109 | 591345 | 78 | + | excisionase, putative |
| 72 | GDC_MTUB_663028 | 663028 | 663426 | 132 | + | PROBABLE RIBONUCLEOSIDE-DIPHOSPHATE REDUCTASE |
| 73 | GDC_MTUB_688806 | 688806 | 689060 | 84 | + | MCE-FAMILY PROTEIN MCE2B |
| 74 | GDC_MTUB_701762 | 701762 | 702643 | 293 | - | u1764ad |
| 75 | GDC_MTUB_731710 | 731710 | 731877 | 55 | + | ribosomal protein L33 |
| 76 | GDC_MTUB_772761 | 772761 | 773402 | 213 | - | ENSANGP00000004917 |
| 77 | GDC_MTUB_868821 | 868821 | 869216 | 131 | - | cold-shock induced protein of the Srp1p/Tip1p |
| 78 | GDC_MTUB_890358 | 890358 | 891254 | 298 | - | orf2 |
| 79 | GDC_MTUB_904043 | 904043 | 904840 | 265 | + | aminoimidazole ribotide synthetase |
| 80 | GDC_MTUB_1045383 | 1045383 | 1046129 | 248 | + | u650i |
| 81 | GDC_MTUB_1068100 | 1068100 | 1068726 | 208 | - | anchorage subunit of a-agglutinin; Aga1p |
| 82 | GDC_MTUB_1115707 | 1115707 | 1116369 | 220 | - | mucin 7 precursor, salivary |
| 83 | GDC_MTUB_1124996 | 1124996 | 1125712 | 238 | - | putative oxidoreductase |
| 84 | GDC_MTUB_1138949 | 1138949 | 1139665 | 238 | - | platelet binding protein GspB |
| 85 | GDC_MTUB_1170285 | 1170285 | 1170749 | 154 | - | MC8 |
| 86 | GDC_MTUB_1176592 | 1176592 | 1176858 88 | | + | gp85 |
| 87 | GDC_MTUB_1202653 | 1202653 | 1203198 | 181 | - | s19 chorion protein |
| 88 | GDC_MTUB_1231843 | 1231843 | 1232460 | 205 | + | carboxylesterase |
| 89 | GDC_MTUB_1241031 | 1241031 | 1241468 | 145 | - | PE |
| 90 | GDC_MTUB_1252888 | 1252888 | 1253748 | 286 | - | ppg3 |
| 91 | GDC_MTUB_1264312 | 1264312 | 1264554 | 80 | + | ketoacyl-CoA thiolase-related protein |
| 92 | GDC_MTUB_1286282 | 1286282 | 1286587 | 101 | - | pterin-4-alpha-carbinolamine dehydratase |
| 93 | GDC_MTUB_1301742 | 1301742 | 1302053 | 103 | - | similar to ORF starts at 87, first start codon |
| 94 | GDC_MTUB_1351907 | 1351907 | 1352614 | 235 | - | ppg3 |
| 95 | GDC_MTUB_1476279 | 1476279 | 1476647 | 122 | - | Cell wall-associated hydrolase |
| 96 | GDC_MTUB_148531 | 1485311 | 1486399 | 362 | - | 4-hydroxyphenylpyruvate dioxygenase C terminal |
| 97 | GDC_MTUB_1486309 | 1486309 | 1487727 | 472 | - | cell wall surface anchor family protein |
| 98 | GDC_MTUB_1515112 | 1515112 | 1515846 | 244 | - | putative ABC transporter ATP binding protein |
| 99 | GDC_MTUB_1515464 | 1515464 | 1516198 | 244 | - | extracellular protein, gamma-D-glutamate-meso-d... |
| 100 | GDC_MTUB_1596569 | 1596569 | 1596892 | 107 | - | putative translation initiation factor IF-2 |
| 101 | GDC_MTUB_1600905 | 1600905 | 1601861 | 318 | - | carboxylesterase family protein |
| 102 | GDC_MTUB_1616064 | 1616064 | 1616951 | 295 | - | PUTATIVE TRANSCRIPTION REGULATOR PROTEIN |
| 103 | GDC_MTUB_1672449 | 1672449 | 1673216 | 255 | + | MAV278 |
| 104 | GDC_MTUB_1673708 | 1673708 | 1675000 | 430 | - | MAV301 |
| 105 | GDC_MTUB_1699549 | 1699549 | 1700226 | 225 | + | gmdA |
| 106 | GDC_MTUB_1742061 | 1742061 | 1742858 | 265 | - | ENSANGP00000020758 |
| 107 | GDC_MTUB_1782153 | 1782153 | 1782932 | 259 | + | GLP_26_54603_52153 |
| 108 | GDC_MTUB_2060659 | 2060659 | 2061114 | 151 | + | nuclear factor of kappa light polypeptide gene |
| 109 | GDC_MTUB_2093062 | 2093062 | 2093994 | 310 | - | PROBABLE 6-PHOSPHOGLUCONATE DEHYDROGENASE GND1 |
| 110 | GDC_MTUB_2105797 | 2105797 | 2106912 | 371 | + | ATP-binding subunit of ABC-transport system |
| 111 | GDC_MTUB_2133554 | 2133554 | 2134069 | 171 | - | KIAA0324 protein |
| 112 | GDC_MTUB_2183418 | 2183418 | 2184026 | 202 | - | putative transport protein |
| 113 | GDC_MTUB_2192571 | 2192571 | 2193488 | 305 | - | putative oxidoreductase |
| 114 | GDC_MTUB_2234641 | 2234641 | 2234889 | 82 | - | DNA-binding protein, CopG family |
| 115 | GDC_MTUB_2320829 | 2320829 | 2321062 | 77 | + | DNA-binding protein, CopG family |
| 116 | GDC_MTUB_2321250 | 2321250 | 2322509 | 419 | - | cell wall surface anchor family protein |
| 117 | GDC_MTUB_2487508 | 2487508 | 2488524 | 338 | - | ORF1 |
| 118 | GDC_MTUB_2567990 | 2567990 | 2568457 | 155 | + | B1158F07.3 |
| 119 | GDC_MTUB_2577106 | 2577106 | 2577699 | 197 | + | POSSIBLE CONSERVED MEMBRANE PROTEIN |
| 120 | GDC_MTUB_2577486 | 2577486 | 2577920 | 144 | + | POSSIBLE CONSERVED MEMBRANE PROTEIN |
| 121 | GDC_MTUB_2690012 | 2690012 | 2690509 | 165 | + | PROBABLE CONSERVED INTEGRAL MEMBRANE PROTEIN |
| 122 | GDC_MTUB_2698040 | 2698040 | 2698243 | 67 | - | POSSIBLE CONSERVED MEMBRANE PROTEIN |
| 123 | GDC_MTUB_2712275 | 2712275 | 2714008 | 577 | + | MLCL536.10 protein |
| 124 | GDC_MTUB_2725593 | 2725593 | 2725859 | 88 | - | PROBABLE HYDROGEN PEROXIDE-INDUCIBLE GENES |
| 125 | GDC_MTUB_2733212 | 2733212 | 2734420 | 402 | - | lycoprotein gp2 |
| 126 | GDC_MTUB_2828257 | 2828257 | 2828937 | 226 | + | MC8 |
| 127 | GDC_MTUB_2895354 | 2895354 | 2897222 | 622 | + | antigen T5 |
| 128 | GDC_MTUB_2983047 | 2983047 | 2984033 | 328 | - | MC8 |
| 129 | GDC_MTUB_3005316 | 3005316 | 3005696 | 126 | - | ABC transporter, ATP-binding protein |
| 130 | GDC_MTUB_048559 | 3048559 3 | 3049095 | 178 | - | recX protein |
| 131 | GDC_MTUB_3065095 | 3065095 | 3066549 | 484 | + | ppg3 |
| 132 | GDC_MTUB_3100192 | 3100192 | 3100452 | 86 | - | IS1537, transposase |
| 133 | GDC_MTUB_3129118 | 3129118 | 3129594 | 158 | - | KIAA1139 protein |
| 134 | GDC_MTUB_3237815 | 3237815 | 3238096 | 93 | - | acylphosphatase |
| 135 | GDC_MTUB_3283182 | 3283182 | 3283718 | 178 | - | Putative mycocerosyl transferase in MAS 5'r... |
| 136 | GDC_MTUB_3289702 | 3289702 | 3290232 | 176 | + | POSSIBLE TRANSPOSASE |
| 137 | GDC_MTUB_3319076 | 3319076 | 3319546 | 156 | - | u0002d |
| 138 | GDC_MTUB_3339006 | 3339006 | 3339851 | 281 | - | membrane glycoprotein |
| 139 | GDC_MTUB_3356995 | 3356995 | 3357831 | 278 | - | sensor histidine kinase |
| 140 | GDC_MTUB_3381198 | 3381198 | 3381755 | 185 | + | MC8 |
| 141 | GDC_MTUB_3388071 | 3388071 | 3389003 | 310 | + | cellulosomal scaffoldin anchoring protein C |
| 142 | GDC_MTUB_3482312 | 3482312 | 3482770 | 152 | - | MC8 |
| 143 | GDC_MTUB_3581973 | 3581973 | 3582620 | 215 | + | similar to mucin, submaxillary - pig |
| 144 | GDC_MTUB_3711717 | 3711717 | 3712613 | 298 | - | orf2 |
| 145 | GDC_MTUB_3716987 | 3716987 | 3718534 | 515 | - | similar to profilaggrin - human (fragments) |
| 146 | GDC_MTUB_3754581 | 3754581 | 3755711 | 376 | - | putative transposase |
| 147 | GDC_MTUB_3794808 | 3794808 | 3795026 | 72 | - | deoxyxylulose-5-phosphate synthase |
| 148 | GDC_MTUB_3796793 | 3796793 | 3797512 | 239 | + | membrane glycoprotein [imported] - equine herpesvirus |
| 149 | GDC_MTUB_3879013 | 3879013 | 3879534 | 173 | - | ribosomal protein S11 |
| 150 | GDC_MTUB_3921024 | 3921024 | 3921665 | 213 | - | 3-oxoacyl-(acyl-carrier-protein) reductase |
| 151 | GDC_MTUB_3974481 | 3974481 | 3975056 | 191 | + | mucin 10 |
| 152 | GDC_MTUB_3994808 | 3994808 | 3995446 | 212 | + | MAV278 |
| 153 | GDC_MTUB_3998938 | 3998938 | 3999642 | 234 | - | protease inhibitor/seed storage/lipid transfer |
| 154 | GDC_MTUB_4021183 | 4021183 | 4021425 | 80 | - | PUTATIVE TRNA/RRNA METHYLTRANSFERASE |
| 155 | GDC_MTUB_4046290 | 4045946 | 4045946 | 114 | - | chalcone/stilbene synthase family protein |
| 156 | GDC_MTUB_4053635 | 4053033 | 4053033 | 200 | + | putative protein (2G313) |
| 157 | GDC_MTUB_4140460 | 4140236 | 4140236 | 74 | - | DNA-binding protein, CopG family |
| 158 | GDC_MTUB_4169706 | 4169350 | 4169350 | 118 | + | PROBABLE CUTINASE PRECURSOR CUT5 |
| 159 | GDC_MTUB_4171211 | 4170798 | 4170798 | 137 | + | PUTATIVE OXIDOREDUCTASE |
| 160 | GDC_MTUB_4252921 | 4252190 | 4252190 | 243 | + | Salivary gland secretion 1 CG3047-PA |
| 161 | GDC_MTUB_4261213 | 4260620 | 4260620 | 197 | + | SPAPB15E9.01c |
| 162 | GDC_MTUB_4302858 | 4302166 | 4302166 | 230 | + | u1764ad |
| 163 | GDC_MTUB_4318309 | 4317863 | 4317863 | 148 | + | POSSIBLE TRANSPOSASE [SECOND PART] |
| 164 | GDC_MTUB_4342388 | 4341852 | 4341852 | 178 | - | GLP_49_64409_65443 |
| 165 | GDC_MTUB_4391988 | 4391527 | 4391527 | 153 | - | AT9S |
| 166 | gi!Sars174_ref seq_OUTPUT F_GDC_701_1 225 | 701 | 1225 | 174 | + | ABC transporter ATP binding protein/Cytochrome c oxidase folding protein |
| 167 | gi!Sars68_refs eq_OUTPUTF _GDC_1397_1 603 | 1397 | 1603 | 68 | + | Major facilitator for superfamily protein or serine/threonine kinase 2 |
| 168 | gi!Sars61_refs eq_OUTPUTF _GDC_8828_9 013 | 8828 | 9013 | 61 | + | Putative protein |
| 169 | gi!Sars78_refs eq_OUTPUTF _GDC_28559_ 28795 | 24492 | 24764 | 90 | + | NADH dehydrogenase I chain |

A systematic sensitivity and specificity analysis of GeneDecipher has been done on 10 microbial genomes (Figure 3). Further analysis of GeneDecipher on viral genomes is presented here. *SARS-CoV genome sequence*:Sequences of the 18 SARS-CoV strains available in the GenBank database (http://www.ncbi.nlm.nih.gov/Entrez/genomes/viruses) were downloaded and analyzed.

These include SARS-CoV Refseq (NC_004718.3),SARS-CoV TWC(AY32118), SIN2774(AY283798),SIN2748(AY283797) SIN267^(AY283796), SIN2677(AY283794), SIN25ti6(AY283794), Frankfurt1(A Y291315), BJ04(AY279354) BJ03(AY278490), BJ02(AY278487), GZ01(AY278848), CUHKW1(AY278554), TOR2(AY274119), TW1(AY291451), BJ01(AY278488), Urban(AY278741), HKU-39849(AY278491). Other information related to protein coding genes was retrieved from .http://www.ncbi.nlm.nih.gov/genomes/SARS/SAks.html *Testing of GeneDecipher on viral genomes:*

To test our method on viral genomes the applicants first analyzed *Human Respiratory Syncytial Virus (HRSV)*, complete genome using GeneDecipher. Comparison of GeneDecipher results with state of the art method ZCURVE_CoV has been done (Table 3). ZCURVE_CoV is able to predict 8 annotated proteins out of 11 reported at NCBI without any false positives. ZCURVE_CoV was unable to predict the following three genes: PID 9629200 (location 626...1000, non-structural protein2 (NS2)); PID 9629205 (location 4690...5589, attachment glycoprotein (G)); and PID 9629208 (location 8171...8443, matrix protein 2(M2)). GeneDecipher predicted 10 out of total 11 annotated proteins of HRSV without any false positives. The gene missed by GeneDecipher was PID 9629208 (location 8171...8443, matrix protein 2) which was notably missed by ZCURVE_CoV too.

This successful prediction of protein coding regions in *HRSV* genome increases our confidence to predict protein coding regions on newly sequenced SARS-CoV genomes.

### Analysis of SARS-CoV using GeneDecipher:

The applicants analyzed all 18 strains of SARS-CoV using GeneDecipher. (Detailed results are available on the website given above). GeneDecipher predicts a total of 15 protein coding regions in SARS-CoV genomes including both the polyproteins 1a, 1ab (Sars2628 C-terminal end of Polyprotein1ab), and all four known structural proteins (M, N, S, and E) for each of the 18 strains. GeneDecipher also predicts 6 to 8 additional coding regions depending on the genome sequence of the strain used. The length of these additional coding regions varied between 61 and 274 amino acids. GeneDecipher predicts 12 coding regions which are common to all 18 strains (Table 4), and one coding region (Sars63, sars6 at NCBI refseq genome) present in 5 strains. GeneDecipher predicts gene Sars90 in GZ01 strain, and Sars154 (Sars 3b at NCBI refseq genome) in BJ02 strain specifically.

These 12 common protein coding regions consist of the 6 basic proteins of SARS-CoV (2 polyproteins and the 4 structural proteins); Sars274 (Sars3a at NCBI refseq database), Sars 122 (Sars7a at NCBI refseq database), Sars78 (already reported with start shifted as ORF14/Sars9c in TOR2 strain); and three newly predicted (false positives with respect to current annotation at NCBI) protein coding regions Sars174, Sars68, and Sars61. The three newly predicted genes lie completely within polyprotein 1a genomic region. Although our method discards such genes in bacterial genomes, possibility of finding such genes in viral genomes has not been ruled out. As these genes are present in all 18 strains it is likely that they are protein coding genes.

The applicants predict three more coding regions Sars63, Sars154, and Sars90 apart from the 12 discussed above. Sars63 is identified in 5 strains and not identified in remaining 13 strains. This coding region is already reported in NCBI refseq (Sars6). Here the applicants can not comment much about the existence of Sars63 (Sars6 at NCBI refseq) because it is identified in 5 strains and not identified in rest 13. This is due to high density of non-synonymous mutations across strains in this region. Two coding regions Sars154 (sars3b at NCBI), and Sars90 (newly predicted in GZ01 starin) are identified in only one strain. Since these two coding regions are identified in only one strain, they are less likely to be protein coding regions, as also suggested by ZCURVE_CoV (Chen *et al.,* 2003) analysis. The locations of these three genes in different strains are provided in Table 5.

Since the peptide libraries are made from the genome sequences of various organisms, the evolutionary origin of a given protein can be traced. If the protein is rich in heptapeptides found occurring in viral genomes then that protein is considered to be of viral origin. The applicants found that 5 core proteins (two polyproteins and three structural proteins M, N, and S) are of viral origin. The remaining, including 3 new predictions, are of prokaryotic origin. It is interesting to that from the same DNA region the applicants are getting proteins in different frames which contain peptides from different origin. Here, how same DNA sequence can code for both bacterial and viral origin is intriguing. This might explain why these new protein coding genes were not detected in primary attempts based on homology to other known viral genome sequences.

### Comparison with the existing system - ZURVE_CoV:

Comparison of GeneDecipher, ZCURVE_CoV results with the known annotations for Urbani and TOR2 strains of SARS-CoV are presented in Tables 6a and 6b.

In general, GeneDecipher results are in good agreement with the known annotations. In case of Urbani strain GeneDecipher predicts all the known genes except Sars84(X5), Sars63(X3) and Sars154(X2). Sars84(X5) and Sars63(X3) are supported by ZCURVE_CoV whereas Sars154(X2) is missed by both the methods. GeneDecipher predicts four new genes in this strain which incidentally are not supported by ZCURVE_CoV. It is noticeable that out of these four genes Sars78 is already known for strain TOR2 as ORF14/Sars9c. This supports the likelihood of the gene being present in Urbani strain. However, ZCURVE_CoV predicts 2 new genes which are not supported by GeneDecipher either.

GeneDecipher predictions for TOR2 strain are identical with those for Urbani strain. In this strain GeneDecipher predicts 9 known genes but fails to predict 6 genes with known annotations. These 6 genes are: Sars154 (ORF4), Sars98 (ORF13), Sars63 (ORF7), Sars44 (ORF9), Sars39 (ORF10), and Sars84 (ORF11). Of these, Sars154 (ORF4) and Sars98 (ORF13) are also missed by ZCURVE_CoV. It is to be noted that both Sars44 (ORF9) and Sars39 (ORF10) are ORFs very small in length (44 and 39 amino acids respectively), and their presence too is not consistent across various SARS strains. Sars63 (ORF7) has been predicted by GeneDecipher in 5 other strains but not in the two strains considered here.

### Mutation Analysis:

Analysis using multiple sequence alignment (ClustalW) for 3 newly predicted protein coding genes Sars 174, Sars68 and Sars61 across all 18 strains shows:
1. Sars68 has one point mutation at location 80 GAT->GGT (D->G) SIN2677 strain.
2. Sars174 has two synonymous point mutations at location 204 CGA->CGC in GZ01 strain and at location 447 CTG->CTT in BJ04 strain.
3. Sars61 has one point mutation at location 119 CTG->CAG (L->Q) in GZ01 strain.

These three newly predicted genes are present in all 18 strains without significant mutations and has no significant hits with BLASTP in non-redundant database. This indicates that these three proteins might have crucial biological functions specific to SARS-CoV. Therefore these coding sequences might serve as candidate drug targets against SARS.

### Function Assignment:

In total the applicants predict 15 coding regions in SARS-CoV out of which functions of the four structural proteins (M, N, S and E) have already been assigned. Although the polyprotein 1ab has been assigned only replicase activity, our analysis implies that the replicase activity is associated with Sars2628 (C terminal of ORF 1ab) fragment. The complete 1ab polyprotein contains 6 functional signatures of which polyprotein I a contains signatures associated with metabolic enzymes (Table 7a). Functions were assigned to the polyproteins on the basis of peptides (length 7 or more amino acids) occurring in proteins having similar functions in at least 5 different organisms. Other predicted genes/protein coding regions contain peptides which occur in fewer genomes. Based on these peptides the applicants suggest functions, albeit with lesser confidence (Table 7b). The biological relevance of these finding remains to be explored.

**Table3. Comparison of GeneDecipher results with ZCURVE_CoV results on HRSV genome, with respect to annotated genes**

| Annotated genes | | | ZCURVE_CoV | | | GeneDecipher | | |
|---|---|---|---|---|---|---|---|---|
| Start | End | Length | Start | End | Length | Start | End | Length |
| 99 | 518 | 139 | 99 | 518 | 139 | 99 | 518 | 139 |
| 626 | 1000 | 124 | -- | -- | -- | 626 | 1000 | 124 |
| 1140 | 2315 | 391 | 1140 | 2315 | 391 | 1140 | 2315 | 391 |
| 2348 | 3073 | 241 | 2348 | 3073 | 241 | 2348 | 3073 | 241 |
| 3263 | 4033 | 256 | 3158 | 4033 | 291 | 3158 | 4033 | 291 |
| 4303 | 4500 | 65 | 4303 | 4500 | 65 | 4303 | 4500 | 65 |
| 4690 | 5589 | 299 | -- | -- | -- | 4690 | 5589 | 299 |
| 5666 | 7390 | 574 | 5666 | 7390 | 574 | 5621 | 7390 | 589 |
| 7618 | 8205 | 195 | 7618 | 8205 | 195 | 7618 | 8205 | 195 |
| 8171 | 8443 | 90 | -- | -- | -- | -- | -- | -- |
| 8509 | 15009 | 2166 | 8443 | 15009 | 2188 | 8443 | 15009 | 2188 |

**Table4: Protein coding genes predicted by GeneDecipher in SARS-CoV Refseq common to all 18 strains.**

| **S.No.** | **Start** | **Stop** | **Frame** | **Length** | | **Feature** |
|---|---|---|---|---|---|---|
| | | | | **bp** | **aa** | |
| 1 | 265 | 13413 | 1+ | 13149 | 4382 | Sars1a polyprotein |
| 2 | 701 | 1225 | 2+ | 525 | 174 | Sars174(new prediction) |
| 3 | 1397 | 1603 | 2+ | 207 | 68 | Sars68(new prediction) |
| 4 | 8828 | 9013 | 2+ | 186 | 61 | Sars61(new prediction) |
| 5 | 13599 | 21485 | 3+ | 7887 | 2628 | Sars2628(C-terminal end of polyprotein 1ab) |
| 6 | 21492 | 25259 | 3+ | 3768 | 1255 | Spike (S) protein |
| 7 | 25268 | 26092 | 2+ | 825 | 274 | Sars274(Sars 3a) |
| 8 | 26117 | 26347 | 2+ | 231 | 76 | Sars76(Sars4) |
| 9 | 26398 | 27063 | 1+ | 666 | 221 | Sars221(Sars5) |
| 10 | 27273 | 27641 | 3+ | 369 | 122 | Sars122(Sars7a) |
| 11 | 28120 | 29388 | 1+ | 1269 | 422 | Sars422(Sars9a) |
| 12 | 28559 | 28795 | 2+ | 237 | 78 | Sars78 ( Identical to ORF 14/Sars9c in TOR2 with shifted start) |

**Table5: Identification of Sars90, Sars63, Sars154 as protein coding genes by GeneDecipher in various strains of SARS-CoV**

| **S.No.** | **Strain name** | **Sars90 (New prediction)** | **Sars63(Sars6 at NCBI)** | **Sars154(Sars 3b at NCBI)** |
|---|---|---|---|---|
| 1 | SIN2748 | -- | -- | -- |
| 2 | BJ01 | -- | 27055..27246 -- | |
| 3 | BJ02 | -- | 27074..27265 | 25689..26153 |
| 4 | BJ03 | -- | 27070..27261 | -- |
| 5 | BJ04 | -- | 27058..27249 | -- |
| 6 | Frankfurtt 1 | -- | -- | -- |
| 7 | Urbani | -- | -- | -- |
| 8 | GZ01 | 24492..24764 | 27058..27249 -- | |
| 9 | SIN2500 | -- | -- | -- |
| 10 | SIN2677 | -- | -- | -- |
| 11 | SIN2679 | -- | -- | |
| 12 | SIN2774 | -- | -- | -- |
| 13 | CHUKW1 | -- | -- | -- |
| 14 | TW1 | -- | -- | -- |
| 15 | TWC | -- | -- | -- |
| 16 | HKU-39849 | -- | -- | -- |
| 17 | Refseq | -- | -- | -- |
| 18 | TOR2 | -- | -- | -- |

**Table 6(a). Comparison of GeneDecipher results with ZCURVE_CoV results on SARS-CoV genome Urbani strain, with respect to annotated genes**

| Annotated genes | | | ZCURVE_CoV | | | GeneDecipher | | | Features |
|---|---|---|---|---|---|---|---|---|---|
| Start | End | Length | Start | End | Leng th | Start | End | Length | |
| 265 | 1339 8 | 4377 | 265 | 13398 | 4377 | 265 | 13413 | 4382 | ORF 1a |
| -- | -- | -- | -- | -- | -- | 701 | 1225 | 174 | Sars 174(New prediction by GeneDecipher) |
| -- | -- | -- | -- | -- | -- | 1397 | 1603 | 68 | Sars68(New prediction by GeneDecipher) |
| -- | -- | -- | -- | -- | -- | 8828 | 9013 | 61 | Sars61(New prediction by GeneDecipher) |
| 13398 | 2148 5 | 2695 | 13398 | 21485 | 2695 | 13599 | 21485 | 2628 | ORF 1b |
| 21492 | 2525 9 | 1255 | 21492 | 25259 | 1255 | 21492 | 25259 | 1255 | S protein |
| 25268 | 2609 2 | 274 | 25268 | 26092 | 274 | 25268 | 26092 | 274 | Sars274(X1) |
| 25689 | 2615 3 | 154 | -- | -- | -- | -- | -- | -- | Sars154(X2) |
| 26117 | 2634 7 | 76 | 26117 | 26347 | 76 | 26117 | 26347 | 76 | E protein |
| 26398 | 2706 3 | 221 | 26398 | 27063 | 221 | 26389 | 27063 | 224 | M protein |
| 27074 | 27265 | 63 | 27074 | 27265 | 63 | -- | -- | -- | Sars63(X3) |
| 27273 | 27641 | 122 | 27273 | 27641 | 122 | 27273 | 27641 | 122 | Sars122(X4) |
| -- | -- | -- | 27638 | 27772 | 44 | -- | -- | -- | Sars44 |
| -- | -- | -- | 27779 | 27898 | 39 | -- | -- | -- | Sars39 |
| 27864 | 28118 | 84 | 27864 | 28118 | 84 | -- | -- | -- | Sars84(X5) |
| 28120 | 29388 | 422 | 28120 | 29388 | 422 | 28120 | 29388 | 422 | N protein |
| -- | -- | -- | -- | -- | -- | 28559 | 28795 | 78 | Sars78(Identica I to ORF 14/Sars9c in TOR2 with shifted start) |

**Table 6(b). Comparison of GeneDecipher results with ZCURVE_CoV results on SARS-CoV genome TOR2 strain, with respect to annotated genes**

| Annotated genes | | | ZCURVE_CoV predicted genes | | | GeneDecipher predicted genes | | | Features |
|---|---|---|---|---|---|---|---|---|---|
| Start | End | Length | Start | End | Length | Start | End | Length | |
| 265 | 13398 | 4377 | 265 | 13398 | 4377 | 265 | 13413 | 4382 | ORF 1a |
| -- | -- | -- | -- | -- | -- | 701 | 1225 | 174 | Sars174(New prediction by GeneDecipher) |
| -- | -- | -- | -- | -- | -- | 1397 | 1603 | 68 | Sars68(New prediction by GeneDecipher) |
| -- | -- | -- | -- | -- | -- | 8828 | 9013 | 61 | Sars61(New prediction by GeneDecipher) |
| 13398 | 21485 | 2695 | 13398 | 21485 | 2695 | 13599 | 21485 | 2628 | ORF 1b |
| 21492 | 25259 | 1255 | 21492 | 25259 | 1255 | 21492 | 25259 | 1255 | S protein |
| 25268 | 26092 | 274 | 25268 | 26092 | 274 | 25268 | 26092 | 274 | ORF3(Sars274) |
| 25689 | 26153 | 154 | -- | -- | -- | -- | -- | -- | ORF4(Sars154) |
| 26117 | 26347 | 76 | 26117 | 26347 | 76 | 26117 | 26347 | 76 | E protein |
| 26398 | 27063 | 221 | 26398 | 27063 | 221 | 26389 | 27063 | 224 | M protein |
| 27074 | 27265 | 63 | 27074 | 27265 | 63 | -- | -- | -- | Sars63(ORF7) |
| 27273 | 27641 | 122 | 27273 | 27641 | 122 | 27273 | 27641 | 122 | Sars122(ORF8) |
| 27638 | 27772 | 44 | 27638 | 27772 | 44 | -- | -- | -- | Sars44(ORF9) |
| 27779 | 27898 | 39 | 27779 | 27898 | 39 | -- | -- | -- | Sars39(ORF10) |
| 27864 | 28118 | 84 | 27864 | 28118 | 84 | -- | -- | -- | Sars84(ORF11) |
| 28120 | 29388 | 422 | 28120 | 29388 | 422 | 28120 | 29388 | 422 | N protein |
| 28130 | 28426 | 98 | -- | -- | -- | -- | -- | -- | ORF13 |
| 28583 | 28795 | 70 | -- | -- | -- | 28559 | 28795 | 78 | Sars78(Identical to ORF 14/Sars9c in TOR2 with shifted start) |

**Table 7(a): Functional assignment of polyproteins in SARS (Urbani) Genome using PLHOST**

| **S.No.** | **NCBI annotation** | **Conserved peptide signature** | **Function assigned** |
|---|---|---|---|
| 1 | Sars1ab (Poly protein 1ab) | RIRASLPT | Phosphoglycerate kinase |
| | | RSETLLPL | Sulfite reductase (NADPH), Flavoprotein beta subunit |
| | | LDKLKSLL | Probable acyl-CoA thiolase |
| | | ATVVIGTS | cell division protein ftsZ |
| | | NVAITRAK | DNA-binding protein, probably DNA helicase |
| | | LQGPPGTGK | DNA helicase related protein |
| 2 | Sars 1a poly protein 1a | RIRASLPT | Phosphoglycerate kinase |
| | | RSETLLPL | Sulfite reductase (NADPH), Flavoprotein beta subunit |
| | | LDKLKSLL | Probable acyl-CoA thiolase |
| 3 | Sars 2628 (C terminal of Sars 1ab) | ATVVIGTS | cell division protein ftsZ |
| | | NVAITRAK | DNA-binding protein, probably DNA helicase |
| | | LQGPPGTGK | DNA helicase related protein |

**Table7 (b): Suggested functions for some of the non-structural genes in SARS-CoV using PLHOST**

| **S.No.** | **Gene** | **Peptide Signature** | **Suggested function** |
|---|---|---|---|
| 1 | Sars174(new prediction) | TLSKGNAQ | ABC transporter ATP binding protein *[Lactococcus lactis subsp. lactis]* |
| | | VAQMGTLL | Cytochrome c oxidase folding protein *[Synechocystis sp. PCC 6803]* |
| 2 | Sars68(new prediction) | LVLVLILA | *putative major facilitator superfamily protein [Schizosaccharomyces pombe]* |
| | | TQTLKLDS | *serine*/*threonine kinase 2: Serine*/*threonine protein kinase-2 [Homo sapiens]* |
| 3^{*} | Sars90(new prediction only in GZ01 strain) | GLLHRGT | NADH Dehydrogenase 1 Chain |
| 4 | Sars61(new prediction) | LLPLLAFL | Putative protein (Conserved across 2 organisms) |
| 5 | Sars274(Sars3a) | LLLFVTIY | Polyamine transport protein; Tpo1p *[Saccharomyces cerevisiae]* |
| 6 | Sars154(Sars3b) | QTLVLKML | K550.3.p *[Caenorhabditis elegans]* |
| 7 | Sars63(Sars6) | DDEELMEL | Elongation factor Tu *[Lactococcus lactis subsp. lactis]* |
| 8 | Sars122(Sars7a) | LIVAALVF | Putative transport transmembrane protein *[Sinorhizobium meliloti]* |
| | | RARSVSPK | Src homology domain 3 *[Caenorhabditis elegans]* |
| 9* | Sars78(Sars9c) | QLLAAVG | Gamma-glutamate kinase (Conserved across 8 organisms) |

| | | | |
|---|---|---|---|
| *: No conserved octapeptide was found. However, function has been assigned on the basis of the only highly conserved heptapeptide. | | | |

From the aforementioned The applicants have disclosed 4 new genes including Sars78 in SARS-CoV. The analysis further corroborates the finding of ZCURVE_CoV (Chen et al., 2003) that ORF Sars154 (listed in Refseq as Sars3b) is unlikely to be a coding region. The applicants have also assigned functions to the two polyproteins 1ab and 1a. In addition to replication associated function of C-terminal of 1ab polyprotein, the applicants' analysis implies that the polyprotein 1a may be associated with metabolic enzyme like functions. In all, six peptide signatures are present in polyprotein 1ab. The applicants have suggested putative function for other 9 proteins including ones newly predicted by GeneDecipher.

### Advantages:

1. Main advantage of the present invention is to provide a new method for prediction of protein coding DNA sequences without using any external evidences like ribosome binding sites, promoter sequences, transcription start sites or codon usage biases.
2. It provides a method for statistical analysis of protein coding DNA sequences that utilizes the biological information retained in the conserved peptides which withstood evolutionary pressure.
3. It provides a simple method for start site prediction of a protein coding gene.
4. It provides a method to detect organism specific, strain specific protein coding DNA sequences.
5. It provides novel protein coding DNA sequences, which could be used as potential drug targets.

### References:

Altschul,S.F., Gish,W., Miller,W., Myers,E.W., Lipman,D.J. (1990 ) Basic local alignment search tool. J. Mol Biol., 215, 403-10
Bird,A.(1987) CpG islands as gene markers in the vertebrate nucleus. Trends Genet., 3, 342-47
Chen,L., Ou,H., Zhang,R. and Zhang,C. (2003) ZCURVE_CoV: a new system to recognize protein coding genes in coronavirus, and its applications in analyzing SARS-CoV genomes. Biochemical and Biophysical Research Communications, 307, 382-8.
Delcher,A.L. ,Harmon,D., Kasif,S., White,O. and Salzberg,S.L.(1999) Improved microbial gene identification with GLIMMER. Nucleic Acid Research, 27, 4636-41.
Kehoe,M.A., et al., (1996)Horizontal gene transfer among group A streptococci: implications for pathogenesis and epidemiology. Trends Microbial., 4, 436-43.
Lukashin,A.V. and Borodovsky,M. (1998) GeneMark.hmm: New solution for gene finding. Nucleic Acid Research, 26, 1107-15.
Mathe,C., Sagot,M.F., Schiex,T. and Rouze,P. (2002) Current Methods of gene prediction their strength and the applicantsaknesses. Nucleic Acid Research, 30, 4103-17
Medigue,C., et al. (1999) Detecting and Analyzing DNA Sequencing Errors:Toward a Higher Quality of the Bacillus subtilis Genome Sequence. Genome Research, 9, 1116-27
Pearson,W.R. (1995) Comparison of methods for searching protein sequence databases. Protein Science, 4, 1145-60.
Salzberg,S.L., Delcher,A.L., Kasif,S. and White,O. (1998) Microbial gene identification using interpolated Markov models. Nucleic Acid Research, 26, 544-8.
Shibuya,T. and Rigoutsos,I.(2002) Dictionary-driven prokaryotic gene finding. Nucleic Acid Research, 30, 2710-25.
Brahmachari, S.K.. and Dash, D. (2001) a computer based method for identifying peptides useful as drug targets. PCT international patent publication (WO 01/74130 A2, 11th October 2001).
Cumulative number of reported cases of severe acute respiratory syndrome (SARS) Geneva: World Health Organization, 2003. (Accessed April 9, 2003 at http://www.who.int/csr./sarscountry/ 2003_04_04/en/.)
Drosten,C., Giinther,S. and Preiser,W., (2003) Identification of a Novel Coronavirus in Patients with Severe Acute Respiratory Syndrome. N Engl J Med., (www.nejm.org on April 10,2003.)
Ksiazek,T.G., Dean Erdman,P.H. and Goldsmith,C.S. (2003) A Novel Coronavirus Associated with Severe Acute Respiratory Syndrome. NEnglJMed, 348, 1947-58.
Marra,M.A., Jones,S.J., Astell,C.R., Holt,R.A., Brooks-Wilson,A. (2003) The Genome sequence of the SARS-associated coronavirus. Science, 300, 1399-404.
Tsang,K.W., Ho,P.L. and Ooi,G.C., (2003) A cluster of cases of severe acute respiratory syndrome in Hong Kong. NEnglJMed, 348, 1977-85.

### Organization Applicant

Street : Rafi Marg
City : New Delhi
State : Delhi
Country : INDIA
PostalCode : 110 001
PhoneNumber :
FaxNumber :
EmailAddress : ipmd@vsnl.net
<110> OrganizationName : Council of Scientific and Industrial Research

### Application Project

<120> Title : A computer based versatile method for identifying protein coding DNA sequences useful as drug target
<130> AppFileReference : US 1729
<140> CurrentAppNumber :
   <141> CurrentFilingDate :

### Sequence

<213> OrganismName : Haemophilus influenzae
<400> PreSequenceString : 
ttgttgttga aaggagtgat tatgcaggtc tcaagaagaa aattcttcaa gatctgtgca
60 ggaggtatgg cgggaacgtc agctgcaatg ttgggctttg ctccagcaaa cgtattagct
120 gcgccacgcg aatataaatt attacgcgcg tttgaatccc gtaacacctg tacatattgc
180 gctgtaagtt gcggtatgtt gttatatagc acaggcaaac cttacaattc attaagcagc
240 catactggca caaatactcg ttcaaaactc tttcatattg agggtgatcc agatcatcca
300 gtcagtcgtg gtgcgctttg cccgaaaggt gctggctcac tcgattatgt caatagtgaa
360 agccgttctt tatatcctca atatcgtgcg ccaggttctg ataaatggga acgaatttct
420 tggaaagatg ccattaaacg tattgctcgt ttaatgaaag atgaccgaga tgccaacttt
480 gttgaaaaag attcaaatgg aaaaacggtt aatcgttggg caacgacagg aattatgact
540 gcatcagcaa tgagcaatga agctgcgtta ttaacacaaa agtggattag aatgctcggt
600 atggtgccag tatgtaacca agcgaatact tga
633
 <212> Type : DNA
   <211> Length : 633
   SequenceName : SEQ ID 1:GDC_HINF_5641
   SequenceDescription :

### Custom Codon

Sequence Name : SEQ ID 1:GDC_HINF_5641

### Sequence

<213> OrganismName : Haemophilus influenzae
<400> PreSequenceString : 
atgacaaata actgggttga tattaaaaat gccaacttaa tcatcgttca aggcggtaac
60 cctgcagaag cccatcctgt tggcttccgt tgggcaattg aagcgaagaa aaacggtgcg
120 aaaatcatcg ttattgatcc gcgttttaac cgtacagcat ccgttgctga tcttcatgcg
180 ccaattcgtt ctggttctga tattacgttc ttaatgggcg tgatccgtta cctattggaa
240 acaaaccaaa ttcaacacga atatgttaaa cactatacca acgcatcatt cttaattgat
300 gaaggtttca aatttgaaga tggtttattt gtagggtata acgaagaaaa acgtaactac
360 gataaatcta aatggaacta ccaatttgat gaaaatggtc acgctaaacg tgatatgaca
420 ttacaacatc ctcgttgtgt cattaacatc ttaaaagagc acgtttctcg ttatacccca
480 gaaatggttg aacgtattac aggcgtaaaa caaaaactct tcttacaaat ctgtgaagaa
540 attggtaaaa cctctgtgcc aaataaaacg atgacgcatc tatatgcatt aggttttaca
600 gagcattcaa tcggtacaca aaatattcgc tcaatggcga taatccagtt acttttaggt
660 aatatgggga tgccaggtgg cggtattaac gcattacgtg gacactccaa tgtgcaaggt
 
720 acgacagata tgggcttatt gccaatgtct ttaccaggtt atatgcgttt gccaaacgat
780 aaagatacct cttacgatca atacattaac gcaattacac caaaagatat cgttccaaac
840 caagtgaact attatcgtca tacttcaaaa ttctttgtta gcatgatgaa aactttctac
900 ggagataatg ccactaagga aaatggctgg ggattcgatt tcttaccaaa agcagatcgc
960 ctatatgatc caattactca cgttaaattg atgaatgaag gcaaattaca cggttggatt
1020 ttac
1024
 <212> Type : DNA
   <211> Length : 1024
   SequenceName : SEQ ID 2:GDC_HINF_6322
   SequenceDescription :

### Custom Codon

Sequence Name : SEQ ID 2:GDC_HINF_6322

### Sequence

<213> OrganismName : Haemophilus influenzae
<400> PreSequenceString : 
gtgatgagcc gacatcgagg tgccaaacac cgccgtcgat atgaactctt gggcggtatc
60 agcctgttat ccccggagta ccttttatcc gttgagcgat ggcccttcca ttcagaacca
120 ccggatcact atgacctact ttcgtacctg ctcgacttgt ctgtctcgca gttaagcttg
180 cttataccat tgcactaa
198
 <212> Type : DNA
   <211> Length : 198
   SequenceName : SEQ ID 3:GDC_HINF_124181
   SequenceDescription :

### Custom Codon

Sequence Name : SEQ ID 3:GDC_HINF_124181

### Sequence

<213> OrganismName : Haemophilus influenzae
<400> PreSequenceString : 
gtgtttatgc tttatttaga atttttattt ttactattaa tgctctatat cggtagccgt
60 tacggcggta tcggattagg tgttgtttct ggtatcggtc ttgctatcga ggttttcgta
120 tttcgtatgc cagtggggaa gcaccgattg atgttatgct tatcattctt gcagtggtga
180
 <212> Type : DNA
   <211> Length : 180
   SequenceName : SEQ ID 4:GDC_HINF_170553
   SequenceDescription :

### Custom Codon

Sequence Name : SEQ ID 4:GDC_HINF_170553

### Sequence

<213> OrganismName : Haemophilus influenzae
<400> PreSequenceString : 
atggctgctg caattcaaca acgtgccgaa cttcaacgcc gtatttggca aa[?]tgctaat
60 gatgtgcgag gctcggtcga tggctgggat ttcaaacaat atgtgcttgg cacacttttt
120 taccgtttta ttagcgaaaa ttttgccaat tacattgaag cgggcgatga aagcgtaaat
180 tatgcccaat tacctgatga aatcattaca cagatgccat taaaacgaaa ggctacttta
240 tttacccaag ccaattattt aagaatgttg cggctaatgc tggcagcaat cctaatttga
300
 <212> Type : DNA
   <211> Length : 300
   SequenceName : SEQ ID 5:GDC_HINF_231874 SequenceDescription :

### Custom Codon

Sequence Name : SEQ ID 5:GDC_HINF_231874

### Sequence

<213> OrganismName : Haemophilus influenzae
<400> PreSequenceString : 
ttgaatactg atttaaaaca gatttttact gatattgaaa actcagcgac gggctttccg
60 tctgaacaag atattaaagg gttatttgcc gattttgata ccaccagcaa tcgcttaggc
120 aataccgtaa aagataaaaa cgaccgctta acggctgttt tgaaaggcgt ggctgaactt
180 gattttggca aatttgaaga taaccacatt gatttatttg gcgatgcata cgaatatctt
240 atttctaact atgccgccaa tgcaggcaaa tctggtggcg aattttttac cccacaaagt
300 gtttccaaac tcattgctca aattgcaatg cacgggcaaa cctcggtcaa taaaatttat
360 gaccctgcag caggttctgg ctcacttttg cttcaagcca aaaaacaatt tgatgaacat
420 attattgaag aaggcttttt cgggcaggaa attaaccata ccacatacaa ccttgcccgt
480 atgaatatgt tttgcataa catcaactac gacaagtttg atattgcttt aggcaacacc
540 ttaatggaac cacaatttgg cgataataaa cctttcgatg ccattgtttc gaacccgcct
600 tactccgtga aatgggctgg ctccgacgat ccaacattga ttaatgatga acgatttgcc
660 ccccgcaggc gtgcttgcac caaaatccaa agcggacttt gcctttattt tacatgcgtt
720 aagttatctt tcagcaaaag gccgcgcggc gattgtttcc ttccctggta tttttatcg
780 tggcggtgcc gagcaaaaaa ttcgtcaata tttggtggat aa
822
 <212> Type : DNA
   <211> Length : 822
   SequenceName : SEQ ID 6:GDC_HINF_232170 SequenceDescription :

### Custom Codon

Sequence Name : SEQ ID 6:GDC_HINF_232170

### Sequence

<213> OrganismName : Haemophilus influenzae
<400> PreSequenceString : 
atgatgaacg atttgccccc cgcaggcgtg cttgcaccaa aatccaaagc ggactttgcc
60 tttattttac atgcgttaag ttatctttca gcaaaaggcc gcgcggcgat tgtttccttc
120 cctggtattt tttatcgtgg cggtgccgag caaaaaattc gtcaatattt ggtggataat
180 aactatgtgg acgcggtgat tgcgcttgcg ccaaatctct tttttggcac cagtattgcg
240 gtgaatattt tggtgctttc caaacacaaa cccaatttat cgatgccagc ggtttattta
300 aatctgccac taataaccac attttag
327
 <212> Type : DNA
   <211> Length : 327
   SequenceName : SEQ ID 7:GDC_HINF_232813 SequenceDescription :

### Custom Codon

Sequence Name : SEQ ID 7:GDC_HINF_232813

### Sequence

<213> OrganismName : Haemophilus influenzae
<400> PreSequenceString : 
gtgccgcatt tggcaaaatc catatccttt gaagaaatcg cccaaaatga ctacaacctt
60 gcagtaagtt cgtatgtgga acaaaaagac actcgtgaag tgattaatat tgatgaactc
120 aatgctcaaa ttcgtgaaac tgttaccaat attgaccact tgcgtgcgga aattgacaag
180 attgttgcag aaattgaagg gtaa
204
 <212> Type : DNA
   <211> Length : 204
   SequenceName : SEQ ID 8:GDC_HINF_233190
   SequenceDescription :

### Custom Codon

Sequence Name : SEQ ID 8:GDC_HINF_233190

### Sequence

<213> OrganismName : Haemophilus influenzae
<400> PreSequenceString : 
atgacccaat acaaaactat cgctgaatcc aataatttta tcgttttaga tcaatataat
60 aaatttgtgg aagaatctaa tgctggttat caaacggaaa ggagccttga gcgtgagttt
120 attcgtgatt tacaggctca aggctatgag tatttacaat ggcttaataa tcacgatgaa
180 ctgattaaaa acttacgggc gcaattacaa cgcttaaata acgtggtttt ctccgatgca
240 gaatggcaac gttttttaga ggaatatttg gataaaccga gcgataatct gattgagaaa
300 acccgcaaaa ttcacgatga ttatatttat gattttgtgt tcgataacgg acgcattcag
360 aacatctatt tgcttgataa gaaaaatctt gccaataatt ctctgcaagt catcaatcaa
420 tttaagcaaa ctggcagcta tgataatcgt tatgatgtga caattttggt gaatggttta
480 ccccttttatt ga
492
 <212> Type : DNA
   <211> Length : 492
   SequenceName : SEQ ID 9:GDC_HINF_235441
   SequenceDescription :

### Custom Codon

Sequence Name : SEQ ID 9:GDC_HINF_235441

### Sequence

<213> OrganismName : Haemophilus influenzae
<400> PreSequenceString : 
atggtttacc cctttattga attaaaaaaa cgcggcgtgg cgattcgtga agcctttaac
60 caaattcacc gttacagcaa agaaagtttc aataaagaaa attctctctt taaatatatt
120 cagatttttg tcatttctaa tggcacggat actcgctatt ttgctaatac gactaaacgc
180 aataagaata gctacgactt cacaatgaat tgggcaacgg caaaaaatac tctgattaaa
240 gatttaaagg attttaccgc gactttcttg caaaagaata ctttgctcaa tgtgttggta
300 aattactgcg tgtttgatgt gagtgatacg ttgttaatta tgcgtccgta tcaaattgcc
360 gcaacagaac gtattttatg gaaaattcaa atttcttact tagcaaaaaa ttggagtaat
420 cgtgaaagtg gtggctatat ttggcatacc acaggttcag gcaaaaccct caccagtttt
480 aaagcctctc gccttgcgac tgaacttgat tttattgata aagtcttttt tgtggtcgat
540 cgtaaagact tagactacca aacgatgaaa gaatatcagc gtttttcgcc tgatagcgtg
600 aatgggtcgg aaagtaccgc tgggcttaaa cgcaatattg aaaaagatga taacaaaatt
660 atcgtaacca ccattcaaaa attgaataat ttaatgaaaa gtgaagaaaa cctgtctatt
720 tatcaaaaac aggtggtctt tattttcgat gaagcacatc gctctcaatt tggcgaagca
780 caaaaaaatc taaaacgtaa attcaaaaaa ttctatcaat ttggttttac tggcacgcct
840 attttccctg aaaacgcatt aggtgcggaa acgacagcaa gtgtgttcgg tgcggaattg
900 cattcttatg tgattaccga tgctattcgt gatgacaaag tactgaaatt caaagtcgat
960 tacaacgatg tccgcccaca atttaaagcc ttagaaacag aaaaagatcc tgaaaaattg
1020 accg
1024
 <212> Type : DNA
   <211> Length : 1024
   SequenceName : SEQ ID 10:GDC_HINF_235913 SequenceDescription :

### Custom Codon

Sequence Name : SEQ ID 10:GDC_HINF_235913

### Sequence

<213> OrganismName : Haemophilus influenzae
<400> PreSequenceString : 
atggatataa taaagcctat atgcacaggt tttttttata acgataataa tgttttagga
60 gatttgatga aaaatttcaa atattttgct cagagttatg tggattgggt tattcgtctt
120 gggcgtcttc gtttttctct tttaggcgtg atgattctcg cggttttagc tctttgtact
180 cagattttat ttagtctatt tattgttcat cagatatctt gggtagatat ttttcgttcg
240 gtaacttttg gcttactcac tgcgcctttt gttatttatt ttttcacttt attagtagaa
300 aaacttgaac attctcgtct tgatctttct agctcggtta atcgattgga aaatgaggtc
360 gccgagcgaa ttgctgctca gaaaaaatta tcccaagcat tggaaaagtt agaaaaaaat
420 agccgtgata aaagtacctt acttgccaca ataagccatg aatttcgcac gccattgaat
480 gggattgtcg ggcttagcca gattttactt gatgatgaat tggatgatct ccagcgtaat
540 tatttaaaaa ctatcaacat aagtgcggtc agtttaggct atatttttag cgatattatt
600 gatttggaaa aaattgatgc cagccgaatt gaattaaatc gccagccaac agatttccct
660 gccttattaa acgatattta taattttgct agtttcctcg ccaaagaaaa aaatcttatt
720 ttttctttag agcttgaacc taatttgcct aattggttga atcttgatcg tgttcgcttg
780 agccaaattt tgtggaactt aattagtaat gcggtgaagt ttacggatca gggaaatatt
840 attcttaaaa ttatgagaaa tcaggattgt taccatttta ttgtgaaaga tacaggaatg
900 gggatttcac ctgaagaaca aaaacatatt tttgaaatgt attatcaagt gaaagaaagc
960 cgccagcaaa gtgcgggtag cggtattggg ttggctattt ctaaaaatct tgctcagtta
1020 atgg
1024
 <212> Type : DNA
   <211> Length : 1024
   SequenceName : SEQ ID 11:GDC_HINF_240336
   SequenceDescription :

### Custom Codon

Sequence Name : SEQ ID 11:GDC_HINF_240336

### Sequence

<213> OrganismName : Haemophilus influenzae
<400> PreSequenceString : 
gtgatgagcc gacatcgagg tgccaaacac cgccgtcgat atgaactctt gggcggtatc
60 agcctgttat ccccggagta ccttttatcc gttgagcgat ggcccttcca ttcagaacca
120 ccggatcact atgacctact ttcgtacctg ctcgacttgt ctgtctcgca gttaagcttg
180 cttataccat tgcactaa
198
 <212> Type : DNA
   <211> Length : 198
   SequenceName : SEQ ID 12:GDC_HINF_243018
   SequenceDescription :

### Custom Codon

Sequence Name : SEQ ID 12:GDC_HINF_243018

### Sequence

<213> OrganismName : Haemophilus influenzae
<400> PreSequenceString : 
gtgaatattc atggtttagc aaaacttaat ggtaatgtca ctttaataga tcacagccaa
60 tttacattga gcaacaatgc cacccaaaca ggcaatatca aactttcaaa tcacgcaaat
120 gcaacggtaa ataatgccac gttaaacggc aatgtgcatt taacggattc tgctcaattt
180 tctttaaaaa acagccattt ttggcaccaa attcagggcg acaaagacac aacagtgacg
240 ttggaaaatg cgacttggac aatgcctagc gatactacat tgcagaattt aacgctaaat
300 aatagtactg ttacgttaaa ttcagcttat tcagctagct caaataatgc gccacgtcac
360 cgccgttcat tagagacgga aacaacgcca acatcggcag aacatcgttt caacacattg
420 acagtaaatg gtaaattgag cgggcaaggc acattccaat ttacttcatc tttatttggc
480 tataaaagcg ataaattaaa attatccaat gacgctgagg gcgattaca[?] attatctgtt
540 cgcaacacag gcaaagaacc tgtgaccctt gagcaattaa ctttgattga aagcttagat
600 aataaaccgt tatcagataa gctcaaattt actttagaaa atgaccacgt tgatgcaggt
660 gcattacgtt ataaattagt gaagaataag ggcgaattcc gcttgcataa cccaataaaa
720 gagcaggaat tgctcaatga tttagtaaga gcagagcaag cagaacaaac attagaagcc
780 aaacaagttg aacagactgc tgaaaaacaa aaaagtaagg caaaagcgcg gtcaagaaga
840 gcggtgttgt ctgatacccc gtctgctcaa agcctgttaa acgcattaga agccaaacaa
900 gttgaacaga ctactgaaac acaaacaagt aagccaaaaa caaaaaaagg gcggtcaaaa
960 agagcattga gtgcagcgtt ttctgatacc ccgtttgatc taagccagtt aaaggtattc
1020 gaag
1024
 <212> Type : DNA
   <211> Length : 1024
   SequenceName : SEQ ID 13:GDC_HINF_274892
   SequenceDescription :

### Custom Codon

Sequence Name : SEQ ID 13:GDC_HINF_274892

### Sequence

<213> OrganismName : Haemophilus influenzae
<400> PreSequenceString : - 
atgaaaaaa ctgtatttcg tcttaatttt ttaaccgctt gtgtttcatt agggatagca
60 tcacaagcct gggcaggtca tacttatttt gggattgact accaatatta tcgtgatttt
120 gccgagaata aagggaagtt cacagttggg gctaaaaata ttgaggttta taacaaagaa
180 gggcaattag ttggcacatc aatgacaaaa gccccgatga ttgatttttc cgtggtgtcg
240 cgtaacggcg tggcggcatt agtaggcgat cagtatattg tgagcgtggc acataacggc
300 ggatataacg atgttgattt tggtgcagaa ggacgaaacc ctgatcagca ccgctttact
360 tatcaaattg taaaaagaaa taattatcaa gcttgggaga gaaagcatcc ttatgatgga
420 gattatcata tgcctcgttt acataaattt gtaactgaag ctgaacctgt gggtatgaca
480 acaaatatgg atggaaaagt atatgctgat agagagaact atcctgagcg tgtacgtata
540 ggctcaggac gtcagtattg gcgtacagat aaagatgaag aaacgaatgt acatagttca
600 tattatgtct caggtgcata tcgttatctt actgcaggaa atacccatac tcagagtgga
660 aatggtaatg gtacagtcaa tcttagtggt aatgtagtta gccctaatca ttatggtcca
720 ttaccaacgg gtggttctaa aggcgatagc ggttcgccaa tgtttattta tgatgcgaag
780 aagaaacaat ggcttataaa tgctgtatta caaactgggc atcctttttt cggaagaggt
840 aatgggtttc agttaatacg tgaagaatgg ttttataatg aagttcttgc ggttgatacc
900 cctagtgttt ttcaacgcta tattccccca ataaatggac attattcctt tgtatcaaat
960 aatgatggta caggtaaatt aactttaact agacctagta aagatggctc taaagcaaaa
1020 tcag
1024
 <212> Type : DNA
   <211> Length : 1024
   SequenceName : SEQ ID 14:GDC_HINF_276992
   SequenceDescription :

### Custom Codon

Sequence Name : SEQ ID 14:GDC_HINF_276992

### Sequence

<213> OrganismName : Haemophilus influenzae
<400> PreSecluenceString : 
gtgggggaaa acgcgatgaa tttaagtcgt cgagacttta tgaaagccaa tgcggctatg
60 gcagccgcaa cggcagcggg gctaaccatc ccagtcaaaa atgtggttgc ggctgaatcc
120 gaaattaaat gggacaaagc agtatgtcgt ttctgtggta ccggttgtgc agtattagtt
180 ggtactaaag atggacgtgt tgtggcatct caaggcgatc ctgatgcaga agtaaaccgt
240 ggtttaaact gtattaaagg ttatttcttg ccaaaaatta tgtacggtaa agaccgttta
300 acgcagccgc ttttacgtat gacaaacgga aaatttgata agaacggcga ttttgcgcca
360 gtttcttggg attttgccgt tcaaaacaat ggctga
396
 <212> Type : DNA
   <211> Length : 396
   SequenceName : SEQ ID 15:GDC_HINF_370413
   SequenceDescription :

### Custom Codon

Sequence Name : SEQ ID 15:GDC_HINF_370413

### Sequence

<213> OrganismName : Haemophilus influenzae
<400> PreSequenceString : 
ttgataagaa cggcgatttt gcgccagttt cttgggattt tgccgttcaa aacaatggct
60 gaaaaattca aagaagcgtt caaaaagaac ggtcaaaatg cagtaggtat gtttagttct
120 ggtcagtcta ccatttggga aggctatgca aagaacaaac tttggaaagc aggttttcgt
180 tctaacaacg tagacccgaa tgcgcgtcac tgtatggcat ctgcagcggt tgcgtttatg
240 cgcaccttcg gtatggatga acctatgggt tgttataacg acattgaaca ggcagatgct
300 tttgttcttt ggggctcaaa tatggcggaa atgcacccaa ttttgtggtc gcgtattact
360 gatcgccgta tttctaatcc tgatgttcgt gtcactgtac tttctactta cgaacatcgt
420 agttttgaac ttgccgatca cggtttgata tttacaccgc aaactgattt ggcaattatg
480 aactacatca tcaattatct tattcaaaat aatgcgatta attgggattt tgttaataaa
540 cataccaaat ttaaacgcgg agaaacgaat attggctatg gtttgcgtcc agagcatcca
600 ttagaaaaag acacgaatcg taaaacagct gggaaaatgc acgattcttc ttttgaagaa
660 ttaaagcaac ttgtatcaga atatacagtg gaaaaagtat cgaaaatgtc tgggttagat
720 aaagtccagt tagaaacttt agcgaaactt tatgctgatc caacgaagaa agtggtttcc
780 tactggacaa tgggctttaa ccaacataca cgtggtgtgt gggtaaacca attaatctac
840 aatattcatt tacttactgg aaaaatttca atcccaggtt gtgggccatt ttcattaact
900 ggtcagcctt ctgcttgtgg tacggcgcgt gaagtaggtt cattccctca tcgtttacct
960 gccgacttag tggtaactaa tccgaaacac cgtgaaattg ctgaacgtat ttggaaatta
1020 ccaa
1024
 <212> Type : DNA
   <211> Length : 1024
   SequenceName : SEQ ID 16:GDC_HINF_370747 SequenceDescription :

### Custom Codon

Sequence Name : SEQ ID 16:GDC_HINF_370747

### Sequence

<213> OrganismName : Haemophilus influenzae
<400> PreSequenceString : 
gtgatgagcc gacatcgagg tgccaaacac cgccgtcgat atgaactctt gggcggtatc
60 agcctgttat ccccggagta ccttttatcc gttgagcgat ggcccttcca ttcagaacca
120 ccggatcact atgacctact ttcgtacctg ctcgacttgt ctgtctcgca gttaagcttg
180 cttataccat tgcactaa
 198
   <212> Type : DNA
   <211> Length : 198
   SequenceName : SEQ ID 17:GDC_HINF_628407
   SequenceDescription :

### Custom Codon

Sequence Name : SEQ ID 17:GDC_HINF_628407

### Sequence

<213> OrganismName : Haemophilus influenzae
<400> PreSequenceString : 
ttggttatgt tcaatgattt tttggcaaca ttcagccagc aattaacacc tcaaatgtgg
60 ggcgttgtcg caaccgcaac ttatgaaact gtttatatca gttttgcatc taccctactt
120 gctgtactag tcggcgtgcc tgttggcata tggacttttt taactggaaa aaatgagatt
180 ttacaaaata accgcactca ttttgtgtta aacacgatta ttaatattgg gcgttccatt
240 ccatttatta ttttgctcct aatcttatta cctgtaactc gtttcatcgt gggaactgta
300 ttaggtacaa cagcagcaat tattccattg agtatttgtg caatgccatt cgtggctcgc
360 ttaactgcta atgcactaat ggaaattcca aatggtttaa ccgaagcagc tcaagcaatg
420 ggggctacta aatggcaaat tgttcgtaaa ttctatttgt cagaagctct acctacgcta
480 attaatggcg ttactcttac gctagtcact ttagttggtt attctgcaat ggcaggaaca
540 caagggggcg gtggtttagg tagcctcgct atcaactacg ggcgtatatc gcaatatgcc
600 ttatgtaact tgggtggcaa ccattattat tgtgctattc gttatgatta g
651
 <212> Type : DNA
   <211> Length : 651 SequenceName : SEQ ID 18:GDC_HINF_654365 SequenceDescription :

### Custom Codon

Sequence Name : SEQ ID 18:GDC_HINF_654365

### Sequence

<213> OrganismName : Haemophilus influenzae
<400> PreSequenceString : 
gtgatgagcc gacatcgagg tgccaaacac cgccgtcgat atgaactctt gggcggtatc
60 agcctgttat ccccggagta ccttttatcc gttgagcgat ggcccttcca ttcagaacca
120 ccggatcact atgacctact ttcgtacctg ctcgacttgt ctgtctcgca gttaagcttg
180 cttataccat tgcactaa
198
 <212> Type : DNA
   <211> Length : 198
   SequenceName : SEQ ID 19:GDC_HINF_661444 SequenceDescription :

### Custom Codon

Sequence Name : SEQ ID 19:GDC_HINF_661444

### Sequence

<213> OrganismName : Haemophilus influenzae
<400> PreSequenceString : 
ttgcgtaaag atgcactacc cgcatttttc acagacgtaa atcaaatgta tgatgcctta
60 ttgaataaat caggggcaac aggtgtattt actgatttcc cagatacttg cgtggaattc
120 ttaaaaggaa taaaataa
138
 <212> Type : DNA
   <211> Length : 138
   SequenceName : SEQ ID 20:GDC_HINF_737160 SequenceDescription :

### Custom Codon

Sequence Name : SEQ ID 20:GDC_HINF_737160

### Sequence

<213> OrganismName : Haemophilus influenzae
<400> PreSequenceString : 
gtgatgagcc gacatcgagg tgccaaacac cgccgtcgat atgaactctt gggcggtatc
60 agcctgttat ccccggagta ccttttatcc gttgagcgat ggcccttcca ttcagaacca
120 ccggatcact atgacctact ttcgtacctg ctcgacttgt ctgtctcgca gttaagcttg
180 cttataccat tgcactaa
198
 <212> Type : DNA
   <211> Length : 198
   SequenceName : SEQ ID 21:GDC_HINF_775792
   SequenceDescription :

### Custom Codon

Sequence Name : SEQ ID 21:GDC_HINF_775792

### Sequence

<213> OrganismName : Haemophilus influenzae
<400> PreSequenceString : 
ttgcctaaac ctgaaccaat accacgaccg aggcgtttag cactatgctt tgcaccttca
60 gccggagata gagtatttaa acgcatctct tactcctcca ctttaaccat gtatgaaact
120 tggttaatca taccacgtac tgcaggcgta tcaattaact caacagtgtg gtgtatatgg
180 cgaagaccaa gaccacgcaa ggtagcttta tgcttcggta aacgagcaat tgagctacga
240 acttgtgtta ctttaatagt tttagccatt attcattacc ccaagatttc atcaacagtt
300 ttaccgcgtt ttgcagcaac catttctggt gatttcatat ttgctaatgc atcaatagtt
360 gcacgaacaa cgttaattgg gttggtagaa ccatacgctt tagaaagaac gttacgtaca
420 cctgcaactt ccaataccgc acgcattgca ccaccagcga tgatacctgt accttcactt
480 gctggctgca taaatacacg tgaaccagta tga
513
 <212> Type : DNA
   <211> Length : 513
   SequenceName : SEQ ID 22:GDC_HINF_848166
   SequenceDescription :

### Custom Codon

Sequence Name : SEQ ID 22:GDC_HINF_848166

### Sequence

<213> OrganismName : Haemophilus influenzae
<400> PreSequenceString : 
ttgcttatat atgggggaat aaatatgcaa attacacttt caaatacctt agcgaatgat
60 gcttggggaa aaaatgcgat tttgagcttt gactctaata aagctatgat tcatttaaaa
120 aataatggaa aaactgaccg cactttagtt caacaagctg ctcgtaaatt gcgtgggcaa
180 ggaatcaaag aggtggagtt ggtcggcgag aaatgggatt tggaattttg ctgggcgttt
240 tatcaaggtt tttataccgc aaaacaagat tacgcgattg agtttccaca tttagatgat
300 gaaccgcaag atgaattgtt agcacgtatt gaatgtggcg attttgtgcg tggaattatt
360 aatgaaccag cacaaagttt aacgcctgtg aaattagtag agcgagcggc tgaatttatc
 
420 ttaaaccaag cggacattta taatgaaaaa agtgcggtaa gttttaagat tatttctggc
480 gaggaacttg agcaacaagg ttatcacgga atttggactg tgggtaaagg ctctgcgaac
540 ttgccagcca tgttgcaact tgatttcaat ccaacacagg attcgaatgc gcccgtgtta
600 gcttgtttag ttggtaaggg gattactttt gatagtggcg gctatagtat caaaccaagt
660 gatggtatga gtacaatgcg aactgatatg ggcggggctg cattattaac ggggcttta
720 ggtttcgcta tcgctcgtgg attaaatcaa cgcgttaagc tgtatttatg ttgcgcagaa
780 aatttggtaa gcaataatgc ctttaagcta ggcgatatta ttacttataa aaatggcgtg
840 agcgcagaag tactgaatac tgatgcggaa ggtcgtttgg tgttagctga tggattgatt
900 gaggctgata accaaaatcc aggttttatt attgattgcg cgactttaac tggcgcagca
960 aaaagtggct gtaggaaacg actatcattc tgtattatct atggatga
1008
 <212> Type : DNA
   <211> Length : 1008
   SequenceName : SEQ ID 23:GDC_HINF_928073
   SequenceDescription :

### Custom Codon

Sequence Name : SEQ ID 23:GDC_HINF_928073

### Sequence

<213> OrganismName : Haemophilus influenzae
<400> PreSequenceString : 
gtggctgtag gaaacgacta tcattctgta ttatctatgg atgatgaact tgtgaaaaat
60 cttttccaat ccgcacaagc agaaaatgaa cctttctggc gtttaccatt tgaagatttt
120 catcgttcac aaattaattc atcttttgcc gatattgcta atattggttc ggttccagtt
180 ggagctgggg caagcactgc aacggcattt ttatcgtatt ttgtaaaaaa ttataaacaa
240 aattggttgc atattgattg ctccgcgact tatcgtaaat ctggtagtga tttatggtct
300 gttggggcaa caggaattgg tgtgcaaact ttagctaatt taatgttatc aagatcattg
360 aagtaa
366
 <212> Type : DNA
   <211> Length : 366
   SequenceName : SEQ ID 24:GDC_HINF_929037
   SequenceDescription :

### Custom Codon

Sequence Name : SEQ ID 24:GDC_HINF_929037

### Sequence

<213> OrganismName : Haemophilus influenzae
<400> PreSequenceString : 
ttgccaattg aattaaaagt agaaggttta gtgggtaaac caaacgagaa aatttctgcg
60 gcagaatttc gtcaaaaatg tcgtgaatac gcggcggaac aggtcgaggg tcaaaagaaa
120 gactttatcc gtttaggtgt gttgggcgat tgggataatc catatctcac gatgaatttc
180 gataccgaag cgaatattat ccgcacttta ggtaaagtga ttgaaaatgg tcatttgtat
240 aaaggctcaa aaccagttca ctggtgtttg gattgcggtt cttctttagc agaagcagaa
300 gtggaatatg aagacaaagt ttctccgtca atttacgttc gtttccctgc ggaaagtgcg
360 gatgaaattg aagctaaatt ttctgcacaa ggtagaggac aaggtaaatt atcagccatc
420 atttggacta ccacaccttg gacgatgcca tctaaccgtg cgattgcggt gaatgcagac
480 ttagaataca acttagtcca acttggcgat gagcgtgtaa ttttagctgc tgaattagtt
540 gagtcagtgg caaaagcggt gggtattgag cacattgaaa ttctgggttc tgtaaaaggt
600 gatgatcttg aattaagccg tttccatcat ccgttctatg attttactgt gccagtgatt
660 ttaggcgatc acgtaaccac tgatggcggt acaggtttag tacataccgc acctgatcac
720 ggtttagacg actttatcgt gggtaaacaa tatgatttac caatggcggg tcttgtatcg
780 aatgatggta aatttatttc aacgaccgaa ttctttgcag gcaaaggcgt atttgaagca
840 aatccgcttg tgatagaaaa attacaagaa gtaggtaact tattaaaagt tgaaaaaatc
900 aaacacagct atccacactg ctggcgtcac aaaacgccaa ttattttccg tgcaacaccg
 
960 caatggttta tcggcatgga aacgcaaggt ttacgccaac aagcattagg cgaaattaaa
1020 caag
1024
 <212> Type : DNA
   <211> Length : 1024
   SequenceName : SEQ ID 25:GDC_HINF_1018846
   SequenceDescription :

### Custom Codon

Sequence Name : SEQ ID 25:GDC_HINF_1018846

### Sequence

<213> OrganismName : Haemophilus influenzae
<400> PreSequenceString : 
ttggaaaata aaatgacagt cgattacaaa aacactctta acctaccgga aaccagcttt
60 ccaatgcgcg gtgatttagc taagcgcgaa cctgataagt ag
102
 <212> Type : DNA
   <211> Length : 102
   SequenceName : SEQ ID 26:GDC_HINF_1021582 SequenceDescription :

### Custom Codon

Sequence Name : SEQ ID 26:GDC_HINF_1021582

### Sequence

<213> OrganismName : Haemophilus influenzae
<400> PreSequenceString : 
atgaagataa ctcattgtaa attaaagaaa tctatacaaa ataagctact tgaatttttt
60 gtattagaag ttacagcccg agcagcggct gatttactcg atatctaa
108
 <212> Type : DNA
   <211> Length : 108
   SequenceName : SEQ ID 27:GDC_HINF_1082407
   SequenceDescription :

### Custom Codon

Sequence Name : SEQ ID 27:GDC_HINF_1082407

### Sequence

<213> OrganismName : Haemophilus influenzae
<400> PreSequenceString : 
ttgtttctgg ttggaaacct tttgaggtgg gtttggcttg cgctttttat cattgcgcaa
60 atttgggctt atgtacaaac acctgattct tggttagcaa tgatttctgg tatttctggt
120 attttgtgtg tggtattggt aagtaaaggt aaaattagta attatttctt tggattgatt
180 tttgcctata cttattttta tgttgcttgg ggatcgaatt tcttaggcga aatgaacacc
240 gtactttacg tatatttgcc ctctcaattt attggttact ttatgtggaa agccaatatg
300 caaaatagcg atggtggaga aagcgtgatt gcaaaagcgt taactgttaa aggatggatg
360 acattaattg ttgtgactac ggttggtact ttgctttttg ttcaagcatt acaagcggct
420 ggtggtagct caacaggttt agatggtcta actacaatta ttacggttg[?] ggcacagatt
480 ttaatgattt tgccgttatc gtga
504
 <212> Type : DNA
   <211> Length : 504
   SequenceName : SEQ ID 28:GDC_HINF_1144501

### SequenceDescription :

### Custom Codon

Sequence Name : SEQ ID 28 : GDC_HINF_1144501

### Sequence

<213> OrganismName : Haemophilus influenzae
<400> PreSequenceString : 
atgtttagtg gcgaacatga tgcttgcgat tgctatgtgg acctacaagc aggttctggc
60 ggcaccgaag ctcaagattg gacagaaatg ttgctccgta tgtatctccg ttgggctgaa
120 agcaaaggtt ttaaaacaga actgatggaa gtctctgacg gcgatgtagc tggattgaaa
180 tcagcaacca ttaaagtgag cggtgaatat gcttttggtt ggttacgaac agaaacgggg
240 attcatcgtt tagtgcgtaa aagtccattt gattccaata accgtcgtca cacatcattc
300 agcgcagcat ttgtctaccc tgaaattgat gatgatattg atattgaaat caatcctgct
360 gatttacgta ttgatgttta tcgtgcatca ggggcaggtg gtcagcacgt aaacaaaact
420 gaaagtgcgg tgcgaattac ccatatgcca agtggcattg tggtgcaatg tcaaaacgac
480 cgttcacagc acaagaacaa agatcaagca atgaaacaat taaaagcgaa attgtatgag
540 cttgaattac aaaagaaaaa tgcggataaa caagcaatgg aagataataa atctgacatt
600 ggttggggaa gccaaattcg ctcttatgta ttagacgatt cacgcattaa agatttacgt
660 actggcgtag aaaaccgtaa tacgcaagcc gtattagacg gggatttaga tcgatttatt
720 gaagcgagtt taaaagcggg cttgtag
747
 <212> Type : DNA
   <211> Length : 747
   SequenceName : SEQ ID 29:GDC_HINF_1279189
   SequenceDescription :

### Custom Codon

Sequence Name : SEQ ID 29:GDC_HINF_1279189 Sequence
<213> OrganismName : Haemophilus influenzae
<400> PreSequenceString : 
ttgcttggta acgaaaaaca agctgaagca caagctaaat atgcggaaga cacgctgaaa
60 caagcacgcg attttgctaa acaacatcat aaaacagcct atttagcgcg taatgcggat
120 ggcttacaaa ctggtcaaaa aggttcgatt catacggaag caatggaatt ggttggcttg
180 gaaaacgtcg cagagggaga acaaaaaggc ttaactcaag tttcaatgga acagctttta
240 ttgtga
246
<212> Type : DNA
<211> Length : 246
SequenceName : SEQ ID 30:GDC_HINF_1347200
SequenceDescription :

### Custom Codon

Sequence Name : SEQ ID 30:GDC_HINF_1347200

### Sequence

<213> OrganismName : Haemophilus influenzae
<400> PreSequenceString : 
ttgccacgta tttttgccgc ttgttttgtc ggggcggcgc ttgcttgtgg gggcgcaact
60 tatcaaggta tgtttaaaaa tccgcttgtt tcgccagata ttttgggtgt ttcagcgggg
120 gcaggttttg gggcaagttt ggcaattttt tataatttgc caatgattta tatccaattt
180 tttgctttta gcggtggcat tttagctgtg ttatgtgtat cgctcattgc ctcgcgtagt
240 cgtacacaag atcctatttt agtgctggtg ctttctggga ttgcaattgg ttctttactt
 
300 ggtgcaggca tttctttgtt aaaaattctt gcggatcctt tcactcaatt accttcaatc
360 actttttggc tacttggtag cctgacggct attaatcaac aagatttaat tcaattgatc
420 ccgatgttgt tgctagggat tgttcccatt tttttattac ttactgatac gctggctcgc
480 acgattgcac cgattgaact gccactcggt attctgactt ctgcttgtgg ttattag
537
 <212> Type : DNA
   <211> Length : 537
   SequenceName : SEQ ID 31:GDC_HINF_1347942 SequenceDescription :

### Custom Codon

Sequence Name SEQ ID 31:GDC_HINF_1347942

### Sequence

<213> OrganismName : Haemophilus influenzae
<400> PreSequenceString : 
ttgaagaact cattacggga gttaaaacnn gattatactg tggttatagt aactcataat
60 atgcaacaag ctacacgttg ctccgactat acggcattta tgtatttggg tgaattagtt
120 gaatttggtc aaacacaaca aatttttgat agacccaaga tacaacgtac agaagattat
180 attcgcggta aaatggggta g
201
 <212> Type : DNA
   <211> Length : 201
   SequenceName : SEQ ID 32:GDC_HINF_1476415
   SequenceDescription :

### Custom Codon

Sequence Name : SEQ ID 32:GDC_HINF_1476415

### Sequence

<213> OrganismName : Haemophilus influenzae 
atgattagtc tacaagaaac caaaatagct gtgcaaaatc taaatttcta ctatgaggat
60 tttcatgcat taaaaaacat taatttacgt atcgctaaga ataaagtgac cgcctttatt
120 ggtccttcag gttgcggtaa atctacttta ttgcggagtt ttaatcggat gtttgaacta
180 tatccaaatc aaaaagctac tggtgaaatt aatttagacg gtgaaaattt actcacaaca
240 aagatggata tttctctgat tcgtgctaag gttggtatgg ttttccaaaa accaacgcca
300 tttccaatgt cgatttatga taatattgca ttcggtgttc gtttgtttga aaaattatca
360 aaagaaaaga tgaatgaacg agtagaatgg gcattgacta aggccgctct ttggaatgaa
420 gtgaaagata aattacataa aagcggagat agtttatctg gcggacaaca gcaacgcttg
480 tgcattgctc gagggattgc tattaaacct agtgtgttgt tgttagatga accttgttcg
540 gcattagatc ctatttcgac tatgaaaatt gaagaactca ttacgggagt taaaacnnga
600 ttatactgtg gttatagtaa ctcataa
627
<212> Type : DNA
<400> PreSequenceString :
<211> Length : 627
   SequenceName : SEQ ID 33:GDC_HINF_1476557
   SequenceDescription :

### Custom Codon

Sequence Name : SEQ ID 33:GDC_HINF_1476557

### Sequence

<213> OrganismName : Haemophilus influenzae
<400> PreSequenceString : 
atgagccagc ttaatattca atttccgaca aaattcaaac cgctctttga atctatttgg
60 cggtttatta ttttctacgg tgggcgaggt tcaggtaaaa gttttagtat cgctagagca
120 ttagtattgc gagcctatca atcgcctgtt cgagttttgt gttccgtgaa attcagaaat
180 cgatttctga ttctgtga
198
 <212> Type : DNA
   <211> Length : 198
   SequenceName : SEQ ID 34:GDC_HINF_1505851
   SequenceDescription :

### Custom Codon

Sequence Name : SEQ ID 34:GDC_HINF_1505851

### Sequence

<213> OrganismName : Haemophilus influenzae
<400> PreSequenceString : 
gtggttcccg agttcattat tgtttcttta atcttggtgg cacagtccat gaaattggcg
60 ttaaacaaat ggcttatcat atttggcaac gctatagctc ttcacataaa gtacgcttta
120 ttgcgattaa actttgaggg agttgttggt gagattttag agaaagtcga taacggccaa
180 atgggcgttg tattaaaacg gatgatggtg cgagccgcaa gtaaagtcgc tcaacgtttc
240 aatattgaag caattgtgac aggggaggca ttagggcaag tttctagcca aactttaacc
300 aatttacgct tgattgatga agccgctgat gccttagtat tgcgtccgtt aattacccat
360 gataaagaac aaattatcgc gatggcgaaa gaaattggca ctgatgatat tgcaaaatct
420 atgccagaat tttgtggcgt gatttcaaaa aatcctacga ttaaagcggt tcgtgaaaag
480 attcttaaag aagaaggcaa ttttaatttt gagattcttg aaagtgcggt acaaaatgca
540 aaatatttag atattcgcca gattgcagaa gaaacagnaa aagcagtcgt ggaagtcgag
600 gcaatttctg tgttaggtga aaatgaagtg attttggata ttcgtagccc agaagaaacg
660 gatgaaaagc catttgaatc aggtacacat gacgtcattc aaatgccgt[?] ctacaaactt
720 tcttctcaat ttggtagcct tgatcaaagt aaaagttacg tgttgtattg tgaacgtggt
780 gtgatgagta aattacaagc cttatatttg aaagaaaatg gtttttcaaa tgtgcgtgta
840 tttgcaaaaa acattcatta a
861
 <212> Type : DNA
   <211> Length : 861
   SequenceName : SEQ ID 35:GDC_HINF_1524561
   SequenceDescription :

### Custom Codon

Sequence Name : SEQ ID 35:GDC_HINF_1524561

### Sequence

<213> OrganismName : Haemophilus influenzae
<400> PreSequenceString : 
ttggccatcg ctattggtgg aggtaataga ggtaatgcaa gcggagtatt gcgccaaaat
60 tttgcagaag ataaagcaaa aaagaccgct tcgaagctcg tgggcgtaat ggctcactat
120 tttggcggta agtcgtttta tctgcccgca ggtgataaaa tcaaagaag[?] cttacgagat
180 gcacaaattt atcaagaatt caacggtaag aatgtacctg acctaataaa aaaataccga
240 ttgtcagaaa gcacaattta tgcgatctta cgcaatcaac gaacgcttca aagaaagcga
300 catcagatgg attttaattt tagttag
327
 <212> Type : DNA
   <211> Length : 327
   SequenceName : SEQ ID 36:GDC_HINF_1568974
   SequenceDescription :

### Custom Codon

Sequence Name : SEQ ID 36:GDC_HINF_1568974

### Sequence

<213> OrganismName : Haemophilus influenzae
<400> PreSequenceString : 
ttgtttaggt ggcactacct tggaggtttt acagtaatgc cagatacaaa taacacagaa
60 accaataata agatcgaact ctatctaaat ggcaaaattt tatccggttg gaaaagcctt
120 aacctgcaac gctcgctgga atcaatgagt ggtcgttttg atttaggcat tgctgtgcga
180 cctgaagatg atatatcagt gcttgccgca ggttcgccac tggtgctgaa aatgggcggg
240 caaaccgtga ttaccggtta cttggatgaa atcaaacaac gcgtaagcgg taacgacaaa
300 actatctctg tgagtggacg agataaaact tgcgacttgg tggattgtgc cattatccac
360 aacagctacc aattcaaaaa ccaaactgcc aaacaaattg ccgaagccat ctgtaaacct
420 tttggcatta gcgtagtatg gcaagtgcaa gcccctgaag ccaatgaacg aatccctgtc
480 tggcaagtag aaccaggcga aaccgccttt gataatttaa gcaaaatcgc ccgacacaaa
540 ggcgtgttag tcaccagcga cgtggacggc aatttgcttt tcaccgagcc gagcaacaag
600 caagtcggta atcttaccct tggcgaaaac ttgctcgaac tggaacaaac cgacagctgg
660 ttgcaacgct tttcgctcta tcgcgtgatt ggtgacgcag aacaaggcgg cgccaaaggt
720 gataccaaaa ccaaaaacaa agcggcaaaa ggcaaggaaa aagatgatgg cgtggtagaa
780 gatcccgata tttacccagg accagcagaa ggaggcaagt aa
822
 <212> Type : DNA
   <211> Length : 822
   SequenceName : SEQ ID 37:GDC_HINF_1586944
   SequenceDescription :

### Custom Codon

Sequence Name : SEQ ID 37:GDC_HINF_1586944

### Sequence

<213> OrganismName : Haemophilus influenzae
<400> PreSequenceString : 
atgaaggttt cttaccggct aaataattgt ctaagtttaa agttagcgct gatcccatta
60 ttaatactat tatttgttgt tatgggatcg gtgctttctt taatcgcaaa attagatttt
120 tatttttttc aacaaatatt atttaattcc gaattgcatt ttgcattgct aatgtcattg
180 ggaacgtctc ttttttcttt gatattagca ttatgtattg ctattccatc tgcatggcga
240 atgagtcaag tgcggttgcc ttttcaatca ttttttgaca ctttgtttga tttaccaatg
300 gttttgccac cattagtcac aggactaagt ttgcttctac tttttagttc acaagggata
360 ttggctgaac tacttccttt tataagtaaa tggatttttt cccctgtagg gatcattatt
420 gctcagactt atattgcgag ttcgatttta ttgcgttgta gcgagccatt aaaactgcga
480 aaaaaaacca ttaaaactac gaaaataaaa ccttga
516
 <212> Type : DNA
   <211> Length : 516
   SequenceName : SEQ ID 38:GDC_HINF_1594339 SequenceDescription :

### Custom Codon

Sequence Name : SEQ ID 38:GDC_HINF_1594339

### Sequence

<213> OrganismName : Haemophilus influenzae
<400> PreSequenceString : 
ttgacaaaac gtaaaaatgt ttcctttact tatgaaaatt atactgttac gccattttgg
60 gatacgctca agttaagcta ttcacaacaa agaattacaa caagagcaag aacagaagat
120 tactgtgatg gtaatgaaaa atgtgactct tataagaatc ctttagggct tcaattaaaa
180 gagggaaaag tcgttgatcg gaatggtgat cctgttgagt tgaagcttgt tgaggatgaa
 
240 caaggtcaga aacgacatca agttgttgat aaatataata atccttttag tgtagcctct
300 ggaactaata atgatgcttt cgtaggtaaa caattatctc cttctgagtt ttggttagat
360 tgctctattt ttaattgtga taagcctgtc agggtttata aatatcagta tagcaaccaa
420 gaaccagagt cgaaggaagt tgagttaaat agaaccatgg aaattaatgg aaagaaattt
480 gctacttatg agtctaataa ttatagagat agataccata tgattttacc aaattctaaa
540 ggttacttgc ctttggatta taaagagcgt gatttaaata caaagacgaa acaaattaat
600 ttagatttaa caaaagcctt tactctcttt gagattgaaa atgaactttc ctatggtggt
660 gtttacgcga aaacgaccaa ggaaatggtg aataaagcag gatattatgg gcgtaatcct
720 acttggtggg cggagagaac gttagggaaa tcattgctta atggattgag aacgtgtaag
780 gaagattctt catataatgg gctactatgt cctcgtcatg aacctaaaac gtctttctta
840 attcctgtag aaacaacaac taagtcttta tattttgcag acaatatcaa gttgcacaat
900 atgttgagcg tagatttagg ttatcgttat gatgatatta aatatcagcc agagtatatt
960 cctggtgtaa cacctaagat tgcagatgat atggtcagag aattatttgt tccactccct
1020 ccag
1024
 <212> Type : DNA
   <211> Length : 1024
   SequenceName : SEQ ID 39:GDC_HINF_1634710
   SequenceDescription :

### Custom Codon

Sequence Name : SEQ ID 39:GDC_HINF_1634710

### Sequence

<213> OrganismName : Haemophilus influenzae
<400> PreSequenceString : 
ttgcgtgaac gtagttcgct ttctgctcta atggccaaaa cgattgaatg ggattttata
60 acagaaaacc ccctaaaata tcttgagaaa ccaaaagcgc cagcaccaag aactcgtcga
120 tataatgaac atgaaattga gcgtctgatt tttgtgtcag gttatgatgt cgaacatatt
180 gaaccgccaa aaaccttaca aaattgcacg ggggcggcat ttctttttg[?] tatagagaca
240 gcaatgagag caggggaaat agcaagttta acttggaata atattaattt tgaaaagcgc
300 accacctttt tgccaattac taaaaatgga cattcacgca cggtgcctct ttcggtaaaa
360 gcaatagaga ttttacaaca tcttacttcg gtaaaaacag aaagtgatcc gcgagtattc
420 caaatggaag cacgccaact ggatcacaac ttccgcaagc tcaaaaagat ggaagggctt
480 gaaaatgcca atttacattt tcacgacacc cgccgtgaac gattggcaga aaaagtggat
540 gtaatggtat tagccaaaat atcgggccat agagatctca gtattctgca aaatacttat
600 tacgcacctg atatggcaga aggctataaa acaaaggcgg gttatgatct gaccccaacc
660 aaaggcttga gccaacggaa tttttcttc tttaatgaaa acttcatcgt tttcacaaca
720 aatccaccga tagtcattaa gctgtaa
747
<212> Type : DNA
<211> Length : 747
   SequenceName : SEQ ID 40:GDC_HINF_1638626
   SequenceDescription :

### Custom Codon

Sequence Name : SEQ ID 40:GDC_HINF_1638626

### Sequence

<213> OrganismName : Haemophilus influenzae
<400> PreSequenceString : 
atggcgacaa ttatcaagaa tggcaagcgt tggcacgcac aagtgcgcaa gtttggcgtg
60 agcaaatcag ccattttttt gactcaagca gacgcaaaaa aatgggcaga aatgctcgaa
120 aaacagcttg aatcaggaaa gtataatgaa atccctgata ttacattgga tgaactcatt
180 gataagtatc taaaagaagt cactgtaacc aagcgcggga aacgtgaaga gcgcataaga
240 ctactgcgtc tttctcgaac tccgcttgcc gcaatatctt tacaagaaat aggaaaagca
300 cactttcgtg agtggtaa
 318
   <212> Type : DNA
   <211> Length : 318
   SequenceName : SEQ ID 41:GDC_HINF_1639409
   SequenceDescription :

### Custom Codon

Sequence Name : SEQ ID 41:GDC_HINF_1639409

### Sequence

<213> OrganismName : Haemophilus influenzae
<400> PreSequenceString : 
atggaagccg ttcaattaga caaaaatcaa gagcctaatt ataaaggtta tagcggtagc
60 ttgattcatc ctgcatttca acagcaaaca acaaaacgtg aaaaaccgag tacaccatta
120 cctagtttgg atttgctttt aaaatatccg ccaaatgaac aacgcattac accagatgaa
180 ataatggaaa cctcacagcg tattgaacaa caattacgca attttaatgt aaaagccagc
240 gtaaaagatg tgcttgttgg ccctgttgtt acgcgttatg aat[?]agaatt acagccgggt
300 gtgaaagcat caaaagtcac gagcatcgat accgatttag caagagcatt gatgtttcgt
360 tctattcgtg tggcagaggt gattccaggt aaaccttata ttggtattga aaccccaaat
420 cttcatcgtc aaatggtgcc attacgtgat gtattagata gcaatgaatt ccgtgatagc
480 aaggcaactt tacctattgc tttaggtaaa gatattagtg gcaaaccagt cattgttgat
540 ttagcgaaaa tgccacattt attggtagca ggttctacgg gatcaggta[?] gtctgttggt
600 gtgaatacga tgattctaag tttactttat cgtgttcaac cagaagatgt gaaatttatt
660 atgattgatc ctaaagtcgt cgaactttct gtttataatg atattccaca tttactgaca
720 ccagttgtaa cggatatgaa aaaagccgct aatgcgttgc gttggtgcgt agatgaaatg
780 gaacgtcgtt atcagttgct ttcagcttta cgcgtacgaa acattgaagg ctttaatgaa
840 aaaattgatg aatacgaagc aatgggaatg cctgtgccaa atccaatttg gcgactgggc
900 gatacgatgg atgcaatgcc accagcgttg aaaaaattga gttatattgt ggttattgtc
960 gatgagtttg ctgatttaat gatggtagcg ggtaagcaaa tcgaagaact gattgcacgg
1020 ttgg
1024
 <212> Type : DNA
   <211> Length : 1024
   SequenceName : SEQ ID 42:GDC_HINF_1660491
   SequenceDescription :

### Custom Codon

Sequence Name : SEQ ID 42:GDC_HINF_1660491

### Sequence

<213> OrganismName : Haemophilus influenzae
<400> PreSequenceString : 
atgaataaaa tttttaaagt tatttggaat gttgtgactc aaacttgggt tgtggtgtct
60 gaactcactc gcgcccacac caaacgcacc tccgcaaccg tggcaaccgc cgtattggcg
120 accgtattgt ctgcaacggt tcaggcgatt aacgacgcag gaactttcgt gaaagtgcaa
180 agtacggaag atgatattga agatagtgct gcaaccaaag atgacaataa aaaccaagct
240 ctcaaagcag gcgacacctt aaccttaaaa gcgggtaaaa acttaaaagc taagttagac
300 caaggtggta aatcagtaac ctttgcttta gcgaaagacc ttgatgtgaa aaccgcgaaa
360 gtgagtgata ctttaacgat tggcgggaat acgcctgctg cgggtggtgc tacgccaaaa
420 gtaagtatta ctagcacggc tgatggcttg aagttagcaa aaggcactaa tggagatact
480 gcagttcatt tgaatggctt ggcttcaact ttgcctgatg tgactacaaa tacaggtgcc
540 tcaacttcag taaccttttc gcctagtgac attgaaaaaa caagagctgc aactattaaa
600 gatgttttaa atgcaggttg gaatattaaa ggagctaaag ttgcgggggg taataccgag
660 aatgttgatt tagtggcggg ttatgacaat gttgagttta ttacaggaga taaaaacaca
720 cttgatgttg tattaacagc taaagaaaac ggtaaaacaa ccgaagtgaa gttcacaccg
780 aaaacttctg ttattaaaga taataatggt aagttgctta caggtaagca gttgaaggat
840 gcgaatactg gtacagcgac caatgcaact gaagatacag acgaggcaat ggcttag
 897
   <212> Type : DNA
   <211> Length : 897
   SequenceName : SEQ ID 43:GDC_HINF_1807963
   SequenceDescription :

### Custom Codon

Sequence Name : SEQ ID 43:GDC_HINF_1807963

### Sequence

<213> OrganismName : Haemophilus influenzae
<400> PreSequenceString : 
gtgatgagcc gacatcgagg tgccaaacac cgccgtcgat atgaactctt gggcggtatc
60 agcctgttat ccccggagta ccttttatcc gttgagcgat ggcccttcca ttcagaacca
120 ccggatcact atgacctact ttcgtacctg ctcgacttgt ctgtctcgca gttaagcttg
180 cttataccat tgcactaa
198
 <212> Type : DNA
   <211> Length : 198
   SequenceName : SEQ ID 44:GDC_HINF_1817220
   SequenceDescription :

### Custom Codon

Sequence Name : SEQ ID 44:GDC_HINF_1817220

### Sequence

<213> OrganismName : Helicobacter pylori-26695
<400> PreSequenceString : 
atgtttgcag tgcatgctgc gatgattacg acattaaaga aagaagtttt ctttctttac
60 ctttatatca aatcactcaa aatcccgatt cctactacac tgaaatacat gatttcttta
120 ggcaaaatca gagaattaga tgttttagca aatcttgcta aactttgccc tacttgtcat
180 agggctttaa aaaaaggatc tagcgaagag gagtttcaaa aacgcttgat tagaaacatt
240 ctcaatcgca ataaagacaa tttagagttt gcgcaattgc gttttgaaac cgatgatttt
300 tcaacgctta ttgatcgtat ttgtgaaagc ttgaaatga
339
 <212> Type : DNA
   <211> Length : 339
   SequenceName : SEQ ID 45:GDC_HPYL_51094 SequenceDescription :

### Custom Codon

Sequence Name : SEQ ID 45:GDC_HPYL_51094

### Sequence

<213> OrganismName : Helicobacter pylori-26695
<400> PreSequenceString : 
atgattaaac aaaccctcat cattcttgcc ccttttttta tcgcaacgct gttgtatttt
60 ttaggcgcac cggatgggtt aagacctaac gcttggcttt atttttgtat tttcatgggc
120 atgattatag ggctaatttt agagccggtg ccatcaggtt taatagcgct aagcgcgtta
180 gtgctgtgta tagcgttaaa aattggagcg agcgataaag tagcgagcgc taataaggct
240 atttcgtggg gtttgagcgg gtatgcgaat aaaacggtgt ggcttgtgtt tgtcgctttc
300 attttgggtt tagggtatga aaaaagcttg ttagggaaac ggatcgctct tttactgatt
360 aggtttttag ggcaaacccc tttaggttta ggctatgcga ttggtttgag cgaattgtgt
420 ctagcccctt ttatccctag caactccgct agaagtggag gcatactcta tcccatcgtt
480 tcatctatcc cgcctttaat gggatctact ccaaataata accctgacaa aatcggcgcg
 
540 tatttgatgt gggtcgcttt ggcttcaact tgcatcactt cgtccatgtt tttaaccgcg
600 ctcgctccta accccctagc aatggaaatc gctgccaaaa tgggcgtgaa tgaaatctca
660 tggttttcgt ggtttttagc gttcttgcct tgtggggtgg ttttgatctt gcttgtgcct
720 ttattggcgt ataaaacctg caaacccacc ttaaaaggct caaaagaagt gagtttgtgg
780 gccaaaaaaa ggaattag
798
<212> Type : DNA
<211> Length : 798
SequenceName : SEQ ID 46:GDC_HPYL_155367
SequenceDescription :

### Custom Codon

Sequence Name : SEQ ID 46:GDC_HPYL_155367

### Sequence

<213> OrganismName : Helicobacter pylori-26695
<400> PreSequenceString : 
atgagccgac atcgaggtgc caaacctccc cgtcgatgtg agctcttggg ggagatcagc
60 ctgttatccc cggggtacct tttatccttt gagcgatggc ccttccacac agaaccaccg
120 gatcactatg accgactttc gtctctgctt gacttgtatg tcttacagtc aggctggctt
180 gtgccattac actcaacttg cgatttccaa ccgcaatga
219
 <212> Type : DNA
   <211> Length : 219
   SequenceName : SEQ ID 47:GDC_HPYL_447632 SequenceDescription :

### Custom Codon

Sequence Name : SEQ ID 47:GDC_HPYL_447632

### Sequence

<213> OrganismName : Helicobacter pylori-26695
<400> PreSequenceString : 
gtgcaacttc attgccacaa cttgccatgc gtttcaattg atattctact aggcggacca
60 ccatgccaga gctattctac ccttggcaaa agaaaaatgg atgaaaaagc gaatctgttt
120 aaagaatatt tgcggctttt agatttagta aaaccaaaaa tatttgtttt tgaaaatgtg
180 gtgggtttaa tgtctatgca aaaagggcaa ttattcaaac aaatttgtaa cgcttttaaa
240 gagagagatt atattttaga gcatgccatt ttgaacgccc tagattatgg tgtgcctcaa
300 atgagagaac gagtgatttt agtgggcgtg cttaaaagct ttaaacaaaa attttacttc
360 cctaaaccca taaaaacgca tttttctctg aaagacgctt taggggattt accacccatt
420 caaagcggtg aaaatggtga tgctttaggt tatcttaaaa atgcggataa tgtttttttg
480 gaatttgtgc gaaattctaa agaattaagc gaacatagca gtcctaaaaa caatgaaaaa
540 ctgataaaaa tcatgcaaac gctaaaagac ggacagagta aagatgattt gccagaaagt
600 ctgcgtccca aaagtggtta tattaatacc tatgccaaaa tgtggtggga aaaaccagcc
660 cccaccatta caagaaattt ttctacccca agcagttcta ggtgtatcca tccaagagac
720 tctagagcgt taagcattag agagggggca agattgcaaa gctttcctga taattataaa
780 ttctgtggga gtggtagcgc taaaagattg caaattggca atgccgtgcc gcctttattg
840 agtgtagcgc tcgcgcaggc ggtctttgac tttttaaagg ggtaa
885
 <212> Type : DNA
   <211> Length : 885
   SequenceName : SEQ ID 48:GDC_HPYL_506250
   SequenceDescription :

### Custom Codon

Sequence Name : SEQ ID 48:GDC_HPYL_506250

### Sequence

<213> OrganismName : Helicobacter pylori-26695
<400> PreSequenceString : 
ttgatggaat ttgatgttac catcatagat gagacaggca gggccacagc accagaaatc
60 ttgattcctg cacttcgcac taaaaaactg atcttaatag gcgatcacaa ccagctccca
120 cctagcattg ataggtacct cctagaacaa ttagagagcg atgatattca aaacttggat
180 gccattgatc gccaattatt ggaagagagt ttttttgaaa atctctataa gtatattcca
240 gagagtaata aggccatgct taatgagtaa
270
 <212> Type : DNA
   <211> Length : 270
   SequenceName : SEQ ID 49:GDC_HPYL_583607 SequenceDescription :

### Custom Codon

Sequence Name : SEQ ID 49:GDC_HPYL_583607

### Sequence

<213> OrganismName : Helicobacter pylori-26695
<400> PreSequenceString : 
atgcctgctt ctattggatc gctagttagt cagctttttt ataaagagaa acttaagaat
60 ggagtgatca aaaatacctc gcaattttac gatcctaaga atattatccg ttggattaat
120 gttgaagggg agcatcaact agaaaaaaca agtagctata acaaaaatca agttcaaaaa
180 atcatagagc ttttagagca aatcaatcgc gttcttaatc aaagaaaaat cagaaaaacc
240 ataggaatta tcacacctta taatgcccaa aaaagatgct tgcgatcaga agtggaaaaa
300 tacggcttca agaattttga tgagctcaaa atagacactg tggatgcctt tcaaggcgag
360 aaggcagata ttattattta ttccaccgtg aaaacttatg gtaatctttc tttcttgata
420 gattctaaac gcttgaatgt agctatttct agggcaaaag aaaatctcat tttgtgggc
480 aaaaagtctt tctttgagaa tttgcgaagc gatgagaaga atatctttag cgctattttg
540 caagtctgta gatag
555
 <212> Type : DNA
   <211> Length : 555
   SequenceName : SEQ ID 50:GDC_HPYL_583883 SequenceDescription :

### Custom Codon

Sequence Name : SEQ ID 50:GDC_HPYL_583883

### Sequence

<213> OrganismName : Helicobacter pylori-26695
<400> PreSequenceString : 
ttgattattg aaacgcaaca agaccccaaa gaactacctg agtcttgcaa aataacgccc
60 caaaaaatct cttttaacca agtggttttt aaaaaaatta aaagaaaact caaccgcttc
120 attggaagca ttttagctcg gacagaagtg tataagaatc tcgtggcaaa atacgatgaa
180 ctcacaggaa aatacgaatc attattggca aaagaggcaa acatcaaaga gaccttttgg
240 gaaaggcgtg ctgatagcga aaaagaagcc ttttttttag agcattttta cctcactagc
300 gtgtatgtgg cttctacagc aggatactat atcacgccta agggcgctaa aacctttata
360 gaagccacgg agcgttttaa aatcatagag ccggtggata tgttcataaa caaccccact
420 taccatgatg tggctaattt tacctatttg ccttgccctg tttctttaaa caagcatgct
480 ttcaatagca ccattcaaaa tgcaaaaaag cctgacattt cattaaaacc ccctagaaaa
540 tcctattttg ataatctttt ttatgatcaa ttaaacacta gaaagtgctt aaaagccttt
600 cacaaataca gcagacgata cgctccttta aaaaccccta aagaggttta a
651
 <212> Type : DNA
   <211> Length : 651
   SequenceName : SEQ TD 51:GDC_HPYL_665045
   SequenceDescription :

### Custom_Codon

Sequence Name : SEQ ID 51:GDC_HPYL_665045

### Sequence

<213> OrganismName : Helicobacter pylori-26695
<400> PreSequenceString : 
ttgatggaaa ttttagtgtt gaatctgggc agttcgtcta ttaagtttaa gttgtttgac
60 atgaaagaaa ataagccctt agcgagcggt ttggctgaaa aaatcggcga agaaataggg
120 cagttgaaaa ttaaatcgca tttgcaccat aacgatcaag aattaaaaga aaagtttgtg
180 attaaagatc atgcgagcgg acttttaatg attcgtgaga atttaacgaa aatggggatt
240 atcaaagatt ttaaccaaat tgacgctata gggcatcgtg tggttcaagg gggggataaa
300 ttccatgccc cagttctagt caatgaaaaa gtcatgcaag aaattggcaa tctttctatt
360 ttagccccct tacacaaccc ggcgaattta gccggtattg agtttgttca aaaagcgcac
420 ccccatatcc ctcaaatcgc tgtttttgac accgcattcc atgccactat gcccagttac
480 gcttacatgt atgcgttacc ttatgaattg tatgaaaagt atcaaatccg gcactatggt
540 ttccatagga cttcacacca ttatgtggcc aaagaagcgg cgaagttttt gaataccgct
600 tatgaggaat ttaacgcgat cagtttgcat ttagggaacg gctcaagtgc agccgccatt
660 caaaagggta aaagcgtgga tacttctatg gggctaaccc ctttagaagg cttgattatg
720 ggcacaaggt gtggggatat tgaccccact gtggtggaat atactgcgca atgcgcgaac
780 aagagcttag aagaagtgat gaaaatgtta aaccatgaaa gcggattgaa aggcatttgt
840 ggggataatg agaaacatag aagccagaaa agaaaaaggt ga
882
 <212> Type : DNA
   <211> Length : 882
   SequenceName : SEQ ID 52:GDC_HPYL_953783
   SequenceDescription :

### Custom Codon

Sequence Name : SEQ ID 52:GDC_HPYL_953783

### Sequence

<213> OrganismName : Helicobacter pylori-26695
<400> PreSequenceString : 
atgcctaaca gccaagtggc tgggcaagct agcgttttta ttttcccgga tttaaacgct
60 gggaacatcg cttataaagc ggtgcaacgg agcgctaaag ccgtggcgat agggcccatt
120 ttacaaggtt tgaataagcc cattaacgat ttgagtaggg gcgctttagt ggaagatatt
180 attaacaccg ttttgattag cgcccttcaa gcgcaagatt aa
222
 <212> Type : DNA
   <211> Length : 222
   SequenceName : SEQ ID 53:GDC_HPYL_954679
   SequenceDescription :

### Custom Codon

Sequence Name : SEQ ID 53:GDC_HPYL_954679

### Sequence

<213> OrganismName : Helicobacter pylori-26695
<400> PreSequenceString : 
gtgagcctgg tttcaagcgt gtttttaatg tgtttagaca ctcaagtgct agtctttggg
60 gattgcgcga ttatccctaa ccctagccct aaagaattag ccgagatcgc taccacttcc
 
120 gcacaaaccg ccaagcaatt caatattgcg cctaaagtgg ccttgctttc ttatgcgaca
180 ggcgattccg ctcaaggcga aatgatagac aaaatcaacg aagctttaac aatcgctcaa
240 aagttggatc cccaattaga aattgatggc cccttacaat ttgacgcttc cattgataaa
300 agcgtagcca agaaaaaatg cctaacagcc aagtggctgg gcaagctagc gtttttattt
360 tcccggattt aa
372
 <212> Type : DNA
   <211> Length : 372
   SequenceName : SEQ ID 54:GDC_HPYL_954846
   SequenceDescription :

### Custom Codon

Sequence Name : SEQ ID 54:GDC_HPYL_954846

### Sequence

<213> OrganismName : Helicobacter pylori-26695
<400> PreSequenceString : 
ttgaaagctg cacatcgttt gaatttaatg ggcgcggtag gattgatctt at[?]aggcgat
60 aaagaagcca ttaattcgaa aaatttgaac ttgaatttag aaaatgtgga aatcattgat
120 cccaacactt ctcattatag agaagaattc gctaaaagct tgtatgaatt acgaaaatca
180 aagggcttga gtgagcaaga agctaagcaa ttagtgctgg ataagactta ttttgcgacc
240 atgctcgtgc attcaggcta tgtgcatgcg atggtttctg gggtgaatca cagctga
297
 <212> Type : DNA
   <211> Length : 297
   SequenceName : SEQ ID 55:GDC_HPYL_955261
   SequenceDescription :

### Custom Codon

Sequence Name : SEQ ID 55:GDC_HPYL_955261

### Sequence

<213> OrganismName : Helicobacter pylori-26695
<400> PreSequenceString : 
gtgaaacaaa ttagtatctc ttgcagccat agaaaatatt ttgttagctt tagcgtggaa
60 tacgaacaag acattactcc cataaaaaac actaaaaatg gtgtggggct agatttgaat
120 atccttgata tagcttgttc ttgtgagata aacaaccatg acaaactaac ggactttaag
180 caataccaaa cagacatgaa agaattacta gggatagaaa tagatgaaga gctggatact
240 aaacgactta tccctactta ttccaaattg tattctttaa aaaaatactc taaaaaattt
300 aaaagattac aaagaaaaca aagccgtagg gtgttaaagt ctaaacaaaa caaaaccaaa
360 ttaggaggta atttttacaa aacccaaaag aaattaaacc aagcctttga caagtctagt
420 catcaaaaaa cagacagata ccataaaatc acaagcgaac tttcaaagca atttgaattg
480 atagtagttg aagatttgca agtaaaaaac atgactaaaa gagctaaact caaaaatgtt
540 aaacaaaaga gtgggcttaa tcaatctatt ttaaacgctt cattctatca aatcatctct
600 tttttagact acaaacaaca gcataatggc aaattgttag tgaaagttcc cccacaatat
660 acgagtaaaa cttgccattg ttgtgggaat atcaaccaca agcttaaatt aaatcatagg
720 caatattggt gtttagaatg cgggtataga gaacacaggg acatcaacgc tgcgaacaac
780 attttaagca aagggttaag tctttttggg gtaggaaata tccatgcaga ctttaaagaa
840 caaagccttt cgtgttag
858
 <212> Type : DNA
   <211> Length : 858
   SequenceName : SEQ ID 56:GDC_HPYL_1068602
   SequenceDescription :

### Custom Codon

Sequence Name : SEQ ID 56:GDC_HPYL_1068602

### Sequence

<213>_OrganismName_: Helicobacter pylori-26695
<400> PreSequenceString : 
atgaaagtca ataagggttt taaattccgc ttgtatccca ctaaagaaca acaagataag
60 ttgcaacact gcttttttgt ctataatcaa gcttataata ttggcttgaa tgaactgcaa
120 gagcaatatg aaaccaacaa agattcacca cctaaagaaa gaaaatacaa aaaatcaagc
180 gaattagaca atgcgatcaa acaatgcttg agagctaggg acttgccctt tagcgctgtg
240 atagcccaac aagcacgcat gaatgttgaa agggctttaa aagatgcttt taaagttaaa
300 aacagaggct ttcctaaatt caaaaactct aaatccgcta aacaatcttt ttcgtggaac
360 aatcaaggct tctctatcaa agagagcgat gatgagtgct tcaagacatt cactctgatg
420 aaaatgcctt tactcatgcg catgcataga gacttccccc taattttaaa gtga
474
 <212> Type : DNA
   <211> Length : 474
   SequenceName : SEQ ID 57:GDC_HPYL_1069456
   SequenceDescription :

### Custom Codon

Sequence Name : SEQ ID 57:GDC_HPYL_1069456

### Sequence

<213> OrganismName : Helicobacter pylori-26695
<400> PreSequenceString : 
ttgatattca tcacccattt ttccacagag cctttacctt tacccatcct ggtttctaag
60 ggtttagcgg tcaaaggctt atcagggaat actctaatcc acaccttacc cgctctttta
120 atgtgccttg tcatggccac ccttgcggat tcaatttggc gtgaatcaat cctcccatgc
180 tctatggctt taatcgcaat atccccaaac gcaatggagt taccccgatg ggctttccca
240 cgattgcgcc ctttcatttg ctttctgtat tttgttcttt ttggcattaa catgattatt
300 gcctccctct tctgcttctt ctag
324
 <212> Type : DNA
   <211> Length : 324
   SequenceName : SEQ ID 58:GDC_HPYL_1376803
   SequenceDescription :

### Custom Codon

Sequence Name : SEQ ID 58:GDC_HPYL_1376803

### Sequence

<213> OrganismName : Helicobacter pylori-26695
<400> PreSequenceString : 
atgagccgac atcgaggtgc caaacctccc cgtcgatgtg agctcttggg ggagatcagc
60 ctgttatccc cggggtacct tttatccttt gagcgatggc ccttccacac agaaccaccg
120 gatcactatg accgactttc gtctctgctt gacttgtatg tcttacagtc aggctggctt
180 gtgccattac actcaacttg cgatttccaa ccgcaatga
219
 <212> Type : DNA
   <211> Length : 219
   SequenceName : SEQ ID 59:GDC_HPYL_1474291
   SequenceDescription :

### Custom Codon

Sequence Name : SEQ ID 59:GDC_HPYL_1474291

### Sequence

<213> OrganismName : Helicobacter pylori-26695
<400> PreSeciuenceString : 
ttgaacgccg catttaaaga aaggcgcttc attctcgtcc agttagatga aaaaattgat
60 cccaaggaag acaaaagcgc ttatgatttt tgtttgaaca ccttaaaatc accctcccca
120 agcatttttg acatcaccga agaaaggatt aaaagagcgg gggctaaaat caaagaagct
180 tgcgcgcatt tagatgtggg gtttagagcg tttgaaatca ttgatgatga aacgcatgct
240 aatgataaaa atctcagtca agcccatcaa aaggatttgt tcgcttattc taaccttgat
300 agaatggaaa cccaaacgat tttaattaag cttttaggct gcgagggttt ggagctcact
360 acccctataa cttgcttgat tgaaaacgcc ttgtatctgg ctttaaatac ggctttcatt
420 gtgggggata tagaaatgag cgaagtttta gaaaacttga aagataaagg ggtggaaaaa
480 atcagcatgt atatgcccgc tatcagtaac gataatttgt gtttggaatt gggcagtaat
540 ttgttggatt tgaaattaga gagtggcgat ttaaagatta gggggtag
588
 <212> Type : DNA
   <211> Length : 588
   SequenceName : SEQ ID 60:GDC_HPYL_1600102
   SequenceDescription :

### Custom Codon

Sequence Name : SEQ ID 60:GDC_HPYL_1600102

### Sequence

<213> OrganismName : Mycobacterium tuberculosis-H37RV
<400> PreSequenceString : 
atgtatatac gtttttatcg cgattctctt gcagagcccg ccacagacat atacgctttt
60 gcctatgttt cgttcaacaa ggaggccggc acatggcaca cccctgcgca accgacccgg
120 aactatggtt cgggtacccc gatgacgacg gcagcgacgg cgccgctaag gcacgcgcct
180 atgagcggtc ggccacccaa gcgcggatcc aatgcctgcg ccggtgcccg ctcctacagc
240 agcgccggtg tgctcaacac gcggtcgagc atcgggtgga gtacggcgta tggccggca
300 tcaagcttcc cggcggccag taccgaaagc gcgaacagct cgcggcagcc cacgacgtgc
360 tgcgtcggat tgccggcggc gagatcaatt ccaggcagct cccggacaat gcggctctgc
420 tggcccgcaa cgaaggactc gaggtcaccc cggtgcccgg ggtcgtggtg cacctgccga
480 tcgcacaggt tggcccacaa ccggccgctt gatgcccggt cggcaagccc ggcagttgcc
540 aaacccagcg tgatcaggct cggctcgcga gttcggcgaa gaagtggctc gcctgatcac
600 ctaccatcgg ccaggatctg cgtgtcatca cgacgctcgc caaggaggtt gttgtggtgc
660 tatcgacggc ctttagccag atgttcggaa tcgactatcc gatag
705
 <212> Type : DNA
   <211> Length : 705
   SequenceName : SEQ ID 61:GDC_MTUB_26830
   SequenceDescription :

### Custom Codon

Sequence Name : SEQ ID 61:GDC_MTUB_26830

### Sequence

<213> OrganismName : Mycobacterium tuberculosis-H37RV
<400> PreSequenceString : 
ttgatgttct gtgcgtcgcg gaaagagatg gcgatgtcga attcgtcttc tagctcggtg
60 atcaactgga acagcttgag cgagtcaaaa cccaggtcgt cgacgagtac ctggttcgcg
120 gtgatgccgc ggtcggttcg caagatccgt tggatggtgg cgttgatggc ctctttcata
180 gcgcggctcc ttgcggggtc aggtcctcgg caaggccggc aaacacgtgc aaggcccggt
240 cgaggtcaga ttgtcggtgg tcggctaggt agctggtgcg gaatcccgaa cgctcctccg
300 gcacggctgg gggggccacc gggttcacat acaccccgga gcgcatcagc cgcagatagc
 
360 ccgcatgcgc cacggtcggg ttgcccagga tcaccggcac gatcgcggtt ccgtgatact
420 cggcctgata gccctgccgt gccaggccgg tggccatgta ctcggccgcg gccagcaccc
480 gagcccgccg gtcgggttca cgccgactga
510
 <212> Type : DNA
   <211> Length : 510
   SequenceName : SEQ ID 62:GDC_MTUB_36276
   SequenceDescription :

### Custom Codon

Sequence Name : SEQ ID 62:GDC_MTUB_36276

### Sequence

<213> OrganismName : Mycobacterium tuberculosis-H37RV
<400> PreSequenceString : 
gtgccgccac cgatcccgcg gtgcgcggcg gccagtactt cggacccgat ggcttcggtg
60 aaatacgggg ctacccgaag gtggtggcct ccagcgccca gtctcacgac gagcagctgc
120 agcgccgcct gtgggctgtg tccgaagagc tcaccggggt cgtctatccc gtcggatgag
180 ccggactcaa cggcaacggt tggtcaacac tcgacgatgt tgactgcgac gttgatggcg
240 agcccgccgg ccgaggtttc cttgtacttg gtgtgcatgt ccgcgccggt ggcgcgcatg
300 gtgtcgatga cctggtcgag ggtgacgcga tggatgccgt cgccgcgcaa tgccatccgt
360 gcggcgttga tggccttgcc ggcggaaatc gcgttgcgtt cgatgcaggg gatctgcacc
420 agcccggcga tggggtcaca ggtcaggccg aggctgtgtt ccatggcgat ctcggcggcg
480 ttttccactt gtcgcggtgt gccgccgagg atttcagcca atccggcggc ggccatggcg
540 gccgcggagc cgacctcgcc ctga
564
 <212> Type : DNA
   <211> Length : 564
   SequenceName : SEQ ID 63:GDC_MTUB_76032
   SequenceDescripcion :

### Custom Codon

Sequence Name : SEQ ID 63:GDC_MTUB_76032

### Sequence

<213> OrganismName : Mycobacterium tuberculosis-H37RV
<400> PreSequenceString : 
atgatcccga tggacgtgat attcggctgc ccgttgtacg ccaatttctg taagccctcg
60 gtcgtgagga agacattggg gatcttggcc agcgcggtgg aattcggcac aatgccaacg
120 acccgcaatc tgcgcgcgcc gacctcgaca gtgtcaccga ggtgtcggcc catcgtgctc
180 gatgccgcga cttcgtccgg tttcgacggt gaccgaccct ctgagacccg tggcatgcca
240 ggtccgtgct cgggcgcgcc gaagaccgtg acgtttcgcg tcgacgtgcc ttctttcatg
300 atcgtcccca cgctgcccaa cggggccgcg gccatgacac cgggttcagc ggccactcgg
360 gccaggtcaa catcgggaaa cggtattgaa cccagaaaag gtccagcagc gccggatctg
420 acgacgaata catcgacacc catggaatcg acggtgtgcc gggcctccac ccggaagccg
480 ttcgcgagtc cggtcaaaac aagcgtcatc ccgaagatca gcccggtgct gatgatcgtg
540 atgaccaggc ggcgctttct ccattgcatg tcacgcaggg ccgcgaagag cattcccaga
600 ggctaccaac gtggcgcact tgtggggcct ggtcttgacg ttttgtggtc agggcgcggc
660 ccgctagtgg tcgaagaggc gttcggggtg gtggtagtcg ttggtgtgc[?] caccgcggtc
720 gaggtggggt ggcgggatcc attccgtttg gccgtcggac cgtttccttg tctgccagcc
780 tttcccgact ag
792
 <212> Type : DNA
   <211> Length : 792
   SequenceName : SEQ ID 64:GDC_MTUB_80423
   SequencDescription :

### Custom Codon

Sequence Name : SEQ ID 64:GDC_MTUB_80423

### Sequence

<213> OrganismName : Mycobacterium tuberculosis-H37RV
<400> PreSequenceString : 
atgtcgcgtg ctatccggac aaagccgaaa tcagcatctt cccggggtag cgcaggctac
60 cgggtatacc tcggccaacg actgggtgtc gctgtattcg cgcagcgaga tgatcatccc
120 gtcacgggtc tcgaagatgc agacgaacgg gctgtcatat cgggtccggt cggcgctcac
180 accgtcgcaa tgcccctcga ccactaccgt ttcaccctcg ttgacgcagc ggatgagttc
240 gatgttgacc tcgaagacct gcttgcgccg ctcgactgct cgccgaaacg tcttcttgtc
300 caattccgta cgggtgacga tgctccagta ggtgaagtcg ttgctgagca gcgcgaagcc
360 ttcgtcgaga tctccgccct cgcagaggct ttgcaggaac atccaggcca gttcggcttg
420 cgggtcgtcg aacggcgtca tcacatcgcc atcttgtctc gggagacagc gtgcggtcaa
480 ttgacgtggt cgtcgaagcg gtggtcacct tcgcgggggc ggccggcttc gcgcacacct
540 tggcgccgtt gcgtcgcggt cagcaggatc catgctttcg ggtccccggt gacggcacta
600 tctggcggac cagcttgctg cccaccgggc cggtcaccgc gcggatcagc cgtgctgggc
660 gcgacgccgc ccgttgcgtg gcgtggggca gcggtgccga ggagtttgtc gacatggcgc
720 ccgccatgct gggcgccgcc gacgacgcca gcgatttcgt gccgctgcat ccggccgtgg
780 ccgccgcgca ccgccggctg ccgaacttgc gcctgggccg caccggccag gtgctggaag
840 ccttga
846
 <212> Type : DNA
   <211> Length : 846
   SequenceName : SEQ ID 65:GDC_MTUB_167239 SequenceDescription :

### Custom Codon

Sequence Name : SEQ ID 65:GDC_MTUB_167239

### Sequence

<213> OrganismName : Mycobacterium tuberculosis-H37RV
<400> PreSequenceString : 
gtgcgaccgg gccaccgcca ggtcgatgga tgccgccgtg gccaaccgtt gtgcggtgct
60 catgaacgcg tcggcctcgt gcgggttgtc ggtgccttcg gcctggcgca gcagggctgc
120 gatgcgggcc agcatcttgt cgttggtcat ggcgccaaaa ctagtggagg gctgcgacag
180 gtcggctcgg cctacaaccg ctcggtgagc caggcgacca catcgtcgag cacctggttg
240 cgctccggct cgttgaacac ctcgtggtac agcccgggat actccttcag ctgcacgtcg
300 gccgatccca cacattcgac caggcgacgg ctgccctcga tggggatcag ccggtcatcg
360 gtgccgtgca gcactagcag cggcgcggtc aatgccggtg ctcgccgcgg catggtctcg
420 cccacctgca gcagcgcgcg gccaatcccg gccggaaccc gtccgtggtg cacgagtggg
480 tcggtgttgt aa
492
 <212> Type : DNA
   <211> Length : 492
   SequenceName : SEQ ID 66:GDC_MTUB_214625 SequenceDescription :

### Custom Codon

Sequence Name : SEQ ID 66:GDC_MTUB_214625

### Sequence

<213> OrganismName : Mycobacterium tuberculosis-H37RV
<400> PreSequenceString : gtgtgtaaag catgtctcgg tcaccatacc catcaccacc gaacatctcg gcccctacga 
60 aatcgatgcc agcacgatca accccgacca gcccatcgac acggctttca cccaaaccct
120 cgatttcgcc ggcagcggca ccgtgggcgc gttccccttc ggcttcggct ggcagcagag
180 cccgggattc ttcaactcga ccacaacccc gtcgtcgggc ttcttcaact ccggcgccgg
240 tggcgcatcg ggcttcctca acgacgccgc agccgccgtg tcgggcctgg gaaacgtctt
300 caccgagact tcgggcttct tcaatgctgg cggcgtagga attcgggctt ccaaaacttc
360 ggcaacctgc tgtcgggctg ggcgaaccta ggcaataccg tctccggttt ctacaacacg
420 agcatgctgg acctcgcgac ccaagccctt atctccggct tcggcaacca cggagcccga
480 ctctccggca tcctcaacaa cggtagcgga ccctaa
516
 <212> Type : DNA
   <211> Length : 516
   SequenceName : SEQ ID 67:GDC_MTUB_424142 SequenceDescription :

### Custom Codon

Sequence Name : SEQ ID 67:GDC_MTUB_424142

### Sequence

<213> OrganismName : Mycobacterium tuberculosis-H37RV
<400> PreSequenceString : 
gtgcttagcc tatccgctgg cggcccggaa ccgagaatgc gaccaggtca caacccagtc
60 accttccacg ccgagcagac gaggaatcgc actgcgcgga cctcacgcgt gcgattccgc
120 gtctgctcgt cagacaaatc agcccaggat cagcgagtcg gcgtcggggc tgacgttgac
180 cggcacggta tcgccgtcgt gcacctggcc ggccaacagc atcttggcca gctggtcacc
240 gatggcctgc tgcaccagcc ggcgcaacgg ccgcgccccg tacaccgggt cgaatccgcg
300 ctgcgccaac cagcgcttgg ccggcagcga gacctgcagc tgcagccgcc gctgcgccag
360 ccgcttgccc agctgcgcca gctggatgtc gacgatgcgc accagctctt cggggttgag
420 accctcaaag atgagcacgt cgtcgagccg gttgatgaac tccggcttga acgtagcgcg
480 caccgcggcc agcacctgct cggcgctgcc acccgacccc aggttggacg tcaggatcaa
540 gatggtgttg cggaagtcga ccgtgcggcc gtgcccgtcg gtgagccggc cctcgtcgag
600 gacctgcagc agcacgtcga acacgtccgg gtgcgccttc tcgatctcgt cgaacagcac
660 caccgtgtag ggacgccggc gcaccgcctc ggtcagctga ccgcccgcct cgtatcccac
720 atagccgggc ggggcgccga tcaaccgagc cacggtgtgc ttctcgccgt actcgctcat
780 gtcgatgcgg accatcgccc gctcgtcgtc gaacaggaag tcggccagcg ccttggccag
840 ctcggtcttg ccgacaccgg tcgggccgag gaacatgaac gccccggtgg gccggttggg
900 gtcggacacc ccggcccggc tgcgccgcac cgcatcagag actgcggtaa ccgcggcctt
960 ctgcccgatg acccgcttgc ccagctcgtc ttccatgcgc agcagcttgg cggtctcgcc
1020 ttcc
1024
 <212> Type : DNA
   <211> Length : 1024
   SequenceName : SEQ ID 68:GDC_MTUB_459316 SequenceDescription :

### Custom Codon

Sequence Name : SEQ ID 68:GDC_MTUB_459316

### Sequence

<213> QrganismName : Mycobacterium tuberculosis-H37RV
<400> PreSequenceString : 
ttgcttgccg atttcgatgt aggacaacac cttttccagc tggtcgttgg aggcctggga
60 acccagcatg gtttcggtgt ccagcgggtc gccctgccgg accgccttgg tccggatcgc
120 cgccagctcc aggaactcgt cgtagatgtc ggcctggatc agactgcgcg acgggcaggt
180 gcacacctcg ccctggttga gggcgaacat ggtgaagcct tccagcgcct tgtcgcagaa
240 gtcgtcgtgg gcggccagca cgtcggcgaa gaagatgttg gggctcttgc cgccgagttc
300 cagggtgacc gggatcaggt tgtgcgaggc gtattgcatg atcagccgcc ccgtggtggt
360 ttccccggtg aacgcgacct tggcgatgcg gtcgctggag gccaacggct tgccggcctc
 
420 ggcgccgaat ccgttgacca cgttgaccac cccgggcggc aacagatcac cgatcagcga
480 catcaggtag agcaccgaag cgggtgtctg ctcggcgggt ttgagcaccg ccgtgttgcc
540 ggccgccaac gccggcgcca gcttccaggc cgccatcagg atggggaagt tccacggaat
600 gatctggccc accacgccga gcggctcgtg gaagtggtag gccacggtgt cctcgtcgat
660 ctggctcagc gcgccctcct gggcgcgaat cgccgcggcg aagtaccgga agtgatcgac
720 cgccaacggg atatcggcgg ccagcgcttc ccggaccggt ttcccgttgt cccagacctc
780 ggccaccgcc agcgcggcgg cgttcttgtc gatgcggtcg gcaatcatgt tgaggatcgc
840 cgcccgttcg gccggtgcgg tcttgcccca ccccggcgcc gccgcgtgcg cggcgtcgag
900 cgccttgtcg atgtcggccg cgtcggagcg cggcacctcg cagaacggct ggccggtcac
960 cggcgtcggg ttctcgaagt agcgcccatg gaccggcgcg acccactggc ccccgatgaa
1020 gttt
1024
 <212> Type : DNA
   <211> Length : 1024
   SequenceName : SEQ ID 69:GDC_MTUB_549643 SequenceDescription :

### Custom Codon

Sequence Name : SEQ ID 69:GDC_MTUB_549643

### Sequence

<213> OrganisinName : Mycobacterium tuberculosis-H37RV
<400> PreSequenceString : 
gtgtatcttc cgcccaagct gatcccgagg cggatcccgg cgcaggtgag gccaactatg
60 gtggcccccc aagttcccca cgtcttgtcg atcacaccga atgggcgcag tggggaagtc
120 tgcccagcct ccgggtctac ccgtcccaag ttgggcgtac agcctcccgc cgcctcggga
180 tggccgctgc cgacgcggcc tgggccgagg ttctcgcgct gtcaccggag gccgacactg
240 ccggcatgcg cgcgcagttc atctgccact ggcagtacgc cgaaatcaga caacccggca
300 aacccagctg gaacctcgag ccgtggcggc cggtcgtcga cgactcggag atgttggctt
360 ccggctgcaa tccgggcagc cctgaagagt cgttttagtg ctcggccaac cgactcgggc
420 gcagttggcc gcgctggtag accacaccct gctcaagcct ga
462
 <212> Type : DNA
   <211> Length : 462
   SequenceName : SEQ ID 70:GDC_MTUB_566823 SequenceDescription :

### Custom Codon

Sequence Name : SEQ ID 70:GDC_MTUB_566823

### Sequence

<213> OrganismName : Mycobacterium tuberculosis-H37RV
<400> PreSequenceString : 
atgacgtcta cgaacgggcc atcggcgcgg gataccggtt ttgttgaggg ccagcaggcc
60 aagacacaac ttctcaccgt ggccgaagtg gcggccctga tgcgggtgtc caagatgacg
120 gtgtaccggc tggtgcacaa tggcgaactg cccgcggttc gggtcgggcg gtcattccgg
180 gtgcatgcca aggccgtcca cgacatgttg gagacttcgt acttcgacgc gggctag
237
 <212> Type : DNA
   <211> Length : 237
   SequenceName : SEQ ID 71:GDC_MTUB_591109 SequenceDescription :

### Custom Codon

Sequence Name : SEQ ID 71:GDC_MTUB_591109

### Sequence

<213> OrganismName : Mycobacterium tuberculosis-H37RV
<400> PreSequenceString : 
gtggcggagt ccgtggctat ccgcggctgc ctgctgaggt gcgggccgcg ttcccgaccg
60 cggcggagat cgcgccgcag tggcatctgc gcatgcaggc cgcggtgcag cgccacgtcg
120 aggccgccgt gtccaagacg gtcaacttgc ccgccacggc gacggtcgat gacgtccgcg
180 ccatctatgt ggccgcctgg aaggcaaagg tcaagggcat cacggtgtat cgctacggca
240 gccgggaagg acaggtactg tcctacgccg cgccgaaacc gctactggcg caggctgaca
300 cggagttcag cggcggctgt gcgggccgct cctgcgagtt ctgacggcgg ctcccatggc
360 gcgagcagac gcagaatcgc acaaaatcag cgattttga
399
 <212> Type : DNA
   <211> Length : 399
   SequenceName : SEQ ID 72:GDC_MTUB_663028 SequenceDescription :

### Custom Codon

Sequence Name : SEQ ID 72:GDC_MTUB_663028

### Sequence

<213> OrganismName : Mycobacterium tuberculosis-H37RV
<400> preSequenceString : 
ttgctgcaca gcagcttcgg gcacctcgag ggcatccagc agccgctcat agacgagctg
60 gcagaactcg accacgtgtt gggcaagctg ccggacgcct accggatcat cggccgcgcc
120 ggcggcatat acggtgactt cttcaacttc tatctgtgtg acatctcact gaaagtcaac
180 ggattacagc ctggaggtcc ggtacgcacc gtcaagttgt tcggccagcc gaccggcagg
240 tgcacaccgc aatga
255
 <212> Type : DNA
   <211> Length : 255
   SequenceName : SEQ ID 73:GDC_MTUB_688806 SequenceDescription :

### Custom Codon

Sequence Name : SEQ ID 73:GDC_MTUB_688806

### Sequence

<213> OrganismName : Mycobacterium tuberculosis-H37RV
<400> PreSequenceString : 
ttgctggggg cgctgcacca gtacccgcac actcgcatcc agccgggtgc cgttgcggcg
60 caccgtgatc gccagcaccc gcgcccggtc tttggcgatg aggcgctcga tgcggcgggt
120 gttctcatgc gtacgcacgc agccgatcac cggcaaagtg aggtgtctac ggtcggctc
180 aacgcgcatc gcacccgtgg tgaacgacac gcgatcggcg tcgcggccct tcttcttgaa
240 tcgagggaag cccattctct tgccgtcgcg cttgccagca cgcctctgct gccagttcca
300 gtacgcgtcg accgcgcccg cgatcccgtc ggcgtaggcc tctttcgagc attccggcca
360 ccacacggtg ccagtctcgg cgttgacaca cacctcgtct ttcaccgtgt tccagcgttt
420 ccgcagtacc cgaagcgacg gcttcgccgt ctgggcgccg gtcgcgcgcc acgcttggat
480 atcggctttc agctgcgcga cggtccagtt gtaggccttg cggcgggcgc cgaaatgccg
540 cgccaacgcg tgtgcctgct cggcggtcgg atcgagtgtg aaccggaacg cttgcacaca
600 ccagccgttg ggatctccaa aacgcggcat ctcaggccgc ctcatgatca tcgacagcgg
660 cagccgcgac ggcccgcttg gcccggttct gagcagcacg tttgccatac aaccttgcgc
720 acatcgaggt cagaatctcg gtcatatccc ataccaggtc atcgtcaacc tcggccgagt
780 ccaccacgac caactcccga ccctgagcgg ccagcgcagc gtggacatac tccgaaccga
840 accggcagaa ccgatcccga tgctcaacca caatccgcgt ga
882
 <212> Type : DNA
   <211> Length : 882
   SequenceName : SEQ ID 74:GDC_MTUB_701762 SequenceDescription :

### Custom Codon

Sequence Name : SEQ ID 74:GDC_MTUB_701762

### Sequence

<213> OrganismName : Mycobacterium tuberculosis-H37RV
<400> PreSequenceString : 
atggcttcca gtaccgacgt gcggccgaag atcactttgg catgcgaggt gtgcaagcac
60 cgtaactaca tcaccaaaaa gaaccgccgc aacgacccgg accggctgga gctgaagaag
120 ttctgcccga attgcggcaa acaccaggcg caccgcgaga cgcggtaa
168
 <212> Type : DNA
   <211> Length : 168
   SequenceName : SEQ ID 75:GDC_MTUB_731710 SequenceDescription :

### Custom Codon

Sequence Name : SEQ ID 75:GDC_MTUB_731710

### Sequence

<213> OrganismName : Mycobacterium tuberculosis-H37RV
<400> PreSequenceString : 
ttggtatgcg ccgccgcccc cggtcgacga cgacccctcg gcgtaggcgg acaggtcgaa
60 gccggcacag aatccctcgc cgcgaccgga caccagaatg acatgcacgc ctggatccag
120 atcggcacgc tccaccagag cagacaactc cagcggggtg tctgcgatga tcgcgttgcc
180 cttctccggc cggttgaagg tgatccgcgc aatccgaccg gtgacctcat aggtcatcgt
240 cttcaggttg tcgaaatcga ccggcctgat cgcgtgtgtc atcagcggcc gctcagcctt
300 ttaccagcgc acgctcgagg atgggcgcga gatccagacc ggccggcatg gtgccgtacg
360 ctccgcccca ctggccgccg agccgagtgg ccagaaacgc ctcggcgacg gcgggatgtc
420 cgtggcgcac caacaacgat ccctgcaacg ccaggcagat gtcttcggca atcttgcggg
480 ctcgataacc gatcgtgtca agatcgccca gctgcggacg cagcctttcg acgtggccgt
540 ccagcctggg gtcctggcct gcgctgcggg ccagctcgtc aaacagcacc tcgacgcatg
600 cgggccgggt tgccatggcg cgcaaggtat ctagcgcgct ga
642
 <212> Type : DNA
   <211> Length : 642
   SequenceName : SEQ ID 76:GDC_MTUB_772761 SequenceDescription :

### Custom Codon

Sequence Name : SEQ ID 76:GDC_MTUB_772761

### Sequence

<213> OrganismName : Mycobacterium tuberculosis-H37RV
<400> PreSequenceString : 
ttgggtctcg ttgcgccggc aggtgacggt cgcgcagcga aaaagcgacc tgcgggccgc
60 cgaggatccg atcgacgccg tcgtatgcgc ctacgtggcg ttgtacgccc aacgccggcc
120 cgccgatgtc acgatctatg gggacttcac caccgggtac attgtcacgc cgtcgctgcc
180 caccgacttc agaacggcac cggacgctgg tcgacgggcg cgagcacgtc gatgaggtcg
240 accaccgtcg ccagcgcagc ggcacgcggg tcccgccctt cgaccagcgc cgagaccacc
300 gatccgtcga ccgcacagat caacgtacac accagttcga tctgtgcgga gcggccggag
360 cgctcgatgg cctcggccac ggcctcagcg cgctga
 396
   <212> Type : DNA
   <211> Length : 396
   SequenceName : SEQ ID 77:GDC_MTUB_868821 SequenceDescription :

### Custom Codon

Sequence Name : SEQ ID 77:GDC_MTUB_868821

### Sequence

<213> OrganismName : Mycobacterium tuberculosis-H37RV
<400> PreSequenceString : 
atgcggtgta gggcggcgtt gagctggcgg ttgcccgagc ggctgagccg catctggccg
60 gcggtgttgc ccgaccacac cgggatggga gccactgcgg catggcaggc gaaggcggct
120 tcgcttttga accgggtcac tccggcggct tcgccgacga ttttggctgc agtcagctcc
180 gcgcagccag ggatttccag cagtgcgggg gcgacctggt ggactcgggc gctgatgcgc
240 tgggctaggg tgttgatctc gccggtgagc cggatgatgt cggtcagctc ggcgcgcgcg
300 agttcggcga ccaatcctgg ctgggtgtcc agccaggtcc gcagggcctg ctggtgcttg
360 gcggcatcga gcgagcgtgc tgccggtgcc cgctcgggat cgagttcatg gacgagccag
420 cgcaaccggt tgatcgccga cgtgcgttgg gccacaagga catctcgacg gtcagtcaac
480 aacttcaact cccgcgacgt ctcgtcgtgg gtggccaggg gtaggtcggt ttcacgcatc
540 accgcccgcg ccaccgccag cgcatcgatc ggatccgact tgccccgact gcgcgccgac
600 ttgcgggtct gggccatcag cttggtgggt acccgcacca cctgctggcc ggccgccagt
660 aggtcacgct ccagacgcgc cgacatgttg cggcagtcct cgatgcccca gatcagctcg
720 aggccgaact gttcacgggc ccacatgatg gctgtggcgt gcccggccgt ggtggccttg
780 acggtcttct caccgagttg gcgacccact tcgtcggtgg ccacaaaggt gtggctgtac
840 ttgtgcgcat cggttccaac aacaaccatg gtggttgcct ctgaaccgcc ccggtga
897
 <212> Type : DNA
   <211> Length : 897
   SequenceName : SEQ ID 78:GDC_MTUB_890358 SequenceDescription :

### Custom Codon

Sequence Name : SEQ ID 78:GDC_MTUB_890358

### Sequence

<213> OrganismName : Mycobacterium tuberculosis-H37RV
<400> PreSequenceString : 
ttgcggcgcc gagccgctgt tcctgttgga ttacatcgcc gtcggtcgga tcgtgccgga
60 gcgactcagc gcgatcgtcg ccggtatcgc cgatgggtgc atgcgtgccg gctgtgcgct
120 gcttggcggc gagaccgcag aacatccggg cctgatcgag cccgatcact acgatatctc
180 tgccaccggc gtcggcgtcg tcgaggcgga caatgtgctg ggtcccgacc gggtcaaacc
240 cggcgacgtc atcatcgcga tgggctcgtc gggtctgcat tccaatgggt actcgctggt
300 ccgcaaggtg ttgctggaga tcgaccggat gaatctggcc ggtcatgtc[?] aggagttcgg
360 tcgcaccttg ggcgaagagt tattggagcc gactcgcatc tacgccaaag actgtttggc
420 cttggccgcc gaaacccgtg tccggacgtt ttgccacgtc accggcggcg ggctcgccgg
480 caacctgcaa cgggtcatcc cgcatggcct catcgccgag gtcgaccgcg gcacctggac
540 acccgcgccg gtattcacca tgattgccca gcgcggccgg gtcaggcgca cagagatgga
600 gaagacgttc aacatgggtg tcggcatgat cgccgtcgtt gcccccgaag acacgacgcg
660 cgccctggcc gtcctgaccg cgcggcacct ggactgctgg gtattgggaa ccgtctgcaa
720 aggcggaaaa caaggcccgc gggcaaaact ggttgggcag cacccgagat tctaagaacc
780 agacctaacc gggtctaa
798
 <212> Type : DNA
   <211> Length : 798
   SequenceName : SEQ ID 79:GDC_MTUB_904043

### SequenceDescription.:

### Custom Codon

Sequence Name : SEQ ID 79:GDC MTUB 904043

### Sequence

<213> OrganismName : Mycobacterium tuberculosis-H37RV
<400> PreSequenceString : 
gtggtagcgg tccggattga agtcgtcggc catcgagtcc accacctggc cggccatctt
60 gagttccgcg ggtttgatct ccaccttctg gtccagcacc gggaagtcgg ggtcgcggat
120 ctcatcgggc cacagcaacg tgtgcaccat catcacctct cgcttgccga aatccttgac
180 gcgcaacgcc gccagcctgg tcttgttgcg cagcgtgaaa tgcacgatcg ccatccggtc
240 ggtctcggcg agtgtcttag ccagcagcac atacgatttc gacgacttcg aatcaggctc
300 caaaaagtag ctgcggtcga acatcatcgg gtccacgtcg gcggcgggga cgaactccaa
360 cacctcgatc tcccggctgc gttcttcagg caagctggcg atgtcgtcgt cggtgatcgc
420 caccatttgg ccgtcgccgg actcgtaggc ccgggcaaga tcgcggtagt cgaccacctc
480 gccacacgcc tcgcagacgc gcttgtaccg gatgcgtccg ttgtccttgg cgtgcacctg
540 gtggaacctg atgtcgtggt ctgcggtagc gctgtacacc ttgaccggca cgttcaccag
600 cccgaaggcg atcgaacccg tccaaatggc tcgcatgtaa gtgagtatgc cttgattgtc
660 cgcgagcgga acgtcacggc gaaattccac gcgatatttg accgtgacgt tacgctcgcg
720 acttgtgtga ccgacaggct acgttga
747
<212> Type : DNA
<211> Length : 747
   SequenceName : SEQ ID 80:GDC_MTUB_1045383
   SequenceDescription :

### Custom Codon

Sequence Name : SEQ ID 80:GDC_MTUB_1045383

### Sequence

<213> OrganismName : Mycobacterium tuberculosis-H37RV
<400> PreSequenceString : 
ttgcgctcgg cgagggtgaa tccgccggcg cgcagtgcgg caagcacgcc atggtaccca
60 agcggatcgg tgaccaccgc cgcgctggga tggtttttgg cggcggcccg caccatcgcc
120 ggcccgccga tatcaatctg ctcgacgcag tcgtcgacac tggcgccgga ttcgacggtc
180 tggctgaacg gatacaagtt gactacaacg agttcgaaag cctcgatccc gagttgctcg
240 agggccgcgg cgtgctcgga cttgcgcagg tcagccagca gcccggcatg cactcgtggg
300 tgcagtgtct tgacccggcc atcgagcacc tcgggaaagc cggtcagctg ctccacgggg
360 gtcaccggaa tcccggtgtc ggcaatggtc ttggccgttg acccagtcga gatgatctcg
420 acgccggccg cgctcaggcc ctgtgccagg tctaccagcc cggtcttgtc gtacacgctg
480 atcagcgcac ggcggatcgg ccgtcttccg tcgtcggtgc tcatcctatg gttacctttc
540 gtcccatcgt cgctgttcgt ccgaccaccg tcacgccatg ggtggccagt gcggccaccg
600 ccgctaccaa cagccgtcgt tcggtga
627
 <212> Type : DNA
   <211> Length : 627
   SequenceName : SEQ ID 81:GDC_MTUB_1068100 SequenceDescription :

### Custom Codon

Sequence Name : SEQ ID 81:GDC_MTUB_1068100

### Sequence

<213> OrganismName : Mycobacterium tubereulosis-H37RV
<400> PreSequenceString : 
gtgcgcgctg acccgccgac gaccgcctgc aacacgcgat gcacgcccag cgtctgtgtc
60 ccgtcgatgt gcggtacatc gaccacctcg atgccgcccc gcagctgcgt cccggaaaaa
120 gtcaccttgc tgcagtcttt cccggggctg ggggccggca gcggctggga cgtctccacc
180 gcgatgacga cgaaccggtt gccgttgccc tcggcggaga cggcggccat gttgccctgc
240 aacccggtcg gcagctgggg cccggccgcc acttgcgcac agttcgccgg atcgaaactc
300 agcccgtcgg gcagtttgcg ggcggaaaag aacccgggat cgatggccct gggagtgaca
360 tcggtgacgg tgtattcagg tccaaagccc gacttcactt cggccacctt ggcgatgtcg
420 ccggtcgagg cggtggtgga gctggcccct gatgagcagc cgacaagcca gcacaccgat
480 ccgactgcca gtaccgcctt gcgcatcgtg gtcaatctac ccaacgcagc ccctgagctg
540 cgcaacgtcg acaccgtttt gactagcaga tcagcggcga actgcggtgc cagcggcgga
600 cgcaccgacc cggggtcggt gatcagccga cggcctcgat cacttgccg[?] gctacccggt
660 tga
663
 <212> Type : DNA
   <211> Length : 663
   SequenceName : SEQ ID 82:GDC_MTUB_1115707 SequenceDescription :

### Custom Codon

Sequence Name : SEQ ID 82:GDC_MTUB_1115707

### Sequence

<213> OrganismName : Mycobacterium tuberculosis-H37RV
<400> PreSequenceString : 
gtgggtactg cgcaagagcg agtccgaagc cgatcaggcc cggttccgca ccacgctcta
60 cgtcacctgc gaggtagtcc gcatcgcggc actgctgatc cagccggtga tgccggagtc
120 ggccggcaaa attttggacc tgctcggcca ggccccaaac cagcggtcgt tcgccgccgt
180 aggtgttcgg ctgacccccg gcacagcgct gccgccgccc accggggtat ttccccgcta
240 ccagccgccg caaccacccg aaggcaagtg agcggaccgc agcgacggga aagccaccta
300 cgaagcgttg accgcggtct gcgcgtcgcg tgggatgtcg agcgtggcga cgggataaaa
360 cccggaatcg tcgcggccgt cgcgggacaa cagcatgggc ggatagttca ccacatggga
420 gccgttcggt ttgtgctgtt gccagtcgat cgcggcccgc agcgtgtagt ggcccgcggg
480 caagccggac agatcaacgc gaaccgtctc ggcgaccgac gccggtgtcg gctggtcgct
540 gctgcgatcg ccgcgctggt cggagaccag cgtcttcagg tccaccgctg ccggcagcgt
600 ccgaaccacc tgtccggtgg aatccaccag ccggtagccg ggcacccact tttcggtggc
660 ggcagcagcg ccgtagttgg tccaggtgac cgagatcgtc gcgaccttgc ccgctag
717
 <212> Type : DNA
   <211> Length : 717
   SequenceName : SEQ ID 83:GDC_MTUB_1124996 SequenceDescription :

### Custom Codon

Sequence Name : SEQ ID 83:GDC_MTUB_1124996

### Sequence

<213> OrganismName : Mycobacterium tuberculosis-H37RV
<400> PreSequenceString : 
atgtcgatct ccggaatcga gcgctggtcg gctaccgaga acatccgcat ctcggtgatc
60 tcgtcgcccc agaactcgac ccgcaccgga tgttcggccg tcggggcaaa gatgtccaga
120 atcccgccgc gcacagcgaa ctcgccgcgc cggccgacca tatccacccg ggtatatgcc
180 agctcgacca gccgcgccac cacgccgtcg aagggggatt cgtcgccaac ggtcagcgtg
240 aggggctcca tcatgcccag ctgcggcgtc atgggctgca gcagcgagcg caccgaggtc
300 accactaccc ccagcggtgg gcccagctgg gcatcgtcgg ggtgggccag ccggcgcagc
360 gccatcaggc gagtgccgac ggtgtcaaca ccgggtgaga gccgttcgtg cggcagtgtc
420 tcccaggacg gcaacaacgc caccgcatcc ccgaacacac cacgcagttc ggcggccagg
 
480 tcgtcggctt cccgcccggt ggcggtgacc accagcaatg gcccctgccg agccagcgca
540 ctggcgacca acagccgcgc gctggccggc gcgatgagcg tcaattcgtc gggtcgaccc
600 ccggcgcgct gcatgagctg ttggaatgtc ggcgcgctca gcgccaatt[?] gacgagcccc
660 gcgatcgggg tatctgagca ggcaggcccc ggtgcggtca tgatgcggcc attctag
717
 <212> Type : DNA
   <211> Length : 717
   SequenceName : SEQ ID 84:GDC_MTUB_1138949 SequenceDescription :

### Custom Codon

Sequence Name : SEQ ID 84:GDC_MTUB_1138949

### Sequence

<213> OrganismName : Mycobacterium tuberculosis-H37RV
<400> PreSequenceString : 
gtgctggcgt tctaccttcg gccaaggcca gggacgtggt gtacgagtga aggttcctcg
60 cgtgatcctt cgggtggcag tctaggtggt cagtgctggg gtgttggtgg tttgctgctt
120 ggcgggttct tcggtgctgg tcagtgctgc tcgggctcgg gtgaggacct cgaggcccag
180 gtagcgccgt ccttcgatcc attcgtcgtg ttgttcggcg aggacggctc cgacgaggcg
240 gatgatcgag gcgcggtcgg ggaagatgcc cacgacgtcg gttcggcgt[?] gtacctctcg
300 gttgaggcgt tcctgggggt tgttggacca gatttggcgc cagatctgct tggggaaggc
360 ggtgaacgcc agcaggtcgg tgcgggcggt gtcgaggtgc tcggccaccg cggggagttt
420 gtcggtcaga gcgtcgagta cccgatcata ttgggcaaca actga
465
 <212> Type : DNA
   <211> Length : 465
   SequenceName : SEQ ID 85:GDC_MTUB_1170285 SequenceDescription :

### Custom Codon

Sequence Name : SEQ ID 85:GDC_MTUB_1170285

### Sequence

<213> OrganismName : Mycobacterium tuberculosis-H37RV
<400> PreSequenceString : 
ttgacgaccg ctggcataag cgggtcaaag ggccggacgg gaacaggcga accgtgcggt
60 ctgctgtctg cggcagggtt tcgcgctggc gcgtcaggtg ggttgacggc ggcggagagg
120 agcacagcaa gagcttccag cgcaaacctg acgcgcaggt acctgaccca tgccgaactg
180 ttgatgctcg ccaggcccac gggccggttc gaaacgctca ccttggtgct cggctactgc
240 ggcttacggc ggtttacggt tcggtga
267
 <212> Type : DNA
   <211> Length : 267
   SequenceName : SEQ ID 86:GDC_MTUB_1176592 SequenceDescription :

### Custom Codon

Sequence Name : SEQ ID 86:GDC_MTUB_1176592

### Sequence

<213> OrganismName : Mycobacterium tuberculosis-H37RV
<400> PreSequenceString : 
atgggtcagt gcccacgacc tgtgcggcac tggccgcctg ccgtaattgt ttgtagccga
60 actaaattgc ggcgcgcctg cctgcgcgac taccgccgtc ccgcccctc cgacaagaag
 
120 cccaacaagt cgtaccgggt aatgacccca accggcttgc cttcctccac caccatcaac
180 gcatcccaat cacgcaacgc cttgccggcc gcactgacca attcaccggc gcctatcatc
240 cgcagcggcg ggctcatgtg tgccgacacg gcgtcggcca acttggcgcg gccctcgaac
300 acggccgaga gcagctcgcg ttccgagacg ctaccggcga cctcgccggc catcaccggc
360 ggctcggcgc cgaccaccgg catctgcgac accccgtact cgcgaagaat cccgatggcg
420 tcgcgcacgg tctccgacgg atgggtgtgc accagggcgg gcagcgcgcc ggacttgcgg
480 cgcaacacat caccgacggt ggattgctcg gtcgacccgt caaggcggct gcgcaggaac
540 ccatag
546
 <212> Type : DNA
   <211> Length : 546
   SequenceName : SEQ ID 87:GDC_MTUB_1202653 SequenceDescription :

### Custom Codon

Sequence Name : SEQ ID 87:GDC_MTUB_1202653

### Sequence

<213> OrganismName : Mycobacterium tuberculosis-H37RV
<400> PreSequenceString : 
ttggcggcga tcccgagaag gtcacgctgt tcggtgaatc cgcgcgggaa tcgtcacgac
60 cctgctcgcc accccggcgg ccgcgggtct gttcgcggcg gcgatcgccc agagctcacc
120 ggcgacatcg gtctacgacc aggtgagggc tcggcgcgtc gcggtttgcg tcctcgacaa
180 gctgggaatc gacccgtccg atgtgcacag gttcatgaag tgccgaccgc ggcaatcctt
240 tccgcgtcca gcgaagtgtt caacgaagtg ccggttcgta accccggcac gctggcgttc
300 gtcccgatcg tcgacggcga tctgctgccc gactacccgg tcaagctggc gcaggagggc
360 cgctcacacc cggttccctt gatcatcggc accaacaagc acgagtcggc gctctttcgg
420 ttgatgcgct cgccgctgat gccgatcacc ccgcgcgatc acgtcgatgt tcacccagat
480 tgccgccgaa cagcccgatc tgcaagtgcc aaccgaggag cagatcggct ccgcgtactc
540 gcgatggcgg cgcaaagcac gctcattgag tatggctacc gacgtcggct tccggatgcc
600 gtcggtgtgg ctcgctga
618
 <212> Type : DNA
   <211> Length : 618
   SequenceName : SEQ ID 88:GDC_MTUB_1231843 SequenceDescription :

### Custom Codon

Sequence Name : SEQ ID 88:GDC_MTUB_1231843

### Sequence

<213> OrganismName : Mycobacterium tuberculosis-H37RV
<400> PreSequenceString : 
gtgctggcct tgaggcccca gcgtcatttc acccagagcc ggagcgcccg gcggctacgc
60 tgtgtgctcg acgatgacgt atgggtgccc tgggcacggt cagggggttg caggacagca
120 acacggcatt tgtcggtgcg ctgcatagcg ggaacctgtt gggggccacc ggtgcggttc
180 tgcaggctcc gggcaacgcc gtcaacggtt tcttgttcgg ccagacgtcg atatcgcagt
240 cgattgacgt gtcaccggag tacggatacg agttggtcgc tgtcagcgac ccggttggcg
300 gaactgctgg ctccgctcga gccggtcacg gttacgttca cgccgacctt cggtgaaccg
360 gacatggtcc atctgagtgg cacgaagttc gggggccttg tcccggccct cttcgaaggg
420 gtgcgcgccg gcttctaa
438
 <212> Type : DNA
   <211> Length : 438
   SequenceName : SEQ ID 89:GDC_MTUB_1241031
   SequenceDescription :

### Custom Codon

Sequence Name : SEQ ID 89:GDC_MTUB_1241031

### Sequence

<213> OrganismName : Mycobacterium tuberculosis-H37RV
<400> PreSequenceString : 
atgaccagct cagcaccgaa gcccgcggcg tcgcgcgcat cggactggcc aactacttcg
60 ccggcgcctt cctgctcccc taccgcgaat tccaccgtgc cgcagagcag ttacgctatg
120 acatcgacct gctgggccgc cggttcggag tgggcttcga aaccgtctgc caccggctct
180 ccacactgca gcgcccgcgg cagcgaggga taccgttcat cttcgtccgc accgacaagg
240 ccggaaacat ctcaaagcga cagtccgcga cggcgtttca cttcagccgg gtcggcggca
300 gctgcccgct gtgggtggtc cacgacgcgt tcgcccagcc agagaggatc gtccgccagg
360 tggcgcaaat gcccgacggc aggtcgtact tctgggtggc caagaccacc gctgccgacg
420 ggctcgggta tctgggcccg cacaagaact tcgcggtcgg gctgggctgc gacctcgcgc
480 acgcccataa actcgtctac tccaccggtg tcgtcctgga cgacccgagc acggaggtcc
540 cgatcggggc gggctgcaag atctgcaacc gaacgtcgtg cgcccaacgt gcgttcccct
600 atctcggtgg tcgcgtcgcg gtcgacgaga acgcgggcag cagcttgcct tattcgtcga
660 ccgagcaatc ggtttgaccg cccgacgcca cagcagacaa cgaaacccct tatattactg
720 tggtttcagc aggctctggg caagcattgt tgtcggtgcc tgcacatagc attcagtcat
780 gtgttccact cgggaggaga tcacggaggc cttcgcgtca ttggctaccg cgctgtcccg
840 cgtgctgggg ctgacctttg a
861
 <212> Type : DNA
   <211> Length : 861
   SequenceName : SEQ ID 90:GDC_MTUB_1252888 SequenceDescription :

### Custom Codon

Sequence Name : SEQ ID 90:GDC_MTUB_1252888

### Sequence

<213> OrganismName : Mycobacterium tuberculosis-H37RV
<400> PreSequenceString : 
atgcagcttg gcaatcaaaa cactatgaga ttcgcagggc ggcctcagcg ttttcgccaa
60 agcgcttacc ccctgttcaa ccccaacagc gcgatcgcgc ttggccaccc attcggcggc
120 tcgggggcac ggttgatgac tacagtgcta caccacatgc cggacaaggg aattcgctac
180 ggcttacaga cgatgtgcga gggccgcggc caagccaatg ccaccattgt ggagttgctg
240 tga
243
 <212> Type : DNA
   <211> Length : 243
   SequenceName : SEQ ID 91:GDC_MTUB_1264312 SequenceDescription :

### Custom Codon

Sequence Name : SEQ ID 91:GDC_MTUB_1264312

### Sequence

<213> OrganismName : Mycobacterium tuberculosis-H37RV
<400> PreSequenceString : 
gtgacggtat accgtcgagg tatggctgtg ttaacggatg agcaggtcga cgccgcactg
60 cacgacctca acggctggca gcgcgccggt ggtgtcctgc gtaggtcaat caagtttccg
120 acgtttatgg ccggtatcga cgccgtacgc cgggtggccg agcgagccga ggaggtaaat
180 catcatccgg acatcgatat ccgttggcga acagtaactt tcgcgctggt tacgcatgcg
240 gtaggtggta tcacggaaaa cgacattgcg atggcgcacg atatcgacgc aatgtttggg
 300 gcctaa
   306
   <212> Type : DNA
   <211> Length : 306
   SequenceName : SEQ ID 92:GDC_MTUB_1286282
   SequenceDescription :

### Custom Codon

Sequence Name : SEQ ID 92:GDC_MTUB_1286282

### Sequence

<213> OrganismName : Mycobacterium tuberculosis-H37RV
<400> PreSequenceString : 
gtgggtgcag tacggcttca acctcaccgc atgggcggtg ggatggctgc cctacatcgg
60 catactggca ccgcagatca acttcttcta ttacctcggc gagcccatcg tgcaggcagt
120 cctgttcaat gcgatcgact tcgtggacgg gacagtcact ttcagccagg cactaaccaa
180 tatcgaaacg gccaccgcgg catcgatcaa ccaattcatc aacaccgaga tcaactggat
240 acgcggcttc ctgccgccgt tgccgccaat cagcccgccg ggattcccgt ctttgcccta
300 acttcggact ag
312
 <212> Type : DNA
   <211> Length : 312
   SequenceName : SEQ ID 93:GDC_MTUB_1301742
   SequenceDescription :

### Custom Codon

Sequence Name : SEQ ID 93:GDC_MTUB_1301742

### Sequence

<213> OrganismName : Mycobacterium tuberculosis-H37RV
<400> PreSequenceString : 
atgccttcgc cggtgagcag cggaccgacc agccatggca caaacaaggg gtgcgggttg
60 atcaggtctg agtcgatgaa caccacgatg tcgccgctgg tggccgccag tgaacgccac
120 aatgcctcac ctttgccggg ccgtaccggc acctcgggca acgcctgttc acggctgaca
180 acccgggcgc cggaggcgat ggcccggatc tcggtgtcgt cggtggaac[?] ggagtccagc
240 acgatcaatt catcgaccag gccatcgacc agcggagaga tgctgtcgat caccgattcg
300 atggtcgctt cctcgttgag ggccggcagc accaccgaaa tcgtccgtcc ggcctttgcc
360 gcttccaact ccccgatcgt ccagccggga cggtgccaag tagtgtccaa gggcagcgcg
420 ccaggggccc tgccaccggc gagatcgccg gcgaccagct ccgatgctgt catgcgagtc
480 ctctcaccgt gcgcgtcggc ggccggaccc cctgaatcga tgccaccatt tccagcaccc
540 gccgggtggc ggcgacctca tgcacccgaa acatgcgcgc cccggcggcc gcagccaacg
600 cggtggctgc cagcgttccc tcaagccgtt cggtcaaatc cacgcccaga gtctccccga
660 caacgtcctt gttgctcaaa gccatcagca cgggccaccc ggtcataa
708
 <212> Type : DNA
   <211> Length : 708
   SequenceName : SEQ ID 94:GDC_MTUB_1351907 SequenceDescription :

### Custom Codon

Sequence Name : SEQ ID 94:GDC_MTUB_1351907

### Sequence

<213> OrganismName : Mycobacterium tuberculosis-H37RV
<400> PreSequenceString : 
atgctttcag cggttatcct gaccgaacgt ggctatccag cggtgcccct ggcgggacaa
60 ctggtgcacc agaggttcgt ccgtcccggt cctctcgtac tagggacagg tttcctcaag
120 tttctgacgc gcgcggcgga tagagaccga actgtctcac gacgttctaa acccagctcg
180 cgtgccgctt taatgggcga acagcccaac ccttgggacc tgctccagcc ccaggatgcg
240 acgagccgac atcgaggtgc caaaccatcc cgtcgatatg gactcttggg gaagatcagc
300 ctgttatccc cggggtacct tttatccgtt gagcgacacc ccttccactc gggggtgccg
360 gatcactaa
369
 <212> Type : DNA
   <211> Length : 369
   SequenceName : SEQ ID 95:GDC_MTUB_1476279 SequenceDescription :

### Custom Codon

Sequence Name : SEQ ID 95:GDC_MTUB_1476279

### Sequence

<213> OrganismName : Mycobacterium tuberculosis-H37RV
<400> PreSequenceString : 
ttggtgggac gcagccgcgt actcgtcctg ttcggagcgg gtgaacatgt cgacgtcgtt
60 gcgttgctcg gtgagcgcgc ccatcggctg atcggtgaac acgtcgtgca gaccgtcgta
120 ggccatgtgg tccaaaaccg taacgtcgcc gtacttgtaa cccgaccggc tattcatcaa
180 caggtggggc gccttcgtca tcgactcctg accgccggcc accaccacgt cgaactctct
240 ggcccgaatg agttgatcag ccagcgcgat tgcgtcgatg ccggacaggc acatcttgtt
300 gatcgtcagc gcagggacat cccaaccgat gccggccgcc actgccgcct gccgtgcggg
360 catttgcccg gcacccgcgg tcaacacctg gcccatgatc acgtactcga ccaaggacgc
420 cggcacgttg gccttctcca gggcgccctt aatggcgatg gcacccagct cgctggcgct
480 gaaatccttc agggagccca tcaacttgcc gatgggtgta cgcgcgccag caacaatcac
540 cgatgtcgtt atgactacct cctcagcgca cccgaaagcc gatctgaccg acccggagaa
600 gcagattctt tcccttcagg ttaccgttgt gtgatgacga ccgatcaagt ccacgcccgt
660 cacatgctgg ctacctcgtt ggtaactgga ctcgatcacg tcggtattgc ggtcgccgac
720 ctggacgttg ccatcgagtg gtatcacgac caccttggca tgatcctggt ccacgaggaa
780 atcaacgacg atcagggcat ccgcgaggca ctgctggcgg tgccgggctc cgcggcgcaa
840 atccagttga tggccccgct cgacgaatcc tcggtgatag cgaagttcct ggacaagcgc
900 gggccaggca tccaacagct ggcgtgccgg gtcagcgatc ttgacgccat gtgtcggcgg
960 ctgcgctccc agggcgtccg gctggtctac gagacggcca ggcgtggcac cgcgaactca
1020 cgga
1024
 <212> Type : DNA
   <211> Length : 1024
   SequenceName : SEQ ID 96:GDC_MTUB_1485311 SequenceDescription :

### Custom Codon

Sequence Name : SEQ ID 96:GDC_MTUB_1485311

### Sequence

<213> OrganismName : Mycobacterium tuberculosis-H37RV
<400> PreSequenceString : 
ttgcgcgcgg caacaaagtc gccatcctcg agctgctggc gcgcctgtgc caccgctgga
60 tcgacttcgg tggactcctc ggaactcgct gcgcccttga gctttccggc tgtcgcagac
120 aacagggaat ccacccagcg actcagttgg tccgcgggct ggaggccctg gaagctcgag
180 atcggctgtc ccgcagccaa ggccaccacg gtcggaaccg cttggacgcc gaatatctgt
240 gccaccctgg gtgcgacgtc aacgttaacc gacgccagcg accacttgcc cttagcggca
300 gcggccaagc cggacagcgt gtcaagcaag tcgacgcata cctcgctgcg gggtgaccac
360 agcaacacca ccaccggcac ttcgtcggac cggacgatca cctcgtcctc gaagttcgcc
420 tcggtgatct cggtcacacc ggacggcgtc gacagtgccc ggtcggcatc cgtgctcgcc
 
480 gcagcgtttt gctgggcacg ttgtttgatg ccggagaggt caacagcacc ggccatggcc
540 ggcccgagcg ggggtcgcgg acgcgtcacg ccgtcaagtc tgtcatgccg ctgcggtcat
600 cgatccaccc ggtggcgccg accctgcggc aggagccgac ataccgcgat cggttggtat
660 gaccaagatc acactggccg ccaccgaccc ctcaaccgct atccggcccg caatatcagt
720 gcgtcgccct gcccgccagc cccgcacaat gcggcaaccc cgacgcccga tccccggcgt
780 gccaactgca gcgccgcatg tagcgtgatt cgcgtccctg acatgccgag gggatgcccg
840 acggcaatcg caccaccgtt gacgttgacg atctgggggt tcagcccgag ttcgcgtatc
900 gaggccaatg ccaccgcagc gaacgcctcg ttgatctcca ccacgtcgag ctggtccacc
960 gagatgccct cgcgatccag cgccttgttg atcgcgttgg ccggctgcga ttgcagtgtg
1020 gaat
1024
 <212> Type : DNA
   <211> Length : 1024
   SequenceName : SEQ ID 97:GDC_MTUB_1486309
   SequenceDescription :

### Custom Codon

Sequence Name : SEQ ID 97:GDC_MTUB_1486309

### Sequence

<213> OrganismName : Mycobacterium tuberculosis-H37RV
<400> PreSequenceString : 
gtgcggtcac ggcgtctagc acccacccgg ccacggtcgc ggcggacagc cagcccagcc
60 acagccacgc gcgctgcggc gcctccccga acaacgccgc catcagcggc accagcaaca
120 cggtgcccac cgctcgcgcg acaacggaac aaaacgcgag cagcgcaaag ccgattagcc
180 tggcgcggtg gtcgttcgga acaagggcta tccaggtgcg gatcatcggg tgccgtcctg
240 cgctgcggcg accgccaccc ggctgccctg gccggtgtcc cacagccggc agtagcgtcc
300 gcccgcggca agcaactcct cgtgggtgcc gcgttcgacg atccgaccat gatcgagcac
360 gacgatctgg tcggcccggg tgatggtatg cagtcgatgg gcgattacca gcacggtgcg
420 gtcccgggtc agccggttaa gcgcctgttg cacaaggtat tccgattccg gatcggcaaa
480 cgcggtggcc tcgtcgagga tgaggaccgg agtgtcgccg aggatggcac gggcaatggt
540 gagccgctgt cgctccccgc ccgaaagacc actgttggct ccgagcacgg tatcgtagcc
600 gtccggcagc cgaagcaccc ggtcgtggat ttgcgcttcg cgggccgcga cctggacctg
660 ttcggcgggg gcatccggta ccgccagcgc gatgttttcg gcggcggtgc catgcacaag
720 ctgggcttcc tgtag
735
 <212> Type : DNA
   <211> Length : 735
   SequenceName : SEQ ID 98:GDC_MTUB_1515112
   SequenceDescription :

### Custom Codon

Sequence Name : SEQ ID 98:GDC_MTUB_1515112

### Sequence

<213> OrganismName : Mycobacterium tuberculosis-H37RV
<400> PreSequenceString : 
gtgagcgcgg tattggcttt gtctgctgcg gtatcggcac gccgcgcaaa ggctgcggag
60 gcccacagcg cccccagcag caacggcacg ccggccagtg cagccacgcc gagctgccag
120 gagatcggca acagggccag cgcgatcact gccggcagca ggatcgcgct ggtcaacggt
180 gtcaccagat taaccaccag gccaacaagt tccggcccgg tggccgcgat cgcctgccgt
240 gccgtcgcgg tgttttcggc ggtaaaccaa tccaaccgga caaccggaag ccggtccgcc
300 acatcatgtt gggtgtggtt aaggacggcg aaacccagct cgataccgat gcgtgcggtc
360 acggcgtcta gcacccaccc ggccacggtc gcggcggaca gccagcccag ccacagccac
420 gcgcgctgcg gcgcctcccc gaacaacgcc gccatcagcg gcaccagcaa cacggtgccc
480 accgctcgcg cgacaacgga acaaaacgcg agcagcgcaa agccgattag cctggcgcgg
540 tggtcgttcg gaacaagggc tatccaggtg cggatcatcg ggtgccgtcc tgcgctgcgg
 
600 cgaccgccac ccggctgccc tggccggtgt cccacagccg gcagtagcgt ccgcccgcgg
660 caagcaactc ctcgtgggtg ccgcgttcga cgatccgacc atgatcgagc acgacgatct
720 ggtcggcccg ggtga
735
 <212> Type : DNA
   <211> Length : 735
   SequenceName : SEQ ID 99:GDC_MTUB_1515464
   SequenceDescription :

### Custom Codon

Sequence Name : SEQ ID 99:GDC_MTUB_1515464

### Sequence

<213> OrganismName : Mycobacterium tuberculosis-H37RV
<400> PreSequenceString : 
atgccatcgg tcattcgcga cccagatccc ggtgcagcgc ccgcaccgac agttgctgat
60 cggagcgcag aagtcccatc agtgcttcag cgatcgcgac gctgcgatgc ttaccaccgg
120 tacagccgat ggcgattgtc atatagcgct tcccctctcg gcggtagccg tcgacaacca
180 gggatagcaa ccgatggtag gactcgagga actcagccgc gcccggccgg tgcagcacat
240 agtcgcgcac ggccggatgt tggccggtca gtggccgcaa ctcgtccacc cagtgcgggt
300 tcggcaggaa ccgcacgtcc atga
324
 <212> Type : DNA
   <211> Length : 324
   SequenceName : SEQ ID 100:GDC_MTUB_1596569 SequenceDescription :

### Custom Codon

Sequence Name : SEQ ID 100:GDC_MTUB_1596569

### Sequence

<213> OrganismName : Mycobacterium tuberculosis-H37RV
<400> PreSequenceString : 
gtgctacggc ccatacgggc gggccaacct ggccgacatc tggcgccgcc gcgacctgcc
60 acgcgacgcc aaggcaccgg tgctggtaca ggtgcccggc ggcgcctggg tactggggtg
120 gcgccgcccg caggcgtatc cgttgatgag ccatctggct gcgcgcggct gggtatgcgt
180 gtcgctgaac taccgggtgt cgccgcgcca cacctggccc gaccacattg tcgacgtgaa
240 gcgcgcgctg gcgtgggtca aggaaaacat cgccgcctac ggcggggatc cgaatttcgt
300 tgccatcagc ggcggttcgg ccggcggcca tctgtgcgcc ctggcggcgt tgacccccaa
360 cgatccgcga tttcagcccg ggttcgaaca ggtcgacacc tcggtggcgg cagcggttcc
420 ggtatacggg cgttacgact ggtttacgac cgatgcgccg gggcgtcggg aattcgtcgg
480 gttgctcgaa acgttcgtgg tgaaacggaa attcagcacg caccgcgaca tcttcgtcga
540 tgcctcaccg atccaccatg tgcgggccga cgccccaccg ttcttcgttc tgcacggccg
600 ccacgactcc ctgatccccg tggccgaagc ccatgcgttc gtcgaggaac tgcgggcggt
660 gtcgaagtcg cccgtcgcct acgcggacct gccccacgcc caacacgcct tcgacgtctt
720 cggctccccg cgggcgcatc acaccgccga ggccgtggcc cgcttcctgt cttgggtgta
780 cgcgaccaac ccgccggcca cgtagtcagc tataggccag ctattgctat tccgcggcac
840 gctccagctc ggccagtgcc ggttcgatgg catcggccat ctcgtcgatg tcgttggcca
900 cctcgggtgt ggtcaccagg ccgaaatcca gataatcctg gtaggagaag caggtga
957
 <212> Type : DNA
   <211> Length : 957 SequenceName : SEQ ID 101:GDC_MTUB_1600905
   SequenceDescription :

### Custom Codon

Sequence Name : SEQ ID 101:GDC_MTUB_1600905

### Sequence

<213> OrganismName : Mycobacterium tuberculosis-H37RV
<400> PreSequenceString : 
atgacggcca gcaggcgctc ggaccacacg gacgcgacgc gtcgagccct cgtcgacgct
60 ggccgttacc tattcgcgcg gcgcgactat ggtgacgtct cgatcgaaga catcgtcacc
120 cgtgcccgag tcacccgtgg cgccctggac taccacttcg acagcaagaa agatctgttc
180 cagacggtac tcgaggttgt cgaagccgac ctggtcgccg acgtcgaagc cgccatagcg
240 aaggtcaccg acgcctggat ctgctggtcg tcggcttcca cgccttcctt gacgcggcga
300 ccaaaccgga tgcgctgcag gtcattgcga ttgacggccc gtcagtgctc gggtggggcg
360 aatggcgccg gatcgacatg cgctagggct tggtctgctg gtcggggctc tcgaacgcgg
420 gatggccgcc ggggtgattc agcgcgtacc gttgccacca ctttcgcatc tgctgctggc
480 cgcgctaacc gaatccgcgc tgcagatcgc ggacgcgacg gacaaagacc ggaccagagt
540 cgaggtcgaa cgcgcattta tggccctact cgaaggtcta cgggtgtagc acgcccgcga
600 tccgctacgg caacggacca ccggccgcaa tcgcggccag cgtcgcgaaa tgctccccgt
660 ccagcgacgc cccgccgacc aggccaccat cgacgtcatc ctggccacgg atgtcgccga
720 cgtttttggc gttcaccgag ccgccgtaga gcacccgcac cgtatcggca atcctcggcg
780 aggccaacga ggccaactct tttcggatcg ccgcacacac ctcctggcgg tcggcggcgc
840 tggccacccg cccggtgccg atcgcccaga ccggttcgta ggcgatga
888
 <212> Type : DNA
   <211> Length : 888
   SequenceName : SEQ ID 102:GDC_MTUB_1616064
   SequenceDescription :

### Custom Codon

Sequence Name : SEQ ID 102:GDC_MTUB_1616064

### Sequence

<213> OrganismName : Mycobacterium tuberculosis-H37RV
<400> PreSequenceString : 
gtgcggttac gctcggaaag cgcgggcctc gcccacgcgg cggatgatgt cagcggggtg
60 gtcctcggcg acgacccgga ccacgatcca cccgtagcgg tgctggactt tctcgtgccg
120 gaggatgtct ttccggtagt ggtagcgact ggtcagatgg tggtcgccgt catactcggc
180 cgcgaccttg atgtcttgcc agcccatatc caaatggcct tccgcccagc cccattcgtt
240 gcgcaccgcg atctgcgtct gggggcgcgg aaagccggcg cggatcaaca acaagcgcag
300 ccaggtttcc ttgggggact gggcaccgcc gtcgacgagg tccagagcgg ctcttgcggc
360 cttcatgcca cggcggcccc gatagcgctc gatcagcggc tcgacgtcgg ccaccttcaa
420 atcggtggcc tgtatcaggg cgtcgacggc cgcgacggcg gggtccaatg gaaatcgact
480 ggtcaggtcg agcgccgttc gctccggtgt ggtcacgcgc atgccctcga tgacgcagat
540 ctcgtcgggc tcgatgcgct cttcccagac ttgcagcccc ggggcacggc ggcggttggt
600 gtcgatgatc gcggcgggaa gatccgcgtc gatccacttg gcgccatgga aggcagaagc
660 cgagtagccg gccagcacgc cgcggcggcg cgagcgcagc cacagcgctt ttgcacgcaa
720 ttgcgcggtc agttccacac cctgcggcac gtacacgtct ttatgtag
768
 <212> Type : DNA
   <211> Length : 768
   SequenceName : SEQ ID 103:GDC_MTUB_1672449 SequenceDescription :

### Custom Codon

Sequence Name : SEQ ID 103:GDC_MTUB_1672449

### Sequence

<213> OrganismName : Mycobacterium tuberculosis-H37RV
<400> PreSequenceString : 
ttgggtgtgc gcgccgccgt cggcgtagat gatgtcaccc gtggtcgccg gcagccagtc
60 agacagcagc gcgcacaccg tcttggcgac cggcgtcgca tccttcatgt tccagccgat
120 cggagcgcgc tgatcccagc cctcctcgag cagctggatc tgggcgccgg cctcctcgcc
180 gagcgcaccg ccgacgatcg cactcatcgc cagcgtccgg atagggcctg cggcaacgag
240 attcgaacgc acaccgtact tgccggcctc gcgcgccacg aacctgttga ccgactccaa
300 cgcgctcttg gcgaccgtca tccagttgta ggccggcatc gcccggctcg ggtcgaagtc
360 catgccgacg atggaacctc cggggttcat gatcggcagc agcgccttgg ccatcgaagc
420 atacgaatac gccgagatgt ggatgccctt ggacacatcc gcgtagggcg cgtcgaagaa
480 cgggttgatg cccatcccgg tctgcggcat gaacccaatc gaatgcacca ccccgtcgag
540 cttgttgccc gccccgatcg cctcggtcac ccggccggcc aagctggcca ggtgctcctc
600 gttttgcacg tcgagttcga gcagcggggc ctttgccggc agccggtcgg tgatgcgctg
660 aatcagccgc agccggtcga acccggtgag caccagctgg gcgccctgct cctgggctac
720 ccgtgcgatg tgaaacgcga tcgacgagtc ggtgatgatt ccgctaacca gaatccgttt
780 gccgtccagc agtcctgtca tgtgcgtcct tgtgttgtgt cagtggccca tacccatgcc
840 gccgtcgacc gggatgaccg caccggagat atagctcgca tcctcggaag ccaggaagct
900 gaccaccccg gcgacctcgg cgggggtgcc gacccgcttc gctgggataa attgcagcgc
960 cccctgctga atccgctcat ccagcgcgcg ggtcatatcg gtgtcgatgt agcccggggc
1020 cacc
1024
 <212> Type : DNA
   <211> Length : 1024
   SequenceName : SEQ ID 104:GDC_MTUB_1673708
   SequenceDescription :

### Custom Codon

Sequence Name : SEQ ID 104:GDC_MTUB_1673708

### Sequence

<213> OrganismName : Mycobacterium tuberculosis-H37RV
<400> PreSequenceString : 
atggtgccga gcatgagggt gcgctcggat tgggagccga tcgcccagag ccgctcccgg
60 ctcgcggtca cggcaccgcg caacacctcc gggggtcgct tcatctggat tctcctcggt
120 tctgcgcgaa acggtagcag agcgccatgg ttgccaacgc ggtcgccggg cagtctagac
180 cggatcttcc tcgtggcaac cgacaacagg acgtcgttgc cgaaagggcg ctgggcaccg
240 acatctagga tgaacccaca gccacgcccc gacgttatgc catggcgaag agcgaccggc
300 aggagcggga acccagtgaa gcgagcgctc atcaccggaa tcacaggacc ggacggctcg
360 tatctcgcta agctcccgct gaagggatat gtggccgctg gtagcccggc cgaggtctat
420 ttctgctggg cgacacggaa ttatcgcgaa ttgtatgggt tgctcgcggt caacagcatc
480 tggttcaatc acgaatcacc gcgtcacggc gagacattca tgactcgtaa tcctgcacca
540 tatcgcggtc ggcaacgagg cgctgatcga tgcgcagacg ctgatgcgcc ggcccacccg
600 gataggtatc agtattgggg cgttccggcc agcgtacgag gcgtgatcga ccgcgcaatg
660 ggtgtttgcg ttgagtaa
678
 <212> Type : DNA
   <211> Length : 678
   SequenceName : SEQ ID 105:GDC_MTUB_1699549 SequenceDescription :

### Custom Codon

Sequence Name SEQ ID 105:GDC_MTUB_1699549

### Sequence

<213> OrganismName : Mycobacterium tuberculosis-H37RV
<400> PreSequenceString : 
ttgagcggtc agccatcggc tttgcgccga cctacggtgt ccccgtcggc gtgtcgccga
60 cctacggtgt cgaagtcaaa gccaaagatc gacaggatga ccagcaggat ggcgccaccg
 
120 actaccgacg gatcggcgac attgaacacc ggccaccagc cgaccgacaa gaaatcgacg
180 acgtgcccgc gcagcggccc cggtgcccga aagaagcgat caaccaggtt gcccatggca
240 ccgcccagga tcatcccaag acccagcgcc caccacggcg ataccagccg ccgccccatc
300 cagaaaattc cgaccacgac acccgtcgca atcagcgtca aaacccaggt gtatccggtc
360 gccatcgaga aggccgcccc agaattacgc accagagtcc aggtcaccgt gtcgccgata
420 atcgacaccg gctggccggg cggcaacagt tggacagcta ccaccttggt gacaatgtcg
480 agtgtgagca ccaccacagc gaccgacagc agcatgcgca gccgtcgcgg cggcgcggga
540 gcgttaggtt cccccgcccc cccggcttcc tcggtcgagg tcagcggatc agccgatcct
600 gttggttcgt caggcacacc atcatcatcc cctagggccg atatggcccg cccagacccc
660 gcggccggat gggagcaaac cacgtgcgca atgatcccat catggcccgc ctcaccgtca
720 tcactactgg agggacaatc tcgaccaccg ccggccccga tggggtgcta cggccaaccc
780 attgcggggc gacgctga
798
 <212> Type : DNA
   <211> Length : 798
   SequenceName : SEQ ID 106:GDC_MTUB_1742061
   SequenceDescription :

### Custom Codon

Sequence Name : SEQ ID 106:GDC_MTUB_1742061

### Sequence

<213> OrganismName : Mycobacterium tuberculosis-H37RV
<400> PreSequenceString : 
gtgcccccga ataggccgga acgccggtta gggaaacctc taacagcgcc gcttcgacgc
60 gcaccagcac atccccttcg cgacggtccc ggatcggtcg gaaacccacc gaaaacgagt
120 cgacgacacc agcttttacg ttcgccaaag cctcgtcgcc gtccggggtg tccgcaatct
180 cgaacgcccc gaacaagccg tgaggctcct cccgcaactc aacggcccgg cccaccgggt
240 agcgggttcg agcgtcgtga gagaccagca gcttcaattt gtggccgcgc tcggcgatgg
300 agcgccgaaa agcgccagga gcgaacattt cctggaactc gccgtcgaag tcgcggacgg
360 tggtcgcctc gttgtagggc acgatggtgc cgtgcacggt tcggccttcg ccagaccgca
420 gctcggccat gcggaaaagg atgctactca aaattcggcc accacctagc agacgcaaga
480 aacgcgcgga atcgcttgtg gcgcatggcg gccgctatcc gggttccagc cgccccgcgg
540 cgactgcccg gcgtcagcgg atgccgagat gccaaactcg attgtatcac acacaaaagg
600 tcatcaccgg tccggggcaa acgggttgag cccgtcgccg tcgtcgcccg gcgccaccgc
660 cagtcgctgc tcggcggccg gggtcaggcc aaactcggag gccaagcgca gcagatgcat
720 gcgcgccgtc tccgcaaccg tcaccgccgg gttccggtgc acgacaccgg atttcggtga
780
 <212> Type : DNA
   <211> Length : 780
   SequenceName : SEQ ID 107:GDC_MTUB_1782153
   SequenceDescription :

### Custom Codon

Sequence Name : SEQ ID 107:GDC_MTUB_1782153

### Sequence

<213> OrganismName : Mycobacterium tuberculosis-H37RV
<400> PreSequenceString : 
ttgtggaaat ggaagccgcg cttggcattc caccgggcaa cctggcggcg acgctggacc
60 gctacaacgc ctacgccgcg cgcggcgcag atcccgattt ccacaagcag ccggaattcc
120 ttgcagcaca agacaacggg ccgtgggggg cgttcgacat gtcgctgggc aaggcgatgt
180 atgccggatt cactctgggc gggctggcca cgtcggtgga cggtcaagta ctgcgcgacg
240 acggcgcggt ggtggccggc ctgtacgcgg tcggggcatg cgcgtccaat atcgcccagg
300 acggcaaggg atatgccagc gggacccagc tgggtgaggg gtcgtttttc gggcgtcgcg
360 ccggagcgca tgcggcagcc cgagcgcagg gcatgtaagc ctcctcgcgc cgcgactggg
420 aatcctgcga cgcgacacgc cgacaaggcg tcgtga
 456
   <212> Type : DNA
   <211> Length : 456
   SequenceName : SEQ ID 108:GDC_MTUB_2060659
   SequenceDescription :

### Custom Codon

Sequence Name : SEQ ID 108:GDC_MTUB_2060659

### Sequence

<213> OrganismName : Mycobacterium tuberculosis-H37RV
<400> PreSequenceString : 
ttgtggcccc gtatttccgc ggcgccgtcg aatcggcgat cgacagttgg cggcgtgtgg
60 tgtcgacggc ggcccaactg ggtatcccga ccccgggatt ctcgtcggcc ctgtcgtatt
120 acgacgcgct gcgcaccgcg cggctgcccg ctgcactcac ccaggcccag cgcgacttct
180 tcggcgcaca cacctacggc cggatcgacg aaccaggcaa gttccacaca ctatggagtt
240 cagaccgcac cgaagtaccg gtgtagcggg ctagaactaa aagggggtaa aggggtaagt
300 gatgagattt ctagacgggc acccacccgg gtacgacctg acatacaacg acgtgttcat
360 cgttccgaac cgatccgagg tcgcgtcgcg cttcgacgtc gatttgtcca ccgccgacgg
420 ctcgggcacc accattccgg tagtggtcgc caatatgacc gcggtagccg ggcggcggat
480 ggccgagacg gtcgcccgcc gcggtggcat cgtaatcctg ccgcaggatc tgccgatccc
540 ggcggtaaag cagacggtgg cgttcgtcaa aagccgggac ctggtgctcg acaccccagt
600 gacgctggca cccgacgatt cggtgtccga cgccatggcg ctcatccaca agcgcgcaca
660 tggcgtcgcg gtggtcatcc tcgagggtcg cccgatcgga ttggtgcgcg aatcgtcctg
720 cctgggcgtg gatcgcttca cccgggtgcg cgatatcgcc gtgacggact atgtgaccgc
780 tccagcggga accgagccac gcaagatctt cgacctgctg gagcacgccc cggtcgacgt
840 tgcggtgctg accgacgccg acggcacgtt ggcgggagtg ctaagccgca ccggggctat
900 ccgcgccggt atctacaccc cggccaccga tag
933
 <212> Type : DNA
   <211> Length : 933
   SequenceName : SEQ ID 109:GDC_MTUB_2093062
   SequenceDescription :

### Custom Codon

Sequence Name : SEQ ID 109:GDC_MTUB_2093062

### Sequence

<213> OrganismName : Mycobacterium tuberculosis-H37RV
<400> PreSequenceString : 
ttgggtatat ctcccggcga tcgcggggat cgtgttcgtg gcaatgccgc tggtcgcgat
60 cgccatccgg gtcgattggc cgcgtttctg ggcgctgatc actactccgt cttctcaaac
120 ggccctgctg ttgagcgtga agaccgccgc ggccagcacg gtgctgtgcg tactgctggg
180 cgtcccgatg gcgctggtgc tggcccgcag ccgcggacga ctggtgcggt cgttacgacc
240 gctgatcctg ttaccgctgg tgctgccgcc ggtagtcggg ggtatcgcgt tgctctacgc
300 gttcggccgg ctcggcctga tcgggcgcta cctggaggcg gccggcatca gcatcgcatt
360 cagtaccgcg gctgtggtgc tggcgcagac ctttgtctcg ctgccgtatc tggtgatttc
420 cctagagggt gcagcccgca ccgccggagc cgactacgag gtggtggcgg cgacacttgg
480 ggcgcggccc ggcactgtct ggtggcgcgt gaccctgccg ttgctgctcc cgggcgtggt
540 gtccggatca gtactggcgt ttgcccgctc gctcggagag tttggcgcga ccctaacctt
600 tgccggttcc cggcaagggg tcacccgtac ccttccgctg gagatttacc tgcagcgggt
660 gaccgatccg gacgcggcgg tggcattgtc actgctgctc gttgtggtag cggcactggt
720 ggtgctgggt gtgggtgctc gtacgccgat cgggaccgat accaggtagc cggtcatgag
780 caagctgcag ctgcgcgcgg tcgtcgccga ccggcgtttg gacgtcgaat tctcggtgtc
840 cgcgggcgag gtgcttgcag tgctcggccc caacggtgcg ggcaagtcca ccgccctgca
900 tgttatcgcg gggctgcttc gccccgacgc gggcttggta cgtttggggg accgggtgtt
960 gaccgacacc gaggccgggg tgaatgtggc gacccacgac cgtcgagtcg ggctgctgtt
 1020 gcaa
   1024
   <212> Type : DNA
   <211> Length : 1024
   SequenceName : SEQ ID 110 : GDC_MTUB_2105797 SequenceDescription :

### Custom Codon

Sequence Name : SEQ ID 110:GDC_MTUB_2105797

### Sequence

<213> OrganismName : Mycobacterium tuberculosis-H37RV
<400> PieSequenceString : 
ttgcccacgc cggtcccagc ccgaactggg acgccgtcgc gcagtgcgaa tccgggggca
60 actgggcggc caacaccgga aacggcaaat acggcggact gcagttcaag ccggccacct
120 gggccgcatt cggcggtgtc ggcaacccag cagctgcctc tcgggaacaa caaatcgcag
180 ttgccaatcg ggttctcgcc gaacagggat tggacgcgtg gccgacgtgc ggcgccgcct
240 ctggccttcc gatcgcactg tggtcgaaac ccgcgcaggg catcaagcaa atcatcaacg
300 agatcatttg ggcaggcatt caggcaagta ttccgcgctg acggttggcg gcgtgtgcgg
360 tctatgacca ggtcgacgta tgtgtttgga tcaggtcatg gaaggttcgg ccacagttca
420 catggcagcg ccgccggaca agatctggac attgatcgcg gatgtccgca ataccggccg
480 gttctcgccg gaaaccttcg aggccgagtg gcttga
516
 <212> Type : DNA
   <211> Length : 516
   SequenceName : SEQ ID 111:GDC_MTUB_2133554 SequenceDescription :

### Custom Codon

Sequence Name : SEQ ID 111:GDC_MTUB_2133554

### Sequence

<213> OrganismName : Mycobacterium tuberculosis-H37RV
<400> PreSequenceString : 
atgcgccggc tccgctcttc agatccacgg tgccatcgcc ttcacgtggg agcacgacct
60 gcacctgtat taccgccggg ccaagaccac cgaggcgctt ttcgggagca gcgctcgaaa
120 tcgtgcgctg ctcgccgaac gcgcggggct tgtgaaagcc taggcgccca gcgcggccag
180 cgccgcttcg tagttgggtt cttgcgcgat ttccggcacc aattccgtgt aggcgacgtt
240 gccgtccgcg ccgatcacca cgattgcgcg ggcgagcagc ccggccatcg gcccgtcggc
300 gatggtcacg ccgtaatcct cgccgaagct gtcccggaat gccgacgcgg gcatgacgtt
360 ttcggtgccc tcggcgccgc agaagcgctt ctgggcgaac ggcagatcct tcgagacaca
420 cagcacggta gcgccacttg ccgccgcacg ctcgtcgaag gttcgcacac tcgtcgcgca
480 caccggtgtg tccacggatg gaaagatgtt cagcaacacg gacttacccc ggaactggtc
540 gctgctgatc acccccagat cgcccccggt cagggtgaag gccggggccg gggatccgac
600 agcaggtag
609
 <212> Type : DNA
   <211> Length : 609
   SequenceName : SEQ ID 112:GDC_MTUB_2183418 SequenceDescription :

### Custom Codon

Sequence Name : SEQ ID 112:GDC_MTUB_2183418

### Sequence

<213> OrganismName : Mycobacterium tuberculosis-H37RV
<400> PreSequenceString : 
ttgcgcgggt ccgggcggac gcagatacaa gaccacgccg ctgccctgag ccgacatcct
60 cgccagcgcg ccgttgagtt cctcgccgca gcggcacgcc gtcgagccga acacgtcgcc
120 cgtcaggcac tcgatgtgga cgtgcagcgg cacgggcacc ccggcaccga ccgcacccac
180 gatgaccgcc aaatgctcgc cgaggtcgta aacgtcacga aagccgatga cacgcgaggc
240 gccggcccag gtgggcagcg tcgctgccgt aaaccggacc acctggggct cgatccgccg
300 gcgatacgcc accagctccc cgatcgagac catggccagt ccgtgttcga cggcgaattc
360 gaccgactcg gcgtggtgcg ccatctggac gggattatcg ggcgagacga tctcgcagag
420 cgcggcggcc ggccgccgtt ccgccaggcg ggccaggtcg acggccgcct cggcgggtcc
480 ccgccgaccc agcacaccgt cggcttgcgc ctgcacgggc accacatggc ccggacgttg
540 gaaatcggcg gcgacggagg tggccgaagc cagtgccgcg atggtccagg cgcgatcgct
600 cgccgagatt ccggtgccgg tgccgcgaac gtcgaccgac acgcaatgcg tggtgtctcg
660 gtcacacatg ggcggcaggt gcagtcgctc gcattcggcg cccggcagcg cgacgcgcaa
720 ataacccgag gtgtgccgga ccgcaaaggc aaccagccgc ggcgtcgcgg cctgggcggc
780 gaagacgaga tagccatcgc cattggggtc gccggtcagg accacggcgt gaccgcccgc
840 catcgccgtg atcgcacgac gtacccgcac atcggtcgtc ttcatcgaga ctccaaccgg
900 cggaaccggc taccgtga
918
 <212> Type : DNA
   <211> Length : 918
   SequenceName : SEQ ID 113:GDC_MTUB_2192571
   SequenceDescription :

### Custom Codon

Sequence Name : SEQ ID 113:GDC_MTUB_2192571

### Sequence

<213> OrganismName : Mycobacterium tuberculosis-H37RV
<400> PreSequenceString : 
atgaagacag ctatttctct gccggatgag acgttcgatc gggtatcgcg gcgtgcgagt
60 gagctcggca tgagtcggtc cgagttcttc acgaaggctg cgcagcgcta cctgcacgag
120 ctggacgccc aattgctcac gggccagatc gacagggctc tagagagcat ccatggcacc
180 gacgaagcgg aggccctcgc cgtggccaac gcataccgcg tgctagaaac catggacgat
240 gagtggtga
249
 <212> Type : DNA
   <211> Length : 249
   SequenceName : SEQ ID 114:GDC_MTUB_2234641
   SequenceDescription :

### Custom Codon

Sequence Name : SEQ ID 114:GDC_MTUB_2234641

### Sequence

<213> OrganismName : Mycobacterium tuberculosis-H37RV
<400> PreSequenceString : 
atgtctacat ccacgacgat tagggtttca acccagactc gggatcgtct ggccgcccaa
60 gcccgcgaac ggggaatctc gatgtcggct ctgctcaccg aactggccgc ccaggccgag
120 cgccaggcaa tcttccgcgc cgaacgcgag gcctcgcacg ccgagacgac cacccaggca
180 gtccgcgacg aggaccgcga gtgggagggc acggtaggcg acggccttgg ctga
234
 <212> Type : DNA
   <211> Length : 234
   SequenceName : SEQ ID 115:GDC_MTUB_2320829 SequenceDescription :

### Custom Codon

Sequence Name : SEQ ID 115:GDC_MTUB_2320829

### Sequence

<213> OrganismName : Mycobacterium tuberculosis-H37RV
<400> PreSequenceString : 
gtggcgacca gcacctcgcc ggccggtggg ctgccgcagg cccgctcgca gccgacgaaa
60 tgccgatgcc cggctgactc cacgttcagt gaccgcgcgg cgtcggcccg tacgtcggcg
120 gccgagtgcg cgcagccggg gctgccggtg caggcgctga tgttcagcca gggggagttc
180 tcgtcgaaca ccaggcccag cggcgccagc acccgcagcg cggcgtcggc cgtcgcgtcg
240 tcgaggtcgc agatcagcac cgatcgccac ggcgtgatca ccagcggggc ctcgatcgcg
300 gccaggcatt ccgcgacccg ggcgggcaag acccccagcg gcaccgcggc gcccagcgtt
360 acccggctgt catcctgggg tatccagccg acgggcgttt tggtgacggg ccgaacggat
420 gggcccagct cgacaccgga ctgcagctcg ccgatatcgg ctaattccgt tactcgccag
480 gcggtttcgc ggatcttgac gaaacgcaac gcgacctcga tcagggtctc ggcgacatcg
540 gccacccgca cgccggtgtc acgtccggtc aacagcagtc ggggaccgtc ggggaacacc
600 tgcacgccga cgtcggcacc caggccggac acgtcggcgc ggccgtcgtc gagaccgaac
660 cagaaccggc cgcccagttc cgccagccgg ggctcggcgc ggatcgccgc gtcgagctca
720 ccgacccatg cccgcacgtc ggctagcccg ccggcccggc cggacagcgg cgaggcgacg
780 atattgcgca cccgctcgtg tgttgccgac ggcagcagcc cggctttggc gaccgcgtcc
840 gcgaccgctg ccacgtcgcg gatcccgcgc aactggacat tgccgcgcgc ggtcagttcc
900 agtgtcgcgg agccgaagtc gctggcgacg ctggccagcg tcgccagttg tgccgcggtg
960 atcatcccgc cgggcagccg gatccgcgcc agcgccccgt cggcggcctg gtgcggccgc
1020 aacg
1024
 <212> Type : DNA
   <211> Length : 1024
   SequenceName : SEQ ID 116:GDC_MTUB_2321250
   SequenceDescription :

### Custom Codon

Sequence Name : SEQ ID 116:GDC_MTUB_2321250

### Sequence

<213> OrganiSmName : Mycobacterium tuberculosis-H37RV
<400> PreSequenceString : 
atgacgggcc gtgtccgaca gaccggcata acccgtctcg tcgtacatca gcggggcccc
60 gtccttccac agcgactgat gacagtgcat gccggacccg ttgtcgccga acagcggctt
120 gggcatgaac gtgaccgttt tgccgttctg ccaggcggtg ttcttgatga tgtacttgta
180 caactgcatg tcgtcggcgg cgtgcagcag cgaattgaac tggtagttga tctcggcctg
240 tccgccgctg cccacctcgt ggtggccctt ctccaggatg aagccggagt tgatcaggtt
300 ggtcagcatc ttgtcgcgca ggtcgacgta ttggtcgttg ggggccactg ggaaataccc
360 gcccttgtgg cggaccttgt agccccggtt gggactgccg tcggcctcgg tcgccgcgcc
420 ggtgttccac caccccgaga tggcgtccac ctcgtagaag gagccgttgg cgcgcgagtc
480 gaagctcacc gaatcgaaaa tgtagaactc ggcctcggcg ccgaagtatg cggtgtcggc
540 gatgccagtg ctgatcaggt agttctcggc cttgcgggcg atgttgcgcg ggtcgcggga
600 gtacggctcc agggtgaacg ggtcgtgcac aaagaagttg atattcagcg tcttggccgc
660 gcggaacggg tcgatgcgcg ccgtctcggg atcgggaaga agcaacatgt cggattcgtg
720 gatcgactgg aacccgcgaa tcgacgagcc gtcaaaggcc aagccgtcgt caaacacgct
780 cttgtcaaag gccgaagccg gaatcgtgaa gtgctgcatg atgccaggca ggtcacagaa
840 ccggacgtcg acatattcga ccttctcgtc cttggcaagt ttgaagacgt cgtcgggcgt
900 cttttccgtc acagaatgct cctttactgt atccgcggcc gacgctatgg agccgatatt
960 gcccgtcagt caaccccgtg ttgcgcagac gttactgacc gtgccgccca ccactga
1017
 <212> Type : DNA
   <211> Length : 1017
   SequenceName : SEQ ID 117:GDC_MTUB_2487508

### SequenceDescription :

### Custom Codon

Sequence Name SEQ ID 117.: GDC_MTUB_2487508

### Sequence

<213> OrganismName : Mycobacterium tuberculosis-H37RV
<400> PreSequenceString : 
gtggcgggcg tttgcgcgct attctccggt gcttcccgct ggccgtctgg tgaacttcgg
60 caccgtccac agggttcccg ccggggtccg agccggctac gatgcacctt tccccgacaa
120 aacgtatcaa gccggcgccc gggcgttccc acggttggtg ccgacctcac ccgacgatcc
180 ggcggtaccg gccaaccgcg cggcatggga agccctgggc cggtgggaca aaccgttcct
240 tgccatcttc ggttatcgcg acccgatact cgggcaagcg gacggtccgc tgatcaagca
300 cattcccggc gcggcgggtc agccgcacgc ccgcatcaag gccagccact tcatccagga
360 ggacagcgga accgaactcg ccgaacgcat gctctcctgg cagcaggcaa cgtaaccgcg
420 acggctgcgg acgaaggatc ggcagaatgg cgatggagat ggcgatga
468
 <212> Type : DNA
   <211> Length : 468
   SequenceName : SEQ ID 118:GDC_MTUB_2567990
   SequenceDescription :

### Custom Codon

Sequence Name : SEQ ID 118:GDC_MTUB_2567990

### Sequence

<213> OrganismName : Mycobacterium tuberculosis-H37RV
<400> PreSequenceString : 
atgaccgaca acgagtgccc ggccgacagc cgacggcgcc atgtcctgcg gctcgccctg
60 ttcgccggga ttttgctggg gctgttctac ctggttgcgg tggcacgagt catccacgtc
120 gacggggtcc gtagcgcgat cgtggtggcg acgggtccga tcgcacccct ggcgtacgtt
180 gtggtgtcgg ccgcactcgg cgcgttgttc gtcccgggcc cgatcctcgc cgccggcagc
240 ggggtgctgt tcgggccgct actagacacc tttgtgaccc tgccagcttt ctcggccggc
300 gcgcaggccg gaatgacgcc caggcgctgc tgggtgtcga tcgcgcccat cgcctcgatg
360 cacagatcga acggcgcgga ttgtgggcgg tggtcggtca gcgcttcgtc cccggcatct
420 cggatgcgct ggcctcgtac accttcgggg cgttcggagt tccgttgtgg cagatggtcg
480 ttgggtcgtt catcgggtcg gcgccacggg tgttcgtcta caccgcgctg ggcgcgtcga
540 tcaccaacct gtcgtcgccg ctggtttact cggcgatcgc ggtgtggtgc gtga
594
 <212> Type : DNA
   <211> Length : 594
   SequenceName : SEQ ID 119:GDC_MTUB_2577106
   SequenceDescription :

### Custom Codon

Sequence Name : SEQ ID 119:GDC_MTUB_2577106

### Sequence

<213> OrganismName : Mycobacterium tuberculosis-H37RV
<400> PreSequenceString : 
ttgtgggcgg tggtcggtca gcgcttcgtc cccggcatct cggatgcgct ggcctcgtac
60 accttcgggg cgttcggagt tccgttgtgg cagatggtcg ttgggtcgtt catcgggtcg
120 gcgccacggg tgttcgtcta caccgcgctg ggcgcgtcga tcaccaacct gtcgtcgccg
180 ctggtttact cggcgatcgc ggtgtggtgc gtgaccgcca tcatcggggc gttcgccgcg
240 cggcgttggt accggaagtg gcgtgcgcgc ccgcgccggc ggtgcggcct ggctcagctc
 
300 acgaccggta gtcagcaacg ccacacgagt caccggacac cggcgggcgt cgtcatgccc
360 ggttcactgt ccgagcaccg ccgtctccgt caagaagcgc cggatcgcat cgagcatcac
420 ccgcccatcg agtag
435
 <212> Type : DNA
   <211> Length : 435
   SequenceName : SEQ ID 120:GDC_MTUB_2577486
   SequenceDescription :

### Custom Codon

Sequence Name : SEQ ID 120:GDC_MTUB_2577486

### Sequence

<213> OrganismName : Mycobacterium tuberculosis-H37RV
<400> PreSequenceString : 
ttgtctgcgg ttttaccggc tcggtgcatt cgcgcgctag ccgatagggt ctatcgccat
60 gtccggtgcc acggtgggtg cgcgcgaaat caccatccgc ggagtcgtcc tgggcgcatt
120 gattaccttg gtgttcaccg cggccaacgt gtacctgggg ctaagggttg gattgacatt
180 cgccacttcc ataccggccg cggtgatctc gatgggcgtg ctgcggttgt tcgccaacca
240 ctcagtggtg gagaacaata ttgttcagac gatcgcgtcg gcggccggca cgctgtcgtc
300 gatcatcttc gtgttaccgg cactgctcat gatcggctgg tggagcgggt ttccgtactg
360 gacaacggcg gcggtgtgtg cactgggcgg gatccttggc gtcatgtact caattccgtt
420 gcgccgcgca ctcgtcaccg gatcagacct gccgtaccca gaaggcgttg ccggagccga
480 ggttctcaag atcggtga
498
 <212> Type : DNA
   <211> Length : 498
   SequenceName : SEQ ID 121:GDC_MTUB_2690012
   SequenceDescription :

### Custom Codon

Sequence Name : SEQ ID 121:GDC_MTUB_2690012

### Sequence

<213> OrganismName : Mycobacterium tuberculosis-H37RV
<400> PreSequenceString : 
gtgggcccga tgaacgggtt cctgagttgg tgggacggcg tcgagctgtg gctgtccgga
60 ctcccgttcg cgctgcaggc gttggcagtc atgccggtcg tgctggcttt ggcctatttc
120 accgcggcat tgctggatgc cctgctcggc cgggtcattc agttgattcg ccgcgcccgc
180 cgccccgatc aggcgcccag gtag
204
 <212> Type : DNA
   <211> Length : 204
   SequenceName : SEQ ID 122:GDC_MTUB_2698040
   SequënceDescription :

### Custom Codon

Sequence Name : SEQ ID 122:GDC_MTUB_2698040

### Sequence

<213> OrganismName : Mycobacterium tuberculosis-H37RV
<400> PreSequenceString : 
atggcggacg atgtgagcgg cgcggtgtac cgggccggca cggcccacgg tcggccgacc
60 ggtcgcattg aacaccgcga ccgtcaggtc gtgacgcgcc gggcgactga tacgcgcgcg
120 gaactggacg ggctgtccga ccatcagctc gccgaagtcc agcgctcgcg cgaaaaccac
 
180 tacccggccg gatgtctcgt catcccgcag ccgttgaacc gtcgcccgga acatcaaccg
240 gccccgcccc agcgacactg ggctctcgct gggggtgacc gtgaccagcg cggaggtgcc
300 aaatgccacg gtgattgggt ggcgatcgac cgcctcggag cgcaacgcga ccgcaagccc
360 gtaccccgcg cccaccatac cgaccgcgac caggccggcg ctgatcgaac ccagtcgcgg
420 agcgtgccac gaccggcgcg ccacacacca ccacagtgcg ccgccgccga gggccaccac
480 gacgcagcac aaggcacaca cgttgccgat cggccacacg atcccggccg ccgtcacaat
540 ccagctgacc agcgccgccg ggaccaggcg tacgtccaaa cggacgcgcc cgaagcccat
600 atggcgcacc ggtatcagac acggaccaga ttgcgccgct tgtccagccg cgccggaccg
660 atgccgtcga cgtcggcaag ctggtcgacg ctggtgaacc taccattgcg ctgccgccac
720 gccacaatcg ctgcggcggt gaccggcccg atgccgggca gggcgtccag ctgctccacg
780 gtcgcagtgt tgaggtcgag cacctcagct gtcttaggag ctgtcttagg gcctgtcgtg
840 gctgtgcccg aggtacccgc cggtcccggc gtccccgcac cgaccgagct gcccagcacc
900 ctcggctgtc ccgagggcgg agctagcccg accacgatct gctcaccgtc accaagctgc
960 cgagccatgt tcagtccgac ggtgtccgcg ccgtctaccg ctccgccggc ggcctgtagc
1020 gcat
1024
 <212> Type : DNA
   <211> Length : 1024
   SequenceName : SEQ ID 123:GDC_MTUB_2712275
   SequenceDescription :

### Custom Codon

Sequence Name : SEQ ID 123:GDC_MTUB_2712275

### Sequence

<213> OrganiSmName : Mycobacterium tuberculosis-H37RV
<400> PreSequenceString : 
atgaggggca ctgcctacgc gaccagacgc tcgatgctgc ccaacacccg ggcggtgtgg
60 ctggccaccg tcgtgcagtg cgtgaccggc gggctggggg tgacactgat tccgcagacc
120 gcggccgccg tcgagaccac gcgaagccgg ctggaactcg cccgattcgt cgcccctgcc
180 cggcgcgacg aatcggtttg gtgtttagct ctttcggcgg ccgcgagaag tcctaccagc
240 gtcttgccgg gattatcggc aagctga
267
 <212> Type : DNA
   <211> Length : 267
   SequenceName : SEQ ID 124:GDC_MTUB_2725593
   SequenceDescription :

### Custom Codon

Sequence Name : SEQ ID 124:GDC_MTUB_2725593

### Sequence

<213> OrganiSmName : Mycobacterium tuberculosis-H37RV
<400> PreSequenceString : 
atgcgcagag tattcagcgg ttggacaacg ttggtccgct gcagcaccgc agcgaccacc
60 gtcacgatca gggcgatgac aaagcacgtc ccggtaatcc actccagcga accgacccgg
120 ccgctgacgc cgcgaaagcc ggtggatccg gtgcgtcggt gctgcagcca actgcgtcag
180 ccgaatccga ccacactgaa aaccgcgaag agtgccagcg ctaagtcggc cgcggtggtc
240 gttcgcatca gcgggtctcc ttcggtgcgt agcagtggtc atgaaccgtt gtggcggttg
300 gctcgcaggg ccgcatcgat cgcggcggcg gccggtgcgc agtcgccgac accggacacc
360 aaagttgcca gcgcacccgc agcgcaggcc cgccgcaatg cgcgcagtcg ctcggccggc
420 gaacctgggt tgcgcggcca attcgcagca aggaccccgg caaatacgtc gccggcgccg
480 gcggtatcca ctggcgttac cgttggggcg ggtacctcga acaccccgtc cgcgccgacg
540 taccgggcac cgcgcacacc cagggtgatc acgaaatgtg ttggtggcga cggccagtcg
600 tttgcctcat gctcgttggc gatcaccacg tcggcgatag cggccaagtc ctgcaaggag
660 cttcgatcct ggccggctgg ggaggcgttg accatgacaa ccgcatcggc cgactgggct
720 gcccgcgcgg ctgccagcgc ggttgcaaca ggaatctcca actgggtcaa cagtacatcg
 
780 cagttggcga cggccgaggg taccggagtc agatgtgcat tggcacccgg cgccaccagc
840 acggtgttct cggcgctggc atcgaccacg ataatcgccg tcccgctcgg tccgggcacc
900 gtgacggtcc tgtccagtcc aacggcgttg gcgcgcaggt gggcccgcag ctgggcggcg
960 gctggatcgt cgccgaatgc accggagaac tgtacctgcg cgcctgcgcg cgctgcggcc
1020 accg
1024
 <212> Type : DNA
   <211> Length : 1024
   SequenceName : SEQ ID 125:GDC_MTUB_2733212
   SequenceDescription :

### Custom Codon

Sequence Name : SEQ ID 125:GDC_MTUB_2733212

### Sequence

<213> OrganismName : Mycobacterium tuberculosis-H37RV
<400> PreSequenceString : 
atgagcgctt ctgcgtcagc cgacaaggtc gtatgcgagt gctgcgagct ctgtgttcct
60 aaacagctcg cgtcagcgat tcgcaaccca tacggactcg tccgtgggtg gcgctgtcgc
120 atctgtaacg agcaccaagg ccagccggtc aagatggcgc aagaccacga agaggaggtc
180 cgcatccgtt ggggcgagac ggtggacgaa ctccacgctg cgctggaccg cgccgggcca
240 aggccaggga cgtggtgtac gagtgaaggt tcctcgcgtg atccttcggg tggcagtcta
300 ggtggtcagt gctggggtgt tggtggtttg ctgcttggcg ggttcttcgg tgctggtcag
360 tgctgctcgg gctcgggtga ggacctcgag gcccaggtag cgccgtcctt cgatccattc
420 gtcgtgttgt tcggcgagga cggctccgac gaggcggatg atcgaggcgc ggtcggggaa
480 gatgcccacg acgtcggttc ggcgtcgtac ctctcggttg aggcgttcct gggggttgtt
540 ggaccagatt tggcgccaga tctgcttggg gaaggcggtg aacgccagca ggtcggtgcg
600 ggcggtgtcg aggtgctcgg ccaccgcggg gagtttgtcg gtcagagcgt cgagtacccg
660 atcatattgg gcaacaactg a
681
 <212> Type : DNA
   <211> Length : 681
   SequenceName : SEQ ID 126:GDC_MTUB_2828257
   SequenceDescription :

### Custom Codon

Sequence Name : SEQ ID 126:GDC_MTUB_2828257

### Sequence

<213> OrganismName : Mycobacterium tuberculosis-H37RV
<400> PreSequenceString : 
gtgggatcgc tcaccgtgtt caccagctcg gcgaggatgt cgcgcacagc ggccaacacg
60 tcggcgcgcg cactgcacag catgaccacc gggtcgggcg ggaagagcag aatgctgaac
120 acgatagcca gcccaccacc gaccagcgcg tcgaagaggc gttcgaaaac cacactgccg
180 ttggacgcga agaccaagac cagcaccgcg gagacggcgg cctggttgat gaacattaag
240 ccttgcgcga ccaacccgcg tgcgcacagc accgcgaccg acaacgcgat gaacaccacc
300 acacccatgg cgatcggtcc ggaaccaagc agagcatgca cgccagcacc cagcacgatc
360 cccagcgcca ccccgacgat catctgttgg gcacgtcgtg cgcgcagcac gttggtcgcc
420 gacatgcaca ccacagccga aatcggcgcg aagaacgcct gcggatggtt gaacacgtca
480 tgggtgagat accacgcgag gccggcgacg accgatgtct gggtgatcgg ccacagcacg
540 gtgcgcaacc gttgggcgac cgcacggccg ccgcaggccg tcctgactag cagcgaagcg
600 ctcatgaacg cctatttatt cacactcggg tgcgacgtcg taaccgcaaa gatctggtca
660 tgcctgctgg acccgcttgg gctgggcatc tattccggac tccttacgtt gctgagcggt
720 aatgggcgcc ggcgcgtcgg tgagcggatc gacgccgccg ccggtcttcg ggaacgcgat
780 cacctcacgg atcgagtcca tcccggccag cagcgcggtg gtccggtccc acccgaacgc
840 gattccgccg tgcggcggtg cgccaaacat gaacgcctcc aacaggaatc cgaacttttc
900 ctccgcctcg gccttgtcca ggcccatcac cgcgaacacc cgttcctgga tatcacggcg
 
960 gtggatacgc accgagccgc caccgatctc gtggccgttg cagacgatgt cgtacgcgtc
1020 ggcc
1024
 <212> Type : DNA
   <211> Length : 1024
   SequenceName : SEQ ID 127:GDC_MTUB_2895354
   SequenceDescription :

### Custom Codon

Sequence Name : SEQ ID 127:GDC_MTUB_2895354

### Sequence

<213> OrganismName : Mycobacterium tuberculosis-H37RV
<400> PreSequenceString : 
gtgatcggcg atttcgccga gatgctcggc ggccaggacg gcgtcgctga gttggtccaa
60 cacgtcgctg tgcacccgtt tgatggcgtt gatgagctcg tcgaggcgga cggggtaggc
120 ggtgggtgtg ggctccggca tgacgtcaac agtaggttga cgttatgcat tgtgtcgacc
180 gtgattggct gcgtagtggg ttctgcagcg ctgccaggcc gctgcgggca gggtggcgcc
240 gatcgcggcc accaggccgg cgtgggcgtc gctggtgacc agcgcgaccc cggacaggcc
300 gcgggcgacc aggtcgcgga agaacgccag ccagccggcc ccgtcctcgg cggaggtgac
360 ctggatgccc aggatctctc ggtagccctc ggcgttgacg ccggtggcga tcaaggtgtg
420 caccccgacg acgcggcctg cctcgcgcac cttgagcacc agggcgtcgg cggcgaggaa
480 ggtatacggg ccggcatcga gcgggcgggt ccgaaacgcc tctacggctt cgtcgagctc
540 tttggccatg atcgacactt gcgacttgga aagctttgtc acaccaagtg tttcgaccag
600 gcgctccatc cggcgagtgg atactcccag caggtagcag gtcgccacca cgctggtcag
660 tgcgcgttca gctcgcttgc ggcgctgcag cagccagtcc gggaaatagc tgccctggcg
720 cagcttgggg atcgcgacgt cgatggttgc ggcacgggtg tcgaaatcac ggtggcggta
780 gccgttgcgc tgattggacc gctcatcgct gcgttcgcgg tagcccgccc cgcacagggc
840 gtcggcttca gcccccatca aggcggcgat gaacgtcgag agcagcccgc gcagcagatc
900 cgggctcgcc tgtgcgagtt ggtcagccag aagctgctcg gtgtcgataa gatgagaaga
960 ggtcattgcg tcatttcctt cgattga
987
 <212> Type : DNA
   <211> Length : 987
   SequenceName : SEQ ID 128:GDC_MTUB_2983047
   SequenceDescription :

### Custom Codon

Sequence Name : SEQ ID 128:GDC_MTUB_2983047

### Sequence

<213> OrganismName : Mycobacterium tuberculosis-H37RV
<400> PreSequenceString : 
ttggatgagc cggcgcaccg cgctcgcccg aaagggaacg gagccaatca tgacggcgct
60 caaccgtgct gtggcatcgg cgcgtgtggg aaccgaggtg atccgcgtgc gcgggctcac
120 cttccgctac ccaaaggcgg ccgagccggc ggtgcgtggc atggagttca ccgtcggccg
180 cggcgaaatc ttcgggcttc taggtcccag cggcgcgggc aagtccacca cccagaagct
240 tctcatcggg ctgctgcgcg accacggcgg ccaggccacg gtgtgggaca aagagccggc
300 cgagtgggga cccgattact acgagcgcat cggggtctcc ttcgagctgc ccaaccacta
360 ccaaaagctc accgggtatg a
381
 <212> Type : DNA
   <211> Length : 381
   SequenceName : SEQ ID 129:GDC_MTUB_3005316
   SequenceDescription :

### Custom Codon

Sequence Name : SEQ ID 129:GDC_MTUB_3005316

### Sequence

<213> OrganismName : Mycobacterium tuberculosis-H37RV
<400> PreSequenceString : 
atgatccctc aaatgacggt gtcctgcccg cccccgtcga cttctgagcg cgaagagcag
60 gcgcgggcac tgtgcctgcg cctgctcacc gcgcgatccc gcacccgcgc cgagttagcc
120 ggccagctgg ccaagcgcgg ctaccccgaa gacatcggca accgggtatt ggatcggctg
180 gccgccgttg gcctggtgga tgacaccgac ttcgccgaac aatgggttca gtccaggcgg
240 gcgaacgcag caaagagcaa gcgcgcgttg gctgccgagc tgcacgccaa gggcgtcgac
300 gacgacgtga tcaccacggt gctcgggggc atcgacgccg gtgccgaacg ggggcgggcg
360 gaaaagctgg tacgggccag gctgcggcgg gaggtgctga tcgacgacgg caccgacgaa
420 gcgcgggtga gccgcaggct ggtggcgatg ttggcgcgcc gtgggtacgg ccagaccttg
480 gcgtgcgagg tggttatcgc cgagctggcc gccgagcggg agcgccgacg cgtctaa
537
 <212> Type : DNA
   <211> Length : 537
   SequenceName : SEQ ID 130:GDC_MTUB_3048559
   SequenceDescription :

### Custom Codon

Sequence Name : SEQ ID 130:GDC_MTUB_3048559

### Sequence

<213> OrganismName : Mycobacterium tuberculosis-H37RV
<400> PreSequenceString : 
ttggtgacga ctctggcgcc gatcttggac agtgcatcga tgactccgaa gaccgcctcc
60 tcgttgccgg ggatcagcga cgacgacaac acgatgagat caccagcagt caacgtgatg
120 ctgcgatgct ccccacgcga cattcgcgac aacgccgaca tcggctcgcc ttgggtgccg
180 gtggtgatca acacaacttg gtcgggcgcc atcgtttcgg cggcggcgat gtcgatgaga
240 tcggaatcag ccactcgtag gaagcccagt tgccttgcga cgcgcatgtt gcgcaccatc
300 gatcggccga cgaacgacac tcgccggccc aatgccactg cggcatcgat gatctgctgt
360 acccgatcca cgttggaggc gaaacacgca actatcaccc gtccgtcggc accccggatg
420 agccggtgca gcgttgggcc cacttcgctt tccgatggcc cgacaccggg gatctcggcg
480 ttcgtcgagt cgcacagcaa caggtccacg ccggtgtcgc cgagccgcga catgcccggt
540 agatcggtgg gacggccgtc cggtggcaat tggtcgaact tgatgtcgcc ggtgtgcagg
600 atggttcccg cgccggtata caccgcgatg gccaacgcgt ccggagtgga atggttgacg
660 gcgaagtact cgcactcaaa cacgccgtgc cgggtgctct ggccctcgcg gacctcgacg
720 aacaccggtg ttatgcggta ctcacgacat ttctctgcaa ccagagccaa ggtgaacttc
780 gagccgacga ccgggatgtc gggtcgcagc ttgagcagaa acggaatcgc cccgatgtgg
840 tcctcgtgcc cgtgggtcaa caccagcgcc tcgatgtcgt caagccggtc ttcgacatgg
900 cgcatgtccg gcaggatcag atcgacaccg ggctcgtcgt ggccaggaaa caacacaccg
960 cagtcgataa tcaacagtcg gcccaggtgt tcgaaaaccg tcatgttgcg gccgatttcg
1020 ttga
1024
 <212> Type : DNA
   <211> Length : 1024
   SequenceName : SEQ ID 131:GDC_MTUB_3065095
   SequenceDescription :

### Custom Codon

Sequence Name : SEQ ID 131:GDC_MTUB_3065095

### Sequence

<213> OrganismName : Mycobacterium tuberculosis-H37RV
<400> PreSequenceString : 
atgtccaaga gatcggatgg gccgagcact ggcaatgcga ttcgtgctcg gcatcgcatc
60 agcgtgatga ctgcgcagcg atcaacctcg cacgctacga ggacaccagt agcgtcgtcg
120 gcccagttgg ggccgccgtc aagcgtggag ccgaccgtaa gacccggcct ggccgggctg
180 gtggccgtga agcgcggaag ggaagcagcc gcaaggctgc cgaacaaccc cgagacgggg
240 tgcaagtcgc gtgaccacta a
261
 <212> Type : DNA
   <211> Length : 261
   SequenceName : SEQ ID 132:GDC_MTUB_3100192
   SequenceDescription :

### Custom Codon

Sequence Name : SEQ ID 132:GDC_MTUB_3100192

### Sequence

<213> OrganismName : Mycobacterium tuberculosis-H37RV
<400> PreSequenceString : 
gtggcaacga agaacgcggc atggccttca tctacaagct gctcgaacta ctcgccgaac
60 gcgacgatcg aatcacaaag gccagatggg tgtacttcct cacgcgcatg cgtaacccca
120 ccggtgacac agcgcctttt cagcagtttg ctaaccggct acaccaatgg ttccaagatc
180 cgacagacgc caagcaactc aagaccgcgc tgcacctcta catctatcgc actcgcaagg
240 aggagtccga atgagcgtca tccaagacga ctatgtgaaa caggccgaag taattcgcgg
300 cctgccaaag aaaaagaacg gcttcgagct gaccacaacc cagctgcggg tgctactcag
360 cctgaccgca cagctcttcg acgaggcgca gcagagcgcc aaccccacgc tcccgcgtca
420 gctgaaggag aaggtccagt acctgcgggt ccggttcgtc taccagtccg ggcgtga
477
 <212> Type : DNA
   <211> Length : 477
   SequenceName : SEQ TD 133:GDC_MTUB_3129118
   SequenceDescription :

### Custom Codon

Sequence Name : SEQ ID 133:GDC_MTUB_3129118

### Sequence

<213> OrganismName : Mycobacterium tuberculosis-H37RV
<400> PreSequenceString : 
atgtcggcgc ctgacgtgcg gctgaccgcc tgggtgcacg ggtgggtgca gggagtcggt
60 ttccgctggt ggacccgctg ccgagcgttg gagctcggcc tgaccggtta cgcggccaac
120 cacgccgacg gacgcgtgct ggtggtcgcc cagggtccgc gcgctgcgtg ccagaagctg
180 ctgcagctgc tgcagggcga cacgacaccg ggccgcgtcg ccaaagtcgt cgccgactgg
240 tcgcagtcga cggagcagat caccgggttc agcgagcggt aa
282
 <212> Type : DNA
   <211> Length : 282
   SequenceName : SEQ ID 134:GDC_MTUB_3237815
   SequenceDescription :

### Custom Codon

Sequence Name : SEQ ID 134:GDC_MTUB_3237815

### Sequence

<213> OrganismName : Mycobacterium tuberculosis-H37RV
<400> PreSequenceString : 
atgttgcacg acgtcgtcca cggcagacga tgtagtgaga atggccaccg gcgacgaatc
60 actcagtacc gaatcggaac gttcatcggt aacgccgcct tgtggaaccg aaagcggcac
120 ggcgatgcgc ccggcctgca acgcgccgag aaaggcgacg acgtactcga gtccctgcgg
180 agcagagatc accacgcggt cacccgtgga accacaacgg ctcagctcct gtgccacatt
240 cagcgttcgc cgatacagct gcgaccacgt cagggttatc gcaacgccgt cccagtcctg
300 ttcgtaatcc ataaacgtga aggccgggtc atggggttgc agacgcgcac acgcgcgcaa
360 cgcagcggga agggaacgca cactcatggg catcacgtta ccggccacgc ttggagttgt
420 cgcagtcgcc gtcggggtgt gctcgcgctc cgcggtctta gccaagtcgc atctggccag
480 ctcagcaggg gtttgccggc tcgccatggg tccaccatcg gacacggtcg gatgtga
537
 <212> Type : DNA
   <211> Length : 537
   SequenceName : SEQ ID 135:GDC_MTUB_3283182
   SequenceDescription :

### Custom Codon

Sequence Name : SEQ ID 135:GDC_MTUB_3283182

### Sequence

<213> OrganismName : Mycobacterium tuberculosis-H37RV
<400> PreSequenceString : 
atgcccacca ccaaagccac ccagcgccgt gatgtttcca ccgagatcgc ttacctgaca
60 agagcattga aagctcccac cctgcgtgag tcagtgtccc ggctggccga tcgcgcccgc
120 gccgagaact ggagccacga agaatacctg gccgcctgcc tgcagcggga agtgtcagcc
180 cgggagtccc atggtggtga gggccgcatc cgcgccgccc gcttcccggc tcggaagtcg
240 ttggaagagt tcgactttga gcatgctcgt ggcctcaaac gcgacaccat cgcacatctg
300 ggcaccctgg atttcatcac cgcccgcgat aacgtcgtgt tttgggcccc cgcctggcac
360 cgggaagact catcttgcgg tcggcctggc gatacgcgcg tgtcaggccg gtcatcgggt
420 gctgttcgcc accgccgccg aatgggtagc acggctcgcc gaggctcacc acgccgggcg
480 catctacgcc gaactcaccc ggctttgccg ctatccgctc ctggtggttg a
531
 <212> Type : DNA
   <211> Length : 531
   SequenceName : SEQ ID 136:GDC_MTUB_3289702
   SequenceDescription :

### Custom Codon

Sequence Name : SEQ ID 136:GDC_MTUB_3289702

### Sequence

<213> OrganismName : Mycobacterium tuberculosis-H37RV
<400> PreSequenceString : 
atgcagtggg ggtaccgccc gcttgcgggg gacgaagcga tgaggtgggg gtaccgcccg
60 cttgcgaggg agagcggcgc acttgacccg gatcatcggc ggtgtcgccg gaggccggcg
120 cattgccgtc ccaccacgcg gaaccagacc taccaccgat cggtgcgcgg agtcgctatt
180 caacatcgtg actgcgcggc gggatctgac cggtctggcg gtgttggacc tctatgcggg
240 ttccggcgcc ctggggctgg aggcgttgtc gcggggagcg gcgtccgtgc tgttcgtgga
300 gtccgaccag cgcagcgcgg ccgtcattgc gcgcaacatc gaggccctag gtctctccgg
360 tgcgacgctg cgccggggcg cggtggcggc cgtcgtggcg gccgggacca cgtccccggt
420 ggatctggtg ttggccgacc cgccctacaa cgtcgactcc gccgacgttg a
471
 <212> Type : DNA
   <211> Length : 471
   SequenceName : SEQ ID 137:GDC_MTUB_3319076
   SequenceDescription :

### Custom Codon

Sequence Name : SEQ ID 137:GDC_MTUB_3319076

### Sequence

<213> OrganismName : Mycobacterium tuberculosis-H37RV
<400> PreSequenceString : 
ttgggtgggg ttgccagcac tcggcaggca tccgttcgcc gttggtctgc cgttcacccc
60 ctggatgcct cgccggcgtt gccccgtccc ggtcaacgat gtgcgaccgc tcgcgcggtc
120 gcgggcccta ccccgagctg gcgtgcggcc gtcaggtcgg cgggggtgtc gacatcgcag
180 cgcaggcccg gccaggctcc tgtcagctcg acagcgcccg aacggcggtg ccgcgcggac
240 gaatccggcc cgaaccgcgg gtgcagcgcg gtgccgaacg cacacagtac cgcggtgccg
300 gtcccaagcc ggtcggcgac gaagctgcgc cgatggtggc gtgcggccga gattgcctcg
360 gcgagttcct gtgtctgtaa tgccggcaaa tcgccttgca gcacaacgat gttggaggcc
420 ccttcggcaa ccacgcgttc ggcagcggtg atggcggtgt tcagtgggtc gggatcgtct
480 tcgggtgtcg ggtcggccag tacatcggcg cccagcccgg ccgccgcagc cgccgcggct
540 tcgtcggggg tgataacagt gatcgagcgc agtgaaccga cacccgccgc ggcggtcaac
600 gtgtcgacga gcatggccag caccacgttc tcgcgagtct gcgccgagaa caccggggcc
660 agcctggttt tggccgcggc caagcgcttg acggcgatga tcaagccgat atcgccgtcg
720 tccggtgtgc cgctcatgaa gtcatcctgc cagcgtcgat ccacgcggca cacttcgacg
780 gcattgccgc cacggtcgtg gccgggcccc aggcacggtc ccgacggcaa ccgcggcgca
840 gattag
846
 <212> Type : DNA
   <211> Length : 846
   SequenceName : SEQ ID 138:GDC_MTUB_3339006
   SequenceDescription :

### Custom Codon

Sequence Name : SEQ ID 138:GDC_MTUB_3339006

### Sequence

<213> OrganismName : Mycobacterium tuberculosis-H37RV
<400> PreSequenceString : 
ttgcgcggca ggttgatccg atacgcggtg ttgttgtctc cgagcttgcc gctacgtccc
60 agcgcgtcgg ccaccggctt ccagtcggca tcggtggtgg tcaccgccga acgagctttg
120 ccggcgtggc cgctgcccgc tccacccttg gagcccgaac tgcacgccgc cagtatcacc
180 gccgccgcgg tggtgatcgc gacgattctc ccagcatgtt tggcgcccgc catgcgcgtt
240 ccctccatcc gttgcatcca cggcgtggat ggcagttcgg ttagccatgg tctatcgggt
300 gattatgaaa ccacgatgaa gctcgatcgc accgatccgg gcacggccag acgtcctcat
360 cgacgccctg ggcgcgtatc tgctggccgc cgcggctctt cgacccgtgg aacgcatgcg
420 catccgcgcc gcgggcatca gcgccaccga cccacatgcc cgtctgccat tgccactggc
480 tcgagacgaa atccggtatc ttggaacaac attcaacgac cttctgcagc ggctgcaaga
540 cgcgctcgag cgagaacgtc aattcgtcag cgatgcgggc cacgaacttc gcaccccctt
600 agcctcctga ccaccgaact cgaactcgcc ctgcggcgtc cacgaagcaa ccccgaactg
660 ctcgccgcaa tccgctcggc tctcgcggaa accaccgaca ccgcgcgcac caccggcggc
720 accgggcttg gactggccat cgtcgacacc ctcagccaac gcaaccacgc cagcgtcacc
780 gcccgaaacc gcgccgcagg cggtgccgaa atctccctcc ggcttgctct tggctga
837
 <212> Type : DNA
   <211> Length : 837
   SequenceName : SEQ ID 139:GDC_MTUB_3356995
   SequenceDescription :

### Custom Codon

Sequence Name : SEQ ID 139:GDC_MTUB_3356995

### Sequence

<213> OrganismName : Mycobacterium tuberculosis-H37RV
<400> PreSequenceString : 
ttgcttgggc tgcccgaccc ccgccccgtc ccacgcaacc cggctgcccg tcgtcgggcg
60 acatcccggt ctctatcggc ggacccgagc agccgcccgg ctagccagtc gcggccaagg
120 ccagggacgt ggtgtacgag tgaaggttcc tcgcgtgatc cttcgggtgg cagtctaggt
180 ggtcagtgct ggggtgttgg tggtttgctg cttggcgggt tcttcggtgc tggtcagtgc
240 tgctcgggct cgggtgagga cctcgaggcc caggtagcgc cgtccttcga tccattcgtc
300 gtgttgttcg gcgaggacgg ctccgacgag gcggatgatc gaggcgcggt cggggaagat
360 gcccacgacg tcggttcggc gtcgtacctc tcggttgagg cgttcctggg ggttgttgga
420 ccagatttgg cgccagatct gcttggggaa ggcggtgaac gccagcaggt cggtgcgggc
480 ggtgtcgagg tgctcggcca ccgcggggag tttgtcggtc agagcgtcga gtacccgatc
540 atattgggca acaactga
558
 <212> Type : DNA
   <211> Length : 558
   SequenceName : SEQ ID 140:GDC_MTUB_3381198
   SequenceDescription :

### Custom Codon

Sequence Name : SEQ ID 140:GDC_MTUB_3381198

### Sequence

<213> OrganismName : Mycobacterium tuberculosis-H37RV
<400> PreSequenceString : 
atgattttct gggcaaccag gtactgcacg atctggttgc cgccttcacc ctcgtcggtg
60 accttctccc cggcagtctt ggccggtttg ggcgtcgacg ccagcacggt ggatccggcg
120 ttggccagcc ccacctcgtc gctctcgaca ccgatctcgg ccagggtcag cacggtaact
180 tccttcttct tggcggccat gatgcctttg aaggacggga agcgcggctc gttgatcttc
240 tcgttcacgc tgatcaccgc gggcagcgtg gcctcgaggg tgaatacgcc ctcatcggtc
300 tcacgctcgc cggtgatctt gccgccctcg atcgacactt tgcgcaggtg ggtgagctgc
360 ggcaggccca ggtactcggc gatgatggcc ggcaccgcac cgcccacccc gtcggtcgat
420 tcgttgcctg cgatcaccag ctcggtgccc tcgatggtgc ccaacgcgcg cgccaaagcc
480 cacccggttt ggatgacgtc cgagccgtgc atgccgtcgt cctttaggtg gacggccttg
540 tcggcaccca tcgacagcgc cttgcggatc gcctcggtgg cgcgctcggg gcccgccgtc
600 agcacggtta ccgacccttc gatgccgtcg gcggcctctt tctcccgaat ctgtagcgct
660 tcctccacgg cgcgctcgtt gatctcgtcc agcaccgcgt cggcggcctc gcggtccagc
720 gtgaaatcgc cgtcggtcag cttgcgctcc gaccaggtat ctgggacctg cttgatcagg
780 accacgatgt tcgtcatgac tgtggttcgt cctcctcgaa ggcggcccgc agcgctcgac
840 tgcggaacct cggtcacacg ttttgcaacc gcacagcgat attactattc ggtaagttcg
900 cgtggtgcgc cctcacacca tagcgggtgg tag
933
 <212> Type : DNA
   <211> Length : 933
   SequenceName : SEQ ID 141:GDC_MTUB_3388071
   SequenceDescription :

### Custom Codon

Sequence Name : SEQ ID 141:GDC_MTUB_3388071

### Sequence

<213> OrganismName : Mycobacterium tuberculosis-H37RV
<400> PreSequenceString : 
ttgctctcct cctggccaag gccagggacg tggtgtacga gtgaaggttc ctcgcgtgat
60 ccttcgggtg gcagtctagg tggtcagtgc tggggtgttg gtggtttgct gcttggcggg
120 ttcttcggtg ctggtcagtg ctgctcgggc tcgggtgagg acctcgaggc ccaggtagcg
180 ccgtccttcg atccattcgt cgtgttgttc ggcgaggacg gctccgacga ggcggatgat
 
240 cgaggcgcgg tcggggaaga tgcccacgac gtcggttcgg cgtcgtacct ctcggttgag
300 gcgttcctgg gggttgttgg accagatttg gcgccagatc tgcttgggga aggcggtgaa
360 cgccagcagg tcggtgcggg cggtgtcgag gtgctcggcc accgcgggga gtttgtcggt
420 cagagcgtcg agtacccgat catattgggc aacaactga
459
 <212> Type : DNA
   <211> Length : 459
   SequenceName : SEQ ID 142:GDC_MTUB_3482312
   SequenceDescription :

### Custom Codon

Sequence Name : SEQ ID 142:GDC_MTUB_3482312

### Sequence

<213> OrganismName : Mycobacterium tuberculosis-H37RV
<400> PreSequenceString : 
ttgatcagat cgatcgatcg ctgggggtcc gctgccgggg gggcggtcgg cacgcccggt
60 gggaccgact gtaatggccg ctcctcccac ccagctcggt ctgcggcgac gaacacatcg
120 atctcggccc agggcgccgc gggtccctgg gtcaagaatc gggggcgttc cagttttccg
180 gtggcctcat gcagccgcac cgccgccgag acgacctcat catgcctagg ctccggcgcg
240 ccggcgacga acgtgtctgc ccgccaacca gacaccacgt accggccgtc ggtcgatcgg
300 acgggccgag ccaggcgtac gccgtcgacg aacaacgtct cgcgcacccg ggccgaccag
360 gccgcgcggg cgttgtcggc caccatcgac aacaccacct cgccgcatcg ccagccacct
420 tcccaaccgg cacccaacag gatgggttgc gcacctgcca aaccgaacgc caccaacacg
480 tgctcggcgg gcggctcgac attcacaccg gtcagcctag tagagcccat cggggtgtat
540 tgggcctgta tcggtcctag tacatcacca tgtcgggctg catctgcttg gcccacgcga
600 cgatcccacc ctgcaggtgt accgcgtcgg agaaaccggc tttcttga
648
 <212> Type : DNA
   <211> Length : 648
   SequenceName : SEQ ID 143:GDC_MTUB_3581973
   SequenceDescription :

### Custom Codon

Sequence Name : SEQ ID 143:GDC_MTUB_3581973

### Sequence

<213> OrganismName : Mycobacterium tuberculosis-H37RV
<400> PreSequenceString : 
atgcggtgta gggcggcgtt gagctggcgg ttgcccgagc ggctgagccg catctggccg
60 gcggtgttgc ccgaccacac cgggatggga gccactgcgg catggcaggc gaaggcggct
120 tcgcttttga accgggtcac tccggcggct tcgccgacga ttttggctgc agtcagctcc
180 gcgcagccag ggatttccag cagtgcgggg gcgacctggt ggactcgggc gctgatgcgc
240 tgggctaggg tgttgatctc gccggtgagc cggatgatgt cggtcagctc ggcgcgcgcg
300 agttcggcga ccaatcctgg ctgggtgtcc agccaggtcc gcagggcctg ctggtgcttg
360 gcggcatcga gcgagcgtgc tgccggtgcc cgctcgggat cgagttcatg gacgagccag
420 cgcaaccggt tgatcgccga cgtgcgttgg gccacaagga catctcgacg gtcagtcaac
480 aacttcaact cccgcgacgt ctcgtcgtgg gtggccaggg gtaggtcggt ttcacgcagc
540 accgcccgcg ccaccgccag cgcatcgatc ggatccgact tgccccgact gcgcgccgac
600 ttgcgggtct gggccatcag cttggtgggt acccgcacca cctgctggcc ggccgccagt
660 aggtcacgct ccagacgcgc cgacatgttg cggcagtcct cgatgcccca gatcagctcg
720 aggccgaact gttcacgggc ccacatgatg gctgtggcgt gcccggccgt ggtggccttg
780 acggtcttct caccgagttg gcgacccact tcgtcggtgg ccacaaaggt gtggctgtac
840 ttgtgcgcat cggttccaac aacaaccatg gtggttgcct ctgaaccgcc ccggtga
897
 <212> Tyre : DNA
   <211> Length : 897
   SequenceName : SEQ ID 144:GDC_MTUB_3711717
   SequenceDescription :

### Custom Codon

Sequence Name : SEQ ID 144:GDC_MTUB_3711717

### Sequence

<213> OrganismName : Mycobacterium tuberculosis-H37RV
<400> PreSequenceString : 
gtgccggatc tcctcgagtt tgcggccctt ggtctccggc gcaaagcggt acacgaccac
60 gaacgcgacg acggcgaacg tgccgaagac cgcgaaaacg cctgcgccgc cgagcacacg
120 cagcatggtg agcgagaagg cggcaacgat cgcgttggcc gtcagtgtcg aggtgagcat
180 cgggctcgat cccatcgacc gcagccggga cgggaagctc tccgcggcgt acacccagac
240 cagcgagccg aatccgaagt tgaacccgat gatgaacagc agcacgccgg cgaaccccaa
300 caccagcccc gtgccaccat cggagtcgtt ggcgaatacg gtgatcagca cggcatctgc
360 ggtgatcatc gtcgcgatgc cggacaacag gatcgggcga cggcccagcc gatcgaccag
420 aaacagcgag gcacacaccg ccgccaagcc ggcgacttgc accatcgcgg gcagggcaag
480 catcgcgaaa tagcccgcga agcccatggc ggcgaaaagt cgcggactgt agtagatgat
540 cgcgttgatc ccggtgatct ggacgaggaa gccgagcgcg atgacgaaca gcgtggcccg
600 cagatacggc cgccgcacca tttcgccgat accgccgccg cgttcgtcga ccgcggccgc
660 catatcggcc agctcggcat cgatgtcggc ctccggctgg atccgccgca gcgcgctacg
720 cgcgtcggcg atccggccct tgagcagata ccagcgggcg gtatcggcca tgcgccacaa
780 caacggcaac agcagcgtgg ccggcgcggc ggccagcccg aacatcgcgc gccagccgtg
840 cgatccggcc aacaggtagc cgaccaggta accgacgacg atgccgctaa gcgtcgccag
900 ctgatacgcg gtcaccaacg acccacgcac cgccgccggc gccgactcgg ccacatacac
960 cggcaccacc accaccgaca ggccgattgt cacacccagc agcagacgcg ccaccaccag
1020 catc
1024
 <212> Type : DNA
   <211> Length : 1024
   SequenceName : SEQ ID 145:GDC_MTUB_3716987
   SequenceDescription :

### Custom Codon

Sequence Name : SEQ ID 145:GDC_MTUB_3716987

### Sequence

<213> OrganismName : Mycobacterium tuberculosis-H37RV
<400> PreSequenceString : 
gtgtctgacg ctacgacagt gttgttcggg ctgccaggag cacgggttga gcgtgtcgag
60 cgccgcagtg acgggacccg ggtggtcgat gtgatcaccg atgagccgac ggcggcggcg
120 tgcccgtcgt gcgggggtgg tctcgatatc agtgaaggaa tacgcggtta cctcaccgaa
180 agatctacct tatggcgaag accgcatcat ggtgcgctgg aacaaaattc gctggcgatg
240 ccgagaagac tactgcaagc tggggccgtt caccgaggcc atcacccagg tacctgcccg
300 cgtccgcagc acgctgcggc tgcgtcggca gatggccaag gcgatcgggg atgcggcccg
360 ctcggtgggc cgaggtcgcc caggctgacg ccgtgtcgtg gccgacggca catcgggcgt
420 ttgttgccta cgccgagacg ggtattgacc gagccgttgc ccaccccggt gctgggcgtt
480 gaccagacac ggcgaggaaa acccagatgg gagcgctgcg ccaagactgg ccggtgggta
540 cgggtcgacc cgtgggatac cgggttcgtc gacctggccg gtgatcaggg gtttatgggg
600 cagcatgaag gccgcggcgg cgcggcggtg ctggcatggc tgcaagcgcg cacaccgcag
660 ttccgggaga gcatccagta cggtggccat cgaccccgcc gctgcctacg cctcggcgat
720 ccgcacgccc gggctgctgc ccaacgccaa gctcgtcgtc gaccacttcc atgtgaccac
780 gctggccaac gacgcgctga ccgcggtgcg ccgccgggtg acctgggcgt tccacgaccg
840 gcgcggccgc aagatcgacc cgcagtgggc caaccgacgt cgcttgctga ccgcccggga
900 acgcttgtcg gacaaaagct tcgccaaaat gcggaatcgg atcaacgccg tcgaccccg
960 cgcgcagatt ctctcggcct ggatcgccaa agaggagctg cgcaccctgc tgtcgaccgt
1020 gcgc
 1024
   <212> Type : DNA
   <211> Length : 1024
   SequenceName : SEQ ID 146:GDC_MTUB_3754581
   SequenceDescription :

### Custom Codon

Sequence Name : SEQ ID 146:GDC_MTUB_3754581

### Sequence

<213> OrganismName : Mycobacterium tuberculosis-H37RV
<400> PreSequenceString : 
gtgcaggcat tgcccgaaag ccagctgcca gagctggccg tgcagatgcg tcggcggctc
60 atagaaacag tgacggctac cggtggccat ctcggcgcgg gacttggcat ggtagagctg
120 accatcgcat tgcatcgggt gttcacctcg ccacacgaca tcggtgttcg acaccgggca
180 ccaaacctat ccgcacaagc tgctcaccgg ccgcggtaa
219
 <212> Type : DNA
   <211> Length : 219
   SequenceName : SEQ ID 147:GDC_MTUB_3794808
   SequenceDescription :

### Custom Codon

Sequence Name : SEQ ID 147:GDC_MTUB_3794808

### Sequence

<213> OrganismName : Mycobacterium tuberculosis-H37RV
<400> PreSequenceString : 
atgtcttcag aggggggttg gcccaacgtc ggaaacctcg cgcgcagcgc atcaatgaca
60 tcggcagttt catcaagtgc cagggttgtc tgggtcagat acgatagctg ggtaccctcg
120 ggcaggttca acgctgccac atcagcgggt gtctgcacca ataatgttga ccgcggagcg
180 acgccaagcg tgccttcggt ctcctcatgt ccggcgtgcc cgatgaagac caccgtgtca
240 ccgcgcgcgg caaaccgtgc ggcttcagcg tggactttcg ccaccagtgg gcaggtcgcg
300 tcgacgacct gcagtccccg ctcatcagcg cccgcgcgca ccgccgggga aaccccatgc
360 gcggagaaca ccacgaccgc ccccggcggc ggcggatcgg gaatctcgtc gagatcctcg
420 acgaacactg ctccccggtc ccgcaactcg gcaaccacaa cagtgttgtg cacgatttgc
480 ttgcgcacat acaccgggcc ttcggccacg tcaagcactc gcttgaccgt ctcgatagca
540 cgctctacac cggcgcaaaa cgaccgcggc gacgccaaca gcaccgtgac ttcacccgaa
600 gcgtatccct gtgcgaccgg tcccacgaac acctcagcca tcagcactcc cggcgacata
660 tcagttgcga caacgcgatc aggtctgggg atcgcaccgc atcgggcagt gccgcaatag
720
 <212> Type : DNA
   <211> Length : 720
   SequenceName : SEQ ID 148:GDC_MTUB_3796793
   SequenceDescription :

### Custom Codon

Sequence Name : SEQ ID 148:GDC_MTUB_3796793

### Sequence

<213> OrganismName : Mycobacterium tuberculosis-H37RV
<400> PreSequenceString : 
ttgcctgggc atcgtcgggg cacgtcggct tcaagggttc ccggaaatcg accccgtttg
60 cggcccagct ggccgcggag aacgccgctc gcaaggccca agaccacggg gtgcgcaagg
120 tcgacgtgtt cgtcaagggc ccgggctcgg gccgcgagac cgcgatccgg tcgctgcagg
 
180 ccgccggcct ggaggtgggc gcgatctcgg atgtcacccc ccagccgcat aacggtgtcc
240 ggccccccaa gcgccggcgc gtctaggaga gaagatggct cgttacaccg gacccgtcac
300 ccgcaaatca cggcggttgc gcaccgacct cgtcggtggc gaccaggcct tcgagaagcg
360 tccctacccg cccggccaac acggtcgcgc gcggatcaag gaaagcgaat atctgcttca
420 gctgcaggag aagcagaagg cccgtttcac atacggcgta atggaaaagc agttccgccg
480 ctactacgaa gaggccgtgc ggcagcccgg caagacgggt ga
522
 <212> Tyre : DNA
   <211> Length : 522
   SequenceName : SEQ ID 149:GDC_MTUB_3879013 SequenceDescription :

### Custom Codon

Sequence Name : SEQ ID 149:GDC_MTUB_3879013

### Sequence

<213> OrganismName : Mycobacterium tuberculosis-H37RV
<400> PreSequenceString : 
gtgggacgcc gtgatcgcgg tgcacctgcg cggccatttt ctgctcaccc gcaacgccgc
60 tgcctactgg cgggacaaag ccaaggatgc cgaaggggga tcggtcttcg gccggctcgt
120 caacacctcg tcggaggcgg gtctggtggg cccggtgggg caggcgaatt acgccgccgc
180 caaggctggc atcaccgcgc taaccctgtc ggcggcgcgg gcgctcgggc gctacggcgt
240 ttgcgccaat gtgatttgtc cgcgggcgcg caccgcgatg acggccgatg tcttcggcgc
300 cgcacccgat gtcgaagcgg gccagatcga cccgctgtcg ccgcagcatg tggtaagcct
360 ggtccagttt ctggcgtccc cggctgccgc ggaagtcaac ggtcaggtgt tcatcgtcta
420 cggtccgcag gtgacgctgg tgtcaccgcc gcacatggag cgccggttca gcgcggacgg
480 cacgtcctgg gatcccaccg agctcaccgc gacgctgcgg gactactttg ctggtcggga
540 tccggaacag agcttttcgg cgaccgatct gatgcgtcag tgacccgtgg atataggcgg
600 ccgattattg gaatcggtgt ccgaatcacc acgccaacat ag
642
 <212> Type : DNA
   <211> Length : 642
   SequenceName : SEQ ID 150:GDC_:MTUB_3921024
   SequenceDescription :

### Custom Codon

Sequence Name : SEQ ID 150:GDC_MTUB_3921024

### Sequence

<213> OrganismName : Mycobacterium tuberculosis-H37RV
<400> PreSequenceString : 
ttgccttgga cggcatgttg ctccccttat tcgaacgaca accggaccaa acccagcccg
60 gtgaagtcgg cgacaaactc gtcgccggcc cgcgcctcga ccgcgaacgt gcatgacccg
120 ggtaacacga tgtcgccttt gcgcagccgc acgccgaaac tctcgacctt gccggccagc
180 caagccaccg cggtcgccgg gttacccaac accgcatcac tgcggccctc ggccaccacc
240 tcgccgttgc gggtcagctt cgcatcgatc gccctgacgt caagatcggc cggcggcacc
300 cgggccgcgc ccaacacgaa gcccgccgcc gaggcgttgt cggcgatggt gtcgcagatc
360 ttgatctgcc aatccttgat cctggtgtcg atcagctcga tggcgggcac cagggcctcg
420 gtggccgcca gcacgtcgtc ctcggtgcag cccgcacccg gtaggtcggc ggccaggatg
480 aagcccacct ccacctcaac ccgcggagac aggtaccggg acgcctggac cggcgtgtct
540 tcgaacacct gcatgtcgtc gagcaggtgt ccgtag
576
 <212> Type : DNA
   <211> Length : 576
   SequenceName : SEQ ID 151:GDC_MTUB_3974481
   SequenceDescription :

### Custom Codon

Sequence Name : SEQ ID 151:GDC_MTUB_3974481

### Sequence

<213> OrganismName : Mycobacterium tuberculosis-H37RV
<400> PreSequenceString : 
gtggttcact ctcggcgctc atgggcgcca tcccgccgcc cgcatcgcgg catcgacgcg
60 gccaacgaac gtgccccggc ggtaccagag cagctcactg gtgaccctga tgatcgtcca
120 gcccagatcc agcaacgcgg tggaccgctc gatgtcccga gcccgctgcg ccgggtctgt
180 ccaatgctgt ggcccgtcat actcgacacc gactcgcaat tgctcgtagc ccaggtcgat
240 gcgggcgacg aagtccccgt agtcgtcaaa cactctgatc tgtgtttgcg gcttcggcag
300 accggcatcg atcaacacca atcgggtcca cgtctcctgt ggggattccg cacccccgtc
360 gatcagcggc agcaccgcac ggaggcggac caggccgcgc gcaccggtat gttcggcaat
420 gacggcctgc acgtcggcga ccttgacatc ggtcgaattc gccaacgcgt ccagccgttg
480 aacggcctgc agccgcgagg gtgtgcgccg cccgatatcg aaggcggtgc gcgccggggt
540 ggttaccgcg acaccgtcaa ccgcaaccgt ctcgtgcggc gccaatcgat ccgtgtgcac
600 gacgatgcgc ggcggaggct ttcgattggc gtgcactaa
639
 <212> Type : DNA
   <211> Length : 639
   SequenceName : SEQ ID 152:GDC_MTUB_3994808
   SequenceDescription : ,

### Custom Cordon

Sequence Name : SEQ ID 152:GDC_MTUB_3994808

### Sequence

<213> OrganismName : Mycobacterium tuberculosis-H37RV
<400> PreSequenceString : 
gtgtcgcgct accccaacag ctggcgcagg ttgaacaacc ccgatatggc ggtgcccatg
60 ttaaacaggc ccgtgttcaa gccgctccgg acggagccaa agaggtgccc cgggacgccg
120 atgttgccaa tgcccgaggt ctggccgttg atgacagtgc ccccgctggc cgtgttgaag
180 aacccggaga cgtcgacggc taagggcccg gtgggggtgt tgaagaagcc cgagacgtcg
240 gtgccggtgt tgccgaagcc cgagttggtc aggccgctgt cggtaatgat cccgaaaccg
300 gtgttcacat tgcccgcatt ccacgagccg gtgttgatgt tgcccgagtt cccattgccg
360 gtgttgacgt tgccggagtt gtcaaacccc gtgttgacga agcccgcgtt tccgaagccg
420 gtgtttaatt cacccgcgtt ccccaagccg gtgttgagga tgctcgcgtt cccgaagccg
480 gtgttgagaa cgcccgcgtt cccgaagccg atgttggcgt tgccggaatt cccgacgccc
540 aggttgttga ggtcgccagg caccagggta ttggctccgg tgttgaagac gccgatgttg
600 ccgctgccgg agttgaacaa gccgatgttg ttggtgccgg agttgccgat gccgatattg
660 ccgctgccgg agttcagcag cccggccagg ttgatgccca tctga
705
 <212> Type : DNA
   <211> Length : 705
   SequenceName : SEQ ID 153:GDC_MTUB_3998938
   SequenceDescription :

### Custom Codon

Sequence Name : SEQ ID 153:GDC_MTUB_3998938

### Sequence

<213> OrganismName : Mycobacterium tuberculosis-H37RV
<400> PreSequenceString : 
ttgagctcaa atcatgcgat tctgcgtctg ctcgcgccct tgcggctaga tccccagaac
60 ctgggcgctg gcccacagcg cgagcaccgc catcgccagg gccgcaggca cggtgcacag
 
120 tcccagtcgg gtgtactcgc cgacgctggc gtcgacgttg tgccggcgca gcacgccccg
180 ccacagcagg ttagacagcg aaccggcata ggtcaggttg ggtccgatgt tgaccccgag
240 tag
243
 <212> Type : DNA
   <211> Length : 243
   SequenceName : SEQ ID 154:GDC_MTUB_4021183
   SequenceDescription :

### Custom Codon

Sequence Name : SEQ ID 154:GDC_MTUB_4021183

### Sequence

<213> OrganismName : Mycobacterium tuberculosis-H37RV
<400> PreSequenceString : 
ttgtgccagg gtgtacccgc ccgattgccg ccggcaaccg acactgttgg tgtagtgacc
60 aaatcagcag tgccccgggt gggtcttgac gtgcaaatcg actacagtct tggtgaccgt
120 ccggtacccg ggcatgggac tggaacgaac caagaaacct gtgaggccgt ctgctatgga
180 gcggttcgac ggtttgcgtc cggccaggct caaggtgggg atcatctcgg ctggccgggt
240 cggcaccgcg ctaggggtcg cgctgcagcg cgccgaccat gttgtggtgg cgtgcagcgc
300 catctctcat gcgtcccggc ggcgcgcgca gcgccggctg cctga
345
 <212> Type : DNA
   <211> Length : 345
   SequenceName : SEQ ID 155:GDC_MTUB_4045946
   SequenceDescription :

### Custom Codon

Sequence Name : SEQ ID 155:GDC_MTUB_4045946

### Sequence

<213> OrganismName : Mycobacterium tuberculosis-H37RV
<400> PreSequenceString : 
atgcggcccg caaaacgggc cgaggaggag ccaggcaatc accccagagc cgggtgcagc
60 gggtcgccac catcagcccc gtggcgatcg caaaccccgc gcctggcgac aatgcggccc
120 gcaaaacggg ccgaggagga gccaggcaat caccccagag ccgggtgcag cgggtcgcca
180 ccatcagccc cgtggcgatc gcaaaccccg cgcctggcga caatgcggcc cgcaaaacgg
240 gccgaggagg agccaggcaa tcaccccaga gccgggtgca gcgggtcgcc accatcagcc
300 ccgtggcgat cgcaaacccc gcgcctggcg acaatgcggc ccgcaaaacg ggccgaggag
360 gagccaggca atcaccccag agccgggtgc agcgggtcgc caccatcagc cccgtggcga
420 tcgcaaaccc cgcgcctggc gacaatgcgg cccgcaaaac gggccgagga ggagccaggc
480 aatcacccca gagccgggtg cagcgggtcg ccactggcta gaccaacgac cggtagttcc
540 cgacggcgtc ggaaaatccg acagctgagc gttcgggtca aacacgcggt gcaccggacc
600 tga
603
 <212> Type : DNA
   <211> Length : 603
   SequenceName : SEQ ID 156:GDC_MTUB_4053033
   SequenceDescription :

### Custom Codon

Sequence Name : SEQ ID 156:GDC_MTUB_4053033

### Sequence

<213> OrganismName : Mycobacterium tuberculosis-H37RV
<400> PreSequenceString : 
atgcgcacta cgatcgacct cgatgacgac atactgcggg cgttgaaacg acgccagcgc
60 gaggagcgca aaacgttagg gcagctcgcc tccgaattgc ttgcgcaagc tctggcggcc
120 gagcctcctc caaacgttga catccgctgg tcgactgccg acttgcggcc ccgtgtggat
180 cttgacgaca aggacgctgt ttgggcgatt ttggaccgtg ggtga
225
 <212> Type : DNA
   <211> Length : 225
   SequenceName : SEQ ID 157:GDC_MTUB_4140236
   SequenceDescription :

### Custom Codon

Sequence Name : SEQ ID 157:GDC_MTUB_4140236

### Sequence

<213> OrganismName : Mycobacterium tuberculosis-H37RV
<400> PreSequenceString : 
gtgtcacgtt gtcggattca ctgtcgccgg ctagcgcttt cccgtcagaa gacgagaagc
60 ctccccgatc tccaactagc atcgagatcg ggcttgcgaa ggttgggttg caaaatggat
120 gtcatcagat gggctcgccg gcttgcggtg gtggcgggca cagcagcggc agtgaccact
180 cctgggctac tgagtgcgca cgttccgatg gtctccgccg aaccgtgtcc cgacgtcgag
240 gtggtgtttg cccgtggcac cggggagcca cctggtattg gcagcgtcgg aggactgttc
300 gtcgacgcac tgcgtttccc aggttggcgc caagtcactc ggggtctacg ccgttaa
357
 <212> Type : DNA
   <211> Length : 357
   SequenceName : SEQ ID 158:GDC_MTUB_4169350
   SequenceDescription :

### Custom Codon

Sequence Name : SEQ ID 158:GDC_MTUB_4169350

### Sequence

<213> OrganismName : Mycobacterium tuberculosis-H37RV
<400> PreSequenceString : 
gtggatgcat gtcattcccg ggcgcggcgc ggcgtggttg atcgtcgacg tccgagatgt
60 ggcggcactg cacgcggcgt tgttggaatc cgggcgtggg ccgcgccgct acactgcggg
120 aggtcatcgg attccggtgc ccgagctcgc gaaaattctg ggcgggtcgc cggcaccacg
180 atgctggccg tcccggtgcc cgattccgcg ctgcgtgtcg cgggatcggt gctggatcaa
240 gccgggccct atctgccttt caatactccg ttcaccgcgg caggtatgca gtactacaca
300 cagatgccgg agtccgacga ttcgccgagc gaaaaagaac taggcatcac ctaccgcgat
360 ccgcgcgaca ccgtggccga caccgtcacg gccctgcgcg gcctgggcag ctaa
414
 <212> Type : DNA
   <211> Length : 414
   SequenceName : SEQ ID 159:GDC_MTUB_4170798
   SequenceOescription :

### Custom Codon

Sequence Name : SEQ ID 159:GDC_MTUB_4170798

### Sequence

<213> OrganismName : Mycobacterium tuberculosis-H37RV
<400> PreSequenceString : ttgatgtgga agccgcgctg gcgatggtgt tcgacggctt cggagcggcg aaccaccgcc 
60 agcccagatg cctgccgcaa cgtatcgcgg tgccggtcac caagcttaag acttgccggc
120 tcgggatcac cgtggcatcg gatgcgatcg agatccacgg cggcaatggc tacatcgaga
180 cctggccggt ggcccggttg ctgcgtgacg cgcaagtcaa cacgatctgg gagggccccg
240 acaacatcct gtgtctggat gtgcggcgcg ggatcgagca gacgcgcgct cacgagacac
300 tgttggcgcg gctgcgcgat gcggtgtcgg tgtccgacga tgacgacacc acgcggctgg
360 tctcgcgccg cattgaggac ctcgacgcgg cgatcaccgc ttggaccaaa ctcgacaggc
420 agctggccga ggcgcggctg ttcccgctgg cccaattcat gggcgacgtc tacgccggcg
480 cgttgctcac cgagcaggcc gcctgggaac gggcaacccg cggcaccgac cgcaaggcac
540 tcgtcgcccg cctgtacgcg cgccggtatc tcgccgacca aggcccgctg cgcggtatcg
600 acgcagattg cgatgaggcg ctgcagcgtt tcgacgaact cgtggcgggc gcgttcactg
660 ccgagcagac gtaaaagccc ccaattcgtg gctcttctga cacttccgtg ggtgagtttg
720 tgtcctgagt ag
732
 <212> Type : DNA
   <211> Length : 732
   SequenceName : SEQ ID 160:GDC_MTUB_4252190
   SequenceDescription :

### Custom Codon

Sequence Name : SEQ ID 160:GDC_MTUB_4252190

### Sequence

<213> OrganismName : Mycobacterium tuberculosis-H37RV
<400> PreSequenceString : 
gtgcgggccc cggcgacccg cgcggccagc cgcggctctt cgaggaattc cgaccagcgc
60 ccgtcgggca ggtcggtgat cccgtcgcgg ccttccagca gcgcctgcca ggtctgctcg
120 ggggtgttca tctcgcccgg gaagcgggtg gacaagccca cgatcgcgat gtcgacgcgc
180 tcggccgggc cggtgcgcga ccagtcttcg gcgtcatcgc ccgctaggtc ggtctccggc
240 tcgccctcga tgatccgggt ggccagcgat tcgatggtcg gatgcgcgaa cgccaccgcg
300 accgacagcg tgaccccggt caggtcttct atgtcggcgg ccatcgcgac ggcatcgcgc
360 gacgacagac ccagctccac catgggcacc gattcgtcga tcgagtccgg tgcctttccg
420 acggccttac ccacccagtt gcgcagccac tggcgcatct cggggaccgt tagctcggcc
480 ctttcggcgg gggcgttctc ctgggattcc gctacgtcag ccatgggtcc tcagtccgaa
540 gtggcgaaga ccgtcgggga acccacgcca ctgcgcaggc tgccgtcgag gtag
594
 <212> Type : DNA
   <211> Length : 594
   SequenceName : SEQ ID 161:GDC_MTUB_4260620
   SequenceDescription :

### Custom Codon

Sequence Name : SEQ ID 161:GDC_MTUB_4260620

### Sequence

<213> OrganismName : Mycobacterium tuberculosis-H37RV
<400> PreSequenceString : 
ttgcacgagg acccgcacac tggcgtcgag ccgggtgccg ttacggcgca ccgagattgc
60 cagcacccgc gcccggcctg tggcgatgag ccgttcaatc cggcgtgtgt tctcgtgcgt
120 acggacggtc ccgacgaccg gaagtgtgag atgacggcga tcaggttcga cgcgcatcgc
180 tccggtcgtg aatgtcacgc ggtcctgatc gcggcctttc ttcttgaacc gggggaagcc
240 cattgtcttg ccctcacgtt taccggatcg ggagttctgc cagttccagt acgcatcgac
300 agcgccgcca atgccgtcgg cgtaagcctc tttcgagcac tccggccacc acaccgcccc
360 ggtctcggcg ttgacacaca cctcgtcctt gacggtgttc caccgtttac gaagcacccg
420 cagcgacggc ttgacagtcc cgataccagt aacgcgccac gcctcgatat cggctttcaa
480 agtagcgacc gcccagttgt aggccttgcg gcgagcgccg aaatgccgcg ccagcgcgcg
540 ggcctggtcc tcggttgggt ccagcgtgaa ccggaacgcc tgcacacacc agccttctgg
600 cacctcgaat ctggccatca agctgcctcc gcgtccccga ccgcagcagc aagggcacgc
 
660 ttggccccgt tctgtgcagc gcgttcacca tag
693
 <212> Type : DNA
   <211> Length : 693
   SequenceName : SEQ ID 162:GDC_MTUB_4302166
   SequenceDescription :

### Custom Codon

Sequence Name : SEQ ID 162:GDC_MTUB_4302166

### Sequence

<213> OrganismName : Mycobacterium tuberculosis-H37RV
<400> PreSequenceString : 
ttgcgcccgt caaggtccac cctgatagcc aaatgcgcca gctggcggca accaccccgt
60 tgtcttcgat ccgcagccgt aaaccgtcgt tcgtcggcgc ccgtcgccca acgtgaactg
120 agggcggaga atcggccgga atctcgccct cagttcacgc tcggcgccgt ttggcctcac
180 ccagtcaatg tgatctgtgc gggcgggcgt tggcgcgtag cgaaccccag tggcgccggc
240 ccgccaagca cgccccggcg cggccagctc atcagcggct acgcaagcgc aacggcgccc
300 gcgatgggct gtggaagaac ccggaggatc tcaccgaaca ccagaatgcc aagctgtcgc
360 gctcatctac tcaaagaagg cctacggcac ctgttttcgg tcaaaggcga agagagtaag
420 caggcactgg accggttgat cttctag
447
 <212> Type : DNA
   <211> Length : 447
   SequenceName : SEQ ID 163:GDC_MTUB_4317863
   SequenceDescription :

### Custom Codon

Sequence Name : SEQ ID 163:GDC_MTUB_4317863

### Sequence

<213> OrganismName : Mycobacterium tuberculosis-H37RV
<400> PreSequenceString : 
gtgcattcgg ctagctcggt tgccacaccc gtcaggggtt cgacgttggc gggttcggcg
60 ggccccagca ccgctgtcac catgcccgcc aagccgacct gcggcgccac caactgcagc
120 accagcatgt cgccgtcgcg cgccgcgatc acatggcggt cgcccctgcg gcacacgacg
180 aagcgcacca tgacgccgcc aatgtcgcgc cgccaccagc gaccctccaa ggtccgatct
240 ggcctgccca gggtttcgac catctccgcg accgtcggtt ggggctcccc gtggaggtcg
300 agcacccctt gcgctgtgag gtcacgctgc acctgttccc agacgatgtc tcgcagatcc
360 tcttgcggga tattcggccg aatcccaagc gtgacaggga aatcaaccag gtgtaaccga
420 tcggcgatca ccaacatgcc gtcgatggtt acctcgacgc cgaccacgtt gtcggcggtg
480 cccgcgcggc ctgcagcgga cggacccgtc atgatcaacc gaaaatcttg tcgataa
537
 <212> Type : DNA
   <211> Length : 537
   SequenceName : SEQ ID 164:GDC_MTUB_4341852
   SequenceDescription :

### Custom Codon

Sequence Name : SEQ ID 164:GDC_MTUB_4341852

### Sequence

<213> OrganismName : Mycobacterium tuberculosis-H37RV
<400> PreSequenceString : atggaccgac tctgcggtgc gccgctatgt caccgacgcc ggggccctac tgccacggct 
60 gcacaagctg gtgcgcgccg actgcacgac ccgcaacaag cgccgggccg cgcggttgca
120 ggccagttac gaccggctgg aagagcggat cgcggagctg gccgcccagg aggatctgga
180 tcgggtgcgc cccgacctgg acggcaacca gatcatggcg gtgctcgaca ttccggcggg
240 cccgcaagtc ggcgaggcgt ggcgctactt gaaggagctg cggctagagc gcggcccgtt
300 gtccaccgag gaggcgacaa ccgagctgct gtcctggtgg aaatcacggg ggaaccgcta
360 gcttgggagt cgcgtcagaa cggttgtgga gtactgcata gccggcgacg acggcagcgc
420 cgggatctgg aaccgcccgt tcgacgtcga cctcgacggt ga
462
 <212> Type : DNA
   <211> Length : 462
   SequenceName : SEQ ID 165:GDC_MTUB_4391527
   SequenceDescription :

### Custom Codon

Sequence Name : SEQ ID 165:GDC_MTUB_4391527

### Sequence

<213> OrganismName : Sars coronavirus
<400> PreSequenceString : 
gtgacgagct tggcactgat cccattgaag attatgaaca aaactggaac actaagcatg
60 gcagtggtgc actccgtgaa ctcactcgtg agctcaatgg aggtgcagtc actcgctatg
120 tcgacaacaa tttctgtggc ccagatgggt accctcttga ttgcatcaaa gattttctcg
180 cacgcgcggg caagtcaatg tgcactcttt ccgaacaact tgattacatc gagtcgaaga
240 gaggtgtcta ctgctgccgt gaccatgagc atgaaattgc ctggttcact gagcgctctg
300 ataagagcta cgagcaccag acacccttcg aaattaagag tgccaagaaa tttgacactt
360 tcaaagggga atgcccaaag tttgtgtttc ctcttaactc aaaagtcaaa gtcattcaac
420 cacgtgttga aaagaaaaag actgagggtt tcatgggcgc tatacgctct gtgtaccctg
480 ttgcatctcc acaggagtgt aacaatatgc acttgtctac cttga
525
 <212> Type : DNA
   <211> Length : 525
   SequenceName : SEQ ID 166:GDC_Sars174_refseq
   SequenceDescription :

### Custom Codon

Sequence Name : SEQ ID 166:GDC_SarS174_refseq

### Sequence

<213> OrganismName : Sars coronavirus
<400> PreSequenceString : 
ttggacctga gcatagtgtt gcagattatc acaaccactc aaacattgaa actcgactcc
60 gcaagggagg taggactaga tgttttggag gctgtgtgtt tgcctatgtt ggctgctata
120 ataagcgtgc ctactgggtt cctcgtgcta gtgctgatat tggctcaggc catactggca
180 ttactggtga caatgtggag accttga
207
 <212> Type : DNA
   <211> Length : 207
   SequenceName : SEQ ID 167:GDC_Sars68_refseq
   SequenceDescription :

### Custom Codon

Sequence Name : SEQ ID 167:GDC_Sars68_refseq

### Sequence

<213> OrganismName : Sars coronavirus
<400> PreSequenceString : 
atggtgactt cttgcatttt ctacctcgtg tttttagtgc tgttggcaac atttgctaca
60 caccttccaa actcattgag tatagtgatt ttgctacctc tgcttgcgtt cttgctgctg
120 agtgtacaat ttttaaggat gctatgggca aacctgtgcc atattgttat gacactaatt
180 tgctag
186
 <212> Type : DNA
   <211> Length : 186
   SequenceName : SEQ ID 168:GDC_Sars61_refseq
   SequenceDescription :

### Custom Codon

Sequence Name : SEQ ID 168:GDC_Sars61_refseq

### Sequence

<213> OrganismName : Sars coronavirus
<400> PreSequenceString : 
ttggcacccg caatcctaat aacaatgctg ccaccgtgct acaacttcct caaggaacaa -
60 cattgccaaa aggcttctac gcagagggaa gcagaggcgg cagtcaagcc tcttctcgct
120 cctcatcacg tagtcgcggt aattcaagaa attcaactcc tggcagcagt aggggaaatt
180 ctcctgctcg aatggctagc ggaggtggtg aaactgccct cgcgctattg ctgctag
237
 <212> Type : DNA
   <211> Length : 237
   SequenceName : SEQ ID 169:GDC_Sars78_refseq
   SequenceDescription :

### Custom Codon

Sequence Name : SEQ ID 169:GDC_Sars78_refseq

### Application Project

<120> Title :
<130> AppFileReference :
<140> CurrentAppNumber :
   <141> CurrentFilingDate ;

### Sequence

<213> OrganismName : Haemophilus influenzae
<400> PreSequenceString : 
VTSLALIPLK IMNKTGTLSM AVVHSVNSLV SSMEVQSLAM STTISVAQMG TLLIASKIFS
60
HARASQCALF PNNLITSSRR EVSTAAVTMS MKLPGSLSAL IRATSTRHPS KLRVPRNLTL
120
SKGNAQSLCF LLTQKSKSFN HVLKRKRLRV SWGVYALCTL LHLHRSVTIC TCLP
174
 <212> Type : PRT
   <211> Length : 174
   SequenceName : SEQ ID 170:GDC_HINF_5641 SequenceDescription :

### Sequence

<213> OrganismName : Haemophilus influenzae
<400> PreSequenceString : 
LDLSIVLQII TTTQTLKLDS AREVGLDVLE AVCLPMLAAI ISVPTGFLVL VLILAQAILA
60
LLVTMWRP
68
 <212>Type : PRT
   <211> Length : 68
   SequenceName : SEQ ID 171:GDC_HINF_6322.
   SequenceDescription :

### Sequence

<213> OrganismName : Haemophilus influenzae
<400> PreSequenceString : 
MVTSCIFYLV FLVLLATFAT HLPNSLSIVI LLPLLAFLLL SVQFLRMLWA NLCHIVMTLI
60
C 6
1
 <212> Type : PRT
   <211> Length : 61
   SequenceName : SEQ ID 172:GDC_HINF_124181
   SequenceDescription :

### Sequence

<213> OrganismName : Haemophilus influenzae
<400> PreSequenceString : 
LAPAILITML PPCYNFLKEQ HCQKASTQRE AEAAVKPLLA PHHVVAVIQE IQLLAAVGEI
60
LLLEWLAEVV KLPSRYCC
78
 <212> Type : PRT
   <211> Length : 78
   SequenceName : SEQ ID 173:GDC_HINF_170553
   SequenceDescription :

### Sequence

<213> OrganismName : Haemophilus influenzae
<400> PreSequenceString : 
LLLKGVIMQV SRRKFFKICA GGMAGTSAAM LGFAPANVLA APREYKLLRA FESRNTCTYC
60
AVSCGMLLYS TGKPYNSLSS HTGTNTRSKL FHIEGDPDHP VSRGALCPKG AGSLDYVNSE
120
SRSLYPQYRA PGSDKWERIS WKDAIKRIAR LMKDDRDANF VEKDSNGKTV NRWATTGIMT
180
ASAMSNEAAL LTQKWIRMLG MVPVCNQANT
210
 <212> Type : PRT
   <211> Length : 210
   SequenceName : SEQ ID 174:GDC_HINF_231874
   SequenceDescription :

### Sequence

<213> OrganismName : Haemophilus influenzae
<400> PreSequenceString : 
MTNNWVDIKN ANLIIVQGGN PAEAHPVGFR WAIEAKKNGA KIIVIDPRFN RTASVADLHA
60
PIRSGSDITF LMGVIRYLLE TNQIQHEYVK HYTNASFLID EGFKFEDGLF VGYNEEKRNY
120
DKSKWNYQFD ENGHAKRDMT LQHPRCVINI LKEHVSRYTP EMVERITGVK QKLFLQICEE
180
IGKTSVPNKT MTHLYALGFT EHSIGTQNIR SMAIIQLLLG NMGMPGGGIN ALRGHSNVQG
240
TTDMGLLPMS LPGYMRLPND KDTSYDQYIN AITPKDIVPN QVNYYRHTSK FFVSMMKTFY
300
GDNATKENGW GFDFLPKADR LYDPITHVKL MNEGKLHGWI LQGFNVLNSL PNKNKTLSGM
360
SKLKYLVVMD PLQTESSEFW RNFGESNNVN PAEIQTEVFR LPTTCFAEEE GSIVNSGRWT
420
QWHWKGCDQP GEALPDVDIL SMLREEMHEL YKKEGGQGIE SFEAMTWNYA QPHSPSAVEL
480
AKELNGYALE DLYDPNGNLM YKKGQLLNGF AHLRDDGTTT SGNWLYVGQW TEKGNQTANR
540
DNSDPSGLGC TIGWGFAWPA NRRVLYSRAS LDINGNPWDK NRQLIKWNGK NWNWFDIADY
600
GTQPPGSDTG PFIMSAEGVG RLFAVDKIAN GPMPEHYEPV ESPIDTNPFH PNVVTDPTLR
660
IYKEDREFIG SNKEYPFVAT TYRLTEHFHS WTAQSALNII AQPQQFVEIG EKLAAEKGIQ
720
KGDMVKITSR RGYIKAVAVV TKRLKDLEID GRVVHHIGLP IHWNMKALNG KGNRGFSTNT
780
LTPSWGEAIT QTPEYKTFLV NIEKVGEA
808
 <212> Type : PRT
   <211> Length : 808
   SequenceName : SEQ ID 175:GDC_HINF_232170
   SequenceDescription :

### Sequence

<213> OrganismName : Haemophilus influenzae
<400> PreSequenceString : 
VMSRHRGAKH RRRYELLGGI SLLSPEYLLS VERWPFHSEP PDHYDLLSYL LDLSVSQLSL
60 .
 
LIPLH
65
 <212> Type : PRT
   <211> Length : 65
   SequenceName : SEQ ID 176: GDC_HINF_232813
   SequenceDescription :

### Sequence

<213> OrganismName : Haemophilus influenzae
<400> PreSequenceString : 
VFMLYLEFLF LLLMLYIGSR YGGIGLGVVS GIGLAIEVFV FRMPVGKHRL MLCLSFLQW
59
 <212> Type : PRT
   <211> Length : 59
   SequenceName : SEQ ID 177:GDC_HINF_233190
   SequenceDescription :

### Sequence

<213> OrganismName : Haemophilus influenzae
<400> PreSequenceString : 
MAAAIQQRAE LQRRIWQIAN DVRGSVDGWD FKQYVLGTLF YRFISENFAN YIEAGDESVN
60
YAQLPDEIIT QMPLKRKATL FTQANYLRML RLMLAAILI
99
 <212> Type : PRT
   <211> Length : 99
   SequenceName : SEQ ID 178:GDC_HINF_235441
   SequenceDescription :

### Sequence

<213> QrganismName : Haemophilus influenzae
<400> PreSequenceString : 
LNTDLKQIFT DIENSATGFP SEQDIKGLFA DFDTTSNRLG NTVKDKNDRL TAVLKGVAEL
60
DFGKFEDNHI DLFGDAYEYL ISNYAANAGK SGGEFFTPQS VSKLIAQIAM HGQTSVNKIY
120
DPAAGSGSLL LQAKKQFDEH IIEEGFFGQE INHTTYNLAR MNMFLHNINY DKFDIALGNT
180
LMEPQFGDNK PFDAIVSNPP YSVKWAGSDD PTLINDERFA PRRRACTKIQ SGLCLYFTCV
240
KLSFSKRPRG DCFLPWYFLS WRCRAKNSSI FGG
273
 <212> Type : PRT
   <211> Length : 273
   SequenceName : SEQ ID 179:GDC_HINF_235913
   SequenceDescription :

### Sequence

<213> OrganismName : Haemophilus influenzae
<400> PreSequenceString : 
MMNDLPPAGV LAPKSKADFA FILHALSYLS AKGRAAIVSF PGIFYRGGAE QKIRQYLVDN
60
NYVDAVIALA PNLFFGTSIA VNILVLSKHK PNLSMPAVYL NLPLITTF
108
 <212> Type : PRT
   <211> Length : 108
   SequenceName : SEQ ID 180:GDC_HINF_240336
   SequenceDescription :

### Sequence

<213> OrganismName : Haemophilus influenzae
<400> PreSequenceString : 
VPHLAKSISF EEIAQNDYNL AVSSYVEQKD TREVINIDEL NAQIRETVTN IDHLRAEIDK
60
IVAEIEG
67
 <212> Type : PRT
   <211> Length : 67
   SequenceName : SEQ ID 181:GDC_HINF_243018
   SequenceDescription :

### Sequence

<213> OrganismName : Haemophilus influenzae
<400> PreSequenceString : 
MTQYKTIAES NNFIVLDQYN KFVEESNAGY QTERSLEREF IRDLQAQGYE YLQWLNNHDE
60
LIKNLRAQLQ RLNNVVFSDA EWQRFLEEYL DKPSDNLIEK TRKIHDDYIY DFVFDNGRIQ
120
NIYLLDKKNL ANNSLQVINQ FKQTGSYDNR YDVTILVNGL PLY
163
 <212> Type : PRT
   <211> Length : 163 1
   SequenceName : SEQ ID 182:GDC_HINF_274892
   SequenceDescription :

### Sequence

<213> OrganismName : Haemophilus influenzae
<400> PreSequenceString: 
MVYPFIELKK RGVAIREAFN QIHRYSKESF NKENSLFKYI QIFVISNGTD TRYFANTTKR
60
NKNSYDFTMN WATAKNTLIK DLKDFTATFL QKNTLLNVLV NYCVFDVSDT LLIMRPYQIA
120
ATERILWKIQ ISYLAKNWSN RESGGYIWHT TGSGKTLTSF KASRLATELD FIDKVFFVVD
180
RKDLDYQTMK EYQRFSPDSV NGSESTAGLK RNIEKDDNKI IVTTIQKLNN LMKSEENLSI
240
YQKQVVFIFD EAHRSQFGEA QKNLKRKFKK FYQFGFTGTP IFPENALGAE TTASVFGAEL
300
HSYVITDAIR DDKVLKFKVD YNDVRPQFKA LETEKDPEKL TALEQKQAFL HPERIKEISQ
360
YLLNNFKQKT HRLNATGKGF NAMFAVSSVE AAKRYYETLQ NLQAEQEYPL KIATIFSFAA
420
NEEQDAIGDI PDETFEPTAL NSTAKEFLTK AIDDYNHYFG TNYGVDSQSF QNYYRDLAKR
480
VKNQEVDLLI VVGMFLTGFD APTLNTLFVD KNLRYHGLMQ AFSRTNRIYD TTKTFGNIVT
540
FRDLEQNTID AITLFGDKNT KNVVLEKSYD SYFNGDDNQR GYAEIVKELK ESFPDPTEIE
600
TEQDKKEFVK LFGEYLRVEN ILQNYDEFAA LQALQAVDLN DPIAMEKFKQ VHYVNDEQIA
660
EMLKVPTLPV RAEQDYRSTY NDIRDWLRQR KEGNDKDNSP INWDDVVFEV DLLKSQEINL
720
DYILALIFEH HKKNQDKEVL IDEIRRTVRS SLGNRAKESL IVDFINQTNL DDIPDKATLI
780
 
DSFFLFAQAE QRKEAESLIQ EENLNVDAAK RYISTSLKRE YASENGTALN EVLPKMSLLK
840
PQYLTKKQKI FQKIAAFVEK FKGVGGKI
868
 <212> Type : PRT
   <211> Length : 868
   SequenceName : SEQ ID 183:GDC_HINF_276992
   SequenceDescription :

### Sequence

<213> OrganismName : Haemophilus influenzae
<400> PreSequenceString : 
MDIIKPICTG FFYNDNNVLG DLMKNFKYFA QSYVDWVIRL GRLRFSLLGV MILAVLALCT
60
QILFSLFIVH QISWVDIFRS VTFGLLTAPF VIYFFTLLVE KLEHSRLDLS SSVNRLENEV
120
AERIAAQKKL SQALEKLEKN SRDKSTLLAT ISHEFRTPLN GIVGLSQILL DDELDDLQRN
180
YLKTINISAV SLGYIFSDII DLEKIDASRI ELNRQPTDFP ALLNDIYNFA SFLAKEKNLI
240
FSLELEPNLP NWLNLDRVRL SQILWNLISN AVKFTDQGNI ILKIMRNQDC YHFIVKDTGM
300
GISPEEQKHI FEMYYQVKES RQQSAGSGIG LAISKNLAQL MGRGFNS
347
 <212> Type : PRT
   <211> Length : 347
   SequenceName : SEQ ID 184:GDC_HINF_370413
   SequenceDescription :

### Sequence

<213> OrganismName : Haemophilus influenzae
<400> PreSequenceString : 
VMSRHRGAKH RRRYELLGGI SLLSPEYLLS VERWPFHSEP PDHYDLLSYL LDLSVSQLSL
60
LIPLH
65
 <212> Type : PRT
   <211> Length : 65
   SequenceName : SEQ ID 185:GDC_HINF_370747
   SequenceDescription :

### Sequence

<213> OrganismName : Haemophilus influenzae
<400> PreSequenceString : 
VNIHGLAKLN GNVTLIDHSQ FTLSNNATQT GNIKLSNHAN ATVNNATLNG NVHLTDSAQF
60
SLKNSHFWHQ IQGDKDTTVT LENATWTMPS DTTLQNLTLN NSTVTLNSAY SASSNNAPRH
120
RRSLETETTP TSAEHRFNTL TVNGKLSGQG TFQFTSSLFG YKSDKLKLSN DAEGDYTLSV
180
RNTGKEPVTL EQLTLIESLD NKPLSDKLKF TLENDHVDAG ALRYKLVKNK GEFRLHNPIK
240
EQELLNDLVR AEQAEQTLEA KQVEQTAEKQ KSKAKARSRR AVLSDTPSAQ SLLNALEAKQ
300
VEQTTETQTS KPKTKKGRSK RALSAAFSDT PFDLSQLKVF EVKLEVINAQ PQVKKEPQDQ
360
EEQGKQKELI SRYSNSALSE LSATVNSMFS VQDELDRLFV DQAQSALWTN IAQDKRRYDS
420
 
DAFRAYQQKT NLRQIGVQKA LDNGRIGAVF SHSRSDNTFD EQVKNHATLT MMSGFAQYQW
480
GDLQFGVNVG AGISASKMAE EQSRKIHRKA INYGVNASYQ FRLGQLGIQP YLGVNRYFIE
540
RENYQSEEVK VQTPSLAFNR YNAGIRVDYT FTPTNNISVK PYFFVNYVDV SNANVQTTVN
600
STMLQQSFGR YWQKEVGLKA EILHFQLSAF ISKSQGSQLG KQQNVGVKLG YRW
653
 <212> Type : PRT
   <211> Length : 653
   SequenceName : SEQ ID 186:GDC_HINF_628407
   SequenceDescription :

### Sequence

<213> OrganismName : Haemophilus influenzae
<400> PreSequenceString : 
MKKTVFRLNF LTACVSLGIA SQAWAGHTYF GIDYQYYRDF AENKGKFTVG AKNIEVYNKE
60
GQLVGTSMTK APMIDFSVVS RNGVAALVGD QYIVSVAHNG GYNDVDFGAE GRNPDQHRFT
120
YQIVKRNNYQ AWERKHPYDG DYHMPRLHKF VTEAEPVGMT TNMDGKVYAD RENYPERVRI
180
GSGRQYWRTD KDEETNVHSS YYVSGAYRYL TAGNTHTQSG NGNGTVNLSG NVVSPNHYGP
240
LPTGGSKGDS GSPMFIYDAK KKQWLINAVL QTGHPFFGRG NGFQLIREEW FYNEVLAVDT
300
PSVFQRYIPP INGHYSFVSN NDGTGKLTLT RPSKDGSKAK SEVGTVKLFN PSLNQTAKEH
360
VKAAAGYNIY QPRMEYGKNI YLGDQGKGTL TIENNINQGA GGLYFEGNFV VKGKQNNITW
420
QGAGVSIGQD ATVEWKVHNP ENDRLSKIGI GTLLVNGKGK NLGSLSAGNG KVILDQQADE
480
AGQKQAFKEV GIVSGRATVQ LNSTDQVDPN NIYFGFRGGR LDLNGHSLTF KRIQNTDEGA
540
MIVNHNTTQV ANITITGNES ITAPSNKKNI NKLDYSKEIA YNGWFGETDK NKHNGRLNLI
600
YKPTTEDRTL LLSGGTNLKG DITQTKGKLF FSGRPTPHAY NHLDKRWSEM EGIPQGEIVW
660
DYDWINRTFK AENFQIKGGS AVVSRNVSSI EGNWTVSNNA NATFGVVPN
709
 <212> Type : PRT
   <211> Length : 709
   SequenceName : SEQ ID 187:GDC_HINF_654365
   SequenceDescription :

### Sequence

<213> OrganismName : Haemophilus influenzae
<400> PreSequenceString : 
VGENAMNLSR RDFMKANAAM AAATAAGLTI PVKNVVAAES EIKWDKAVCR FCGTGCAVLV
60
GTKDGRVVAS QGDPDAEVNR GLNCIKGYFL PKIMYGKDRL TQPLLRMTNG KFDKNGDFAP
120
VSWDFAVQNN G
131
 <212> Type : PRT
   <211> Length : 131
   SequenceName : SEQ ID 188:GDC_HINF_661444
   SequenceDescription :

### Sequence

<213> OrganismName : Haemophilus influenzae
<400> PreSequenceString : 
LIRTAILRQF LGILPFKTMA EKFKEAFKKN GQNAVGMFSS GQSTIWEGYA KNKLWKAGFR
60
SNNVDPNARH CMASAAVAFM RTFGMDEPMG CYNDIEQADA FVLWGSNMAE MHPILWSRIT
120
DRRISNPDVR VTVLSTYEHR SFELADHGLI FTPQTDLAIM NYIINYLIQN NAINWDFVNK
180
HTKFKRGETN IGYGLRPEHP LEKDTNRKTA GKMHDSSFEE LKQLVSEYTV EKVSKMSGLD
240
KVQLETLAKL YADPTKKVVS YWTMGFNQHT RGVWVNQLIY NIHLLTGKIS IPGCGPFSLT
300
GQPSACGTAR EVGSFPHRLP ADLVVTNPKH REIAERIWKL PKGTVSEKVG LHTIAQDRAM
360
NDGEMNVLWQ MCNNNMQAGP NINQERLPGW RKEGNFVIVS DPYPTVSALS ADLILPTAMW
420
VEKEGAYGNA ERRTQFWRQQ VKAPGEAKSD LWQLMEFAKY FTTDEMWTED LLAQMPEYRG
480
KTLYEVLFKN GQVDKFPLSE LAEGQLNDES EYFGYYVHKG LFEEYAEFGR GHGHDLAPFD
540
MYHKARGLRW PVVEGKETLW RYREGYDPYV KEGEGVAFYG YPDKKAIILA VPYEPPAESP
600
DNEYDLWLST GRVLEHWHTG TMTRRVPELH RAFPNNLVWM HPLDAQARGL RHGDKIKISS
660
RRGEMISYLD TRGRNKPPRG LVFTTFFDAG QLANSLTLDA TDPISKETDF KKCAVKVEKA
720
A 72
1
 <212> Type : PRT
   <211> Length : 721
   SequenceName : SEQ ID 189:GDC_HINF_737160
   SequenceDescription :

### Sequence

<213> OrganismName : Haemophilus influenzae
<400> PreSequenceString : 
VMSRHRGAKH RRRYELLGGI SLLSPEYLLS VERWPFHSEP PDHYDLLSYL LDLSVSQLSL
60
LIPLH
65
 <212> Type : PRT
   <211> Length : 65
   SequenceName : SEQ ID 190:GDC_HINF_775792
   SequenceDescription :

### Sequence

<213> OrganismName : Haemophilus influenzae
<400> PreSequenceString : 
LVMFNDFLAT FSQQLTPQMW GVVATATYET VYISFASTLL AVLVGVPVGI WTFLTGKNEI
60
LQNNRTHFVL NTIINIGRSI PFIILLLILL PVTRFIVGTV LGTTAAIIPL SICAMPFVAR
120
LTANALMEIP NGLTEAAQAM GATKWQIVRK FYLSEALPTL INGVTLTLVT LVGYSAMAGT
180
QGGGGLGSLA INYGRISQYA LCNLGGNHYY CAIRYD
216
 <212> Type : PRT
   <211> Length : 216
   SequenceName : SEQ ID 191:GDC_HINF_848166
   SequenceDescription :

### Sequence

<213> OrganismName : Haemophilus influenzae
<400> PreSequenceString : 
VMSRHRGAKH RRRYELLGGI SLLSPEYLLS VERWPFHSEP PDHYDLLSYL LDLSVSQLSL
60
LIPLH
65
 <212> Type : PRT
   <211> Length : 65
   SequenceName : SEQ ID 192:GDC_HINF_928073
   SequenceDescription :

### Sequence

<213> OrganismName : Haemophilus influenzae <400> PreSeguenceString : 
LRKDALPAFF TDVNQMYDAL LNKSGATGVF TDFPDTCVEF LKGIK
45
 <212> Type : PRT
   <211> Length : 45
   SequenceName : SEQ ID 193:GDC_HINF_929037
   SequenceDescription :

### Sequence

<213> OrganismName : Haemophilus influenzae
<400> PreSequenceString : 
VMSRHRGAKH RRRYELLGGI SLLSPEYLLS VERWPFHSEP PDHYDLLSYL LDLSVSQLSL
60
LIPLH
65
 <212> Type : PRT
   <211> Length : 65
   SequenceName : SEQ ID 194:GDC_HINF_1018846
   SequenceDescription :

### Sequence

<213> OrganismName : Haemophilus influenzae
<400> PreSequenceString : 
LPKPEPIPRP RRLALCFAPS AGDRVFKRIS YSSTLTMYET WLIIPRTAGV SINSTVWCIW
60
RRPRPRKVAL CFGKRAIELR TCVTLIVLAI IHYPKISSTV LPRFAATISG DFIFANASIV
120
ARTTLIGLVE PYALERTLRT PATSNTARIA PPAMIPVPSL AGCINTREPV
170
 <212> Type : PRT
   <211> Length : 170
   SequenceName : SEQ ID 195:GDC_HINF_1021582
   SequenceDescription :

### Sequence

<213> OrganismName : Haemophilus influenzae
<400> PreSequenceString : 
LFIYGGINMQ ITLSNTLAND AWGKNAILSF DSNKAMIHLK NNGKTDRTLV QQAARKLRGQ
60
GIKEVELVGE KWDLEFCWAF YQGFYTAKQD YAIEFPHLDD EPQDELLARI ECGDFVRGII
120
NEPAQSLTPV KLVERAAEFI LNQADIYNEK SAVSEKIISG EELEQQGYHG IWTVGKGSAN
180
LPAMLQLDFN PTQDSNAPVL ACLVGKGITF DSGGYSIKPS DGMSTMRTDM GGAALLTGAL
240
GFAIARGLNQ RVKLYLCCAE NLVSNNAFKL GDIITYKNGV SAEVLNTDAE GRLVLADGLI
300
EADNQNPGFI IDCATLTGAA KSGCRKRLSF CIIYG
335
 <212> Type : PRT
   <211> Length : 335
   SequenceName : SEQ ID 196:GDC_HINF_1082407
   SequenceDescription :

### Sequence

<213> OrganismName : Haemophilus influenzae
<400> PreSequenceString : 
VAVGNDYHSV LSMDDELVKN LFQSAQAENE PFWRLPFEDF HRSQINSSFA DIANIGSVPV
60
GAGASTATAF LSYFVKNYKQ NWLHIDCSAT YRKSGSDLWS VGATGIGVQT LANLMLSRSL
120
K 12
1
 <212> Type : PRT
   <211> Length : 121
   SequenceName : SEQ ID 197:GDC_HINF_1144501
   SequenceDescription :

### Sequence

<213> OrganismName : Haemophilus influenzae
<400> PreSequenceString : 
LPIELKVEGL VGKPNEKISA AEFRQKCREY AAEQVEGQKK DFIRLGVLGD WDNPYLTMNF
60
DTEANIIRTL GKVIENGHLY KGSKPVHWCL DCGSSLAEAE VEYEDKVSPS IYVRFPAESA
120
DEIEAKFSAQ GRGQGKLSAI IWTTTPWTMP SNRAIAVNAD LEYNLVQLGD ERVILAAELV
180
ESVAKAVGIE HIEILGSVKG DDLELSRFHH PFYDFTVPVI LGDHVTTDGG TGLVHTAPDH
240
GLDDFIVGKQ YDLPMAGLVS NDGKFISTTE FFAGKGVFEA NPLVIEKLQE VGNLLKVEKI
300
KHSYPHCWRH KTPIIFRATP QWFIGMETQG LRQQALGEIK QVRWIPDWGQ ARIEKMVENR
360
PDWCISRQRT WGVPMTLFVH KETEELHPRT LDLLEEVAKR VERAGIQAWW DLDEKELLGA
420
DAETYRKVPD TLDVWFDSGS TYSSVVANRL EFNGQDIDMY LEGSDQHRGW FMSSLMLSTA
480
TDSKAPYKQV LTHGFTVDGQ GRKMSKSIGN IVTPQEVMDK FGGDILRLWV ASTDYTGEMT
540
VSDEILKRAA DSYRRIRNTA RFLLANLNGF DPKRDLVKPE KMISLDRWAV ACALDAQNEI
600
KDAYDNYQFH TVVQRLMRFC SVEMGSFYLD IIKDRQYTTK ADSLARRSCQ TALWHIAEAL
660
VRWMAPILSF TADEIWQHLP QTESARAEFV FTEEFYQGLF GLGEDEKLDD AYWQQLIKVR
720
SEVNRVLEIS RNNKEIGGGL EAEVTVYAND EYRALLAQLG NELRFVLITS KVDVKSLSEK
 
780
PADLADSELE GIAVSVTRSN AEKCPRCWHY SDEIGVSPEH PTLCARCVEN VVGNGEVRYF
840
A 84
1
 <212> Type : PRT
   <211> Length : 841
   SequenceName : SEQ ID 198:GDC_HINF_1279189
   SequenceDescription :

### Sequence

<213> OrganismName : Haemophilus influenzae
<400> PreSequenceString : 
LENKMTVDYK NTLNLPETSF PMRGDLAKRE PDK
33
 <212> Type : PRT
   <211> Length : 33
   SequenceName : SEQ ID 199:GDC_HINF_1347200
   SequenceDescription :

### Sequence

<213> OrganismName : Haemophilus influenzae
<400> PreSequenceString : 
MKITHCKLKK SIQNKLLEFF VLEVTARAAA DLLDI
35
 <212> Type : PRT
   <211> Length : 35
   SequenceName : SEQ ID 200:GDC_HINF_1347942
   SequenceDescription :

### Sequence

<213> OrganismName : Haemophilus influenzae
<400> PreSequenceString : 
LFLVGNLLRW VWLALFIIAQ IWAYVQTPDS WLAMISGISG ILCVVLVSKG KISNYFFGLI
60
FAYTYFYVAW GSNFLGEMNT VLYVYLPSQF IGYFMWKANM QNSDGGESVI AKALTVKGWM
120
TLIVVTTVGT LLFVQALQAA GGSSTGLDGL TTIITVAAQI LMILPLS
167
 <212> Type : PRT
   <211> Length : 167
   SequenceName : SEQ ID 201:GDC_HINF_1476415
   SequenceDescription :

### Sequence

<213> OrganismName : Haemophilus influenzae
<400> PreSequenceString : 
MFSGEHDACD CYVDLQAGSG GTEAQDWTEM LLRMYLRWAE SKGFKTELME VSDGDVAGLK
60
SATIKVSGEY AFGWLRTETG IHRLVRKSPF DSNNRRHTSF SAAFVYPEID DDIDIEINPA
120
DLRIDVYRAS GAGGQHVNKT ESAVRITHMP SGIVVQCQND RSQHKNKDQA MKQLKAKLYE
180
LELQKKNADK QAMEDNKSDI GWGSQIRSYV LDDSRIKDLR TGVENRNTQA VLDGDLDRFI
240
EASLKAGL
248
 <212> Type : PRT
   <211> Length : 248
   SequenceName : SEQ ID 202:GDC_HINF_1476557
   SequenceDescription :

### Sequence

<213> OrganismName : Haemophilus influenzae
<400> PreSequenceString : 
LLGNEKQAEA QAKYAEDTLK QARDFAKQHH KTAYLARNAD GLQTGQKGSI HTEAMELVGL
60
ENVAEGEQKG LTQVSMEQLL L
81
 <212> Type : PRT
   <211> Length : 81
   SequenceName : SEQ ID 203:GDC_HINF_1505851
   SequenceDescription :

### Sequence

<213> OrganismName : Haemophilus influenzae
<400> PreSequenceString : 
LPRIFAACFV GAALACGGAT YQGMFKNPLV SPDILGVSAG AGFGASLAIF YNLPMIYIQF
60
FAFSGGILAV LCVSLIASRS RTQDPILVLV LSGIAIGSLL GAGISLLKIL ADPFTQLPSI
120
TFWLLGSLTA INQQDLIQLI PMLLLGIVPI FLLLTDTLAR TIAPIELPLG ILTSACGY
178
 <212> Type : PRT
   <211> Length : 178
   SequenceName : SEQ ID 204:GDC_HINF_1524561
   SequenceDescription :

### Sequence

<213> OrganismName : Haemophilus influenzae
<400> PreSequenceString : 
LKNSLRELKD YTVVIVTHNM QQATRCSDYT AFMYLGELVE FGQTQQIFDR PKIQRTEDYI
60
RGKMG
65
 <212> Type : PRT
   <211> Length : 65
   SequenceName : SEQ ID 205:GDC_HINF_1568974
   SequenceDescription :

### Sequence

<213> OrganismName : Haemophilus influenzae
<400> PreSequenceString : 
MISLQETKIA VQNLNFYYED FHALKNINLR IAKNKVTAFI GPSGCGKSTL LRSFNRMFEL
60
YPNQKATGEI NLDGENLLTT KMDISLIRAK VGMVFQKPTP FPMSIYDNIA FGVRLFEKLS
120
KEKMNERVEW ALTKAALWNE VKDKLHKSGD SLSGGQQQRL CIARGIAIKP SVLLLDEPCS
180
ALDPISTMKI EELITGVKLY CGYSNS
206
 <212> Type : PRT
   <211> Length : 206
   SequenceName : SEQ ID 206:GDC_HINF_1586944
   SequenceDescription :

### Sequence

<213> OrganismName : Haemophilus influenzae
<400> PreSequenceString : 
MSQLNIQFPT KFKPLFESIW RFIIFYGGRG SGKSFSIARA LVLRAYQSPV RVLCSVKFRN
60
RFLIL
65
 <212> Type : PRT
   <211> Length : 65
   SequenceName : SEQ ID 207:GDC_HINF_1594339
   SequenceDescription :

### Sequence

<213> OrganismName : Haemophilus influenzae
<400> PreSequenceString : 
VVPEFIIVSL ILVAQSMKLA LNKWLIIFGN AIALHIKYAL LRLNFEGVVG EILEKVDNGQ
60
MGVVLKRMMV RAASKVAQRF NIEAIVTGEA LGQVSSQTLT NLRLIDEAAD ALVLRPLITH
120
DKEQIIAMAK EIGTDDIAKS MPEFCGVISK NPTIKAVREK ILKEEGHFNF EILESAVQNA
180
KYLDIRQIAE ETKAVVEVEA ISVLGENEVI LDIRSPEETD EKPFESGTHD VIQMPFYKLS
240
SQFGSLDQSK SYVLYCERGV MSKLQALYLK ENGFSNVRVF AKNIH
285
 <212> Type : PRT
   <211> Length : 285
   SequenceName : SEQ ID 208:GDC_HINF_1634710
   SequenceDescription :

### Sequence

<213> OrganismName : Haemophilus influenzae
<400> PreSequenceString : 
LAIAIGGGNR GNASGVLRQN FAEDKAKKTA SKLVGVMAHY FGGKSFYLPA GDKIKEALRD
60
AQIYQEFNGK NVPDLIKKYR LSESTIYAIL RNQRTLQRKR HQMDFNFS
108
 <212> Type : PRT
   <211> Length : 108
   SequenceName : SEQ ID 209:GDC_HINF_1638626
   SequenceDescription :

### Sequence

<213> OrganismName : Haemophilus influenzae
<400> PreSequenceString : 
LFRWHYLGGF TVMPDTNNTE TNNKIELYLN GKILSGWKSL NLQRSLESMS GRFDLGIAVR
60
PEDDISVLAA GSPLVLKMGG QTVITGYLDE IKQRVSGNDK TISVSGRDKT CDLVDCAIIH
120
NSYQFKNQTA KQIAEAICKP FGISVVWQVQ APEANERIPV WQVEPGETAF DNLSKIARHK
180
GVLVTSDVDG NLLFTEPSNK QVGNLTLGEN LLELEQTDSW LQRFSLYRVI GDAEQGGAKG
240
DTKTKNKAAK GKEKDDGVVE DPDIYPGPAE GGK
 273 <212> Type : PRT
   <211> Length : 273
   SequenceName : SEQ ID 210:GDC_HINF_1639409
   SequenceDescription

### Sequence

<213> OrganismName : Haemophilus influenzae
<400> PreSequenceString : 
MKVSYRLNNC LSLKLALIPL LILLFVVMGS VLSLIAKLDF YFFQQILFNS ELHFALLMSL
60
GTSLFSLILA LCIAIPSAWR MSQVRLPFQS FFDTLFDLPM VLPPLVTGLS LLLLFSSQGI
120
LAELLPFISK WIFSPVGIII AQTYIASSIL LRCSEPLKLR KKTIKTTKIK P
171
 <212> Type : PRT
   <211> Length : 171
   SequenceName : SEQ ID 211:GDC_HINF_1660491
   SequenceDescription :

### Sequence

<213> OrganismName : Haemophilus influenzae
<400> PreSequenceString : 
LTKRKNVSFT YENYTVTPFW DTLKLSYSQQ RITTRARTED YCDGNEKCDS YKNPLGLQLK
60
EGKVVDRNGD PVELKLVEDE QGQKRHQVVD KYNNPFSVAS GTNNDAFVGK QLSPSEFWLD
120
CSIFNCDKPV RVYKYQYSNQ EPESKEVELN RTMEINGKKF ATYESNNYRD RYHMILPNSK
180
GYLPLDYKER DLNTKTKQIN LDLTKAFTLF EIENELSYGG VYAKTTKEMV NKAGYYGRNP
240
TWWAERTLGK SLLNGLRTCK EDSSYNGLLC PRHEPKTSFL IPVETTTKSL YFADNIKLHN
300
MLSVDLGYRY DDIKYQPEYI PGVTPKIADD MVRELFVPLP PANGKDWQGN PVYTPEQIRK
360
NAEENIAYIA QEKRFKKHSY SLGATFDPLN FLRVQVKYSK GFRTPTSDEL YFTFKHPDFT
420
ILPNPNMKPE EAKNQEIALT FHHDWGFFST NVFQTKYRQF IDLAYLGSRN LSNSVGGQAQ
480
ARDFQVYQNV NVDRAKVKGV EINSRLNIGY FFEKLDGFNV SYKFTYQRGR LDGNRPMNAI
540
QPKTSVIGLG YDHKEQRFGA DLYVTHVSAK KAKDTYNMFY KEQGYKDSAV RWRSDDYTLV
600
DFVTYIKPVK NVTLQFGVYN LTDRKYLTWE SARSIKPFGT SNLINQGTGA GINRFYSPGR
660
NYKLSAEITF
670
 <212> Type : PRT
   <211> Length : 670
   SequenceName : SEQ ID 212:GDC_HINF_1807963
   SequenceDescription :

### Sequence

<213> OrganismName : Haemophilus influenzae
<400> PreSequenceString : 
LRERSSLSAL MAKTIEWDFI TENPLKYLEK PKAPAPRTRR YNEHEIERLI FVSGYDVEHI
60
 
EPPKTLQNCT GAAFLFAIET AMRAGEIASL TWNNINFEKR TTFLPITKNG HSRTVPLSVK
120
AIEILQHLTS VKTESDPRVF QMEARQLDHN FRKLKKMEGL ENANLHFHDT RRERLAEKVD
180
VMVLAKISGH RDLSILQNTY YAPDMAEGYK TKAGYDLTPT KGLSQRNFFF ENENEIVETT
240
NPPIVIKL
248
 <212> Type : PRT
   <211> Length : 248
   SequenceName : SEQ ID 213:GDC_HINF_1817220
   SequenceDescription :

### Sequence

<213> OrganismName : Helicobacter pylori-26695
<400> PreSequenceString : 
MATIIKNGKR WHAQVRKFGV SKSAIFLTQA DAKKWAEMLE KQLESGKYNE IPDITLDELI
60
DKYLKEVTVT KRGKREERIR LLRLSRTPLA AISLQEIGKA HFREW
105
 <212> Type : PRT
   <211> Length : 105
   SequenceName : SEQ ID 214:GDC_HPYL_51094
   SequenceDescription :

### Sequence

<213> OrganismName : Helicobacter pylori-26695
<400> PreSequenceString : 
MEAVQLDKNQ EPNYKGYSGS LIHPAFQQQT TKREKPSTPL PSLDLLLKYP PNEQRITPDE
60
IMETSQRIEQ QLRNFNVKAS VKDVLVGPVV TRYELELQPG VKASKVTSID TDLARALMFR
120
SIRVAEVIPG KPYIGIETPN LHRQMVPLRD VLDSNEFRDS KATLPIALGK DISGKPVIVD
180
LAKMPHLLVA GSTGSGKSVG VNTMILSLLY RVQPEDVKFI MIDPKVVELS VYNDIPHLLT
240
PVVTDMKKAA NALRWCVDEM ERRYQLLSAL RVRNIEGFNE KIDEYEAMGM PVPNPIWRLG
300
DTMDAMPPAL KKLSYIVVIV DEFADLMMVA GKQIEELIAR LAQKARAIGI HLILATQRPS
360
VDVITGLIKA NIPSRIAFTV ASKIDSRTIL DQGGAEALLG RGDMLYSGQG SSDLIRVHGA
420
YMSDDEVINI ADDWRARGKP DYIDGILESA DDEESSEKGI SSGGELDPLF DEVMDFVINT
480
GTTSVSSIQR KFSVGFNRAA RIMDQMEEQG IVSPMQNGKR EILSHRPEY
529
<212> Type : PRT
<211> Length : 529
   SequenceName : SEQ ID 215:GDC_HPYL_155367
   SequenceDescription :

### Sequence

<213> OrganismName : Helicobacter pylori-26695
<400> PreSequenceString : 
MNKIFKVIWN VVTQTWVVVS ELTRAHTKRT SATVATAVLA TVLSATVQAI NDAGTFVKVQ
60
STEDDIEDSA ATKDDNKNQA LKAGDTLTLK AGKNLKAKLD QGGKSVTFAL AKDLDVKTAK
120
 
VSDTLTIGGN TPAAGGATPK VSITSTADGL KLAKGTNGDT AVHLNGLAST LPDVTTNTGA
180
STSVTFSPSD IEKTRAATIK DVLNAGWNIK GAKVAGGNTE NVDLVAGYDN VEFITGDKNT
240
LDVVLTAKEN GKTTEVKFTP KTSVIKDNNG KLLTGKQLKD ANTGTATNAT EDTDEAMA
298
 <212> Type : PRT
   <211> Length : 298
   SequenceName : SEQ ID 216:GDC_HPYL_447632
   SequenceDescription :

### Sequence

<213> OrganismName : Helicobacter pylori-26695
<400> PreSequenceString : 
VMSRHRGAKH RRRYELLGGI SLLSPEYLLS VERWPFHSEP PDHYDLLSYL LDLSVSQLSL
60
LIPLH
65
 <212> Type : PRT
   <211> Length : 65
   SequenceName : SEQ ID 217:GDC_HPYL_506250
   SequenceDescription :

### Sequence

<213> OrganismName : Helicobacter pylori-26695
<400> PreSequenceString : 
MFAVHAAMIT TLKKEVFFLY LYIKSLKIPI PTTLKYMISL GKIRELDVLA NLAKLCPTCH
60
RALKKGSSEE EFQKRLIRNI LNRNKDNLEF AQLRFETDDF STLIDRICES LK
112
 <212> Type : PRT
   <211> Length : 112
   SequenceName : SEQ ID 218:GDC_HPYL_583607
   SequenceDescription :

### Sequence

<213> OrganismName : Helicobacter pylori-26695
<400> PreSequenceString : 
MIKQTLIILA PFFIATLLYF LGAPDGLRPN AWLYFCIFMG MIIGLILEPV PSGLIALSAL
60
VLCIALKIGA SDKVASANKA ISWGLSGYAN KTVWLVFVAF ILGLGYEKSL LGKRIALLLI
120
RFLGQTPLGL GYAIGLSELC LAPFIPSNSA RSGGILYPIV SSIPPLMGST PNNNPDKIGA
180
YLMWVALAST CITSSMFLTA LAPNPLAMEI AAKMGVNEIS WFSWFLAFLP CGVVLILLVP
240
LLAYKTCKPT LKGSKEVSLW AKKRN
265
 <212> Type : PRT
   <211> Length : 265
   SequenceName : SEQ ID 219:GDC_HPYL_583883
   SequenceDescription :

### Sequence

<213> OrganismName : Helicobacter pylori-26695
<400> PreSequenceString : 
MSRHRGAKPP RRCELLGEIS LLSPGYLLSF ERWPFHTEPP DHYDRLSSLL DLYVLQSGWL
60
VPLHSTCDFQ PQ
72
 <212> Type : PRT
   <211> Length : 72
   SequenceName : SEQ ID 220:GDC_HPYL_665045
   SequenceDescription :

### Sequence

<213> OrganismName : Helicobacter pylori-26695
<400> PreSequenceString : 
VQLHCHNLPC VSIDILLGGP PCQSYSTLGK RKMDEKANLF KEYLRLLDLV KPKIFVFENV
60
VGLMSMQKGQ LFKQICNAFK ERDYILEHAI LNALDYGVPQ MRERVILVGV LKSFKQKFYF
120
PKPIKTHFSL KDALGDLPPI QSGENGDALG YLKNADNVFL EFVRNSKELS EHSSPKNNEK
180
LIKIMQTLKD GQSKDDLPES LRPKSGYINT YAKMWWEKPA PTITRNFSTP SSSRCIHPRD
240
SRALSIREGA RLQSFPDNYK FCGSGSAKRL QIGNAVPPLL SVALAQAVFD FLKG
294
 <212> Type : PRT
   <211> Length : 294
   SequenceName : SEQ ID 221:GDC_HPYL_953783
   SequenceDescription :

### Sequence

<213> OrganismName : Helicobacter pylori-26695
<400> PreSequenceString : 
LMEFDVTIID ETGRATAPEI LIPALRTKKL ILIGDHNQLP PSIDRYLLEQ LESDDIQNLD
60
AIDRQLLEES FFENLYKYIP ESNKAMLNE
89
 <212> Type : PRT
   <211> Length : 89
   SequenceName : SEQ ID 222:GDC_HPYL_954679
   SequenceDescription :

### Sequence

<213> OrganismName : Helicobacter pylori-26695
<400> PreSequenceString : 
MPASIGSLVS QLFYKEKLKN GVIKNTSQFY DPKNIIRWIN VEGEHQLEKT SSYNKNQVQK
60
IIELLEQINR VLNQRKIRKT IGIITPYNAQ KRCLRSEVEK YGFKNFDELK IDTVDAFQGE
120
KADIIIYSTV KTYGNLSFLI DSKRLNVAIS RAKENLIFVG KKSFFENLRS DEKNIFSAIL
180
QVCR
184
 <212> Type : PRT
   <211> Length : 184
   SequenceName : SEQ ID 223:GDC_HPYL_954846
   SequenceDescription :

### Sequence

<213> OrganismName : Helicobacter pylori-26695
<400> PreSequenceString : 
LIIETQQDPK ELPESCKITP QKISFNQVVF KKIKRKLNRF IGSILARTEV YKNLVAKYDE
60
LTGKYESLLA KEANIKETFW ERRADSEKEA FFLEHFYLTS VYVASTAGYY ITPKGAKTFI
120
EATERFKIIE PVDMFINNPT YHDVANFTYL PCPVSLNKHA FNSTIQNAKK PDISLKPPRK
180
SYFDNLFYDQ LNTRKCLKAF HKYSRRYAPL KTPKEV
216
 <212> Type : PRT
   <211> Length : 216
   SequenceName : SEQ ID 224:GDC_HPYL_955261
   SequenceDescription :

### Sequence

<213> OrganismName : Helicobacter pylori-26695
<400> PreSequenceString : 
LMEILVLNLG SSSIKFKLFD MKENKPLASG LAEKIGEEIG QLKIKSHLHH NDQELKEKFV
60
IKDHASGLLM IRENLTKMGI IKDFNQIDAI GHRVVQGGDK FHAPVLVNEK VMQEIGNLSI
120
LAPLHNPANL AGIEFVQKAH PHIPQIAVFD TAFHATMPSY AYMYALPYEL YEKYQIRHYG
180
FHRTSHHYVA KEAAKFLNTA YEEFNAISLH LGNGSSAAAI QKGKSVDTSM GLTPLEGLIM
240
GTRCGDIDPT VVEYTAQCAN KSLEEVMKML NHESGLKGIC GDNEKHRSQK RKR
293
<212> Type : PRT
<211> Length : 293
SequenceName : SEQ ID 225:GDC_HPYL_1068602
SequenceDescription :

### Sequence

<213> OrganismName : Helicobacter pylori-26695
<400> PreSequenceString : 
MPNSQVAGQA SVFIFPDLNA GNIAYKAVQR SAKAVAIGPI LQGLNKPIND LSRGALVEDI
60
INTVLISALQ AQD
73
 <212> Type : PRT
   <211> Length : 73
   SequenceName : SEQ ID 226:GDC_HPYL_1069456
   SequenceDescription :

### Sequence

<213> OrganismName : Helicobacter pylori-26695
<400> PreSequenceString : 
VSLVSSVFLM CLDTQVLVFG DCAIIPNPSP KELAEIATTS AQTAKQFNIA PKVALLSYAT
60
GDSAQGEMID KINEALTIAQ KLDPQLEIDG PLQFDASIDK SVAKKKCLTA KWLGKLAFLF
120
SRI
123
 <212> Type : PRT
   <211> Length : 123
   SequenceName : SEQ ID 227:GDC_HPYL_1376803
   SequenceDescription :

### Sequence

<213> OrganismName : Helicobacter pylori-26695
<400> PreSequenceString : 
LKAAHRLNLM GAVGLILLGD KEAINSKNLN LNLENVEIID PNTSHYREEF AKSLYELRKS
60
KGLSEQEAKQ LVLDKTYFAT MLVHSGYVHA MVSGVNHS
98
 <212> Type : PRT
   <211> Length : 98
   SequenceName : SEQ ID 228:GDC_HPYL_1474291
   SequenceDescription :

### Sequence

<213> OrganismName : Helicobacter pylori-26695
<400> PreSequenceString : 
VKQISISCSH RKYFVSFSVE YEQDITPIKN TKNGVGLDLN ILDIACSCEI NNHDKLTDFK
60
QYQTDMKELL GIEIDEELDT KRLIPTYSKL YSLKKYSKKF KRLQRKQSRR VLKSKQNKTK
120
LGGNFYKTQK KLNQAFDKSS HQKTDRYHKI TSELSKQFEL IVVEDLQVKN MTKRAKLKNV
180
KQKSGLNQSI LNASFYQIIS FLDYKQQHNG KLLVKVPPQY TSKTCHCCGN INHKLKLNHR
240
QYWCLECGYR EHRDINAANN ILSKGLSLFG VGNIHADFKE QSLSC
285
 <212> Type : PRT
   <211> Length : 285
   SequenceName : SEQ ID 229:GDC_HPYL_1600102
   SequenceDescription :

### Sequence

<213> OrganismName : Mycobacterium tuberculosis-H37RV
<400> PreSequenceString : 
MKVNKGFKFR LYPTKEQQDK LQHCFFVYNQ AYNIGLNELQ EQYETNKDSP PKERKYKKSS
60
ELDNAIKQCL RARDLPFSAV IAQQARMNVE RALKDAFKVK NRGFPKFKNS KSAKQSFSWN
120
NQGFSIKESD DECFKTFTLM KMPLLMRMHR DFPLILK
157
 <212> Type : PRT
   <211> Length : 157
   SequenceName : SEQ ID 230:GDC_MTUB_26830
   SequenceDescription :

### Sequence

<213> OrganismName : Mycobacterium tuberculosis-H37RV
<400> PreSequenceString : 
LIFITHFSTE PLPLPILVSK GLAVKGLSGN TLIHTLPALL MCLVMATLAD SIWRESILPC
60
SMALIAISPN AMELPRWAFP RLRPFICFLY FVLFGINMII ASLFCFF
107
 <212> Type : PRT
   <211> Length : 107
   SequenceName : SEQ ID 231:GDC_MTUB_36276

### SequenceDescription :

<213> OrganismName : Mycobacterium tuberculosis-H37RV
<400> PreSequenceString : 
MSRHRGAKPP RRCELLGEIS LLSPGYLLSF ERWPFHTEPP DHYDRLSSLL DLYVLQSGWL
60
VPLHSTCDFQ PQ
72
 <212> Type : PRT
   <211> Length : 72
   SequenceName : SEQ ID 232:GDC_MTUB_76032
   SequenceDescription :

### Sequence

<213> OrganismName : Mycobacterium tuberculosis-H37RV
<400> PreSequenceString : 
LNAAFKERRF ILVQLDEKID PKEDKSAYDF CLNTLKSPSP SIFDITEERI KRAGAKIKEA
60
CAHLDVGFRA FEIIDDETHA NDKNLSQAHQ KDLFAYSNLD RMETQTILIK LLGCEGLELT
120
TPITCLIENA LYLALNTAFI VGDIEMSEVL ENLKDKGVEK ISMYMPAISN DNLCLELGSN
180
LLDLKLESGD LKIRG
195
 <212> Type : PRT
   <211> Length : 195
   SequenceName : SEQ ID 233:GDC_MTUB_80423
   SequenceDescription :

### Sequence

<213> OrganismName : Mycobacterium tuberculosis-H37RV
<400> PreSequenceString : 
MYIRFYRDSL AEPATDIYAF AYVSFNKEAG TWHTPAQPTR NYGSGTPMTT AATAPLRHAP
60
MSGRPPKRGS NACAGARSYS SAGVLNTRSS IGWSTAYGPA SSFPAASTES ANSSRQPTTC
120
CVGLPAARSI PGSSRTMRLC WPATKDSRSP RCPGSWCTCR SHRLAHNRPL DARSASPAVA
180
KPSVIRLGSR VRRRSGSPDH LPSARICVSS RRSPRRLLWC YRRPLARCSE STIR
234
 <212> Type : PRT
   <211> Length : 234
   SequenceName : SEQ ID 234:GDC_MTUB_167239
   SequenceDescription :

### Sequence

<213> OrganismName : Mycobacterium tuberculosis-H37RV
<400> PreSequenceString : 
LMFCASRKEM AMSNSSSSSV INWNSLSESK PRSSTSTWFA VMPRSVRKIR WMVALMASFI
60
ARLLAGSGPR QGRQTRARPG RGQIVGGRLG SWCGIPNAPP ARLGGPPGSH TPRSASAADS
120
PHAPRSGCPG SPARSRFRDT RPDSPAVPGR WPCTRPRPAP EPAGRVHAD
169
<212> Type : PRT
 <211> Length : 169
   SequenceName : SEQ ID 235:GDC_MTUB_214625
   SequenceDescription :

### Sequence

<213> OrganismName : Mycobacterium tuberculosis-H37RV
<400> PreSequenceString : 
VPPPIPRCAA ASTSDPMASV KYGATRRWWP PAPSLTTSSC SAACGLCPKS SPGSSIPSDE
60
PDSTATVGQH STMLTATLMA SPPAEVSLYL VCMSAPVARM VSMTWSRVTR WMPSPRNAIR
120
AALMALPAEI ALRSMQGICT SPAMGSQVRP RLCSMAISAA FSTCRGVPPR ISANPAAAMA
180
AAEPTSP
187
 <212> Type : PRT
   <211> Length : 187
   SequenceName : SEQ ID 236:GDC_MTUB_424142
   SequenceDescription :

### Sequence

<213> OrganismName : Mycobacterium tuberculosis-H37RV
<400> PreSequenceString : 
MIPMDVIFGC PLYANFCKPS VVRKTLGILA SAVEFGTMPT TRNLRAPTST VSPRCRPIVL
60
DAATSSGFDG DRPSETRGMP GPCSGAPKTV TFRVDVPSFM IVPTLPNGAA AMTPGSAATR
120
ARSTSGNGIE PRKGPAAPDL TTNTSTPMES TVCRASTRKP FASPVKTSVI PKISPVLMIV
180
MTRRRFLHCM SRRAAKSIPR GYQRGALVGP GLDVLWSGRG PLVVEEAFGV VVVVGVGTAV
240
EVGWRDPFRL AVGPFPCLPA FPD
263
 <212> Type : PRT
   <211> Length : 263
   SequenceName : SEQ ID 237:GDC_NTUB_459316
   SequenceDescription :

### Sequence

<213> OrganismName : Mycobacterium tuberculosis-H37RV
<400> PreSequenceString : 
MSRAIRTKPK SASSRGSAGY RVYLGQRLGV AVFAQRDDHP VTGLEDADER AVISGPVGAH
60
TVAMPLDHYR FTLVDAADEF DVDLEDLLAP LDCSPKRLLV QFRTGDDAPV GEVVAEQREA
120
FVEISALAEA LQEHPGQFGL RVVERRHHIA ILSRETACGQ LTWSSKRWSP SRGRPASRTP
180
WRRCVAVSRI HAFGSPVTAL SGGPACCPPG RSPRGSAVLG ATPPVAWRGA AVPRSLSTWR
240
PPCWAPPTTP AISCRCIRPW PPRTAGCRTC AWAAPARCWK P
281
 <212> Type : PRT
   <211> Length : 281
   SequenceName : SEQ ID 238:GDC_MTUB_549643
   SequenceDescription :

### Sequence

<213> OrganismName : Mycobacterium tuberculosis-H37RV
<400> PreSequenceString : 
VRPGHRQVDG CRRGQPLCGA HERVGLVRVV GAFGLAQQGC DAGQHLVVGH GAKTSGGLRQ
60
VGSAYNRSVS QATTSSSTWL RSGSLNTSWY SPGYSFSCTS ADPTHSTRRR LPSMGISRSS
120
VPCSTSSGAV NAGARRGMVS PTCSSARPIP AGTRPWCTSG SVL
163
 <212> Type : PRT
   <211> Length : 163
   SequenceName : SEQ ID 239:GDC_MTUB_566823
   SequenceDescription :

### Sequence

<213> OrganismName : Mycobacterium tuberculosis-H37RV
<400> PreSequenceString : 
VCKACLGHHT HHHRTSRPLR NRCQHDQPRP AHRHGFHPNP RFRRQRHRGR VPLRLRLAAE
60
PGILQLDHNP VVGLLQLRRR WRIGLPQRRR SRRVGPGKRL HRDFGLLQCW RRRNSGFQNF
120
GNLLSGWANL GNTVSGFYNT SMLDLATQAL ISGFGNHGAR LSGILNNGSG P
171
 <212> Type : PRT
   <211> Length : 171
   SequenceName : SEQ ID 240:GDC_MTUB_591109
   SequenceDescription :

### Sequence

<213> OrganismName : Mycobacterium tuberculosis-H37RV
<400> PreSequenceString : 
VLSLSAGGPE PRMRPGHNPV TFHAEQTRNR TARTSRVRFR VCSSDKSAQD QRVGVGADVD
60
RHGIAVVHLA GQQHLGQLVT DGLLHQPAQR PRPVHRVESA LRQPALGRQR DLQLQPPLRQ
120
PLAQLRQLDV DDAHQLFGVE TLKDEHVVEP VDELRLERSA HRGQHLLGAA TRPQVGRQDQ
180
DGVAEVDRAA VPVGEPALVE DLQQHVEHVR VRLLDLVEQH HRVGTPAHRL GQLTARLVSH
240
IAGRGADQPS HGVLLAVLAH VDADHRPLVV EQEVGQRLGQ LGLADTGRAE EHERPGGPVG
300
VGHPGPAAPH RIRDCGNRGL LPDDPLAQLV FHAQQLGGLA FQQPTGRDAG PRRHHVGDVV
360
GTDLLLEHHL LPGLRLRQRR VELLLHLGDA SVAQLGGLGQ VAVAFGPLGF PAQGFQLLLE
420
VADDFDRVLL VLPAGGELGQ LLFLVGQLGA QLGQPLRRRL VFFFGQRHLF DLQPAHQPLD
480
LVDLDGPRVD LHPQPAGRLV DQVDGLVGQE AGGDIPVAQS GSCHQRRVGD AHPVVHLVAV
540
FEPAQDADGV LHRRLADVHL LETALERGVL LDVLAVFVQR GRPDQP
586
 <212> Type : PRT
   <211> Length : 586
   SequenceName : SEQ ID 241:GDC_MTUB_663028
   SequenceDescription :

### Sequence

<213> OrganismName : Mycobacterium tuberculosis-H37RV
<400> PreSequenceString : 
LLADFDVGQH LFQLVVGGLG TQHGFGVQRV ALPDRLGPDR RQLQELVVDV GLDQTARRAG
60
AHLALVEGEH GEAFQRLVAE VVVGGQHVGE EDVGALAAEF QGDRDQVVRG VLHDQPPRGG
120
FPGERDLGDA VAGGQRLAGL GAESVDHVDH PGRQQITDQR HQVEHRSGCL LGGFEHRRVA
180
GRQRRRQLPG RHQDGEVPRN DLAHHAERLV EVVGHGVLVD LAQRALLGAN RRGEVPEVID
240
RQRDIGGQRF PDRFPVVPDL GHRQRGGVLV DAVGNHVEDR RPFGRCGLAP PRRRRVRGVE
300
RLVDVGRVGA RHLAERLAGH RRRVLEVAPM DRRDPLAPDE VLVPGFIGHQ RPGGTGTGKD
360
SHRIRLLVKI M
371
 <212> Type : PRT
   <211> Length : 371
   SequenceName : SEQ ID 242:GDC_MTUB_688806
   SequenceDescription :

### Sequence

<213> OrganismName : Mycobacterium tuberculosis-H37RV
<400> PreSequenceString : 
VYLPPKLIPR RIPAQVRPTM VAPQVPHVLS ITPNGRSGEV CPASGSTRPK LGVQPPAASG
60
WPLPTRPGPR FSRCHRRPTL PACARSSSAT GSTPKSDNPA NPAGTSSRGG RSSTTRRCWL
120
PAAIRAALKS RFSARPTDSG AVGRAGRPHP AQA
153
 <212> Type : PRT
   <211> Length : 153 -
   SequenceName : SEQ ID 243:GDC_MTUB_701762
   SequenceDescription :

### Sequence

<213> OrganismName : Mycobacterium tuberculosis-H37RV
<400> PreSequenceString : 
MTSTNGPSAR DTGFVEGQQA KTQLLTVAEV AALMRVSKMT VYRLVHNGEL PAVRVGRSFR
60
VHAKAVHDML ETSYFDAG
78
 <212> Type : PRT
   <211> Length : 78
   SequenceName : SEQ ID 244:GDC_MTUB_731710
   SequenceDescription :

### Sequence

<213> OrganismName : Mycobacterium tuberculosis-H37RV
<400> PreSequenceString : 
VAESVAIRGC LLRCGPRSRP RRRSRRSGIC ACRPRCSATS RPPCPRRSTC PPRRRSMTSA
60
PSMWPPGRQR SRASRCIATA AGKDRYCPTP RRNRYWRRLT RSSAAAVRAA PASSDGGSHG
120
ASRRRIAQNQ RF
132
 <212> Type : PRT
   <211> Length : 132
   SequenceName : SEQ ID 245:GDC_MTUB_772761

### SequenceDescription :

### Sequence

<213> OrganismName : Mycobacterium tuberculosis-H37RV
<400> PreSequenceString : 
LLHSSFGHLE GIQQPLIDEL AELDHVLGKL PDAYRIIGRA GGIYGDFFNF YLCDISLKVN
60
GLQPGGPVRT VKLFGQPTGR CTPQ
84
 <212> Type : PRT
   <211> Length : 84
   SequenceName : SEQ ID 246:GDC_MTUB_868821
   SequenceDescription :

### Sequence

<213> OrganismName : Mycobacterium tuberculosis-H37RV
<400> PreSequenceString : 
LLGALHQYPH TRIQPGAVAA HRDRQHPRPV FGDEALDAAG VLMRTHAADH RQSEVSTVGL
60
NAHRTRGERH AIGVAALLLE SREAHSLAVA LASTPLLPVP VRVDRARDPV GVGLFRAFRP
120
PHGASLGVDT HLVFHRVPAF PQYPKRRLRR LGAGRAPRLD IGFQLRDGPV VGLAAGAEMP
180
RQRVCLLGGR IECEPERLHT PAVGDLQTRH LRPPHDHRQR QPRRPAWPGS EQHVCHTTLR
240
TSRSESRSYP IPGHRQPRPS PPRPTPDPER PAQRGHTPNR TGRTDPDAQP QSA
293
 <212> Type : PRT
   <211> Length : 293
   SequenceName : SEQ ID 247:GDC_MTUB_890358
   SequenceDescription :

### Sequence

<213> OrganismName : Mycobacterium tuberculosis-H37RV
<400> PreSequenceString : 
MASSTDVRPK ITLACEVCKH RNYITKKNRR NDPDRLELKK FCPNCGKHQA HRETR
55
 <212> Type : PRT
   <211> Length : 55
   SequenceName : SEQ ID 248:GDC_MTUB_904043
   SequenceDescription :

### Sequence

<213> OrganismName : Mycobacterium tuberculosis-H37RV
<400> PreSequenceString : 
LVCAAAPGRR RPLGVGGQVE AGTESLAATG HQNDMHAWIQ IGTLHQSRQL QRGVCDDRVA
60
LLRPVEGDPR NPTGDLIGHR LQVVEIDRPD RVCHQRPLSL LPAHARGWAR DPDRPAWCRT
120
LRPTGRRAEW PETPRRRRDV RGAPTTIPAT PGRCLRQSCG LDNRSCQDRP AADAAFRRGR
180
PAWGPGLRCG PARQTAPRRM RAGLPWRARY LAR
213
 <212> Type : PRT
   <211> Length : 213
   SequenceName : SEQ ID 249:GDC_MTUB_1045383
   SequenceDescription :

### Sequence

<213> OrganismName : Mycobacterium tuberculosis-H37RV
<400> PreSequenceString : 
LGLVAPAGDG RAAKKRPAGR RGSDRRRRMR LRGVVRPTPA RRCHDLWGLH HRVHCHAVAA
60
HRLQNGTGRW STGASTSMRS TTVASAAARG SRPSTSAETT DPSTAQINVH TSSICAERPE
120
RSMASATASA R
131
 <212> Type : PRT
   <211> Length : 131
   SequenceName : SEQ ID 250:GDC_MTUB_1068100
   SequenceDescription :

### Sequence

<213> OrganismName : Mycobacterium tuberculosis-H37RV
<400> PreSequenceString : 
MRCRAALSWR LPERLSRIWP AVLPDHTGMG ATAAWQAKAA SLLNRVTPAA SPTILAAVSS
60
AQPGISSSAG ATWWTRALMR WARVLISPVS RMMSVSSARA SSATNPGWVS SQVRRACWCL
120
AASSERAAGA RSGSSSWTSQ RNRLIADVRW ATRTSRRSVN NFNSRDVSSW VARGRSVSRI
180
TARATASASI GSDLPRLRAD LRVWAISLVG TRTTCWPAAS RSRSRRADML RQSSMPQISS
240
RPNCSRAHMM AVACPAVVAL TVFSPSWRPT SSVATKVWLY LCASVPTTTM VVASEPPR
298
 <212> Type : PRT
   <211> Length : 298
   SequenceName : SEQ ID 251:GDC_MTUB_1115707
   SequenceDescription :

### Sequence

<213> OrganismName : Mycobacterium tuberculosis-H37RV
<400> PreSequenceString : 
LRRRAAVPVG LHRRRSDRAG ATQRDRRRYR RWVHACRLCA AWRRDRRTSG PDRARSLRYL
60
CHRRRRRRGG QCAGSRPGQT RRRHHRDGLV GSAFQWVLAG PQGVAGDRPD ESGRSCGGVR
120
SHLGRRVIGA DSHLRQRLFG LGRRNPCPDV LPRHRRRARR QPATGHPAWP HRRGRPRHLD
180
TRAGIHHDCP ARPGQAHRDG EDVQHGCRHD RRRCPRRHDA RPGRPDRAAP GLLGIGNRLQ
240
RRKTRPAGKT GWAAPEILRT RPNRV
265
 <212> Type : PRT
   <211> Length : 265
   SequenceName : SEQ ID 252:GDC_MTUB_1124996
   SequenceDescription :

### Sequence

<213> OrganismName : Mycobacterium tuberculosis-H37RV
<400> PreSequenceString : 
VVAVRIEVVG HRVHHLAGHL EFRGFDLHLL VQHREVGVAD LIGPQQRVHH HHLSLAEILD
60
AQRRQPGLVA QREMHDRHPV GLGECLSQQH IRFRRLRIRL QKVAAVEHHR VHVGGGDELQ
120
HLDLPAAFER QAGDVVVGDR HHLAVAGLVG PGKIAVVDHL ATRLADALVP DASVVLGVHL
180
VEPDVVVCGS AVHLDRHVHQ PEGDRTRPNG SHVSEYALIV RERNVTAKFH AIFDRDVTLA
240
TCVTDRLR
248
 <212> Type : PRT
   <211> Length : 248
   SequenceName : SEQ ID 253:GDC_MTUB_1138949
   SequenceDescription :

### Sequence

<213> OrganismName : Mycobacterium tuberculosis-H37RV
<400> PreSequenceString : 
LRSARVNPPA RSAASTPWYP SGSVTTAALG WFLAAARTIA GPPISICSTQ SSTLAPDSTV
60
WLNGYKLTTT SSKASIPSCS RAAACSDLRR SASSPACTRG CSVLTRPSST SGKPVSCSTG
120
VTGIPVSAMV LAVDPVEMIS TPAALRPCAR STSPVLSYTL ISARRIGRLP SSVLILWLPF
180
VPSSLFVRPP SRHGWPVRPP PLPTAVVR
208
 <212> Type : PRT
   <211> Length : 208
   SequenceName : SEQ ID 254:GDC_MTUB_1170285
   SequenceDescription :

### Sequence

<213> OrganismName : Mycobacterium tuberculosis-H37RV
<400> PreSequenceString : 
VRADPPTTAC NTRCTPSVCV PSMCGTSTTS MPPRSCVPEK VTLLQSFPGL GAGSGWDVST
60
AMTTNRLPLP SAETAAMLPC NPVGSWGPAA TCAQFAGSKL SPSGSLRAEK NPGSMALGVT
120
SVTVYSGPKP DFTSATLAMS PVEAVVELAP DEQPTSQHTD PTASTALRIV VNLPNAAPEL
180
RNVDTVLTSR SAANCGASGG RTDPGSVISR RPRSLAGLPG
220
 <212> Type : PRT
   <211> Length : 220
   SequenceName : SEQ ID 255:GDC_MTUB_1176592
   SequenceDescription :

### Sequence

<213> OrganismName : Mycobacterium tuberculosis-H37RV
<400> PreSequenceString : 
VGTAQERVRS RSGPVPHHAL RHLRGSPHRG TADPAGDAGV GRQNFGPARP GPKPAVVRRR
60
RCSADPRHSA AAAHRGISPL PAAATTRRQV SGPQRRESHL RSVDRGLRVA WDVERGDGIK
120
PGIVAAVAGQ QHGRIVHHMG AVRFVLLPVD RGPQRVVARG QAGQINANRL GDRRRCRLVA
180
AAIAALVGDQ RLQVHRCRQR PNHLSGGIHQ PVAGHPLFGG GSSAVVGPGD RDRRDLAR
238
 <212> Type : PRT
   <211> Length : 238
   SequenceName : SEQ ID 256:GDC_MTUB_1202653
   SequenceDescription :

### Sequence

<213> OrganismName : Mycobacterium tuberculosis-H37RV
<400> PreSequenceString : 
MSISGIERWS ATENIRISVI SSPQNSTRTG CSAVGAKMSR IPPRTANSPR RPTISTRVYA
60
SSTSRATTPS KGDSSPTVSV RGSIMPSCGV MGCSSERTEV TTTPSGGPSW ASSGWASRRS
120
AIRRVPTVST PGESRSCGSV SQDGNNATAS PNTPRSSAAR SSASRPVAVT TSNGPCRASA
180
LATNSRALAG AMSVNSSGRP PARCMSCWNV GALSANSTSP AIGVSEQAGP GAVMMRPF
238
 <212> Type : PRT
   <211> Length : 238
   SequenceName : SEQ ID 257:GDC_MTUB_1231843
   SequenceDescription :

### Sequence

<213> OrganismName : Mycobacterium tuberculosis-H37RV
<400> PreSequenceString : 
VLAFYLRPRP GTWCTSEGSS RDPSGGSLGG QCWGVGGLLL GGFFGAGQCC SGSGEDLEAQ
60
VAPSFDPFVV LFGEDGSDEA DDRGAVGEDA HDVGSASYLS VEAFLGVVGP DLAPDLLGEG
120
GERQQVGAGG VEVLGHRGEF VGQSVEYPII LGNN
154
 <212> Type : PRT
   <211> Length : 154
   SequenceName : SEQ ID 258:GDC_MTUB_1241031
   SequenceDescription :

### Sequence

<213> OrganismName : Mycobacterium tuberculosis-H37RV
<400> PreSequenceString : 
LTTAGISGSK GRTGTGEPCG LLSAAGFRAG ASGGLTAAER STARASSANL TRRYLTHAEL
60
LMLARATGRF ETLTLVLGYC GLRRFTVR
88
 <212> Type : PRT
   <211> Length : 88
   SequenceName : SEQ ID 259:GDC_MTUB_1252888
   SequenceDescription :

### Sequence

<213> OrganismName : Mycobacterium tuberculosis-H37RV
<400> PreSequenceString : 
MGQCPRPVRH WPPAVIVCSR TKLRRACLRD YRRPAPSDKK PNKSYRVMTP TGLPSSTTIN
60
ASQSRNALPA ALTNSPAPII RSGGLMCADT ASANLARPSN TAESSSRSET LPATSPAITG
120
GSAPTTGICD TPYSRRIPMA SRTVSDGWVC TRAGSAPDLR RNTSPTVDCS VDPSRRLRRN
 
180
P                                                                                    18
1
 <212> Type : PRT
   <211> Length : 181
   SequenceName : SEQ ID 260:GDC_MTUB_1264312
   SequenceDescription :

### Sequence

<213> OrganismName : Mycobacterium tuberculosis-H37RV
<400> PreSequenceString : 
LAAIPRRSRC SVNPRGNRHD PARHPGGRGS VRGGDRPELT GDIGLRPGEG SARRGLRPRQ
60
AGNRPVRCAQ VHEVPTAAIL SASSEVFNEV PVRNPGTLAF VPIVDGDLLP DYPVKLAQEG
120
RSHPVPLIIG TNKHESALFR LMRSPLMPIT PRDHVDVHPD CRRTARSASA NRGADRLRVL
180
AMAAQSTLIE YGYRRRLPDA VGVAR
205
 <212> Type : PRT
   <211> Length : 205
   SequenceName : SEQ ID 261:GDC_MTUB_1286282
   SequenceDescription :

### Sequence

<213> OrganismName : Mycobacterium tuberculosis-H37RV
<400> PreSequenceString : 
VLALRPQRHF TQSRSARRLR CVLDDDVWVP WARSGGCRTA TRHLSVRCIA GTCWGPPVRF
60
CRLRATPSTV SCSARRRYRS RLTCHRSTDT SWSLSATRLA ELLAPLEPVT VTFTPTFGEP
120
DMVHLSGTKF GGLVPALFEG VRAGF
145
 <212> Type : PRT
   <211> Length : 145
   SequenceName : SEQ ID 262:GDC_MTUB_1301742
   SequenceDescription :

### Sequence

<213> OrganismName : Mycobacterium tuberculosis-H37RV
<400> PreSequenceString : 
MTSSAPKPAA SRASDWPTTS PAPSCSPTAN STVPQSSYAM TSTCWAAGSE WASKPSATGS
60
PHCSARGSEG YRSSSSAPTR PETSQSDSPR RRFTSAGSAA AARCGWSTTR SPSQRGSSAR
120
WRKCPTAGRT SGWPRPPLPT GSGIWARTRT SRSGWAATSR TPINSSTPPV SSWTTRARRS
180
RSGRAARSAT ERRAPNVRSP ISVVASRSTR TRAAACLIRR PSNRFDRPTP QQTTKPLILL
240
WFQQALGKHC CRCLHIAFSH VFHSGGDHGG LRVIGYRAVP RAGADL
286
 <212> Type : PRT
   <211> Length : 286
   SequenceName : SEQ ID 263:GDC_MTUB_1351907
   SequenceDescription :

### Sequence

<213> OrganismName : Mycobacterium tuberculosis-H37RV
<400> PreSequenceString : 
MQLGNQNTMR FAGRPQRFRQ SAYPLFNPNS AIALGHPFGG SGARLMTTVL HHMPDKGIRY
60
GLQTMCEGRG QANATIVELL
80
 <212> Type : PRT
   <211> Length : 80
   SequenceName : SEQ ID 264:GDC_MTUB_1476279
   SequenceDescription :

### Sequence

<213> OrganismName : Mycobacterium tuberculosis-H37RV
<400> PreSequenceString : 
VTVYRRGMAV LTDEQVDAAL HDLNGWQRAG GVLRRSIKFP TFMAGIDAVR RVAERAEEVN
60
HHPDIDIRWR TVTFALVTHA VGGITENDIA MAHDIDAMFG A
101
 <212> Type : PRT
   <211> Length : 101
   SequenceName : SEQ ID 265:GDC_MTUB_1485311
   SequenceDescription :

### Sequence

<213> OrganismName : Mycobacterium tuberculosis-H37RV
<400> PreSequenceString : 
VGAVRLQPHR MGGGMAALHR HTGTADQLLL LPRRAHRAGS PVQCDRLRGR DSHFQPGTNQ
60
YRNGHRGIDQ PIHQHRDQLD TRLPAAVAAN QPAGIPVFAL TSD
103
 <212> Type : PRT
   <211> Length : 103
   SequenceName : SEQ ID 266:GDC_MTUB_1486309
   SequenceDescription :

### Sequence

<213> OrganismName : Mycobacterium tuberculosis-H37RV
<400> PreSequenceString : 
MPSPVSSGPT SHGTNKGCGL IRSESMNTTM SPLVAASERH NASPLPGRTG TSGNACSRLT
60
TRAPEAMARI SVSSVEPESS TINSSTRPST SGEMLSITDS MVASSLRAGS TTEIVRPAFA
120
ASNSPIVQPG RCQVVSKGSA PGALPPARSP ATSSDAVMRV LSPCASAAGP PESMPPFPAP
180
AGWRRPHAPE TCAPRRPQPT RWLPAFPQAV RSNPRPESPR QRPCCSKPSA RATRS
235
 <212> Type : PRT
   <211> Length : 235
   SequenceName : SEQ ID 267:GDC_MTUB_1515112
   SequenceDescription :

### Sequence

<213> OrganismName : Mycobacterium tuberculosis-H37RV
<400> PreSequenceString : 
MLSAVILTER GYPAVPLAGQ LVHQRFVRPG PLVLGTGFLK FLTRAADRDR TVSRRSKPSS
60
 
RAALMGEQPN PWDLLQPQDA TSRHRGAKPS RRYGLLGKIS LLSPGYLLSV ERHPFHSGVP
120
DH
122
 <212> Type : PRT
   <211> Length : 122
   SequenceName : SEQ ID 268:GDC_MTUB_1515464
   SequenceDescription :

### Sequence

<213> OrganismName : Mycobacterium tuberculosis-H37RV
<400> PreSequenceString : 
LVGRSRVLVL FGAGEHVDVV ALLGERAHRL IGEHVVQTVV GHVVQNRNVA VLVTRPAIHQ
60
QVGRLRHRLL TAGHHHVELS GPNELISQRD CVDAGQAHLV DRQRRDIPTD AGRHCRLPCG
120
HLPGTRGQHL AHDHVLDQGR RHVGLLQGAL NGDGTQLAGA EILQGAHQLA DGCTRASNNH
180
RCRYDYLLSA PESRSDRPGE ADSFPSGYRC VMTTDQVHAR HMLATSLVTG LDHVGIAVAD
240
LDVAIEWYHD HLGMILVHEE INDDQGIREA LLAVPGSAAQ IQLMAPLDES SVIAKFLDKR
300
GPGIQQLACR VSDLDAMCRR LRSQGVRLVY ETARRGTANS RINFIHPKDA GGVLIELVEP
360
AP
362
 <212> Type : PRT
   <211> Length : 362
   SequenceName : SEQ ID 269:GDC_MTUB_1596569
   SequenceDescription :

### Sequence

<213> OrganismName : Mycobacterium tuberculosis-H37RV
<400> PreSequenceString : 
LRAATKSPSS SCWRACATAG STSVDSSELA APLSFPAVAD NRESTQRLSW SAGWRPWKLE
60
IGCPAAKATT VGTAWTPNIC ATLGATSTLT DASDHLPLAA AAKPDSVSSK STHTSLRGDH
120
SNTTTGTSSD RTITSSSKFA SVISVTPDGV DSARSASVLA AAFCWARCLM PERSTAPAMA
180
GPSGGRGRVT PSSLSCRCGH RSTRWRRPCG RSRHTAIGWY DQDHTGRHRP LNRYPARNIS
240
ASPCPPAPHN AATPTPDPRR ANCSAACSVI RVPDMPRGCP TAIAPPLTLT IWGFSPSSRI
300
EANATAANAS LISTTSSWST EMPSRSSALL IALAGCDCSV ESGPATTPWA PISASQVSPS
360
SWAFSWFMTT TAAAPSEICD ADPAVMVPSP RNAGFRPASA AAVVLARIPS SSVNCSGSPV
420
RCGMFTGITS SANTPSFHAA AAFWWDAAAY SSCSERVNMS TSLRCSVSAP IG
472
 <212> Type : PRT
   <211> Length : 472
   SequenceName : SEQ ID 270:GDC_MTUB_1600905
   SequenceDescription :

### Sequence

<213> OrganismName : Mycobacterium tuberculosis-H37RV
<400> PreSequenceString : 
VRSRRLAPTR PRSRRTASPA TATRAAAPPR TTPPSAAPAT RCPPLARQRN KTRAAQSRLA
60
WRGGRSEQGL SRCGSSGAVL RCGDRHPAAL AGVPQPAVAS ARGKQLLVGA AFDDPTMIEH
120
DDLVGPGDGM QSMGDYQHGA VPGQPVKRLL HKVFRFRIGK RGGLVEDEDR SVAEDGTGNG
180
EPLSLPARKT TVGSEHGIVA VRQPKHPVVD LRFAGRDLDL FGGGIRYRQR DVFGGGAMHK
240
LGFL
244
 <212> Type : PRT
   <211> Length : 244
   SequenceName : SEQ ID 271:GDC_MTUB_1616064
   SequenceDescription :

### Sequence

<213> OrganismName : Mycobacterium tuberculosis-H37RV
<400> PreSequenceString : 
VSAVLALSAA VSARRAKAAE AHSAPSSNGT PASAATPSCQ EIGNRASAIT AGSRIALVNG
60
VTRLTTRPTS SGPVAAIACR AVAVFSAVNQ SNRTTGSRSA TSCWVWLRTA KPSSIPMRAV
120
TASSTHPATV AADSQPSHSH ARCGASPNNA AISGTSNTVP TARATTEQNA SSAKPISLAR
180
WSFGTRAIQV RIIGCRPALR RPPPGCPGRC PTAGSSVRPR QATPRGCRVR RSDHDRARRS
240
GRPG
244
 <212> Type : PRT
   <211> Length : 244
   SequenceName : SEQ ID 272:GDC_MTUB_1672449
   SequenceDescription :

### Sequence

<213> OrganismName : Mycobacterium tuberculosis-H37RV
<400> PreSequenceString : 
MPSVIRDPDP GAAPAPTVAD RSAEVPSVLQ RSRRCDAYHR YSRWRLSYSA SPLGGSRRQP
60
GIATDGRTRG TQPRPAGAAH SRARPDVGRS VAATRPPSAG SAGTARP
107
 <212> Type : PRT
   <211> Length : 107
   SequenceName : SEQ ID 273:GDC_MTUB_1673708
   SequenceDescription :

### Sequence

<213> OrganismName : Mycobacterium tuberculosis-H37RV
<400> PreSequenceString : 
VLRPIRAGQP GRHLAPPRPA TRRQGTGAGT GARRRLGTGV APPAGVSVDE PSGCARLGMR
60
VAELPGVAAP HLARPHCRRE ARAGVGQGKH RRLRRGSEFR CHQRRFGRRP SVRPGGVDPQ
120
RSAISARVRT GRHLGGGSGS GIRALRLVYD RCAGASGIRR VARNVRGETE IQHAPRHLRR
180
CLTDPPCAGR RPTVLRSARP PRLPDPRGRS PCVRRGTAGG VEVARRLRGP APRPTRLRRL
240
RLPAGASHRR GRGPLPVLGV RDQPAGHVVS YRPAIAIPRH APARPVPVRW HRPSRRCRWP
 
300
PRVWSPGRNP DNPGRRSR
318
 <212> Type : PRT
   <211> Length : 318
   SequenceName : SEQ ID 274:GDC_MTUB_1699549
   SequenceDescription :

### Sequence

<213> OrganismName : Mycobacterium tuberculosis-H37RV
<400> PreSequenceString : 
MTASRRSDHT DATRRALVDA GRYLFARRDY GDVSIEDIVT RARVTRGALD YHFDSKKDLF
60
QTVLEVVEAD LVADVEAAIA KVTDAWICWS SASTPSLTRR PNRMRCRSLR LTARQCSGGA
120
NGAGSTCARA WSAGRGSRTR DGRRGDSART VATTFASAAG RANRIRAADR GRDGQRPDQS
180
RGRTRIYGPT RRSTGVARPR SATATDHRPQ SRPASRNAPR PATPRRPGHH RRHPGPRCRR
240
RFWRSPSRRR APAPYRQSSA RPTRPTLFGS PHTPPGRRRR WPPARCRSPR PVRRR
295
 <212> Type : PRT
   <211> Length : 295
   SequenceName : SEQ ID 275:GDC_MTUB_1742061
   SequenceDescription :

### Sequence

<213> OrganismName : Mycobacterium tuberculosis-H37RV
<400> PreSequenceString : 
VRLRSESAGL AHAADDVSGV VLGDDPDHDP PVAVLDFLVP EDVFPVVVAT GQMVVAVILG
60
RDLDVLPAHI QMGFRPAPFV AHRDLRLGAR KAGADQQQAQ PGFLGGLGTA VDEVQSGSCG
120
LHATAAPIAL DQRLDVGHLQ IGGLYQGVDG RDGGVQWKST GQVERRSLRC GHAHALDDAD
180
LVGLDALFPD LQPRGTAAVG VDDRGGKIRV DPLGAMEGRS RVAGQHAAAA RAQPQRFCTQ
240
LRGQFHTLRH VHVFM
255
 <212> Type : PRT
   <211> Length : 255
   SequenceName : SEQ ID 276:GDC_MTUB_1782153
   SequenceDescription :

### Sequence

<213> OrganismName : Mycobacterium tuberculosis-H37RV
<400> PreSequenceString : 
LGVRAAVGVD DVTRGRRQPV RQQRAHRLGD RRRILHVPAD RSALIPALLE QLDLGAGLLA
60
ERTADDRTHR QRPDRACGNE IRTHTVLAGL ARHEPVDRLQ RALGDRHPVV GRHRPARVEV
120
HADDGTSGVH DRQQRLGHRS IRIRRDVDAL GHIRVGRVEE RVDAHPGLRH EPNRMHHPVE
180
LVARPDRLGH PAGQAGQVLL VLHVEFEQRG LCRQPVGDAL NQPQPVEPGE HQLGALLLGY
240
PCDVKRDRRV GDDSANQNPF AVQQSCHVRP CVVSVAHTHA AVDRDDRTGD IARILGSQEA
300
 
DHPGDLGGGA DPLRWDKLQR PLLNPLIQRA GHIGVDVARG HHIRGHVCLR QLAGDRAGHA
360
NHSGLGGCVV GLVADAPAAG DRTYEYHSTE FVALHAARCP LSHPERPGEV GVDDLLELFL
420
GHPHEECVRG
430
 <212> Type : PRT
   <211> Length : 430
   SequenceName : SEQ ID 277:GDC_MTUB_2060659
   SequenceDescription :

### Sequence

<213> OrganismName : Mycobacterium tuberculosis-H37RV
<400> PreSequenceString : 
MVPSMRVRSD WEPIAQSRSR LAVTAPRNTS GGRFIWILLG SARNGSRAPW LPTRSPGSLD
60
RIFLVATDNR TSLPKGRWAP TSRMNPQPRP DVMPWRRATG RSGNPVKRAL ITGITGPDGS
120
YLAKLPLKGY VAAGSPAEVY FCWATRNYRE LYGLLAVNSI WFNHESPRHG ETFMTRNPAP
180
YRGRQRGADR CADADAPAHP DRYQYWGVPA SVRGVIDRAM GVCVE
225
 <212> Type : PRT
   <211> Length : 225
   SequenceName : SEQ ID 278:GDC_MTUB_2093062
   SequenceDescription :

### Sequence

<213> OrganismName : Mycobacterium tuberculosis-H37RV
<400> PreSequenceString : 
LSGQPSALRR PTVSPSACRR PTVSKSKPKI DRMTSRMAPP TTDGSATLNT GHQPTDKKST
60
TCPRSGPGAR KKRSTRLPMA PPRIIPRPSA HHGDTSRRPI QKIPTTTPVA ISVKTQVYPV
120
AIEKAAPELR TRVQVTVSPI IDTGWPGGNS WTATTLVTMS SVSTTTATDS SMRSRRGGAG
180
ALGSPAPPAS SVEVSGSADP VGSSGTPSSS PRADMARPDP AAGWEQTTCA MIPSWPASPS
240
SLLEGQSRPP PAPMGCYGQP IAGRR
265
 <212> Type : PRT
   <211> Length : 265
   SequenceName : SEQ ID 279:GDC_MTUB_2105797
   SequenceDescription :

### Sequence

<213> OrganismName : Mycobacterium tuberculosis-H37RV
<400> PreSequenceString : 
VPPNRPERRL GKPLTAPLRR APAHPLRDGP GSVGNPPKTS RRHQLLRSPK PRRRPGCPQS
60
RTPRTSREAP PATQRPGPPG SGFERRERPA ASICGRARRW SAEKRQERTF PGTRRRSRGR
120
WSPRCRARWC RARFGLRQTA ARPCGKGCYS KFGHHLADAR NARNRLWRMA AAIRVPAAPR
180
RLPGVSGCRD AKLDCITHKR SSPVRGKRVE PVAVVARRHR QSLLGGRGQA KLGGQAQQMH
240
ARRLRNRHRR VPVHDTGFR
259
 <212> Type : PRT
   <211> Length : 259
   SequenceName : SEQ ID 280:GDC_MTUB_2133554
   SequenceDescription :

### Sequence

<213> OrganismName : Mycobacterium tuberculosis-H37RV
<400> PreSequenceString : 
LWKWKPRLAF HRATWRRRWT ATTPTPRAAQ IPISTSSRNS LQHKTTGRGG RSTCRWARRC
60
MPDSLWAGWP RRWTVKYCAT TARWWPACTR SGHARPISPR TARDMPAGPS WVRGRFSGVA
120
PERMRQPERR ACKPPRAATG NPATRHADKA S
151
 <212> Type : PRT
   <211> Length : 151
   SequenceName : SEQ ID 281:GDC_MTUB_2183418
   SequenceDescription :

### Sequence

<213> OrganismName : Mycobacterium tuberculosis-H37RV
<400> PreSequenceString : 
LWPRISAAPS NRRSTVGGVW CRRRPNWVSR PRDSRRPCRI TTRCAPRGCP LHSPRPSATS
60
SAHTPTAGST NQASSTHYGV QTAPKYRCSG LELKGGKGVS DEISRRAPTR VRPDIQRRVH
120
RSEPIRGRVA LRRRFVHRRR LGHHHSGSGR QYDRGSRAAD GRDGRPPRWH RNPAAGSADP
180
GGKADGGVRQ KPGPGARHPS DAGTRRFGVR RHGAHPQART WRRGGHPRGS PDRIGARIVL
240
PGRGSLHPGA RYRRDGLCDR SSGNRATQDL RPAGARPGRR CGADRRRRHV GGSAKPHRGY
300
PRRYLHPGHR
310
 <212> Type : PRT
   <211> Length : 310
   SequenceName : SEQ ID 282:GDC_MTUB_2192571
   SequenceDescription :

### Sequence

<213> OrganismName : Mycobacterium tuberculosis-H37RV
<400> PreSequenceString : 
LGISPGDRGD RVRGNAAGRD RHPGRLAAFL GADHYSVFSN GPAVEREDRR GQHGAVRTAG
60
RPDGAGAGPQ PRTTGAVVTT ADPVTAGAAA GSRGYRVALR VRPARPDRAL PGGGRHQHRI
120
QYRGCGAGAD LCLAAVSGDF PRGCSPHRRS RLRGGGGDTW GAARHCLVAR DPAVAAPGRG
180
VRISTGVCPL ARRVWRDPNL CRFPARGHPY PSAGDLPAAG DRSGRGGIVV TAARCGSGTG
240
GAGCGCSYAD RDRYQVAGHE QAAAARGRRR PAFGRRILGV RGRGACSARA QRCGQVHRPA
300
CYRGAASPRR GLGTFGGPGV DRHRGRGECG DPRPSSRAAV ARPVVVSTPE RGQKRGLRTT
360
MPSRDVWVRA R
371
 <212> Type : PRT
   <211> Length : 371
   SequenceName : SEQ ID 283:GDC_MTUB_2234641
   SequenceDescription :

### Sequence

<213> OrganismName : Mycobacterium tuberculosis-H37RV
<400> PreSequenceString : 
LPTPVPARTG TPSRSANPGA TGRPTPETAN TADCSSSRPP GPHSAVSATQ QLPLGNNKSQ
60
LPIGFSPNRD WTRGRRAAPP LAFRSHCGRN PRRASSKSST RSFGQAFRQV FRADGWRRVR
120
SMTRSTYVFG SGHGRFGHSS HGSAAGQDLD IDRGCPQYRP VLAGNLRGRV A
171
 <212> Type : PRT
   <211> Length : 171
   SequenceName : SEQ ID 284:GDC_MTUB_2320829
   SequenceDescription :

### Sequence

<213> OrganismName : Mycobacterium tuberculosis-H37RV
<400> PreSequenceString : 
MRRLRSSDPR CHRLHVGARP APVLPPGQDH RGAFREQRSK SCAARRTRGA CESLGAQRGQ
60
RRFVVGFLRD FRHQFRVGDV AVRADHHDCA GEQPGHRPVG DGHAVILAEA VPECRRGHDV
120
FGALGAAEAL LGERQILRDT QHGSATCRRT LVEGSHTRRA HRCVHGWKDV QQHGLTPELV
180
AADHPQIAPG QGEGRGRGSD SR
202
 <212> Type : PRT
   <211> Length : 202
   SequenceName : SEQ ID 285:GDC_MTUB_2321250
   SequenceDescription :

### Sequence

<213> OrganismName : Mycobacterium tuberculosis-H37RV
<400> PreSequenceString : 
LRGSGRTQIQ DHAAALSRHP RQRAVEFLAA AARRRAEHVA RQALDVDVQR HGHPGTDRTH
60
DDRQMLAEVV NVTKADDTRG AGPGGQRRCR KPDHLGLDPP AIRHQLPDRD HGQSVFDGEF
120
DRLGVVRHLD GIIGRDDLAE RGGRPPFRQA GQVDGRLGGS PPTQHTVGLR LHGHHMARTL
180
EIGGDGGGRS QCRDGPGAIA RRDSGAGAAN VDRHAMRGVS VTHGRQVQSL AFGARQRDAQ
240
ITRGVPDRKG NQPRRRGLGG EDEIAIAIGV AGQDHGVTAR HRRDRTTYPH IGRLHRDSNR
300
RNRLP
305
 <212> Type : PRT
   <211> Length : 305
   SequenceName : SEQ ID 286:GDC_MTUB_2487508
   SequenceDescription :

### Sequence

<213> OrganismName : Mycobacterium tuberculosis-H37RV
<400> PreSequenceString : 
MKTAISLPDE TFDRVSRRAS ELGMSRSEFF TKAAQRYLHE LDAQLLTGQI DRALESIHGT
60
DEAEALAVAN AYRVLETMDD EW
82
 <212> Type : PRT
   <211> Length : 82
   SequenceName : SEQ ID 287:GDC_MTUB_2567990
   SequenceDescription :

### Sequence

<213> OrganismName : Mycobacterium tuberculosis-H37RV
<400> PreSequenceString : 
MSTSTTIRVS TQTRDRLAAQ ARERGISMSA LLTELAAQAE RQAIFRAERE ASHAETTTQA
60
VRDEDREWEG TVGDGLG
77
 <212> Type : PRT
   <211> Length : 77
   SequenceName : SEQ ID 288:GDC_MTUB_2577106
   SequenceDescription :

### Sequence

<213> OrganismName : Mycobacterium tuberculosis-H37RV
<400> PreSequenceString : 
VATSTSPAGG LPQARSQPTK CRCPADSTFS DRAASARTSA AECAQPGLPV QALMFSQGEF
60
SSNTRPSGAS TRSAASAVAS SRSQISTDRH GVITSGASIA ARHSATRAGK TPSGTAAPSV
120
TRLSSWGIQP TGVLVTGRTD GPSSTPDCSS PISANSVTRQ AVSRILTKRN ATSIRVSATS
180
ATRTPVSRPV NSSRGPSGNT CTPTSAPRPD TSARPSSRPN QNRPPSSASR GSARIAASSS
240
PTHARTSASP PARPDSGEAT ILRTRSCVAD GSSPALATAS ATAATSRIPR NWTLPRAVSS
300
SVAEPKSLAT LASVASCAAV IIPPGSRIRA SAPSAAWCGR NAPGQASASR VPATRPPYGR
360
MGRRLAALRS RREAEDQGQG VFDCAHRGGF EGAESLHESG TSDRADAAAH RDAIGSYTF
419
 <212> Type : PRT
   <211> Length : 419
   SequenceName : SEQ ID 289:GDC_MTUB_2577486
   SequenceDescription :

### Sequence

<213> OrganismName : Mycobacterium tuberculosis-H37RV
<400> PreSequenceString : 
MTGRVRQTGI TRLVVHQRGP VLPQRLMTVH AGPVVAEQRL GHERDRFAVL PGGVLDDVLV
60
QLHVVGGVQQ RIELVVDLGL SAAAHLVVAL LQDEAGVDQV GQHLVAQVDV LVVGGHWEIP
120
ALVADLVAPV GTAVGLGRRA GVPPPRDGVH LVEGAVGARV EAHRIENVEL GLGAEVCGVG
180
DASADQVVLG LAGDVARVAG VRLQGERVVH KEVDIQRLGR AERVDARRLG IGKKQHVGFV
240
DRLEPANRRA VKGQAVVKHA LVKGRSRNRE VLHDARQVTE PDVDIFDLLV LGKFEDVVGR
300
LFRHRMLLYC IRGRRYGADI ARQSTPCCAD VTDRAAHH
 338 <212> Type : PRT
   <211> Length : 338
   SequenceName : SEQ ID 290:GDC_MTUB_2690012
   SequenceDescription :

### Sequence

<213> OrganismName : Mycobacterium tuberculosis-H37RV
<400> PreSequenceString : 
VAGVCALFSG ASRWPSGELR HRPQGSRRGP SRLRCTFPRQ NVSSRRPGVP TVGADLTRRS
60
GGTGQPRGMG SPGPVGQTVP CHLRLSRPDT RASGRSADQA HSRRGGSAAR PHQGQPLHPG
120
GQRNRTRRTH ALLAAGNVTA TAADEGSAEW RWRWR
155
 <212> Type : PRT
   <211> Length : 155
   SequenceName : SEQ ID 291:GDC_MTUB_2698040
   SequenceDescription :

### Sequence

<213> OrganismName : Mycobacterium tuberculosis-H37RV
<400> PreSequenceString : 
MTDNECPADS RRRHVLRLAL FAGILLGLFY LVAVARVIHV DGVRSAIVVA TGPIAPLAYV
60
VVSAALGALF VPGPILAAGS GVLFGPLLDT FVTLPAFSAG AQAGMTPRRC WVSIAPIASM
120
HRSNGADCGR WSVSASSPAS RMRWPRTPSG RSEFRCGRWS LGRSSGRRHG CSSTPRWARR
180
SPTCRRRWFT RRSRCGA
197
 <212> Type : PRT
   <211> Length : 197
   SequenceName : SEQ ID 292:GDC_MTUB_2712275
   SequenceDescription :

### Sequence

<213> OrganismName : Mycobacterium tuberculosis-H37RV
<400> PreSequenceString : 
LWAVVGQRFV PGISDALASY TFGAFGVPLW QMVVGSFIGS APRVFVYTAL GASITNLSSP
60
LVYSAIAVWC VTAIIGAFAA RRWYRKWRAR PRRRCGLAQL TTGSQQRHTS HRTPAGVVMP
120
GSLSEHRRLR QEAPDRIEHH PPIE
144
 <212> Type : PRT
   <211> Length : 144
   SequenceName : SEQ ID 293:GDC_MTUB_2725593
   SequenceDescription :

### Sequence

<213> OrganismName : Mycobacterium tuberculosis-H37RV
<400> PreSequenceString : 
LSAVLPARCI RALADRVYRH VRCHGGCARN HHPRSRPGRI DYLGVHRGQR VPGAKGWIDI
60
RHFHTGRGDL DGRAAVVRQP LSGGEQYCSD DRVGGRHAVV DHLRVTGTAH DRLVERVSVL
120
 
DNGGGVCTGR DPWRHVLNSV APRTRHRIRP AVPRRRCRSR GSQDR
165
 <212> Type : PRT
   <211> Length : 165
   SequenceName : SEQ ID 29.4:GDC_MTUB-2733212
   SequenceDescription :

### Sequence

<213> OrganismName : Mycobacterium tuberculosis-H37RV
<400> PreSequenceString : 
VGPMNGFLSW WDGVELWLSG LPFALQALAV MPVVLALAYF TAALLDALLG RVIQLIRRAR
60
RPDQAPR
67
 <212> Type : PRT
   <211> Length : 67
   SequenceName : SEQ ID 295:GDC_MTUB_2828257
   SequenceDescription :

### Sequence

<213> OrganismName : Mycobacterium tuberculosis-H37RV
<400> PreSequenceString : 
MADDVSGAVY RAGTAHGRPT GRIEHRDRQV VTRRATDTRA ELDGLSDHQL AEVQRSRENH
60
YPAGCLVIPQ PLNRRPEHQP APPQRHWALA GGDRDQRGGA KCHGDWVAID RLGAQRDRKP
120
VPRAHHTDRD QAGADRTQSR SVPRPARHTP PQCAAAEGHH DAAQGTHVAD RPHDPGRRHN
180
PADQRRRDQA YVQTGRAEAH MAHRYQTRTR LRRLSSRAGP MPSTSASWST LVNLPLRCRH
240
ATIAAAVTGP MPGRASSCST VAVLRSSTSA VLGAVLGPVV AVPEVPAGPG VPAPTELPST
300
LGCPEGGASP TTICSPSPSC RAMFSPTVSA PSTAPPAACS ASAIRAPGAR VTSPGVCTRP
360
TTLTTTGRPE RSGEPGLADD LGFVGETGST GGSLADITGS VRSRIKVNTV TSTARAAITA
420
NATAPARPGS ARILSAQPCP REVSGSQRGS SEFGSSRGSS WSGPSSVGSC GSGSKCADAA
480
CESISGTAPS RLCSRSAGSS VRMGRPQLRG PPEPARTTAS RCPAVDQSEA VDKPLWRWIK
540
MGQTAPTSPN NQHRAATSIR TRLTAIESVL GNAIREC
577
 <212> Type : PRT
   <211> Length : 577
   SequenceName : SEQ ID 296:GDC_MTUB_2895354
   SequenceDescription :

### Sequence

<213> OrganismName : Mycobacterium tuberculosis-H37RV
<400> PreSequenceString : 
MRGTAYATRR SMLPNTRAVW LATVVQCVTG GLGVTLIPQT AAAVETTRSR LELARFVAPA
60
RRDESVWCLA LSAAARSPTS VLPGLSAS
88
 <212> Type : PRT
   <211> Length : 88
   SequenceName : SEQ ID 297:GDC_MTUB_2983047
   SequenceDescription :

### Sequence

<213> OrganismName : Mycobacterium tuberculosis-H37RV
<400> PreSequenceString : 
MRRVFSGWTT LVRCSTAATT VTIRAMTKHV PVIHSSEPTR PLTPRKPVDP VRRCCSQLRQ
60
PNPTTLKTAK SASAKSAAVV VRISGSPSVR SSGHEPLWRL ARRAASIAAA AGAQSPTPDT
120
KVASAPAAQA RRNARSRSAG EPGLRGQFAA RTPANTSPAP AVSTGVTVGA GTSNTPSAPT
180
YRAPRTPRVI TKCVGGDGQS FASCSLAITT SAIAAKSCKE LRSWPAGEAL TMTTASADWA
240
ARAAASAVAT GISNWVNSTS QLATAEGTGV RCALAPGATS TVFSALASTT IIAVPLGPGT
300
VTVLSSPTAL ARRWARSWAA AGSSPNAPEN CTCAPARAAA TAWLAPFPPG VRVNDAASTV
360
SPGRGSASTT NVRSMFTLPT THTRGAMGPT LVSLAFAMLA VG
402
 <212> Type : PRT
   <211> Length : 402
   SequenceName : SEQ ID 298:GDC_MTUB_3005316
   SequenceDescription :

### Sequence

<213> OrganismName : Mycobacterium tuberculosis-H37RV
<400> PreSequenceString : 
MSASASADKV VCECCELCVP KQLASAIRNP YGLVRGWRCR ICNEHQGQPV KMAQDHEEEV
60
RIRWGETVDE LHAALDRAGP RPGTWCTSEG SSRDPSGGSL GGQCWGVGGL LLGGFFGAGQ
120
CCSGSGEDLE AQVAPSFDPF VVLFGEDGSD EADDRGAVGE DAHDVGSASY LSVEAFLGVV
180
GPDLAPDLLG EGGERQQVGA GGVEVLGHRG EFVGQSVEYP IILGNN
226
 <212> Type : PRT
   <211> Length : 226
   SequenceName : SEQ ID 299:GDC_MTUB_3048559
   SequenceDescription :

### Sequence

<213> OrganismName : Mycobacterium tuberculosis-H37RV
<400> PreSequenceString : 
VGSLTVFTSS ARMSRTAANT SARALHSMTT GSGGKSRMLN TIASPPPTSA SKRRSKTTLP
60
LDAKTKTSTA ETAAWLMNIK PCATNPRAHS TATDNAMNTT TPMAIGPEPS RACTPAPSTI
120
PSATPTIICW ARRARSTLVA DMHTTAEIGA KNACGWLNTS WVRYHARPAT TDVWVIGHST
180
VRNRWATARP PQAVLTSSEA LMNAYLFTLG CDVVTAKIWS CLLDPLGLGI YSGLLTLLSG
240
NGRRRVGERI DAAAGLRERD HLTDRVHPGQ QRGGPVPPER DSAVRRCAKH ERLQQESELF
300
LRLGLVQAHH REHPFLDITA VDTHRAATDL VAVADDVVRV GQHAAGIGFD AVLPFRFRRG
360
EGMVHRGPGP RADRDLTGGG RFVGRLEQRR VNDPDECPRI GVNQAQPVGD LDAGRAQQCP
420
RRFDRTGREE DAIAGFGPDM VGQSGALGLG QVFGHRTAQR AVFGDQHVGQ SAVAALLGPV
480
 
LPAVQRAPRL RRPARHHHRA HIRCLEDTKC GVGEEIRAFD ELQPEPQVGF VRTESAHRFG
540
IADPRDGRRN PVAYQRPQLG QNFLGDRDDV LGVDEAHLHI ELGEFGLAVG AEVLVAVAAG
600
DLVVAFHPRH HQQLLEQLRA LR
622
 <212> Type : PRT
   <211> Length : 622
   SequenceName : SEQ ID 300:GDC_MTUB_3065095
   SequenceDescription :

### Sequence

<213> OrganismName : Mycobacterium tuberculosis-H37RV
<400> PreSequenceString : 
VIGDFAEMLG GQDGVAELVQ HVAVHPFDGV DELVEADGVG GGCGLRHDVN SRLTLCIVST
60
VIGCVVGSAA LPGRCGQGGA DRGHQAGVGV AGDQRDPGQA AGDQVAEERQ PAGPVLGGGD
120
LDAQDLSVAL GVDAGGDQGV HPDDAACLAH LEHQGVGGEE GIRAGIERAG PKRLYGFVEL
180
FGHDRHLRLG KLCHTKCFDQ ALHPASGYSQ QVAGRHHAGQ CAFSSLAALQ QPVREIAALA
240
QLGDRDVDGC GTGVEITVAV AVALIGPLIA AFAVARPAQG VGFSPHQGGD ERREQPAQQI
300
RARLCELVSQ KLLGVDKMRR GHCVISFD
328
 <212> Type : PRT
   <211> length : 328
   SequenceName : SEQ ID 301:GDC_MTUB_3100192
   SequenceDescription :

### Sequence

<213> OrganismName : Mycobacterium tuberculosis-H37RV
<400> PreSequenceString : 
LDEPAHRARP KGNGANHDGA QPCCGIGACG NRGDPRARAH LPLPKGGRAG GAWHGVHRRP
60
RRNLRASRSQ RRGQVHHPEA SHRAAARPRR PGHGVGQRAG RVGTRLLRAH RGLLRAAQPL
120
PKAHRV
126
 <212> Type : PRT
   <211> Length : 126
   SequenceName : SEQ ID 302:GDC_MTUB_3129118
   SequenceDescription :

### Sequence

<213> OrganismName : Mycobacterium tuberculosis-H37RV
<400> PreSequenceString : 
MIPQMTVSCP PPSTSEREEQ ARALCLRLLT ARSRTRAELA GQLAKRGYPE DIGNRVLDRL
60
AAVGLVDDTD FAEQWVQSRR ANAAKSKRAL AAELHAKGVD DDVITTVLGG IDAGAERGRA
120
EKLVRARLRR EVLIDDGTDE ARVSRRLVAM LARRGYGQTL ACEVVIAELA AERERRRV
178
 <212> Type : PRT
   <211> Length : 178
   SequenceName : SEQ ID 303:GDC_MTUB_3237815
   SequenceDescription :

### Sequence

<213> OrganismName : Mycobacterium tuberculosis-H37RV
<400> PreSequenceString : 
LVTTLAPILD SASMTPKTAS SLPGISDDDN TMRSPAVNVM LRCSPRDIRD NADIGSPWVP
60
VVINTTWSGA IVSAAAMSMR SESATRRKPS CLATRMLRTI DRPTNDTRRP NATAASMICC
120
TRSTLEAKHA TITRPSAPRM SRCSVGPTSL SDGPTPGISA FVESHSNRST PVSPSRDMPG
180
RSVGRPSGGN WSNLMSPVCR MVPAPVYTAM ANASGVEWLT AKYSHSNTPC RVLWPSRTST
240
NTGVMRYSRH FSATRAKVNF EPTTGMSGRS LSRNGIAPMW SSCPWVNTSA SMSSSRSSTW
300
RMSGRIRSTP GSSWPGNNTP QSIINSRPRC SKTVMLRPIS LMPPSAVTRN PPEVRGPGGG
360
RSTSTSGPPF GSPLDHRSTE AARMSAANAS ICSGVAATWG SRGSPTSMPC SRKPALDNVT
420
PPRRLIALHS GATAMLILRA VAISPEPKAD NNSRSCPAAR WAITLMKPVA PMASQGRLSA
480
SSPE
484
 <212> Type : PRT
   <211> Length : 484
   SequenceName : SEQ ID 304:GDC_MTUB_3283182
   SequenceDescription :

### Sequence

<213> OrganismName : Mycobacterium tuberculosis-H37RV
<400> PreSequenceString : 
MSKRSDGPST GNAIRARHRI SVMTAQRSTS HATRTPVASS AQLGPPSSVE PTVRPGLAGL
60
VAVKRGREAA ARLPNNPETG CKSRDH
86
 <212> Type : PRT
   <211> Length : 86
   SequenceName : SEQ ID 305:GDC_MTUB_3289702
   SequenceDescription :

### Sequence

<213> OrganismName : Mycobacterium tuberculosis-H37RV
<400> PreSequenceString : 
VATKNAAWPS STSCSNYSPN ATIESQRPDG CTSSRACVTP PVTQRLFSSL LTGYTNGSKI
60
RQTPSNSRPR CTSTSIALAR RSPNERHPRR LCETGRSNSR PAKEKERLRA DHNPAAGATQ
120
PDRTALRRGA AERQPHAPAS AEGEGPVPAG PVRLPVRA
158
 <212> Type : PRT
   <211> Length : 158
   SequenceName : SEQ ID 306:GDC_MTUB_3319076 SequenceDescription :

### Sequence

<213> OrganismName : Mycobacterium tuberculosis-H37RV
<400> PreSequenceString : 
MSAPDVRLTA WVHGWVQGVG FRWWTRCRAL ELGLTGYAAN HADGRVLVVA QGPRAACQKL
60
LQLLQGDTTP GRVAKVVADW SQSTEQITGF SER
93
 <212> Type : PRT
   <211> Length : 93
   SequenceName : SEQ ID 307:GDC_MTUB_3339006
   SequenceDescription :

### Sequence

<213> OrganismName : Mycobacterium tuberculosis-H37RV
<400> PfeSequenceString : 
MLHDVVHGRR CSENGHRRRI TQYRIGTFIG NAALWNRKRH GDAPGLQRAE KGDDVLESLR
60
SRDHHAVTRG TTTAQLLCHI QRSPIQLRPR QGYRNAVPVL FVIHKREGRV MGLQTRTRAQ
120
RSGKGTHTHG HHVTGHAWSC RSRRRGVLAL RGLSQVASGQ LSRGLPARHG STIGHGRM
178
 <212> Type : PRT
   <211> Length : 178
   SequenceName : SEQ ID 308:GDC_MTUB_3356995
   SequenceDescription :

### Sequence

<213> OrganismName : Mycobacterium tuberculosis-H37RV
<400> PreSequenceString : 
MPTTKATQRR DVSTEIAYLT RALKAPTLRE SVSRLADRAR AENWSHEEYL AACLQREVSA
60
RESHGGEGRI RAARFPARKS LEEFDFEHAR GLKRDTIAHL GTLDFITARD NVVFLGPAWH
120
REDSSCGRPG DTRVSGRSSG AVRHRRRMGS TARRGSPRRA HLRRTHPALP LSAPGG
176
 <212> Type : PRT
   <211> Length : 176
   SequenceName : SEQ ID 309:GDC_MTUB_3381198
   SequenceDescription :

### Sequence

<213> OrganismName : Mycobacterium tuberculosis-H37RV
<400> PreSequenceString : 
MQWGYRPLAG DEAMRWGYRP LARESGALDP DHRRCRRRPA HCRPTTRNQT YHRSGARVAI
60
QHRDCAAGSD RSGGVGPLCG FRRPGAGGVV AGSGVRAVRG VRPAQRGRHC AQHRGPRSLR
120
CDAAPGRGGG RRGGRDHVPG GSGVGRPALQ RRLRRR
156
 <212> Type : PRT
   <211> Length : 156
   SequenceName : SEQ ID 310:GDC_MTUB_3388071
   SequenceDescription :

### Sequence

<213> OrganismName : Mycobacterium tuberculosis-H37RV
<400> PreSequenceString : LGGVASTRQA SVRRWSAVHP LDASPALPRP GQRCATARAV AGPTPSWRAA VRSAGVSTSQ 
60
RRPGQAPVSS TAPERRCRAD ESGPNRGCSA VPNAHSTAVP VPSRSATKLR RWWRAAEIAS
120
ASSCVCNAGK SPCSTTMLEA PSATTRSAAV MAVFSGSGSS SGVGSASTSA PSPAAAAAAA
180
SSGVITVIER SEPTPAAAVN VSTSMASTTF SRVCAENTGA SLVLAAAKRL TAMIKPISPS
240
SGVPLMKSSC QRRSTRHTST ALPPRSWPGP RHGPDGNRGA D
281
 <212> Type : PRT
   <211> Length : 281
   SequenceName : SEQ ID 311:GDC_MTUB_3482312
   SequenceDescription :

### Sequence

<213> OrganismName : Mycobacterium tuberculosis-H37RV
<400> PreSequenceString : 
LRGRLIRYAV LLSPSLPLRP SASATGFQSA SVVVTAERAL PAWPLPAPPL EPELHAASIT
60
AAAVVIATIL PACLAPAMRV PSIRCIHGVD GSSVSHGLSG DYETTMKLDR TDPGTARRPH
120
RRPGRVSAGR RGSSTRGTHA HPRRGHQRHR PTCPSAIATG SRRNPVSWNN IQRPSAAAAR
180
RARARTSIRQ RCGPRTSHPL SLLTTELELA LRRPRSNPEL LAAIRSALAE TTDTARTTGG
240
TGLGLAIVDT LSQRNHASVT ARNRAAGGAE ISLRLALG
278
 <212> Type : PRT
   <211> Length : 278
   SequenceName : SEQ ID 312:GDC_MTUB_3581973
   SequenceDescription :

### Sequence

<213> OrganismName : Mycobacterium tuberculosis-H37RV
<400> PreSequenceString : 
LLGLPDPRPV PRNPAARRRA TSRSLSADPS SRPASQSRPR PGTWCTSEGS SRDPSGGSLG
60
GQCWGVGGLL LGGFFGAGQC CSGSGEDLEA QVAPSFDPFV VLFGEDGSDE ADDRGAVGED
120
AHDVGSASYL SVEAFLGVVG PDLAPDLLGE GGERQQVGAG GVEVLGHRGE FVGQSVEYPI
180
ILGNN
185
 <212> Type : PRT
   <211> Length : 185
   SequenceName : SEQ ID 313:GDC_MTUB_3711717
   SequenceDescription :

### Sequence

<213> OrganismName : Mycobacterium tuberculosis-H37RV
<400> PreSequenceString : 
MIFWATRYCT IWLPPSPSSV TFSPAVLAGL GVDASTVDPA LASPTSSLST PISARVSTVT
60
SFFLAAMMPL KDGKRGSLIF SFTLITAGSV ASRVNTPSSV SRSPVILPPS IDTLRRWVSC
120
GRPRYSAMMA GTAPPTPSVD SLPAITSSVP SMVPNARAKA HPVWMTSEPC MPSSFRWTAL
180
SAPIDSALRI ASVARSGPAV STVTDPSMPS AASFSRICSA SSTARSLISS STASAASRSS
 
240
VKSPSVSLRS DQVSGTCLIR TTMFVMTVVR PPRRRPAALD CGTSVTRFAT AQRYYYSVSS
300
RGAPSHHSGW
310
 <212> Type : PRT
   <211> Length : 310
   SequenceName : SEQ ID 314:GDC_MTUB_3716987
   SequenceDescription :

### Sequence

<213> OrganismName : Mycobacterium tuberculosis-H37RV
<400> PreSequenceString : 
LLSSWPRPGT WCTSEGSSRD PSGGSLGGQC WGVGGLLLGG FFGAGQCCSG SGEDLEAQVA
60
PSFDPFVVLF GEDGSDEADD RGAVGEDAHD VGSASYLSVE AFLGVVGPDL APDLLGEGGE
120
RQQVGAGGVE VLGHRGEFVG QSVEYPIILG NN
152
 <212> Type : PRT
   <211> Length : 152
   SequenceName : SEQ ID 315:GDC_MTUB_3754581
   SequenceDescription :

### Sequence

<213> OrganismName : Mycobacterium tuberculosis-H37RV
<400> PreSequenceString : 
LIRSIDRWGS AAGGAVGTPG GTDCNGRSSH PARSAATNTS ISAQGAAGPW VKNRGRSSFP
60
VASCSRTAAE TTSSCLGSGA PATNVSARQP DTTYRPSVDR TGRARRTPST NNVSRTRADQ
120
AARALSATID NTTSPHRQPP SQPAPNRMGC APAKPNATNT CSGGGSTFTP VSLVEPIGVY
180
WACIGPSTSP CRAASAWPTR RSHPAGVPRR RNRLS
215
 <212> Type : PRT
   <211> Length : 215
   SequenceName : SEQ ID 316:GDC_MTUB_3794808
   SequenceDescription :

### Sequence

<213> OrganismName : Mycobacterium tuberculosis-H37RV
<400> PreSequenceString : 
MRCRAALSWR LPERLSRIWP AVLPDHTGMG ATAAWQAKAA SLLNRVTPAA SPTILAAVSS
60
AQPGISSSAG ATWWTRALMR WARVLISPVS RMMSVSSARA SSATNPGWVS SQVRRACWCL
120
AASSERAAGA RSGSSSWTSQ RNRLIADVRW ATRTSRRSVN NFNSRDVSSW VARGRSVSRS
180
TARATASASI GSDLPRLRAD LRVWAISLVG TRTTCWPAAS RSRSRRADML RQSSMPQISS
240
RPNCSRAHMM AVACPAVVAL TVFSPSWRPT SSVATKVWLY LCASVPTTTM VVASEPPR
298
 <212> Type : PRT
   <211> Length : 298
   SequenceName : SEQ ID 317:GDC_MTUB_3796793
   SequenceDescription :

### Sequence

<213> OrganismName : Mycobacterium tuberculosis-H37RV
<400> PreSequenceString : 
VPDLLEFAAL GLRRKAVHDH ERDDGERAED RENACAAEHT QHGEREGGND RVGRQCRGEH
60
RARSHRPQPG REALRGVHPD QRAESEVEPD DEQQHAGEPQ HQPRATIGVV GEYGDQHGIC
120
GDHRRDAGQQ DRATAQPIDQ KQRGTHRRQA GDLHHRGQGK HREIAREAHG GEKSRTVVDD
180
RVDPGDLDEE AERDDEQRGP QIRPPHHFAD TAAAFVDRGR HIGQLGIDVG LRLDPPQRAT
240
RVGDPALEQI PAGGIGHAPQ QRQQQRGRRG GQPEHRAPAV RSGQQVADQV TDDDAAKRRQ
300
LIRGHQRPTH RRRRRLGHIH RHHHHRQADC HTQQQTRHHQ HRYGHRGRAE QGEHCVAGDD
360
EHHRFLASDR VGEDAAAKRP GDLAEHRRGG QQLLFSSGEF EFLAERQQRT RDGGKVVPVE
420
DADAGGGEPD EERPAPRSGQ LTGTGALSTS TTRSGSSGAP AGVNPASWYR AVVISMRLPQ
480
RRHAVNRWSS PDFGADQGRL GCPPANDAEG IGVSS
515
 <212> Type : PRT
   <211> Length : 515
   SequenceName : SEQ ID 318:GDC_MTUB_3879013
   SequenceDescription :

### Sequence

<213> OrganismName : Mycobacterium tuberculosis-H37RV
<400> PreSequenceString : 
VSDATTVLFG LPGARVERVE RRSDGTRVVD VITDEPTAAA CPSCGGGLDI SEGIRGYLTE
60
RSTLWRRPHH GALEQNSLAM PRRLLQAGAV HRGHHPGTCP RPQHAAAASA DGQGDRGCGP
120
LGGPRSPRLT PCRGRRHIGR LLPTPRRVLT EPLPTPVLGV DQTRRGKPRW ERCAKTGRWV
180
RVDPWDTGFV DLAGDQGFMG QHEGRGGAAV LAWLQARTPQ FRESIQYGGH RPRRCLRLGD
240
PHARAAAQRQ ARRRPLPCDH AGQRRADRGA PPGDLGVPRP ARPQDRPAVG QPTSLADRPG
300
TLVGQKLRQN AESDQRRRPP RADSLGLDRQ RGAAHPAVDR AHRRGPPPGA PSPTPLPAWR
360
IDSQIPELLT LATTID
376
 <212> Type : PRT
   <211> Length : 376
   SequenceName : SEQ ID 319:GDC_MTUB_3921024
   SequenceDescription :

### Sequence

<213> OrganismName : Mycobacterium tuberculosis-H37RV
<400> PreSequenceString : 
VQALPESQLP ELAVQMRRRL IETVTATGGH LGAGLGMVEL TIALHRVFTS PHDIGVRHRA
60
PNLSAQAAHR PR
72
 <212> Type : PRT
   <211> Length : 72
   SequenceName : SEQ ID 320:GDC_MTUB_3974481
   SequenceDescription :

### Sequence

<213> OrganismName : Mycobacterium tuberculosis-H37RV
<400> PreSequenceString : 
MSSEGGWPNV GNLARSASMT SAVSSSARVV WVRYDSWVPS GRFNAATSAG VCTNNVDRGA
60
TPSVPSVSSC PACPMKTTVS PRAANRAASA WTFATSGQVA STTCSPRSSA PARTAGETPC
120
AENTTTAPGG GGSGISSRSS TNTAPRSRNS ATTTVLCTIC LRTYTGPSAT SSTRLTVSIA
180
RSTPAQNDRG DANSTVTSPE AYPCATGPTN TSAISTPGDI SVATTRSGLG IAPHRAVPQ
239
 <212> Type : PRT
   <211> Length : 239
   SequenceName : SEQ ID 321:GDC_MTUB_3994808
   SequenceDescription :

### Sequence

<213> OrganismName : Mycobacterium tuberculosis-H37RV
<400> PreSequenceString : 
LPGHRRGTSA SRVPGNRPRL RPSWPRRTPL ARPKTTGCAR STCSSRARAR AARPRSGRCR
60
PPAWRWARSR MSPPSRITVS GPPSAGASRR EDGSLHRTRH PQITAVAHRP RRWRPGLREA
120
SLPARPTRSR ADQGKRISAS AAGEAEGPFH IRRNGKAVPP LLRRGRAAAR QDG
173
 <212> Type : PRT
   <211> Length : 173
   SequenceName : SEQ ID 322:GDC_MTUB_3998938
   SequenceDescription.:

### Sequence

<213> OrganismName : Mycobacterium tuberculosis-H37RV
<400> PreSequenceString : 
VGRRDRGAPA RPFSAHPQRR CLLAGQSQGC RRGIGLRPAR QHLVGGGSGG PGGAGELRRR
60
QGWHHRANPV GGAGARALRR LRQCDLSAGA HRDDGRCLRR RTRCRSGPDR PAVAAACGKP
120
GPVSGVPGCR GSQRSGVHRL RSAGDAGVTA AHGAPVQRGR HVLGSHRAHR DAAGLLCWSG
180
SGTELFGDRS DASVTRGYRR PIIGIGVRIT TPT
213
 <212> Type : PRT
   <211> Length : 213
   SequenceName : SEQ ID 323:GDC_MTUB_4021183
   SequenceDescription :

### Sequence

<213> OrganismName : Mycobacterium tuberculosis-H37RV
<400> PreSequenceString : 
LPWTACCSPY SNDNRTKPSP VKSATNSSPA RASTANVHDP GNTMSPLRSR TPKLSTLPAS
60
QATAVAGLPN TASLRPSATT SPLRVSFASI ALTSRSAGGT RAAPNTKPAA EALSAMVSQI
 
120
LICQSLILVS ISSMAGTRAS VAASTSSSVQ PAPGRSAARM KPTSTSTRGD RYRDAWTGVS
180
SNTCMSSSRC P
191
 <212> Type : PRT
   <211> Length : 191.
   SequenceName : SEQ ID 324:GDC_MTUB_4045946
   SequenceDescription :

### Sequence

<213> OrganismName : Mycobacterium tuberculosis-H37RV
<400> PreSequenceString : 
VVHSRRSWAP SRRPHRGIDA ANERAPAVPE QLTGDPDDRP AQIQQRGGPL DVPSPLRRVC
60
PMLWPVILDT DSQLLVAQVD AGDEVPVVVK HSDLCLRLRQ TGIDQHQSGP RLLWGFRTPV
120
DQRQHRTEAD QAARTGMFGN DGLHVGDLDI GRIRQRVQPL NGLQPRGCAP PDIEGGARRG
180
GYRDTVNRNR LVRRQSIRVH DDARRRLSIG VH
212
 <212> Type : PRT
   <211> Length : 212
   SequenceName : SEQ ID 325:GDC_MTUB_4053033
   SequenceDescription :

### Sequence

<213> OrganismName : Mycobacterium tuberculosis-H37RV
<400> PreSequenceString : 
VSRYPNSWRR LNNPDMAVPM LNRPVFKPLR TEPKRVPGTP MLPMPEVWPL MTVPPLAVLK
60
NPETSTAKGP VGVLKKPETS VPVLPKPELV RPLSVMIPKP VFTLPAFHEP VLMLPEFPLP
120
VLTLPELSNP VLTKPAFPKP VFNSPAFPKP VLRMLAFPKP VLRTPAFPKP MLALPEFPTP
180
RLLRSPGTRV LAPVLKTPML PLPELNKPML LVPELPMPIL PLPEFSSPAR LMPI
234
 <212> Type : PRT
   <211> Length : 234
   SequenceName : SEQ ID 326:GDC_MTUB_4140236
   SequenceDescription :

### Sequence

<213> OrganismName : Mycobacterium tuberculosis-H37RV
<400> PreSequenceString : 
LSSNHAILRL LAPLRLDPQN LGAGPQREHR HRQGRRHGAQ SQSGVLADAG VDVVPAQHAP
60
PQQVRQRTGI GQVGSDVDPE
80
 <212> Type : PRT
   <211> Length : 80
   SequenceName : SEQ ID 327:GDC_MTUB_4169350
   SequenceDescription :

### Sequence

<213> OrganismName : Mycobacterium tuberculosis-H37RV
<400> PreSequenceString : 
LCQGVPARLP PATDTVGVVT KSAVPRVGLD VQIDYSLGDR PVPGHGTGTN QETCEAVCYG
60
AVRRFASGQA QGGDHLGWPG RHRARGRAAA RRPCCGGVQR HLSCVPAARA APAA
114
 <212> Type : PRT
   <211> Length : 114
   SequenceName : SEQ ID 328:GDC_MTUB_4170798
   SequenceDescription :

### Sequence

<213> OrganismName : Mycobacterium tuberculosis-H37RV
<400> PreSequenceString : 
MRPAKRAEEE PGNHPRAGCS GSPPSAPWRS QTPRLATMRP AKRAEEEPGN HPRAGCSGSP
60
PSAPWRSQTP RLATMRPAKR AEEEPGNHPR AGCSGSPPSA PWRSQTPRLA TMRPAKRAEE
120
EPGNHPRAGC SGSPPSAPWR SQTPRLATMR PAKRAEEEPG NHPRAGCSGS PLARPTTGSS
180
RRRRKIRQLS VRVKHAVHRT
200
 <212> Type : PRT
   <211> Length : 200
   SequenceName : SEQ ID 329:GDC_MTUB_4252190
   SequenceDescription :

### Sequence

<213> OrganismName : Mycobacterium tuberculosis-H37RV
<400> PreSequenceString : 
MRTTIDLDDD ILRALKRRQR EERKTLGQLA SELLAQALAA EPPPNVDIRW STADLRPRVD
60
LDDKDAVWAI LDRG
74
 <212> Type : PRT
   <211> Length : 74
   SequenceName : SEQ ID 330:GDC_MTUB_4260620
   SequenceDescription :

### Sequence

<213> OrganismName : Mycobacterium tuberculosis-H37RV
<400> PreSequenceString : 
VSRCRIHCRR LALSRQKTRS LPDLQLASRS GLRRLGCKMD VIRWARRLAV VAGTAAAVTT
60
PGLLSAHVPM VSAEPCPDVE VVFARGTGEP PGIGSVGGLF VDALRFPGWR QVTRGLRR
118
 <212> Type : PRT
   <211> Length : 118
   SequenceName : SEQ ID 331:GDC_MTUB_4302166
   SequenceDescription :

### Sequence

<213> OrganismName : Mycobacterium tuberculosis-H37RV
<400> PreSequenceString : 
VDACHSRARR GVVDRRRPRC GGTARGVVGI RAWAAPLHCG RSSDSGARAR ENSGRVAGTT
60
 
MLAVPVPDSA LRVAGSVLDQ AGPYLPFNTP FTAAGMQYYT QMPESDDSPS EKELGITYRD
120
PRDTVADTVT ALRGLGS
137
 <212> Type : PRT
   <211> Length : 137
   SequenceName : SEQ ID 332:GDC_MTUB_4317863
   SequenceDescription :

### Sequence

<213> OrganismName : Mycobacterium tuberculosis-H37RV
<400> PreSequenceString : 
LMWKPRWRWC STASERRTTA SPDACRNVSR CRSPSLRLAG SGSPWHRMRS RSTAAMATSR
60
PGRWPGCCVT RKSTRSGRAP TTSCVWMCGA GSSRRALTRH CWRGCAMRCR CPTMTTPRGW
120
SRAALRTSTR RSPLGPNSTG SWPRRGCSRW PNSWATSTPA RCSPSRPPGN GQPAAPTARH
180
SSPACTRAGI SPTKARCAVS TQIAMRRCSV STNSWRARSL PSRRKSPQFV ALLTLPWVSL
240
CPE
243
 <212> Type : PRT
   <211> Length : 243
   SequenceName : SEQ ID 333:GDC_MTUB_4341852
   SequenceDescription :

### Sequence

<213> OrganismName : Mycobacterium tuberculosis-H37RV
<400> PreSequenceString : 
VRAPATRAAS RGSSRNSDQR PSGRSVIPSR PSSSACQVCS GVFISPGKRV DKPTIAMSTR
60
SAGPVRDQSS ASSPARSVSG SPSMIRVASD SMVGCANATA TDSVTPVRSS MSAAIATASR
120
DDRPSSTMGT DSSIESGAFP TALPTQLRSH WRISGTVSSA LSAGAFSWDS ATSAMGPQSE
180
VAKTVGEPTP LRRLPSR
197
 <212> Type : PRT
   <211> Length : 197
   SequenceName : SEQ ID 334:GDC_MTUB_4391527
   SequenceDescription :

### Sequence

<213> OrganismName : Sars coronavirus
<400> PreSequenceString : 
LHEDPHTGVE PGAVTAHRDC QHPRPACGDE PFNPACVLVR TDGPDDRKCE MTAIRFDAHR
60
SGRECHAVLI AAFLLEPGEA HCLALTFTGS GVLPVPVRID SAANAVGVSL FRALRPPHRP
120
GLGVDTHLVL DGVPPFTKHP QRRLDSPDTS NAPRLDIGFQ SSDRPVVGLA ASAEMPRQRA
180
GLVLGWVQRE PERLHTPAFW HLESGHQAAS ASPTAAARAR LAPFCAARSP
230
 <212> Type : PRT
   <211> Length : 230
   SequenceName : SEQ ID 335:GDC_Sars174_refseq
   SequenceDescription :

### Sequence

<213> OrganismName : Sars coronavirus
<400> PreSequenceString : 
LRPSRSTLIA KCASWRQPPR CLRSAAVNRR SSAPVAQREL RAENRPESRP QFTLGAVWPH
60
PVNVICAGGR WRVANPSGAG PPSTPRRGQL ISGYASATAP AMGCGRTRRI SPNTRMPSCR
120
AHLLKEGLRH LFSVKGEESK QALDRLIF
148
 <212> Type : PRT
   <211> Length : 148
   SequenceName : SEQ ID 336:GDC_Sars68_refseq
   SequenceDescription :

### Sequence

<213> OrganismName : Sars coronavirus
<400> PreSequenceString : 
VHSASSVATP VRGSTLAGSA GPSTAVTMPA KPTCGATNCS TSMSPSRAAI TWRSPLRHTT
60
KRTMTPPMSR RHQRPSKVRS GLPRVSTISA TVGWGSPWRS STPCAVRSRC TCSQTMSRRS
120
SCGIFGRIPS VTGKSTRCNR SAITNMPSMV TSTPTTLSAV PARPAADGPV MINRKSCR
178
 <212> Type : PRT
   <211> Length : 178
   SequenceName : SEQ ID 337:GDC_Sars61_refseq
   SequenceDescription :

### Sequence

<213> OrganismName : Sars coronavirus
<400> PreSequenceString : 
MDRLCGAPLC HRRRGPTATA AQAGARRLHD PQQAPGRAVA GQLRPAGRAD RGAGRPGGSG
60
SGAPRPGRQP DHGGARHSGG PASRRGVALL EGAAARARPV VHRGGDNRAA VLVEITGEPL
120
AWESRQNGCG VLHSRRRRQR RDLEPPVRRR PRR
153
 <212> Type : PRT
   <211> Length : 153
   SequenceName : SEQ ID 338:GDC_Sars78_refseq
   SequenceDescription :

## Claims

1. A computer based versatile method for identifying protein coding DNA sequences useful as drug targets, said method comprising steps of:
a. generating peptide libraries from the known genomes with oligopeptide of length 'N' computationally arranged in an alphabetical order,
b. artificially translating the test genome to obtain a polypeptide in each reading frame,
c. converting each polypeptide sequence into an alphanumeric sequence with one corresponding to each reading frame on the basis of occurrence of these oligopeptides in the peptide libraries,
d. training Artificial Neural Network (ANN) with sigmoidal learning function to the alphanumeric sequences corresponding to known protein coding DNA sequences and known non-coding regions,
e. deciphering the protein coding regions in the test genome by applying the trained Artificial Neural Network of step (d), and
f. identifying stretches of peptides longer or equal to octomers mapped to large number of known genes in other genomes serving as functional signatures.

2. A method claimed in claim 1 wherein the artificial neural network has one or more input layer, one or more hidden layer with varying number of neurons, and one or more output layer.

3. A method claimed in claim 1 wherein the number of neurons in the hidden layer is preferably 30.

4. A method claimed in claim 1 wherein the value of the 'N' is 4 or more.

5. A method claimed in claim 1 wherein the sigmoidal learning function has five parameters comprising total score, mean, fraction of zeroes, maximum continuous non-zero stretch, and variance.

6. A method claimed in claim 1, wherein the method of identifying genes using oligopeptides that are found to occur in the ORFs of other genomes but not limited to
genomes such as *H.influenzae, M.genitalium, E.coli, B.subtilis, A.fulgidis, M.tuberculosis, T.pallidum, T.maritima, Synecho cystis, H.pylori,* and *SARS-CoV.*

7. A method claimed in claim 1, wherein the peptide library data may be taken from any organism but not specifically limited to those used in the invention.

## Patentansprüche

1. Computer-gestütztes vielseitiges Verfahren zum Identifizieren von Protein-kodierenden DNA-Sequenzen, die als Wirkstoffziele nützlich sind, wobei das Verfahren die Schritte umfasst, dass:
a. Peptid-Bibliotheken aus den bekannten Genomen mit Oligopeptiden der Länge 'N', die durch den Computer in alphabetischer Reihenfolge geordnet werden, erzeugt werden,
b. das Testgenom künstlich übersetzt wird, um ein Polypeptid in jedem Leserahmen zu erhalten,
c. jede Polypeptidsequenz in eine alphanumerische Sequenz, wobei eine jedem Leserahmen auf der Grundlage vom Auftreten dieser Oligopeptide in den Peptid-Bibliotheken entspricht, umgewandelt wird,
d. ein Künstliches Neuronales Netzwerk (KNN) mit sigmoider Lernfunktion auf die alphanumerischen Sequenzen, die bekannten Protein-kodierenden DNA-Sequenzen und bekannten nichtkodierenden Bereichen entsprechen, trainiert wird,
e. die Protein-kodierenden Bereiche im Testgenom durch Einsatz des trainierten Künstlichen Neuronalen Netzwerkes aus Schritt (d) dechiffriert werden, und
f. Peptidabschnitte, die länger als oder gleich Octamere sind, die einer großen Anzahl von bekannten Genen in anderen Genomen, die als Funktionssignaturen dienen, zugeordnet sind, identifiziert werden.

2. Verfahren nach Anspruch 1, wobei das Künstliche Neuronale Netzwerk eine oder mehrere Eingabeschichten, eine oder mehrere verdeckte Schichten mit einer variierenden Anzahl an Neuronen, und eine oder mehrer Ausgabeschichten hat.

3. Verfahren nach in Anspruch 1, wobei die Anzahl der Neuronen in der verdeckten Schicht vorzugsweise 30 ist.

4. Verfahren nach Anspruch 1, wobei der Wert von 'N' 4 oder mehr ist.

5. Verfahren nach Anspruch 1, wobei die sigmoide Lernfunktion fünf Parameter hat, die totale Punktzahl, Mittelwert, einen Anteil von Nullen, einen maximalen ununterbrochenen Abschnitt von Nicht-Nullen und Varianz umfassen.

6. Verfahren nach Anspruch 1, wobei das Verfahren zum Identifizieren von Genen Oligopeptide verwendet, von denen bekannt ist, dass sie in ORFs anderer Genome auftreten wie *H. influenzae, M. genitalium, E. coli, B. subtilis, A. fulgidis, M. tuberculosis, T. pallidum, T. maritima, Synecho cystis, H. pylori* und *SARS-CoV,* ohne auf diese beschränkt zu sein.

7. Verfahren nach Anspruch 1, wobei die Peptid-Bibliothekdaten von allen Organismen genommen werden können, ohne auf die in der Erfindung verwendeten beschränkt zu sein.

## Revendications

1. Procédé informatique polyvalent d'identification de séquences d'ADN codant des protéines et utilisables comme cibles de médicaments, lequel procédé comporte les étapes suivantes :
a) générer des bibliothèques de peptides à partir de génomes connus, avec des oligopeptides de longueur N rangés par ordinateur dans un ordre alphabétique ;
b) traduire artificiellement le génome de test, de manière à obtenir un polypeptide correspondant à chaque cadre de lecture ;
c) convertir chaque séquence polypeptidique en une séquence alphanumérique, une pour chaque cadre de lecture, en se basant sur la présence de ces oligopeptides dans les bibliothèques de peptides ;
d) former un réseau d'intelligence artificielle (RIA), muni d'une fonction d'apprentissage sigmoïde, à reconnaître les séquences alphanumériques correspondant à des séquences connues d'ADN codant des protéines et à des régions non-codantes connues ;
e) déchiffrer les régions codant des protéines dans le génome de test,
à l'aide du réseau d'intelligence artificielle formé dans l'étape (d) ;
f) et identifier des fragments de peptides, plus longs ou aussi longs que des octomères, correspondant à un grand nombre de gènes connus dans d'autres génomes et servant de signatures fonctionnelles.

2. Procédé conforme à la revendication 1, dans lequel le réseau d'intelligence artificielle comporte une ou plusieurs couches d'entrée,
une ou plusieurs couches cachées, dotées chacune d'un nombre variable de neurones, et une ou plusieurs couches de sortie.

3. Procédé conforme à la revendication 1, dans lequel le nombre de neurones dans la couche cachée vaut de préférence 30.

4. Procédé conforme à la revendication 1, dans lequel le nombre N vaut 4 ou plus.

5. Procédé conforme à la revendication 1, dans lequel la fonction d'apprentissage sigmoïde comporte cinq paramètres, qui sont le score total, la moyenne, la fraction de zéros, la longueur maximale de suite continue sans zéros, et la variance.

6. Procédé conforme à la revendication 1, lequel procédé est un procédé d'identification de gènes à l'aide d'oligopeptides que l'on voit apparaître dans les cadres ouverts de lecture d'autres génomes, mais sans se limiter à des génomes tels que ceux de *H. influenzae, M. genitalium, E. coli, B. subtilis, A. fulgidis, M. tuber-culosis, T. pallidum, T. maritima, Synecho cystis, H. pylori,* et *SARS-CoV.*

7. Procédé conforme à la revendication 1, pour lequel on peut tirer de n'importe quels organismes les données des bibliothèques de peptides, sans se limiter spécifiquement aux organismes utilisés pour l'invention.
